# EUROPEAN PATENT APPLICATION

(11) **EP 1 762 568 A1**
(43) Date of publication of application: **14.03.2007**
(21) Application number: 05755841.3
(22) Date of filing: 01.07.2005
(51) Int. Cl.: C07D 401/14, C07D 417/14, A61K 31/4545, A61K 31/496, A61K 31/497, A61K 31/501, A61K 31/5377, A61K 31/5513, A61P 3/10, A61P 7/02, A61P 9/10, A61P 9/14, A61P 13/12, A61P 25/04, A61P 25/28, A61P 27/02, A61P 29/02

(54) **PYRAZOLE DERIVATIVES**

(30) Priority: 01.07.2004 JP 2004195497
(71) Applicant: DAIICHI PHARMACEUTICAL CO., LTD., Chuo-ku, Tokyo 103-8234 (JP)
(72) Inventor: KANAYA, Naoaki, Daiichi Pharma. Co., Ltd., Edogawa-ku, Tokyo 134-8630 (JP); ISHIYAMA, Takashi, Daiichi Pharma. Co., Ltd., Edogawa-ku, Tokyo 134-8630 (JP); MUTO, Ryo, Daiichi Pharma. Co., Ltd., Edogawa-ku, Tokyo 134-8630 (JP); WATANABE, Toshiyuki, Daiichi Pharma. Co., Ltd., Edogawa-ku, Tokyo 134-8630 (JP); OCHIAI, Yuichi, Daiichi Pharma. Co., Ltd., Tokyo 134-8630 (JP)
(74) Representative: Hartz, Nikolai
(86) International application number: PCT/JP2005/012176
(87) International publication number: WO 2006/004027

(57) **Abstract**

A compound represented by formula (I): 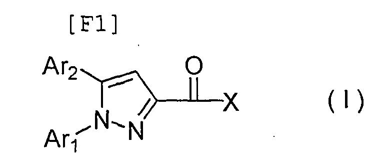 (wherein Ar₁ represents a phenyl group which may have 1 to 3 substituents, or a non-substituted 5- or 6-membered aromatic heterocyclic group; Ar₂ represents (i) a non-substituted phenyl group, (ii) a phenyl group which has been substituted by a lower alkyl group having 1 to 3 groups or atoms selected from among a carbamoyl group, an amino group, a hydroxyl group, a lower alkoxy group, and a halogen atom, or (iii) a 5- or 6-membered nitrogen-containing aromatic heterocyclic group which has been substituted by 1 to 3 groups or atoms selected from among a lower alkyl group, a lower alkynyl group, a lower alkanoyl group, a carbamoyl group, a cyano group, an amino group, a hydroxyl group, a lower alkoxy group, and a halogen atom; and X represents a group represented by formula (II): 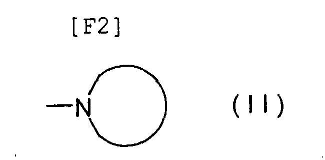 (wherein the ring structure represents a 4- to 7-membered heterocyclic group which may have, in addition to the nitrogen atom shown in formula (II), one heteroatom selected from among nitrogen, oxygen, and sulfur, and which may be substituted by 1 to 4 groups or atoms selected from among a lower alkyl group, a carbamoyl group, an amino group, a hydroxyl group, a lower alkoxy group, an oxo group, a lower alkanoyl group, a lower alkylsulfonyl group, and a halogen atom)), a salt thereof, a solvate of the compound or the salt, and a drug.

## Description

### Technical Field

The present invention relates to pyrazole derivatives having platelet aggregation-inhibiting activity.

### [Background Art]

Platelets play an important role in stopping hemorrhage caused by damage to blood vessels through aggregation to form thrombi. On the other hand, platelets are known to aggregate at sites where vascular endothelium is injured or a blood vessel is narrowed and to trigger formation of thrombi or emboli. The formed thrombi or emboli cause ischemic diseases such as myocardial infarction, angina pectoris, ischemic cerebrovascular disorder, and peripheral vascular disease. Therefore, platelet aggregation inhibitors are administered for prevention and treatment of such ischemic diseases. Aspirin, administered at a low dose, is one such platelet aggregation inhibitor that has been used for a long time, and the effects of aspirin have been demonstrated by APT (Antiplatelet Trialists' Collaboration) in which clinical test results obtained by administering aspirin to 1,000,000 patients had been subjected to metaanalysis (see Non-Patent Document 1).
Aspirin, however, is known to cause side effects such as hemorrhage in gastrointestine or like organs, namely, the so-called "aspirin-induced ulcer." This side effect occurs at a rate of one in 100 patients in dose-independent manner (see Non-Patent Document 2).

Aspirin's inhibitory effect on platelet aggregation is known to be due to the activity to inhibit the cyclooxygenase activity. Cyclooxygenases include cyclooxygenase-1 (COX-1) and cyclooxygenase-2 (COX-2), and aspirin specifically and irreversibly inhibits COX-1 at a low dose, resulting in inhibition of platelet aggregation. This inhibition of COX-1 in turn causes an aspirin-induced ulcer (see Non-Patent Documents 3 and 4). Nonsteroidal antiinflammatory drugs have been known to inhibit COX-2 in a COX-2-selective manner and exhibit antiinflammatory effect.

As described above, although aspirin is useful as a platelet aggregation inhibitor, it produces a side effect of gastrointestinal dysfunction attributable to the COX-1-inhibitory action, which is a mechanism of action of platelet aggregation inhibition. Therefore, there exists a strong demand for a platelet aggregation inhibitor exhibiting no COX-1-inhibiting effect.

Meanwhile, as pyrazole derivatives having antithrombotic activity, there have been known compound (A) (see Patent Document 1 and Non-Patent Document 5) and compound (B) (see Patent Document 2).

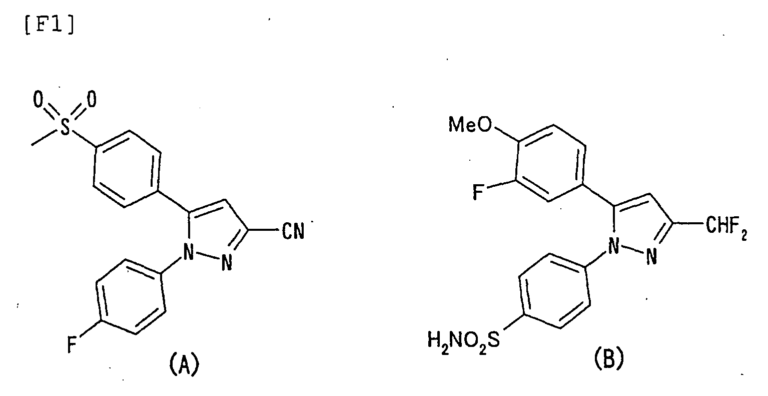

[Patent Document 1] Specification of Japanese Patent No. 2586713
[Patent Document 2] International Publication WO 97/29774 pamphlet
[Non-Patent Document 1] BMJ, vol. 308, pp. 81-106, 1994
[Non-Patent Document 2] BMJ, vol. 321, pp. 1183-1187, 2000
[Non-Patent Document 3] Neurology, vol. 57, Suppl. 2, pp. S5-S7, 2001
[Non-Patent Document 4] Drugs Today, vol. 35, pp. 251-265, 1999
[Non-Patent Document 5] Chem. Pharm. Bull., vol. 45, pp. 987-995, 1997

### [Disclosure of the Invention]

### [Problems to be Solved by the Invention]

Compound (A), however, exhibits an IC₅₀ value of 5.3 × 10⁻⁶ M against collagen-induced platelet aggregation, and even stronger inhibitory activity against COX-2 (IC₅₀ value = 2.4 × 10⁻⁷ M). The situation is similar to the case of compound (B). The platelet aggregation inhibitory activity of compound (B) is not so potent compared with its inhibitory activity against COX-2. As described above, inhibition of COX-2 may lead to an antiinflammatory action, and therefore, inhibition of COX-2 is not necessarily favorable as a platelet aggregation inhibitor. In view of the above, an object of the present invention is to provide a potent inhibitor of platelet aggregation which neither inhibits COX-1 nor COX-2.

### [Means for Solving the Problems]

The present inventors have performed extensive research in search of such a platelet aggregation inhibitor, and have found that pyrazole derivatives represented by the following formula (I) exhibit strong platelet aggregation inhibitory activity without inhibiting COX-1 or COX-2, to thereby complete the present invention.
Accordingly, the present invention provides a compound represented by formula (I):

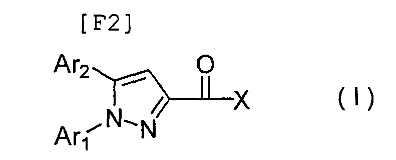

(wherein Ar₁ represents a phenyl group which may have 1 to 3 substituents, or a non-substituted 5- or 6-membered aromatic heterocyclic group; Ar₂ represents (i) a non-substituted phenyl group, (ii) a phenyl group which has been substituted by a lower alkyl group having 1 to 3 groups or atoms selected from among a carbamoyl group which may be substituted, an amino group which may be substituted, a hydroxyl group, a lower alkoxy group, and a halogen atom, or (iii) a 5- or 6-membered aromatic heterocyclic group which has been substituted by 1 to 3 groups or atoms selected from among a lower alkyl group which may be substituted, a lower alkynyl group, a lower alkanoyl group, a carbamoyl group which may be substituted, a cyano group, an amino group which may be substituted, a hydroxyl group, a lower alkoxy group which may be substituted, and a halogen atom; and X represents a group represented by formula (II): 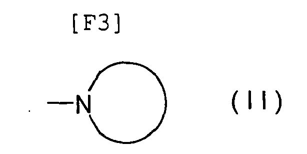 (wherein the ring structure represents a 4- to 7-membered heterocyclic group which may have, in addition to the nitrogen atom shown in formula (II), one heteroatom selected from among nitrogen, oxygen, and sulfur, and which may be substituted by 1 to 4 groups or atoms selected from among a lower alkyl group which may be substituted, an alkylidene group which may be substituted, a carbamoyl group which may be substituted, an amino group which may be substituted, a hydroxyl group, a lower alkoxy group, an oxo group, a lower alkanoyl group, a lower alkylsulfonyl group, and a halogen atom)), a salt thereof, or a solvate of the compound or the salt.

The present invention also provides a drug containing, as an active ingredient, the aforementioned compound, a salt thereof, or a solvate of the compound or the salt; a preventive and/or therapeutic agent for ischemic diseases, the agent containing, as an active ingredient, the aforementioned compound, a salt thereof, or a solvate of the compound or the salt; a platelet aggregation-inhibiting agent containing, as an active ingredient, the aforementioned compound, a salt thereof, or a solvate of the compound or the salt; and a drug composition containing the aforementioned compound, a salt thereof, or a solvate of the compound or the salt.

The present invention also provides a method for preventing and/or treating an ischemic disease, characterized by administering an effective amount of the aforementioned compound, a salt thereof, or a solvate of the compound or the salt.

The present invention also provides use of the aforementioned compound, a salt thereof, or a solvate of the compound or the salt for production of a preventive and/or therapeutic agent for ischemic diseases.

### [Effects of the Invention]

The compound (I) of the present invention, a salt of the compound, and a solvate of the compound or the salt potently inhibit platelet aggregation, and accordingly, also inhibit thrombogenesis without inhibiting COX-1 or COX-2. Therefore, they are useful in prevention and/or treatment of ischemic diseases caused by thrombus or embolus such as myocardial infarction, angina pectoris (chronic stable angina, unstable angina, etc.), ischemic cerebrovascular disorder (transient ischemic attack (TIA), cerebral infarction, etc.), peripheral vascular disease, embolism after replacement with an artificial vessel, thrombotic embolism after coronary artery intervention (coronary artery bypass grafting (CABG), percutaneous transluminal coronary angioplasty (PTCA), stent placement, etc.), diabetic retinopathy and nephropathy, and embolism after replacement with an artificial heart valve, and are also useful in prevention and/or treatment of thrombus and embolus associated with vascular operation, blood extracorporeal circulation, and the like. Moreover, the compounds (I) of the present invention, salts thereof, and solvates of the compounds or the salts are useful for ameliorating ischemic conditions such as ulcer, pain, and chill, which accompany chronic arteriosclerosis obliterans.

### [Best Mode for Carrying Out the Invention]

Formula (I) will next be described.

Ar₁ represents a substituted or non-substituted phenyl group or a non-substituted 5- or 6-membered aromatic heterocyclic group.

Examples of the substituent(s) of the phenyl group include lower alkyl, cyano, amino which may be substituted by one alkyl group or two lower alkyl groups which are identical to or different from each other, hydroxyl, lower alkoxy, and halogen atom. The phenyl group may have one substituent or 2 or 3 substituents which are identical to or different from one another. Preferably, the phenyl group has one substituent, and the substituent locates at the p-position.

The lower alkyl group refers to a linear, branched, or cyclic C1-C6 alkyl group. Specific examples include methyl, ethyl, n-propyl, isopropyl, n-butyl, isobutyl, tert-butyl, n-pentyl, isopentyl, n-hexyl, cyclopropyl, cyclobutyl, cyclopentyl, cyclohexyl, cyclopropylmethyl, and cyclopentylmethyl. Of these, methyl, ethyl, and n-propyl are preferred, and methyl is particularly preferred.

The amino group which may be substituted by one alkyl group or two lower alkyl groups which are identical to or different from each other refers to a non-substituted amino group or an amino group which has been substituted by the above lower alkyl group(s). Specific examples include methylamino, ethylamino, n-propylamino, dimethylamino, diethylamino, and N-methyl-N-ethylamino.

The lower alkoxy group refers to an alkoxy group having the above lower alkyl group in its structure. Specific examples include methoxy, ethoxy, n-propoxy, isopropoxy, n-butoxy, isobutoxy, tert-butoxy, n-pentoxy, and cyclopentyloxy. Of these, methoxy and ethoxy are preferred, and methoxy is particularly preferred.

Examples of the halogen atom include fluorine, chlorine, bromine, and iodine. Of these, fluorine and chlorine are preferred, and fluorine is particularly preferred.

When Ar₁ is a phenyl group, Ar₁ is preferably non-substituted or has one substituent at the p-position thereof.

Examples of the 5- or 6-membered aromatic heterocyclic group include pyrrolyl, pyrazolyl, imidazolyl, triazolyl, pyridyl, pyridazinyl, pyrimidinyl, pyrazinyl, furyl, thienyl, oxazolyl, isoxazolyl, and thiazolyl. Of these, preferred are pyrrolyl, pyrazolyl, imidazolyl, triazolyl, oxazolyl, thiazolyl, pyridyl, pyridazinyl, pyrimidinyl, and pyrazinyl, and particularly preferred are pyrrolyl, pyrazolyl, imidazolyl, thiazolyl, pyridyl, pyridazinyl, pyrimidinyl, and pyrazinyl. Still more preferred are thiazolyl, pyridyl, pyridazinyl, and pyrimidinyl.

Next, Ar₂ will be described.

When Ar₂ is a phenyl group, Ar₂ may be non-substituted or substituted by a lower alkyl group which may have a substituent. The lower alkyl group which may have a substituent refers to a lower alkyl group which has been substituted one group (or atom) or 2 or 3 groups (or atoms) which are identical to or different from one another, the group(s) or atom(s) being selected from among the following 1) to 5). Examples of the lower alkyl group are the same as those mentioned above for a substituent of the Ar₁.

1) Carbamoyl group which may be substituted by one lower alkyl group or two lower alkyl groups which are identical to or different from each other: The carbamoyl refers to a non-substituted carbamoyl group or a carbamoyl group which has been substituted by one of the lower alkyl groups or two of the lower alkyl groups, the two groups being identical to or different from each other. Specific examples include methylcarbamoyl, ethylcarbamoyl, dimethylcarbamoyl, and N-methyl-N-ethylcarbamoyl. Of these, non-substituted carbamoyl, methylcarbamoyl, and dimethylcarbamoyl are preferred.

2) Amino group which may be substituted by one substituent or two substituents which are identical to or different from each other, the substituent(s) being selected from among lower alkyl, lower alkanoyl, and lower alkylsulfonyl: The amino group refers to a non-substituted amino group or an amino group which has been substituted by one substituent or two substituents which are identical to or different from each other, the substituent(s) being selected from lower alkyl, lower alkanoyl, and lower alkylsulfonyl.
The lower alkyl group is the same as that described above.
The lower alkanoyl group refers to a C1-C6 linear or branched alkanoyl group. Specific examples include formyl, acetyl, n-propionyl, n-butyryl, and isobutyryl.
The lower alkylsulfonyl group is a sulfonyl group which has been substituted by the above lower alkyl group.
Specific examples includes methylsulfonyl, ethylsulfonyl, n-propylsulfonyl, isopropylsulfonyl, n-butylsulfonyl, isobutylsulfonyl, tert-butylsulfonyl, n-pentylsulfonyl, isopentylsulfonyl, cyclopropylsulfonyl, and cyclohexylsulfonyl.
Accordingly, the amino group which has been substituted by one substituent or two substituents which are identical to or different from each other, the substituent(s) being selected from the group consisting of lower alkyl, lower alkanoyl, and lower alkylsulfonyl include methylamino, ethylamino, n-propylamino, isopropylamino, cyclopropylamino, n-butylamino, isobutylamino, cyclopentylmethylamino, dimethylamino, diethylamino, di-n-propylamino, di-n-butylamino, N-methyl-N-ethylamino, N-ethyl-N-n-propylamino, N-methyl-N-cyclopentylmethylamino, formylamino, acetylamino, n-propionylamino, N-methyl-N-acetylamino, N-ethyl-N-acetylamino, methylsulfonylamino, ethylsulfonylamino, isopropylsulfonylamino, n-butylsulfonylamino, cyclopropylsulfonylamino, cyclobutanesulfonylamino, N-methyl-N-methylsulfonylamino, and N-ethyl-N-methylsulfonylamino.

3) Hydroxyl group

4) Lower alkoxy group: The lower alkoxy group is the same as that described above in relation to the substituent of Ar₁; i.e., an alkoxy group containing the above lower alkyl group in its structure. Specific examples include methoxy, ethoxy, n-propoxy, isopropoxy, n-butoxy, isobutoxy, n-pentoxy, and cyclopentyloxy. Of these, methoxy and ethoxy are preferred, and methoxy is particularly preferred.

5) Halogen atom: Examples of the halogen atom include, similar to those described above, fluorine, chlorine, bromine, and iodine. Of these, fluorine and chlorine are preferred, and fluorine is particularly preferred.

When Ar₂ is a phenyl group, representative examples of the substituent on the phenyl group include carbamoylmethyl, methylcarbamoylmethyl, dimethylcarbamoylmethyl, N-methyl-N-ethylcarbamoylmethyl, carbamoylethyl, methylcarbamoylethyl, dimethylcarbamoylethyl, aminomethyl, methylaminomethyl, dimethylaminomethyl, ethylaminomethyl, diethylaminomethyl, N-methyl-N-ethylamiriomethyl, aminoethyl, methylaminoethyl, dimethylaminoethyl, formylaminomethyl, acetylaminomethyl, formylaminoethyl, acetylaminoethyl, N-methyl-N-acetylaminomethyl, N-ethyl-N-acetylaminomethyl, methylsulfonylaminomethyl, ethylsulfonylaminomethyl, isopropylsulfonylaminomethyl, n-butylsulfonylaminomethyl, cyclopropylsulfonylaminomethyl, cyclobutanesulfonylaminomethyl, N-methyl-N-methylsulfonylaminomethyl, N-ethyl-N-methylsulfonylaminomethyl, hydroxymethyl, hydroxyethyl, hydroxypropionyl, methoxymethyl, methoxyethyl, ethoxymethyl, ethoxyethyl, fluoromethyl, difluoromethyl, and trifluoromethyl.

When Ar₂ is a phenyl group, Ar₂ is preferably non-substituted or substituted only at the p-position with respect to the pyrazole ring by any of the above substituents.

When Ar₂ is a 5- or 6-membered aromatic heterocyclic group, the aromatic heterocyclic group is substituted by one group or atom or 2 or 3 groups or atoms which are identical to or different from one another, the group(s) or atom(s) being selected from the following (a) to (i). In this case, the 5- or 6-membered aromatic heterocyclic ring is preferably a 5- or 6-membered nitrogen-containing aromatic heterocyclic group. Examples of the nitrogen-containing aromatic heterocyclic group include pyrrolyl, pyrazolyl, imidazolyl, triazolyl, oxazolyl, thiazolyl, pyridyl, pyridazinyl, pyrimidinyl, pyrazinyl, furyl, thienyl, and isoxazolyl. Of these, preferred are pyrrolyl, pyrazolyl, imidazolyl, triazolyl, oxazolyl, thiazolyl, pyridyl, pyridazinyl, pyrimidinyl, and pyrazinyl, particularly preferred are pyrrolyl, pyrazolyl, imidazolyl, thiazolyl, pyridyl, pyridazinyl, pyrimidinyl, and pyrazinyl.

(a) Lower alkyl group: Examples of the lower alkyl group include those as described above. Of these, methyl, ethyl, and n-propyl are preferred, and methyl is particularly preferred.
   The lower alkyl group may be substituted by 1 to 3 groups or atoms selected from among 1) to 5) above, like the case where Ar₂ is a phenyl group that is substituted by a lower alkyl group as described above.

(b) Lower alkynyl group: The lower alkynyl group is a C2-C6 linear, branched, or cyclic alkynyl group. Specific examples include ethynyl, 1-propynyl, 2-propynyl, 1-butynyl, 2-butynyl, 1-pentynyl, and 2-pentynyl. Of these, ethynyl, 1-propynyl, and 2-propynyl are preferred, with ethynyl being more preferred.

(c) Lower alkanoyl group: The lower alkanoyl is the same as listed as a substituent of Ar₁ when Ar₁ is a phenyl group. Of these, acetyl and n-propionyl are preferred, with acetyl being particularly preferred.

(d) Carbamoyl group which may be substituted by 1 or 2 lower alkyl groups: The carbamoyl group is the same as listed 1) above as a substituent of the lower alkyl group when Ar₂ is a phenyl group. Of these, non-substituted carbamoyl, methylcarbamoyl, and ethylcarbamoyl are preferred, with non-substituted carbamoyl being particularly preferred.

(e) Cyano group

(f) Amino group which may be substituted by one substituent or two substituents which are identical to or different from each other, the substituent(s) being selected from among lower alkyl, lower alkanoyl, and lower alkylsulfonyl: The amino group is the same as listed 2) above as a substituent of the lower alkyl group when Ar₂ is a phenyl group. Of these, non-substituted amino, dimethylamino, and diethylamino are preferred, with non-substituted amino and dimethylamino being particularly preferred.

(g) Hydroxyl group

(h) Lower alkoxy group which may be substituted: The lower alkoxy group of the lower alkoxy group which may be substituted is the same as described above as a substituent of Ar₁; i.e., an alkoxy group having the lower alkyl group in its structure. Specific examples include methoxy, ethoxy, n-propoxy, isopropoxy, n-butoxy, isobutoxy, n-pentoxy, and cyclopentyloxy. Of these, methoxy and ethoxy are preferred, with methoxy being particularly preferred.
The lower alkoxy group may be substituted by 1 to 3 groups or atoms selected from among the above 1) to 5), like the case where Ar₂ is a phenyl group that is substituted by a lower alkyl group as described above.

(i) Halogen atom: Examples of the halogen atom include those as described above. Of these, chlorine and fluorine are preferred, with fluorine being particularly preferred.

Specific examples of Ar₂ which is a 5- or 6-membered nitrogen-containing heterocyclic group which may have a substituent include methylpyrrolyl, carbamoylpyrrolyl, dimethylcarbamoylpyrrolyl, cyanopyrrolyl, hydroxypyrrolyl, methoxypyrrolyl, fluoropyrrolyl, chloropyrrolyl, aminopyrrolyl, methylaminopyrrolyl, dimethylaminopyrrolyl, hydroxymethylpyrrolyl, aminomethylpyrrolyl, methylaminomethylpyrrolyl, dimethylaminomethylpyrrolyl, methylpyrazolyl, carbamoylpyrazolyl, dimethylcarbamoylpyrazolyl, cyanopyrazolyl, hydroxypyrazolyl, methoxypyrazolyl, fluoropyrazolyl, chloropyrazolyl, aminopyrazolyl, methylaminopyrazolyl, dimethylaminopyrazolyl, hydroxymethylpyrazolyl, aminomethylpyrazolyl, methylaminomethylpyrazolyl, dimethylaminomethylpyrazolyl, methylimidazolyl, carbamoylimidazolyl, dimethylcarbamoylimidazolyl, cyanoimidazolyl, hydroxyimidazolyl, methoxyimidazolyl, fluoroimidazolyl, chloroimidazolyl, aminoimidazolyl, methylaminoimidazolyl, dimethylaminoimidazolyl, hydroxymethylimidazolyl, aminomethylimidazolyl, methylaminomethylimidazolyl, dimethylaminomethylimidazolyl, methyltriazolyl, carbamoyltriazolyl, dimethylcarbamoyltriazolyl, cyanotriazolyl, hydroxytriazolyl, methoxytriazolyl, fluorotriazolyl, chlorotriazolyl, aminotriazolyl, methylaminotriazolyl, dimethylaminotriazolyl, hydroxymethyltriazolyl, aminomethyltriazolyl, methylaminomethyltriazolyl, dimethylaminomethyltriazoryl, methylpyridyl, carbamoylpyridyl, dimethylcarbamoylpyridyl, cyanopyridyl, hydroxypyridyl, methoxypyridyl, fluoropyridyl, chloropyridyl, aminopyridyl, methylaminopyridyl, dimethylaminopyridyl, hydroxymethylpyridyl, aminomethylpyridyl, methylaminomethylpyridyl, dimethylaminomethylpyridyl, fluoromethylpyridyl, methoxymethylpyridyl, ethynylpyridyl, acetylpyridyl, carbamoylmethoxypyridyl, methylpyridazinyl, carbamoylpyridazinyl, dimethylcarbamoylpyridazinyl, cyanopyridazinyl, hydroxypyridazinyl, methoxypyridazinyl, fluoropyridazinyl, chloropyridazinyl, aminopyridazinyl, methylaminopyridazinyl, dimethylaminopyridazinyl, hydroxymethylpyridazinyl, aminomethylpyridazinyl, methylaminomethylpyridazinyl, dimethylaminomethylpyridazinyl, methylpyrimidinyl, carbamoylpyrimidinyl, dimethylcarbamoylpyrimidinyl, cyanopyrimidinyl, hydroxypyrimidinyl, methoxypyrimidinyl, fluoropyrimidinyl, chloropyrimidinyl, aminopyrimidinyl, methylaminopyrimidinyl, dimethylaminopyrimidinyl, hydroxymethylpyrimidinyl, aminomethylpyrimidinyl, methylaminomethylpyrimidinyl, dimethylaminomethylpyrimidinyl, methylpyrazinyl, carbamoylpyrazinyl, dimethylcarbamoylpyrazinyl, cyanopyrazinyl, hydroxypyrazinyl, methoxypyrazinyl, fluoropyrazinyl, chloropyrazinyl, aminopyrazinyl, methylaminopyrazinyl, dimethylaminopyrazinyl, hydroxymethylpyrazinyl, aminomethylpyrazinyl, methylaminomethylpyrazinyl, dimethylaminomethylpyrazinyl, methyloxazolyl, carbamoyloxazolyl, dimethylcarbamoyloxazolyl, cyanooxazolyl, hydroxyoxazolyl, methoxyoxazolyl, fluorooxazolyl, chlorooxazolyl, aminooxazolyl, methylaminooxazolyl, dimethylaminooxazolyl, hydroxymethyloxazolyl, aminomethyloxazolyl, methylaminomethyloxazolyl, dimethylaminomethyloxazolyl, methylthiazolyl, carbamoylthiazolyl, dimethylcarbamoylthiazolyl, cyanothiazolyl, hydroxythiazolyl, methoxythiazolyl, fluorothiazolyl, chlorothiazolyl, aminothiazolyl, methylaminothiazolyl, dimethylaminothiazolyl, hydroxymethylthiazolyl, aminomethylthiazolyl, methylaminomethylthiazolyl, and dimethylaminomethylthiazolyl.
Of these, particularly preferred are methylpyrrolyl, methylpyrazolyl, methylimidazolyl, methylpyridyl, carbamoylpyridyl, cyanopyridyl, aminopyridyl, hydroxypyridyl, methoxypyridyl, fluoropyridyl, chloropyridyl, hydroxymethylpyridyl, aminomethylpyridyl, fluoromethylpyridyl, methoxymethylpyridyl, ethynylpyridyl, acetylpyridyl, carbamoylmethoxypyridyl, methoxypyridazinyl, methylpyrazinyl, carbamoylpyrazinyl, and aminopyrazinyl.

The group represented by formula (II) is a 4- to 7-membered heterocyclic group which may have, in addition to the nitrogen atom shown in formula (II), one heteroatom selected from among nitrogen, oxygen, and sulfur.
The 4- to 7-membered heterocyclic group is preferably a saturated heterocyclic group, such as azetidino, pyrrolidino, piperidino, piperazino, hexahydropyridazino, hexahydropyrimidino, pyrazolidino, imidazolidino, homopiperazino, morpholino, or thiomorpholino. Of these, particularly preferred are azetidino, pyrrolidino, piperidino, piperazino, homopiperazino, hexahydropyridazin-1-yl, pyrazolidino, and morpholino.

The heterocyclic group may further be substituted by one group or atom or 2 to 4 groups or atoms which are identical to or different from one another, the group(s) or atom(s) being selected from the following substituents (i) to (x). In the case where the heterocyclic group is substituted by a plurality of groups or atoms, the groups or atoms may link to the same atom of the heterocyclic group or different atoms of the heterocyclic group.

(i) Lower alkyl group which may be substituted: The lower alkyl group which may be substituted is a lower alkyl group which may be substituted by 1 to 3 groups or atoms selected from 1) to 5) above, like the case where Ar₂ is a phenyl group that is substituted by a lower alkyl group as described above. The lower alkyl group may be substituted by a single oxo group. Examples of the lower alkyl group include those as described above. Of these, methyl and cyclopropyl are particularly preferred. The substituent linked to the lower alkyl group is preferably halogeno, amino, or hydroxyl, more preferably halogeno or amino. Accordingly, the lower alkyl group which may be substituted is preferably a non-substituted lower alkyl, a halogeno(lower alkyl) group, an amino(lower alkyl) group, or a hydroxy(lower alkyl) group, more preferably a non-substituted lower alkyl group, a halogeno(lower alkyl) group, or an amino(lower alkyl) group. The halogeno(lower alkyl) group is a group corresponding to the above lower alkyl group which has been substituted by the above halogen atom. Specific examples include fluoromethyl, difluoromethyl, trifluoromethyl, chloromethyl, dichloromethyl, and trichloromethyl. Of these, fluoromethyl, difluoromethyl, and trifluoromethyl are preferred, with fluoromethyl being particularly preferred. The amino(lower alkyl) group is a group corresponding to the above lower alkyl group which has been substituted by an amino group. Specific examples include aminomethyl, 1-aminoethyl, 2-aminoethyl, and 1-aminocyclopropyl. Of these, 1-aminocyclopropyl is preferred. The hydroxy(lower alkyl) group is a group corresponding to the above lower alkyl group which has been substituted by a hydroxyl group. Specific examples include hydroxymethyl, 1-hydroxyethyl, 1-hydroxypropyl, 2-hydroxypropyl, and 3-hydroxypropyl.
(ii) Alkylidene group which may be substituted: The alkylidene group is a C1-C6 linear, branched, or cyclic alkylidene group. Specific examples include methylene, ethylidene, isopropylidene, cyclopropylidene, cyclobutylidene, and cyclohexylidene. Of these, methylene and ethylidene are preferred, with methylene being particularly preferred.
Herein, the alkylidene group may further be substituted by one substituent or two substituents which are identical to or different from each other, the substituent(s) being selected from the above halogeno group, lower alkanoyl group, and lower alkylsulfonyl group.

(iii) Lower alkanoyl group: The lower alkanoyl group is a C1-C6 linear or branched alkanoyl group. Specific examples include formyl, acetyl, n-propionyl, n-butyryl, isobutyryl, and pivaloyl. Of these, formyl is particularly preferred.

(iv) Carbamoyl group which may be substituted by 1 or 2 lower alkyl groups: Examples include those as described above. Of these, non-substituted carbamoyl, methylcarbamoyl, and ethylcarbamoyl are preferred, with non-substituted carbamoyl being particularly preferred.

(v) Amino group which may be substituted by one substituent or two substituents which are identical to or different from each other, the substituent(s) being selected from among lower alkyl, lower alkanoyl, and lower alkylsulfonyl: The amino group is similar to that described in 2) above in relation to a substituent of the lower alkyl group when Ar₂ is a phenyl group or to that described in f) above in relation to a substituent of Ar₂ when Ar₂ is a 5- or 6-membered nitrogen-containing aromatic heterocyclic group. Of these, non-substituted amino, dimethylamino, and diethylamino are preferred, with non-substituted amino and dimethylamino being particularly preferred.

(vi) Hydroxyl group

(vii) Lower alkoxy group: Examples of the lower alkoxy group include those as described above. Methoxy and ethoxy are preferred, with methoxy being more preferred.

(viii) Oxo group

(ix) Lower alkylsulfonyl group: Examples of the lower alkylsulfonyl group include those species already mentioned. Of these, methylsulfonyl, ethylsulfonyl, and n-propylsulfonyl are preferred.

(x) Halogen atom: Examples include those species already mentioned. Of these, fluorine and chlorine are preferred, with fluorine being particularly preferred.

The heterocyclic group may be substituted by a single group or atom selected from among (i) to (x), or may be substituted by 2 to 4 groups or atoms selected from among (i) to (x), the groups or atoms being identical to or different from one another, so long as the heterocyclic group has positions available for substitution. In the case where the heterocyclic group is substituted by a plurality of groups, the groups may link to the same atom of the heterocyclic group or different atoms of the heterocyclic group.

Examples of the compounds represented by formula (II) include, but are not limited to, 3-aminoazetidin-1-yl, 3-methylaminoazetidin-1-yl, 3-dimethylaminoazetidin-1-yl, 3,3-difluoroazetidin-1-yl, 3-methoxyazetidin-1-yl, 2-fluoromethylpyrrolidino, 3-fluoropyrrolidino; 3-methoxypyrrolidino, 2-hydroxymethylpyrrolidino, 2-methoxymethylpyrrolidino, 2-carbamoylpyrrolidino, 2-methylcarbamoylpyrrolidino, 2-dimethylcarbamoylpyrrolidino, 3-carbamoylpyrrolidino, 3-methylcarbamoylpyrrolidino, 3-dimethylcarbamoylpyrrolidino, 2-methoxymethyl-5-methylpyrrolidino, 3-aminopiperidino, 4-aminopiperidino, 3-methylaminopiperidino, 4-methylaminopiperidino, 3-dimethylaminopiperidino, 4-dimethylaminopiperidino, 2-methylpiperidino, 3-methylpiperidino, 4-methylpiperidino, 2,2-dimethylpiperidino, 3,3-dimethylpiperidino, 4,4-dimethylpiperidino, 4-fluoromethylpiperidino, 2-(1-aminocyclopropyl)piperazino, 2-carbamoylpiperidino, 3-carbamoylpiperidino, 4-carbamoylpiperidino, 2-methylcarbamoylpiperidino, 3-methylcarbamoylpiperidino, 4-methylcarbamoylpiperidino, 2-dimethylcarbamoylpiperidino, 3-dimethylcarbamoylpiperidino, 4-dimethylcarbamoylpiperidino, 3-fluoropiperidino, 4-fluoropiperidino, 4,4-difluoropiperidino, 4,4-difluoro-2-carbamoylpiperidino, 4-methylenepiperidino, 4-(difluoromethylene)piperidino, 4-methoxypiperidino, 4-methylpiperazino, 4-cyclopropylpiperazino, 2-carbamoyl-4-methylpiperazino, 3-oxo-4-methylpiperazino, 3-oxo-4-ethylpiperazino, 4,6-dimethyl-3-oxopiperazino, 3,5-dioxo-4-methylpiperazino, morpholino, 2,2-dimethylmorpholino, 3,3-dimethylmorpholino, hexahydropyridazino, 2-carbamoylhexahydropyridazino, pyrazolidino, 2-methylpyrazolidinyl, 2-formylpyrazolidinyl, 3-methyl-4-oxoimidazolidino, 4-methyl-3-oxohomopiperazinyl, and 4-methyl-1,2-dihydropyrazin-3-on-1-yl.

Next will be described compounds (I) of the present invention in more detail.

Preferred species of Ar₁ include 2-thiazolyl, 5-thiazolyl, phenyl, 2-pyridyl, 3-pyridyl, 4-pyridyl, 3-pyridazinyl, 2-pyrimidinyl, 5-pyrimidinyl, and 2-pyrazinyl, with 2-thiazolyl, 3-pyridyl, 3-pyridazinyl, 2-pyrazinyl, and 2-pyrimidinyl being particularly preferred.

Preferred species of Ar₂ include phenyl, 4-aminomethylphenyl, 4-formylmethylphenyl, 4-acetylmethylphenyl, 4-(methylsulfonylaminomethyl)phenyl, 4-(hydroxymethyl)phenyl, 4-(methoxymethyl)phenyl, 4-(fluoromethyl)phenyl, 4-(difluoromethyl)phenyl, 4-trifluoromethyl-phenyl, 1H-pyrrol-2-yl, 1-methyl-1H-pyrrol-2-yl, 1H-pyrrol-1-yl, 3-methyl-1H-pyrrol-1-yl, 3-carbamoyl-1H-pyrrol-1-yl, 3-hydroxymethyl-1H-pyrrol-1-yl, 3-aminomethyl-1H-pyrrol-1-yl, 3-methylaminomethyl-1H-pyrrol-1-yl, 3-dimethylaminomethyl-1H-pyrrol-1-yl, 1H-pyrrol-3-yl, 1-methyl-1H-pyrrol-3-yl, 1H-pyrazol-3-yl, 1-methyl-1H-pyrazol-3-yl, 1H-pyrazol-4-yl, 1-methyl-1H-pyrazol-4-yl, 1H-imidazol-4-yl, 1-methyl-1H-imidazol-4-yl, thiazol-2-yl, thiazol-3-yl, thiazol-4-yl, 2-pyridyl, 3-pyridyl, 4-pyridyl, 3-methoxy-2-pyridyl, 3-methyl-2-pyridyl, 3-fluoro-2-pyridyl, 4-methyl-2-pyridyl, 4-chloromethyl-2-pyridyl, 4-ethyl-2-pyridyl, 4-cyano-2-pyridyl, 4-carbamoyl-2-pyridyl, 4-methoxy-2-pyridyl, 4-ethoxy-2-pyridyl, 4-chloro-2-pyridyl, 4-amino-2-pyridyl, 4-methylamino-2-pyridyl, 4-dimethylamino-2-pyridyl, 4-hydroxy-2-pyridyl, 4-aminomethyl-2-pyridyl, 4-methylaminomethylpyridyl, 4-dimethylaminomethylpyridyl, 5-methyl-2-pyridyl, 5-ethynyl-2-pyridyl, 5-hydroxy-2-pyridyl, 5-methoxy-2-pyridyl, 5-carbamoylmethoxy-2-pyridyl, 5-cyano-2-pyridyl, 5-amino-2-pyridyl, 5-methylamino-2-pyridyl, 5-dimethylamino-2-pyridyl, 5-carbamoyl-2-pyridyl, 5-methylcarbamoyl-2-pyridyl, 5-dimethylcarbamoyl-2-pyridyl, 5-chloro-2-pyridyl, 5-fluoro-2-pyridyl, 5-hydroxy-2-pyridyl, 5-aminomethyl-2-pyridyl, 5-methylaminomethylpyridyl, 5-dimethylaminomethylpyridyl, 5-hydroxymethyl-2-pyridyl, 5-aminomethyl-2-pyridyl, 5-fluoromethyl-2-pyridyl, 5-methoxymethyl-2-pyridyl, 5-acetyl-2-pyridyl, 6-methoxy-2-pyridyl, 6-methyl-2-pyridyl, 6-fluoro-2-pyridyl, 6-methoxy-3-pyridyl, 3-pyridazinyl, 6-methoxy-3-pyridazinyl, 6-methyl-3-pyridazinyl, 2-pyrimidinyl, 5-methoxy-2-pyrimidinyl, 5-methyl-2-pyrimidinyl, 5-methoxy-2-pyrazinyl, 5-methyl-2-pyrazinyl, and 5-amino-2-pyrazinyl. Of these, particularly preferred are phenyl, 2-fluorophenyl, 2-chlorophenyl, 2-methylphenyl, 2-methoxyphenyl, 2-hydroxyphenyl, 3-fluorophenyl, 3-chlorophenyl, 3-methylphenyl, 3-methoxyphenyl, 3-hydroxyphenyl, 4-fluorophenyl, 4-chlorophenyl, 4-methylphenyl, 4-methoxyphenyl, 4-hydroxyphenyl, 4-hydroxymethylphenyl, 4-aminomethylphenyl, 4-methylaminomethylphenyl, 4-dimethylaminomethylphenyl, 1-methyl-1H-pyrazol-3-yl, 1-methyl-1H-imidazol-4-yl, 2-pyridyl, 3-pyridyl, 4-pyridyl, 6-methoxy-3-pyridyl, 6-methyl-3-pyridyl, 6-methoxy-2-pyridyl, 6-methyl-2-pyridyl, 3-fluoro-2-pyridyl, 4-fluoro-2-pyridyl, 5-fluoro-2-pyridyl, 6-fluoro-2-pyridyl, 5-methyl-2-pyridyl, 5-ethyl-2-pyridyl, 5-ethynyl-2-pyridyl, 5-methoxy-2-pyridyl, 5-carbamoylmethoxy-2-pyridyl, 5-cyano-2-pyridyl, 5-carbamoyl-2-pyridyl, 5-amino-2-pyridyl, 5-hydroxymethyl-2-pyridyl, 5-aminomethyl-2-pyridyl, 5-fluoromethyl-2-pyridyl, 5-methoxymethyl-2-pyridyl, 5-acetyl-2-pyridyl, 4-amino-2-pyridyl, 4-pyrrolidinyl-2-pyridyl, 3-methoxy-2-pyridyl, 3-methyl-2-pyridyl, 6-methoxy-3-pyridazinyl, 6-methyl-3-pyridazinyl, 5-methoxy-2-pyrimidinyl, 5-methyl-2-pyrimidinyl, 5-methoxy-2-pyrazinyl, 5-methyl-2-pyrazinyl, and 5-amino-2-pyrazinyl.

Typical examples of the compounds of formula (II) include azetidin-1-yl, 3-oxoazetidin-1-yl, 2-oxoazetidin-1-yl, 3-aminoazetidin-1-yl, 3-methylaminoazetidin-1-yl, 3-dimethylaminoazetidin-1-yl, 2-methylazetidin-1-yl, 3-methylazetidin-1-yl, 2,2-dimethylazetidin-1-yl, 3,3-dimethylazetidin-1-yl, 2,2-dimethyl-3-dimethylaminoazetidin-1-yl, 2-hydroxymethylazetidin-1-yl, 3-hydroxymethylazetidin-1-yl, 2-fluoromethylazetidin-1-yl, 3-fluoromethylazetidin-1-yl, 3-methoxyazetidin-1-yl, 2-carbamoylazetidin-1-yl, 2-methylcarbamoylazetidin-1-yl, 2-dimethylcarbamoylazetidin-1-yl, 3-carbamoylazetidin-1-yl, 3-methylcarbamoylazetidin-1-yl, 3-dimethylcarbamoylazetidin-1-yl; pyrrolidino, 2-oxopyrrolidino, 3-oxopyrrolidino, 2,5-dioxopyrrolidino, 3-aminopyrrolidino, 3-methylaminopyrrolidino, 2-dimethylaminomethylpyrrolidino, 3-dimethylaminopyrrolidino, 2-methylpyrrolidino, 3-methylpyrrolidino, 2,2-dimethylpyrrolidino, 3,3-dimethylpyrrolidino, 2,2-dimethyl-3-dimethylaminopyrrolidino, 2-hydroxymethylpyrrolidino, 3-hydroxymethylpyrrolidino, 3-methoxypyrrolidino, 2-methoxymethylpyrrolidino, 3-methoxymethylpyrrolidino, 2-carbamoylpyrrolidino, 2-methylcarbamoylpyrrolidino, 2-dimethylcarbamoylpyrrolidino, 3-carbamoylpyrrolidino, 3-methylcarbamoylpyrrolidino, 3-dimethylcarbamoylpyrrolidino, 3-fluoropyrrolidino, 2-fluoromethylpyrrolidino;imidazolidin-1-yl, 3-methylimidazolidin-1-yl, 2-oxoimidazolidin-1-yl, 4-oxoimidazolidin-1-yl, 3-methyl-2-oxoimidazolidin-1-yl, 3-methyl-4-oxoimidazolidin-1-yl, 2,2-dimethylimidazolin-1-yl ; pyrazolidin-1-yl, 2-methylpyrazolidin-1-yl, 3-oxopyrazolidin-1-yl, 3,5-dioxopyrazolidin-1-yl, 2-formyl-pyrazolidin-1-yl, 2-carbamoylpyrazolidin-1-yl; piperidino, 2-oxopiperidino, 3-oxopiperidino, 4-oxopiperidino, 2-hydroxypiperidino, 3-hydroxypiperidino, 4-hydroxypiperidino, 2-methoxypiperidino, 3-methoxypiperidino, 4-methoxypiperidino, 3-aminopiperidino, 4-aminopiperidino, 3-methylaminopiperidino, 4-methylaminopiperidino, 3-dimethylaminopiperidino, 4-dimethylaminopiperidino, 2-methylpiperidino, 3-methylpiperidino, 4-methylpiperidino, 2,2-dimethylpiperidino, 3,3-dimethylpiperidino, 4,4-dimethylpiperidino, 3-fluoropiperidino, 4-fluoropiperidino, 4-chloropiperidino, 3,3-difluoropiperidino, 4,4-difluoropiperidino, 3,3-dichloropiperidino, 4,4-dichloropiperidino, 4-fluoromethylpiperidino, 2-hydroxymethylpiperidino, 3-hydroxymethylpiperidino, 4-hydroxymethylpiperidino, 2-(1-aminocyclopropyl)piperidino, 2-carbamoylpiperidino, 3-carbamoylpiperidino, 4-carbamoylpiperidino, 2-methylcarbamoylpiperidino, 3-methylcarbamoylpiperidino, 4-methylcarbamoylpiperidino, 2-dimethylcarbamoylpiperidino, 3-dimethylcarbamoylpiperidino, 4-dimethylcarbamoylpiperidino, 2-methoxymethylpiperidino, 3-methoxymethylpiperidino, 4-methoxymethylpiperidino, 2-aminomethylpiperidino, 3-aminomethylpiperidino, 4-aminomethylpiperidino, 2-methylaminomethylpiperidino, 3-methylaminomethylpiperidino, 4-methylaminomethylpiperidino, 2-dimethylaminomethylpiperidino, 3-dimethylaminomethylpiperidino, 4-dimethylaminomethylpiperidino, 2-aminoethylpiperidino, 3-aminoethylpiperidino, 4-aminoethylpiperidino, 2-methylaminoethylpiperidino, 3-methylaminoethylpiperidino, 4-methylaminoethylpiperidino, 2-dimethylaminoethylpiperidino, 3-dimethylaminoethylpiperidino, 4-dimethylaminoethylpiperidino, 4,4-difluoro-2-carbamoylpiperidino, 4-methylenepiperidino, 4-(difluoromethylene)piperidino; piperazino, 2-oxopiperazino, 3-oxopiperazino, 2-oxo-4-methylpiperazino, 3-oxo-4-methylpiperazino, 3-oxo-4-ethylpiperazino, 4-formylpiperazino, 2,3-dioxopiperazino, 3,5-dioxopiperazino, 2,6-dioxopiperazino, 2,3-dioxo-4-methylpiperazino, 3,5-dioxo-4-methylpiperazino, 2,6-dioxo-4-methylpiperazino, 2-methylpiperazino, 3-methylpiperazino, 4-methylpiperazino, 2-ethylpiperazino, 3-ethylpiperazino, 4-ethylpiperazino, 2-isopropylpiperazino, 3-isopropylpiperazino, 4-isopropylpiperazino, 2-cyclopropylpiperazino, 3-cyclopropylpiperazino, 4-cyclopropylpiperazino, 4-cyclobutylpiperazino, 2-cyclopropanespiropiperazino, 3-cyclopropanespiropiperazino, 2,2-dimethylpiperazino, 3,3-dimethylpiperazino, 2,3-dimethylpiperazino, 2,4-dimethylpiperazino, 3,4-dimethylpiperazino, 3,5-dimethylpiperazino, 2,6-dimethylpiperazino, 2-ethyl-4-methylpiperazino, 3-ethyl-4-methylpiperazino, 2-isopropyl-4-methylpiperazino, 3-isopropyl-4-methylpiperazino, 2-cyclopropyl-4-methylpiperazino, 3-cyclopropyl-4-methylpiperazino, 1,2,6-trimethylpiperazino, 3,4,5-trimethylpiperazino, 2,2,4-trimethylpiperazino, 3,3,4-trimethylpiperazino, 4,6-dimethyl-3-oxopiperazino, 3,3,4-trimethyl-5-oxopiperazino, 2,2,4-trimethyl-3-oxopiperazino, 2-cyclopropanespiro-4-methylpiperazino, 3-cyclopropanespiro-4-methylpiperazino, 2-cyclopropanespiro-4-methyl-3-oxopiperazino, 3-cyclopropanespiro-4-methyl-5-oxopiperazino, 4-acetylpiperazino, 4-acetyl-3-cyclopropanespiropiperazino, 2-hydroxymethylpiperazino, 3-hydroxymethylpiperazino, 4-methoxypiperazino, 2-methoxymethylpiperazino, 3-methoxymethylpiperazino, 2-hydroxyethylpiperazino, 3-hydroxyethylpiperazino, 4-hydroxyethylpiperazino, 2-hydroxymethyl-4-methylpiperazino, 3-hydroxymethyl-4-methylpiperazino, 2-methoxymethyl-4-methylpiperazino, 3-methoxymethyl-4-methylpiperazino, 2-hydroxyethyl-4-methylpiperazino, 3-hydroxyethyl-4-methylpiperazino, 2-methoxyethyl-4-methylpiperazino, 3-methoxyethyl-4-methylpiperazino, 2-carbamoylpiperazino, 3-carbamoylpiperazino, 4-carbamoylpiperazino, 2-methylcarbamoylpiperazino, 3-methylcarbamoylpiperazino, 4-methylcarbamoylpiperazino, 2-dimethylcarbamoylpiperazino, 3-dimethylcarbamoylpiperazino, 4-dimethylcarbamoylpiperazino, 2-carbamoylmethylpiperazino, 3-carbamoylmethylpiperazino, 4-carbamoylmethylpiperazino, 2-methylcarbamoylmethylpiperazino, 3-methylcarbamoylmethylpiperazino, 4-methylcarbamoylpiperazino, 2-dimethylcarbamoylmethylpiperazino, 3-dimethylcarbamoylmethylpiperazino, 2-carbamoyl-4-methylpiperazino, 3-carbamoyl-4-methylpiperazino, 4-carbamoylpiperazino, 2-methylcarbamoyl-4-methylpiperazino, 3-methylcarbamoyl-4-methylpiperazino, 4-methylcarbamoylpiperazino, 2-dimethylcarbamoyl-4-methylpiperazino, 3-dimethylcarbamoyl-4-methylpiperazino, 4-dimethylcarbamoylpiperazino, 2-carbamoylmethyl-4-methylpiperazino, 3-carbamoylmethyl-4-methylpiperazino, 4-carbamoylmethylpiperazino, 2-methylcarbamoylmethyl-4-methylpiperazino, 3-methylcarbamoylmethyl-4-methylpiperazino, 4-methylcarbamoylpiperazino, 2-dimethylcarbamoylmethyl-4-methylpiperazino, 3-dimethylcarbamoylmethyl-4-methylpiperazino, 2-aminomethylpiperazino, 3-aminomethylpiperazino, 2-methylaminomethylpiperazino, 3-methylaminomethylpiperazino, 2-dimethylaminomethylpiperazino, 3-dimethylaminomethylpiperazino, 2-aminoethylpiperazino, 3-aminoethylpiperazino, 4-aminoethylpiperazino, 2-methylaminoethylpiperazino, 3-methylaminoethylpiperazino, 4-methylaminoethylpiperazino, 2-dimethylaminoethylpiperazino, 3-dimethylaminoethylpiperazino, 4-dimethylaminoethylpiperazino, 2-aminomethyl-4-methylpiperazino, 3-aminomethyl-4-methylpiperazino, 2-methylaminomethyl-4-methylpiperazino, 3-methylaminomethyl-4-methylpiperazino, 2-dimethylaminomethyl-4-methylpiperazino, 3-dimethylaminomethyl-4-methylpiperazino, 2-aminoethyl-4-methylpiperazino, 3-aminoethyl-4-methylpiperazino, 2-methylaminoethyl-4-methylpiperazino,.3-methylaminoethyl-4-methylpiperazino, 2-dimethylaminoethyl-4-methylpiperazino, 3-dimethylaminoethyl-4-methylpiperazino, 4-methanesulfonylpiperazino; 1,2-dihydropyrazin-3-on-1-yl, 4-methyl-1,2-dihydropyrazin-3-on-1-yl; morpholino, 2-methylmorpholino, 3-methylmorpholino, 2-ethylmorpholino, 3-ethylmorpholino, 2-cyclopropanespiromorpholino, 3-cyclopropanespiromorpholino, 2,2-dimethylmorpholino, 3,3-dimethylmorpholino, 2-hydroxymethylmorpholino, 3-hydroxymethylmorpholino, 2-methoxymethylmorpholino, 3-methoxymethylmorpholino, 2-hydroxyethylmorpholino, 3-hydroxyethylmorpholino, 2-methoxyethylmorpholino, 3-methoxyethylmorpholino, 2-carbamoylmorpholino, 3-carbamoylmorpholino, 2-methylcarbamoylmorpholino, 3-methylcarbamoylmorpholino, 2-dimethylcarbamoylmorpholino, 3-dimethylcarbamoylmorpholino, 2-carbamoylmethylmorpholino, 3-carbamoylmethylmorpholino, 2-methylcarbamoylmethylmorpholino, 3-methylcarbamoylmethylmorpholino, 2-dimethylcarbamoylmethylmorpholino, 3-dimethylcarbamoylmethylmorpholino, 2-carbamoylethylmorpholino, 3-carbamoylethylmorpholino, 2-methylcarbamoylethylmorpholino, 3-methylcarbamoylethylmorpholino, 2-dimethylcarbamoylethylmorpholino, 3-dimethylcarbamoylethylmorpholino, 2-aminomethylmorpholino, 3-aminomethylmorpholino, 2-methylaminomethylmorpholino, 3-methylaminomethylmorpholino, 2-dimethylaminomethylmorpholino, 3-dimethylaminomethylmorpholino, 2-aminoethylmorpholino, 3-aminoethylmorpholino, 2-methylaminoethylmorpholino, 3-methylaminoethylmorpholino, 2-dimethylaminoethylmorpholino, 3-dimethylaminoethylmorpholino; thiomorpholino, 3-oxothiomorpholino, 1,1-dioxothiomorpholino, 2-methylthiomorpholino, 3-methylthiomorpholino, 2-ethylthiomorpholino, 3-ethylthiomorpholino, 2-cyclopropanespirothiomorpholino, 3-cyclopropanespirothiomorpholino, 2,2-dimethylthiomorpholino, 3,3-dimethylthiomorpholino, 2-hydroxymethylthiomorpholino, 3-hydroxymethylthiomorpholino, 2-methoxymethylthiomorpholino, 3-methoxymethylthiomorpholino, 2-hydroxyethylthiomorpholino, 3-hydroxyethylthiomorpholino, 2-methoxyethylthiomorpholino, 3-methoxyethylthiomorpholino, 2-carbamoylthiomorpholino, 3-carbamoylthiomorpholino, 2-methylcarbamoylthiomorpholino, 3-methylcarbamoylthiomorpholino, 2-dimethylcarbamoylthiomorpholino, 3-dimethylcarbamoylthiomorpholino, 2-carbamoylmethylthiomorpholino, 3-carbamoylmethylthiomorpholino, 2-methylcarbamoylmethylthiomorpholino, 3-methylcarbamoylmethylthiomorpholino, 2-dimethylcarbamoylmethylthiomorpholino, 3-dimethylcarbamoylmethylthiomorpholino, 2-carbamoylethylthiomorpholino, 3-carbamoylethylthiomorpholino, 2-methylcarbamoylethylthiomorpholino, 3-methylcarbamoylethylthiomorpholino, 2-dimethylcarbamoylethylthiomorpholino, 3-dimethylcarbamoylethylthiomorpholino, 2-aminomethylthiomorpholino, 3-aminomethylthiomorpholino, 2-methylaminomethylthiomorpholino, 3-methylaminomethylthiomorpholino, 2-dimethylaminomethylthiomorpholino, 3-dimethylaminomethylthiomorpholino, 2-aminoethylthiomorpholino, 3-aminoethylthiomorpholino, 2-methylaminoethylthiomorpholino, 3-methylaminoethylthiomorpholino, 2-dimethylaminoethylthiomorpholino, 3-dimethylaminoethylthiomorpholino;hexahydropyridazin-1-yl, 2-formylhexahydropyridazin-1-yl, 2-acetylhexahydropyridazin-1-yl, 3-oxohexahydropyridazin-1-yl, 6-oxohexahydropyridazin-1-yl, 4-aminohexahydropyridazin-1-yl, 4-methylaminohexahydropyridazin-1-yl, 4-dimethylaminohexahydropyridazin-1-yl, 2-methylhexahydropyridazin-1-yl, 3-methylhexahydropyridazin-1-yl, 4-methylhexahydropyridazin-1-yl, 2,3-dimethylhexahydropyridazin-1-yl, 3,3-dimethylhexahydropyridazin-1-yl, 4,4-dimethylhexahydropyridazin-1-yl, 3-hydroxymethylhexahydropyridazin-1-yl, 4-hydroxymethylhexahydropyridazin-1-yl, 5-hydroxymethylhexahydropyridazin-1-yl, 6-hydroxymethylhexahydropyridazin-1-yl, 2-carbamoylhexahydropyridazin-1-yl, 3-carbamoylhexahydropyridazin-1-yl, 4-carbamoylhexahydropyridazin-1-yl, 5-carbamoylhexahydropyridazin-1-yl, 6-carbamoylhexahydropyridazin-1-yl, 2-methylcarbamoylhexahydropyridazin-1-yl, 3-methylcarbamoylhexahydropyridazin-1-yl, 4-methylcarbamoylhexahydropyridazin-1-yl, 5-methylcarbamoylhexahydropyridazin-1-yl, 6-methylcarbamoylhexahydropyridazin-1-yl, 2-dimethylcarbamoylhexahydropyridazin-1-yl, 3-dimethylcarbamoylhexahydropyridazin-1-yl, 4-dimethylcarbamoylhexahydropyridazin-1-yl, 5-dimethylcarbamoylhexahydropyridazin-1-yl, 6-dimethylcarbamoylhexahydropyridazin-1-yl, 3-methoxymethylhexahydropyridazin-1-yl, 4-methoxymethylhexahydropyridazin-1-yl, 5-methoxymethylhexahydropyridazin-1-yl, 6-methoxymethylhexahydropyridazin-1-yl, 2-aminoethylhexahydropyridazin-1-yl, 3-aminoethylhexahydropyridazin-1-yl, 4-aminoethylhexahydropyridazin-1-yl, 5-aminoethylhexahydropyridazin-1-yl, 6-aminoethylhexahydropyridazin-1-yl, 2-methylaminoethylhexahydropyridazin-1-yl, 3-methylaminoethylhexahydropyridazin-1-yl, 4-methylaminoethylhexahydropyridazin-1-yl, 5-methylaminoethylhexahydropyridazin-1-yl, 6-methylaminoethylhexahydropyridazin-1-yl, 3-aminomethylhexahydropyridazin-1-yl, 4-aminomethylhexahydropyridazin-1-yl, 5-aminomethylhexahydropyridazin-1-yl, 6-aminomethylhexahydropyridazin-1-yl, 3-methylaminomethylhexahydropyridazin-1-yl, 4-methylaminomethylhexahydropyridazin-1-yl, 5-methylaminomethylhexahydropyridazin-1-yl, 6-methylaminomethylhexahydropyridazin-1-yl, 3-dimethylaminomethylhexahydropyridazin-1-yl, 4-dimethylaminomethylhexahydropyridazin-1-yl, 5-dimethylaminomethylhexahydropyridazin-1-yl, 6-dimethylaminomethylhexahydropyridazin-1-yl, 2-dimethylaminoethylhexahydropyridazin-1-yl, 3-dimethylaminoethylhexahydropyridazin-1-yl, 4-dimethylaminoethylhexahydropyridazin-1-yl, 5-dimethylaminoethylhexahydropyridazin-1-yl, 6-dimethylaminoethylhexahydropyridazin-1-yl;hexahydropyrimidin-1-yl, 2-oxohexahydropyrimidin-1-yl, 4-oxohexahydropyrimidin-1-yl, 5-oxohexahydropyrimidin-1-yl, 6-oxohexahydropyrimidin-1-yl, 2-methylhexahydropyrimidin-1-yl, 3-methylhexahydropyrimidin-1-yl, 4-methylhexahydropyrimidin-1-yl, 4-methylhexahydropyrimidin-1-yl, 2,2-dimethylhexahydropyrimidin-1-yl, 4,4-dimethylhexahydropyrimidin-1-yl, 5,5-dimethylhexahydropyrimidin-1-yl, 6,6-dimethylhexahydropyrimidin-1-yl, 2-hydroxymethylhexahydropyrimidin-1-yl, 4-hydroxymethylhexahydropyrimidin-1-yl, 5-hydroxymethylhexahydropyrimidin-1-yl, 6-hydroxymethylhexahydropyrimidin-1-yl, 2-carbamoylhexahydropyrimidin-1-yl, 3-carbamoylhexahydropyrimidin-1-yl, 4-carbamoylhexahydropyrimidin-1-yl, 5-carbamoylhexahydropyrimidin-1-yl, 6-carbamoylhexahydropyrimidin-1-yl, 2-methylcarbamoylhexahydropyrimidin-1-yl, 3-methylcarbamoylhexahydropyrimidin-1-yl, 4-methylcarbamoylhexahydropyrimidin-1-yl, 5-methylcarbamoylhexahydropyrimidin-1-yl, 6-methylcarbamoylhexahydropyrimidin-1-yl, 2-dimethylcarbamoylhexahydropyrimidin-1-yl, 3-dimethylcarbamoylhexahydropyrimidin-1-yl, 4-dimethylcarbamoylhexahydropyrimidin-1-yl, 5-dimethylcarbamoylhexahydropyrimidin-1-yl, 6-dimethylcarbamoylhexahydropyrimidin-1-yl, 3-methoxymethylhexahydropyrimidin-1-yl, 4-methoxymethylhexahydropyrimidin-1-yl, 5-methoxymethylhexahydropyrimidin-1-yl, 6-methoxymethylhexahydropyrimidin-1-yl, 2-aminoethylhexahydropyrimidin-1-yl, 3-aminoethylhexahydropyrimidin-1-yl, 4-aminoethylhexahydropyrimidin-1-yl, 5-aminoethylhexahydropyrimidin-1-yl, 6-aminoethylhexahydropyrimidin-1-yl, 2-methylaminoethylhexahydropyrimidin-1-yl, 3-methylaminoethylhexahydropyrimidin-1-yl, 4-methylaminoethylhexahydropyrimidin-1-yl, 5-methylaminoethylhexahydropyrimidin-1-yl, 6-methylaminoethylhexahydropyrimidin-1-yl, 2-dimethylaminoethylhexahydropyrimidin-1-yl, 3-dimethylaminoethylhexahydropyrimidin-1-yl, 4-dimethylaminoethylhexahydropyrimidin-1-yl, 5-dimethylaminoethylhexahydropyrimidin-1-yl, 6-dimethylaminoethylhexahydropyrimidin-1-yl;homopiperazino, 2-oxohomopiperazino, 3-oxohomopiperazino, 5-oxohomopiperazino, 6-oxohomopiperazino, 7-oxohomopiperazino, 2-oxo-4-methylhomopiperazino, 3-oxo-4-methylhomopiperazino, 5-oxo-4-methylhomopiperazino, 6-oxo-4-methylhomopiperazino, 7-oxo-4-methylhomopiperazino, 2,3-dioxohomopiperazino, 2,7-dioxohomopiperazino, 3,5-dioxohomopiperazino, 3,7-dioxohomopiperazino, 2,3-dioxo-4-methylhomopiperazino, 2,7-dioxo-4-methylhomopiperazino, 3,5-dioxo-4-methylhomopiperazino, 3,7-dioxo-4-methylhomopiperazino, 2-methylhomopiperazino, 3-methylhomopiperazino, 4-methylhomopiperazino, 5-methylhomopiperazino, 6-methylhomopiperazino, 7-methylhomopiperazino, 2-ethylhomopiperazino, 3-ethylhomopiperazino, 4-ethylhomopiperazino, 5-ethylhomopiperazino, 6-ethylhomopiperazino, 7-ethylhomopiperazino, 4-cyclopropylhomopiperazino, 2-cyclopropanespirohomopiperazino, 3-cyclopropanespirohomopiperazino, 5-cyclopropanespirohomopiperazino, 6-cyclopropanespirohomopiperazino, 7-cyclopropanespirohomopiperazino, 2-cyclopropanespiro-4-methylhomopiperazino, 3-cyclopropanespiro-4-methylhomopiperazino, 5-cyclopropanespiro-4-methylhomopiperazino, 6-cyclopropanespiro-4-methylhomopiperazino, 7-cyclopropanespiro-4-methylhomopiperazino, 2-cyclopropanespiro-4-methyl-3-oxohomopiperazino, 2-cyclopropanespiro-4-methyl-5-oxohomopiperazino, 2-cyclopropanespiro-4-methyl-7-oxohomopiperazino, 3-cyclopropanespiro-4-methyl-2-oxohomopiperazino, 3-cyclopropanespiro-4-methyl-5-oxohomopiperazino, 3-cyclopropanespiro-4-methyl-7-oxohomopiperazino, 5-cyclopropanespiro-4-methyl-2-oxohomopiperazino, 5-cyclopropanespiro-4-methyl-3-oxohomopiperazino, 5-cyclopropanespiro-4-methyl-7-oxohomopiperazino, 6-cyclopropanespiro-4-methyl-2-oxohomopiperazino, 6-cyclopropanespiro-4-methyl-3-oxohomopiperazino, 6-cyclopropanespiro-4-methyl-5-oxohomopiperazino, 6-cyclopropanespiro-4-methyl-7-oxohomopiperazino,7-cyclopropanespiro-4-methyl-2-oxohomopiperazino, 7-cyclopropanespiro-4-methyl-3-oxohomopiperazino, 7-cyclopropanespiro-4-methyl-5-oxohomopiperazino, 2,2-dimethylhomopiperazino, 3,3-dimethylhomopiperazino, 5,5-dimethylhomopiperazino, 6,6-dimethylhomopiperazino, 7,7-dimethylhomopiperazino, 2,3-dimethylhomopiperazino, 2,4-dimethylhomopiperazino, 3,4-dimethylhomopiperazino, 3,5-dimethylhomopiperazino, 3,4,5-trimethylhomopiperazino, 2-hydroxymethylhomopiperazino, 3-hydroxymethylhomopiperazino, 5-hydroxymethylhomopiperazino, 6-hydroxymethylhomopiperazino, 7-hydroxymethylhomopiperazino, 2-hydroxymethyl-4-methylhomopiperazino, 3-hydroxymethyl-4-methylhomopiperazino, 5-hydroxymethyl-4-methylhomopiperazino, 6-hydroxymethyl-4-methylhomopiperazino, 7-hydroxymethyl-4-methylhomopiperazino, 2-methoxymethylhomopiperazino, 3-methoxymethylhomopiperazino, 5-methoxymethylhomopiperazino, 6-methoxymethylhomopiperazino, 7-methoxymethylhomopiperazino, 2-methoxymethyl-4-methylhomopiperazino, 3-methoxymethyl-4-methylhomopiperazino, 5-methoxymethyl-4-methylhomopiperazino, 6-methoxymethyl-4-methylhomopiperazino, 7-methoxymethyl-4-methylhomopiperazino, 2-hydroxyethylhomopiperazino, 3-hydroxyethylhomopiperazino, 4-hydroxyethylhomopiperazino, 5-hydroxyethylhomopiperazino, 6-hydroxyethylhomopiperazino, 7-hydroxyethylhomopiperazino, 2-hydroxyethyl4-methylhomopiperazino, 3-hydroxyethyl-4-methylhomopiperazino, 5-hydroxyethyl-4-methylhomopiperazino, 6-hydroxyethyl-4-methylhomopiperazino, 7-hydroxyethyl-4-methylhomopiperazino, 2-methoxyethylhomopiperazino, 3-methoxyethylhomopiperazino, 4-methoxyethylhomopiperazino, 5-methoxyethylhomopiperazino, 6-methoxyethylhomopiperazino, 7-methoxyethylhomopiperazino, 2-methoxyethyl-4-methylhomopiperazino, 3-methoxyethyl-4-methylhomopiperazino, 5-methoxyethyl-4-methylhomopiperazino, 6-methoxyethyl-4-methylhomopiperazino, 7-methoxyethyl-4-methylhomopiperazino, 2-carbamoylhomopiperazino, 3-carbamoylhomopiperazino, 4-carbamoylhomopiperazino, 5-carbamoylhomopiperazino, 6-carbamoylhomopiperazino, 7-carbamoylhomopiperazino, 2-carbamoyl-4-methylhomopiperazino, 3-carbamoyl-4-methylhomopiperazino, 4-carbamoylhomopiperazino, 5-carbamoyl-4-methylhomopiperazino, 6-carbamoyl-4-methylhomopiperazino, 7-carbamoyl-4-methylhomopiperazino, 2-methylcarbamoylhomopiperazino, 3-methylcarbamoylhomopiperazino, 4-methylcarbamoylhomopiperazino, 5-methylcarbamoylhomopiperazino, 6-methylcarbamoylhomopiperazino, 7-methylcarbamoylhomopiperazino, 2-methylcarbamoyl-4-methylhomopiperazino, 3-methylcarbamoyl-4-methylhomopiperazino, 5-methylcarbamoyl-4-methylhomopiperazino, 6-methylcarbamoyl-4-methylhomopiperazino, 7-methylcarbamoyl-4-methylhomopiperazino, 2-dimethylcarbamoylhomopiperazino, 3-dimethylcarbamoylhomopiperazino, 4-dimethylcarbamoylhomopiperazino, 5-dimethylcarbamoylhomopiperazino, 6-dimethylcarbamoylhomopiperazino, 7-dimethylcarbamoylhomopiperazino, 2-dimethylcarbamoyl-4-methylhomopiperazino, 3-dimethylcarbamoyl-4-methylhomopiperazino, 5-dimethylcarbamoyl-4-methylhomopiperazino, 6-dimethylcarbamoyl-4-methylhomopiperazino, 7-dimethylcarbamoyl-4-methylhomopiperazino, 2-carbamoylmethylhomopiperazino, 3-carbamoylmethylhomopiperazino, 4-carbamoylmethylhomopiperazino, 5-carbamoylmethylhomopiperazino, 6-carbamoylmethylhomopiperazino, 7-carbamoylmethylhomopiperazino, 2-carbamoylmethyl-4-methylhomopiperazino, 3-carbamoylmethyl-4-methylhomopiperazino, 5-carbamoylmethyl-4-methylhomopiperazino, 6-carbamoylmethyl-4-methylhomopiperazino, 7-carbamoylmethyl-4-methylhomopiperazino, 2-methylcarbamoylmethylhomopiperazino, 3-methylcarbamoylmethylhomopiperazino, 4-methylcarbamoylhomopiperazino, 5-methylcarbamoylhomopiperazino, 6-methylcarbamoylhomopiperazino, 7-methylcarbamoylhomopiperazino, 2-methylcarbamoylmethyl-4-methylhomopiperazino, 3-methylcarbamoylmethyl-4-methylhomopiperazino, 5-methylcarbamoyl-4-methylhomopiperazino, 6-methylcarbamoyl-4-methylhomopiperazino, 7-methylcarbamoyl-4-methylhomopiperazino, 2-dimethylcarbamoylmethylhomopiperazino, 3-dimethylcarbamoylmethylhomopiperazino, 4-dimethylcarbamoylmethylhomopiperazino, 5-dimethylcarbamoylmethylhomopiperazino, 6-dimethylcarbamoylmethylhomopiperazino, 7-dimethylcarbamoylmethylhomopiperazino, 2-dimethylcarbamoylmethyl-4-methylhomopiperazino, 3-dimethylcarbamoylmethyl-4-methylhomopiperazino, 5-dimethylcarbamoylmethyl-4-methylhomopiperazino, 6-dimethylcarbamoylmethyl-4-methylhomopiperazino, 7-dimethylcarbamoylmethyl-4-methylhomopiperazino, 2-aminomethylhomopiperazino, 3-aminomethylhomopiperazino, 5-aminomethylhomopiperazino, 6-aminomethylhomopiperazino, 7-aminomethylhomopiperazino, 2-aminomethyl-4-methylhomopiperazino, 3-aminomethyl-4-methylhomopiperazino, 5-aminomethyl-4-methylhomopiperazino, 6-aminomethyl-4-methylhomopiperazino, 7-aminbmethyl-4-methylhomopiperazino, 2-methylaminomethylhomopiperazino, 3-methylaminomethylhomopiperazino, 4-methylaminomethylhomopiperazino, 5-methylaminomethylhomopiperazino, 6-methylaminomethylhomopiperazino, 7-methylaminomethylhomopiperazino, 2-methylaminomethyl-4-methylhomopiperazino, 3-methylaminomethyl-4-methylhomopiperazino, 5-methylaminomethyl-4-methylhomopiperazino, 6-methylaminomethyl-4-methylhomopiperazino, 7-methylaminomethyl-4-methylhomopiperazino, 2-dimethylaminomethylhomopiperazino, 3-dimethylaminomethylhomopiperazino, 4-dimethylaminomethylhomopiperazino, 5-dimethylaminomethylhomopiperazino, 6-dimethylaminomethylhomopiperazino, 7-dimethylaminomethylhomopiperazino, 2-dimethylaminomethyl-4-methylhomopiperazino, 3-dimethylaminomethyl-4-methylhomopiperazino, 5-dimethylaminomethyl-4-methylhomopiperazino, 6-dimethylaminomethyl-4-methylhomopiperazino, 7-dimethylaminomethyl-4-methylhomopiperazino, 2-aminoethylhomopiperazino, 3-aminoethylhomopiperazino, 4-aminoethylhomopiperazino, 5-aminoethylhomopiperazino, 6-aminoethylhomopiperazino, 7-aminoethylhomopiperazino, 2-aminoethyl-4-methylhomopiperazino, 3-aminoethyl-4-methylhomopiperazino, 5-aminoethyl-4-methylhomopiperazino, 6-aminoethyl-4-methylhomopiperazino, 7-aminoethyl-4-methylhomopiperazino, 2-methylaminoethylhomopiperazino, 3-methylaminoethylhomopiperazino, 4-methylaminoethylhomopiperazino, 5-methylaminoethylhomopiperazino, 6-methylaminoethylhomopiperazino, 7-methylaminoethylhomopiperazino, 2-methylaminoethyl-4-methylhomopiperazino, 3-methylaminoethyl-4-methylhomopiperazino, 5-methylaminoethyl-4-methylhomopiperazino, 6-methylaminoethyl-4-methylhomopiperazino, 7-methylaminoethyl-4-methylhomopiperazino, 2-dimethylaminoethylhomopiperazino, 3-dimethylaminoethylhomopiperazino, 4-dimethylaminoethylhomopiperazino, 5-dimethylaminoethylhomopiperazino, 6-dimethylaminoethylhomopiperazino, 7-dimethylaminoethylhomopiperazino, 2-dimethylaminoethyl-4-methylhomopiperazino, 3-dimethylaminoethyl-4-methylhomopiperazino, 5-dimethylaminoethyl-4-methylhomopiperazino, 6-dimethylaminoethyl-4-methylhomopiperazino, 7-dimethylaminoethyl-4-methylhomopiperazino, 4-methanesulfonylhomopiperazino, 4-methanesulfonylaminohomopiperazino, 4-(azetidin-1-yl)homopiperazino, 4-pyrrolidinohomopiperazino, 4-piperidinohomopiperazino; and 1,4-oxazepan-4-yl.

Of the above-listed compounds, preferred ones include the following:
Azetidin-1-yl, 3-dimethylaminoazetidin-1-yl, 2-methylazetidin-1-yl, 3-methylazetidin-1-yl, 2,2-dimethylazetidin-1-yl, 3,3-dimethylazetidin-1-yl, 2,2-dimethyl-3-dimethylaminoazetidin-1-yl, 2-hydroxymethylazetidin-1-yl, 3-hydroxymethylazetidin-1-yl, 2-carbamoylazetidin-1-yl, 2-methylcarbamoylazetidin-1-yl, 2-dimethylcarbamoylazetidin-1-yl, 3-methoxyazetidin-1-yl; pyrrolidino, 2-fluoropyrrolidino, 3-fluoropyrrolidino, 2-oxopyrrolidino, 2-dimethylaminomethylpyrrolidino, 3-dimethylaminomethylpyrrolidino, 2,5-dioxopyrrolidino, 2-methylpyrrolidino, 3-methylpyrrolidino, 2,2-dimethylpyrrolidino, 3,3-dimethylpyrrolidino, 2-hydroxymethylpyrrolidino, 3-hydroxymethylpyrrolidino, 3-methoxypyrrolidino, 2-methoxymethylpyrrolidino, 3-methoxymethylpyrrolidino, 2-carbamoylpyrrolidino, 2-methylcarbamoylpyrrolidino, 2-dimethylcarbamoylpyrrolidino, 2-fluoromethylpyrrolidino; 2-oxoimidazolidin-1-yl, 4-oxoimidazolidin-1-yl, 3-methyl-2-oxoimidazolidin-1-yl, 3-methyl-4-oxoimidazolidin-1-yl; pyrazolidino, 2-methylpyrazolidin-1-yl, 3-oxopyrazolidin-1-yl, 3,5-dioxopyrazolidin-1-yl, 2-formylpyrazolidin-1-yl, 2-carbamoylpyrazolidin-1-yl; piperidino, 2-oxopiperidino, 3-oxopiperidino, 4-oxopiperidino, 2-hydroxymethylpiperidino, 3-hydroxymethylpiperidino, 4-hydroxymethylpiperidino, 2-methoxypiperidino, 3-methoxypiperidino, 4-methoxypiperidino, 2-methylpiperidino, 3-methylpiperidino, 4-methylpiperidino, 2,2-dimethylpiperidino, 3,3-dimethylpiperidino, 4,4-dimethylpiperidino, 3-fluoropiperidino, 4-fluoropiperidino, 4-chloropiperidino, 3,3-difluoropiperidino, 4,4-difluoropiperidino, 2-fluoromethylpiperidino, 3-fluoromethylpiperidino, 4-fluoromethylpiperidino, 3,3-dichloropiperidino, 4,4-dichloropiperidino, 4-fluoromethylpiperidino, 2-hydroxymethylpiperidino, 2-(1-aminocyclopropyl)piperidino, 2-carbamoylpiperidino, 2-methylcarbamoylpiperidino, 2-dimethylcarbamoylpiperidino, 2-methoxymethylpiperidino, 4-methyl-4-methoxypiperidino, 2-aminomethylpiperidino, 2-methylaminomethylpiperidino, 2-dimethylaminomethylpiperidino, 2-aminoethylpiperidino, 2-methylaminoethylpiperidino, 2-dimethylaminoethylpiperidino, 4,4-difluoro-2-carbamoylpiperidino, 4-methylenepiperidino, 4-(difluoromethylene)piperidino; piperazino, 2-oxo-4-methylpiperazino, 3-oxo-4-methylpiperazino, 3-oxo-4-ethylpiperazino, 4-formylpiperazino, 2,3-dioxo-4-methylpiperazino, 3,5-dioxo-4-methylpiperazino, 2,6-dioxo-4-methylpiperazino, 4-methylpiperazino, 4-ethylpiperazino, 4-isopropylpiperazino, 4-cyclopropylpiperazino, 2,4-dimethylpiperazino, 3,4-dimethylpiperazino, 2-ethyl-4-methylpiperazino, 3-ethyl-4-methylpiperazino, 2-isopropyl-4-methylpiperazino, 3-isopropyl-4-methylpiperazino, 2-cyclopropyl-4-methylpiperazino, 3-cyclopropyl-4-methylpiperazino, 3,4,5-trimethylpiperazino, 2,2,4-trimethylpiperazino, 3,3,4-trimethylpiperazino, 4,6-dimethyl-3-oxopiperazino, 3,3,4-trimethyl-5-oxopiperazino, 2,2,4-trimethyl-3-oxopiperazino, 2-cyclopropanespiro-4-methylpiperazino, 3-cyclopropanespiro-4-methylpiperazino, 2-cyclopropanespiro-4-methyl-3-oxopiperazino, 3-cyclopropanespiro-4-methyl-5-oxopiperazino, 4-acetyl-3-cyclopropanespiropiperazino, 2-hydroxymethyl-4-methylpiperazino, 3-hydroxymethyl-4-methylpiperazino, 4-methoxypiperazino, 2-methoxymethyl-4-methylpiperazino, 3-methoxymethyl-4-methylpiperazino, 2-hydroxyethyl-4-methylpiperazino, 3-hydroxyethyl-4-methylpiperazino, 2-methoxyethyl-4-methylpiperazino, 3-methoxyethyl-4-methylpiperazino, 2-carbamoyl-4-methylpiperazino, 3-carbamoyl-4-methylpiperazino, 4-carbamoylpiperazino, 2-methylcarbamoyl-4-methylpiperazino, 3-methylcarbamoyl-4-methylpiperazino, 4-methylcarbamoylpiperazino, 2-dimethylcarbamoyl-4-methylpiperazino, 3-dimethylcarbamoyl-4-methylpiperazino, 4-dimethylcarbamoylpiperazino, 2-carbamoylmethyl-4-methylpiperazino, 3-carbamoylmethyl-4-methylpiperazino, 4-carbamoylmethylpiperazino, 2-methylcarbamoylmethyl-4-methylpiperazino, 3-methylcarbamoylmethyl-4-methylpiperazino, 4-methylcarbamoylpiperazino, 2-dimethylcarbamoylmethyl-4-methylpiperazino, 3-dimethylcarbamoylmethyl-4-methylpiperazino, 2-aminomethyl-4-methylpiperazino, 2-methylaminomethyl-4-methylpiperazino, 2-dimethylaminomethyl-4-methylpiperazino, 2-aminoethyl-4-methylpiperazino, 2-methylaminoethyl-4-methylpiperazino, 2-dimethylaminoethyl-4-methylpiperazino; morpholino, 2-methylmorpholino, 3-methylmorpholino, 2-ethylmorpholino, 3-ethylmorpholino, 2-cyclopropanespiromorpholino, 3-cyclopropanespiromorpholino, 2,2-dimethylmorpholino, 3,3-dimethylmorpholino, 3-hydroxymethylmorpholino, 3-methoxymethylmorpholino, 3-hydroxyethylmorpholino, 3-methoxyethylmorpholino, 3-carbamoylmorpholino, 3-methylcarbamoylmorpholino, 3-dimethylcarbamoylmorpholino, 3-carbamoylmethylmorpholino, 3-methylcarbamoylmethylmorpholino, 3-dimethylcarbamoylmethylmorpholino, 3-carbamoylethylmorpholino, 3-methylcarbamoylethylmorpholino, 3-dimethylcarbamoylethylmorpholino, 3-aminomethylmorpholino, 3-methylaminomethylmorpholino, 3-dimethylaminomethylmorpholino, 3-aminoethylmorpholino, 3-methylaminoethylmorpholino, 3-dimethylaminoethylmorpholino; thiomorpholino, 3-oxothiomorpholino, 1,1-dioxothiomorpholino, 2-methylthiomorpholino, 3-methylthiomorpholino, 2-ethylthiomorpholino, 3-ethylthiomorpholino, 2-cyclopropanespirothiomorpholino, 3-cyclopropanespirothiomorpholino, 2,2-dimethylthiomorpholino, 3,3-dimethylthiomorpholino, 3-hydroxymethylthiomorpholino, 3-methoxymethylthiomorpholino, 3-hydroxyethylthiomorpholino, 3-methoxyethylthiomorpholino, 3-carbamoylthiomorpholino, 3-methylcarbamoylthiomorpholino, 3-dimethylcarbamoylthiomorpholino, 3-carbamoylmethylthiomorpholino, 3-methylcarbamoylmethylthiomorpholino, 3-dimethylcarbamoylmethylthiomorpholino, 3-carbamoylethylthiomorpholino, 3-methylcarbamoylethylthiomorpholino, 3-dimethylcarbamoylethylthiomorpholino, 3-methoxycarbonylthiomorpholino, 3-methoxycarbonylmethylthiomorpholino, 3-ethoxycarbonylmethylthiomorpholino; hexahydropyridazin-1-yl, 2-acetylhexahydropyridazin-1-yl, 2-formylhexahydropyridazin-1-yl, 2-methylhexahydropyridazin-1-yl, 3-oxohexahydropyridazin-1-yl, 6-oxohexahydropyridazin-1-yl, 2,3-dimethylhexahydropyridazin-1-yl, 3-hydroxymethylhexahydropyridazin-1-yl, 5-hydroxymethylhexahydropyridazin-1-yl, 6-hydroxymethylhexahydropyridazin-1-yl, 2-carbamoylhexahydropyridazin-1-yl, 2-methylcarbamoylhexahydropyridazin-1-yl, 2-dimethylcarbamoylhexahydropyridazin-1-yl; 2-oxohexahydropyrimidin-1-yl, 4-oxohexahydropyrimidin-1-yl, 6-oxohexahydropyrimidin-1-yl, 2-methylhexahydropyrimidin-1-yl, 3-methylhexahydropyrimidin-1-yl, 3-carbamoylhexahydropyrimidin-1-yl, 3-methylcarbamoylhexahydropyrimidin-1-yl, 3-dimethylcarbamoylhexahydropyrimidin-1-yl, 6-hydroxymethylpyrimidin-1-yl; 2-oxo-4-methylhomopiperazino, 3-oxo-4-methylhomopiperazino, 5-oxo-4-methylhomopiperazino, 6-oxo-4-methylhomopiperazino, 7-oxo-4-methylhomopiperazino, 2,3-dioxohomopiperazino, 2,7-dioxohomopiperazino, 3,5-dioxohomopiperazino, 3,7-dioxohomopiperazino, 2,3-dioxo-4-methylhomopiperazino, 2,7-dioxo-4-methylhomopiperazino, 3,5-dioxo-4-methylhomopiperazino, 3,7-dioxo-4-methylhomopiperazino, 4-methylhomopiperazino, 4-ethylhomopiperazino, 4-cyclopropylhomopiperazino, 2-cyclopropanespirohomopiperazino, 3-cyclopropanespirohomopiperazino, 5-cyclopropanespirohomopiperazino, 6-cyclopropanespirohomopiperazino, 7-cyclopropanespirohomopiperazino, 2,4-dimethylhomopiperazino, 3,4-dimethylhomopiperazino, 3,9,5-trimethylhomopiperazino, 2-hydroxymethyl-4-methylhomopiperazino, 7-hydroxymethyl-4-methylhomopiperazino, 2-methoxymethyl-4-methylhomopiperazino, 3-methoxymethyl-4-methylhomopiperazino, 5-methoxymethyl-4-methylhomopiperazino, 6-methoxymethyl-4-methylhomopiperazino, 7-methoxymethyl-4-methylhomopiperazino, 2-hydroxyethyl4-methylhomopiperazino, 7-hydroxyethyl-4-methylhomopiperazino, 2-methoxyethyl-4-methylhomopiperazino, 3-methoxyethyl-4-methylhomopiperazino, 5-methoxyethyl-4-methylhomopiperazino, 6-methoxyethyl-4-methylhomopiperazino, 7-methoxyethyl-4-methylhomopiperazino, 2-carbamoyl-4-methylhomopiperazino, 7-carbamoyl-4-methylhomopiperazino, 2-methylcarbamoyl-4-methylhomopiperazino, 7-methylcarbamoyl-4-methylhomopiperazino, 2-dimethylcarbamoylhomopiperazino, 7-dimethylcarbamoylhomopiperazino; 1,4-oxazepan-4-yl; 3-methyl-4-oxoimidazolidin-1-yl.

Of the above-listed compounds, the below-listed ones are more preferred.
Azetidin-1-yl, 3-dimethylaminoazetidin-1-yl, 2,2-dimethyl-3-dimethylaminoazetidin-1-yl, 2-hydroxymethylazetidin-1-yl, 2-carbamoylazetidin-1-yl, 2-methylcarbamoylazetidin-1-yl, 2-dimethylcarbamoylazetidin-1-yl, 3-methoxyazetidin-1-yl; pyrrolidino, 2-oxopyrrolidino, 2,5-dioxopyrrolidino, 2-methylpyrrolidino, 3-methylpyrrolidino, 2,2-dimethylpyrrolidino, 3,3-dimethylpyrrolidino, 2-dimethylaminomethylpyrrolidino, 3-dimethylaminomethylpyrrolidino, 2-hydroxymethylpyrrolidino, 3-methoxymethylpyrrolidino, 2-carbamoylpyrrolidino, 2-methylcarbamoylpyrrolidino, 2-dimethylcarbamoylpyrrolidino, 2-fluoropyrrolidino, 3-fluoropyrrolidino, 2-fluoromethylpyrrolidino; 3-methyl-2-oxoimidazolidin-1-yl, 3-methyl-4-oxoimidazolidin-1-yl; pyrazolidino, 2-formylpyrazolidin-1-yl, 2-carbamoylpyrazolidin-1-yl; piperidino, 2-oxopiperidino, 2-methoxypiperidino, 3-methoxypiperidino, 4-methoxypiperidino, 2-hydroxymethylpiperidino, 2-(1-aminocyclopropyl)piperidino, 2-carbamoylpiperidino, 2-methylcarbamoylpiperidino, 2-dimethylcarbamoylpiperidino, 2-methoxymethylpiperidino, 2-aminomethylpiperidino, 2-methylaminomethylpiperidino, 2-dimethylaminomethylpiperidino, 2-aminoethylpiperidino, 2-methylaminoethylpiperidino, 2-dimethylaminoethylpiperidino, 3-fluoropiperidino, 4-fluoropiperidino, 4-methylpiperidino, 4-methoxypiperidino, 3,3-difluoropiperidino, 4,4-difluoropiperidino, 3-fluoromethylpiperidino, 4-fluoromethylpiperidino, 4-methyl-4-methoxypiperidino, 4,4-difluoro-2-carbamoylpiperidino, 4-methylenepiperidino, 4-(difluoromethylene)piperidino; piperazino, 2-oxo-4-methylpiperazino, 3-oxo-4-methylpiperazino, 3-oxo-4-ethylpiperazino, 4-formylpiperazino, 2,3-dioxo-4-methylpiperazino, 3,5-dioxo-4-methylpiperazino, 2,6-dioxo-4-methylpiperazino, 4-methylpiperazino, 4-ethylpiperazino, 4-isopropylpiperazino, 4-cyclopropylpiperazino, 2,4-dimethylpiperazino, 3,4-dimethylpiperazino, 2-ethyl-4-methylpiperazino, 3-ethyl-4-methylpiperazino, 3,4,5-trimethylpiperazino, 2,2,4-trimethylpiperazino, 3,3,4-trimethylpiperazino, 4,6-dimethyl-3-oxopiperazino, 3,3,4-trimethyl-5-oxopiperazino, 2,2,4-trimethyl-3-oxopiperazino, 2-cyclopropanespiro-4-methylpiperazino, 3-cyclopropanespiro-4-methylpiperazino, 2-cyclopropanespiro-4-methyl-3-oxopiperazino, 3-cyclopropanespiro-4-methyl-5-oxopiperazino, 4-acetyl-3-cyclopropanespiropiperazino, 2-hydroxymethyl-4-methylpiperazino, 3-hydroxymethyl-4-methylpiperazino, 4-methoxypiperazino, 2-methoxymethyl-4-methyl-piperazino, 3-methoxymethyl-4-methylpiperazino, 2-hydroxyethyl-4-methylpiperazino, 3-hydroxyethyl-4-methylpiperazino, 2-methoxyethyl-4-methylpiperazino, 3-methoxyethyl-4-methylpiperazino, 2-carbamoyl-4-methylpiperazino, 2-methylcarbamoyl-4-methylpiperazino, 2-dimethylcarbamoyl-4-methylpiperazino, 2-carbamoylmethyl-4-methylpiperazino, 2-methylcarbamoylmethyl-4-methylpiperazino; 2-dimethylcarbamoylmethyl-4-methylpiperazino, 2-aminomethyl-4-methylpiperazino, 2-methylaminomethyl-4-methylpiperazino, 2-dimethylaminomethyl-4-methylpiperazino, 2-aminoethyl-4-methylpiperazino, 2-methylaminoethyl-4-methylpiperazino, 2-dimethylaminoethyl-4-methylpiperazino; 1,2-dihydropyrazin-3-on-1-yl; morpholino, 4-methyl-1,2-dihydropyrazin-3-on-1-yl; morpholino, 3-methylmorpholino, 2-cyclopropanespiromorpholino, 3-cyclopropanespiromorpholino, 2,2-dimethylmorpholino, 3,3-dimethylmorpholino, 3-hydroxymethylmorpholino, 3-methoxymethylmorpholino, 3-hydroxyethylmorpholino, 3-methoxyethylmorpholino, 3-carbamoylmorpholino, 3-methylcarbamoylmorpholino, 3-dimethylcarbamoylmorpholino, 3-aminomethylmorpholino, 3-methylaminomethylmorpholino, 3-dimethylaminomethylmorpholino, 3-aminoethylmorpholino, 3-methylaminoethylmorpholino, 3-dimethylaminoethylmorpholino; thiomorpholino, 3-oxothiomorpholino, 1,1-dioxothiomorpholino, 3-hydroxymethylthiomorpholino, 3-hydroxyethylthiomorpholino; hexahydropyridazin-1-yl, 2-acetylhexahydropyridazin-1-yl, 2-formylhexahydropyridazin-1-yl, 3-oxohexahydropyridazin-1-yl, 2-methylhexahydropyridazin-1-yl, 2-carbamoylhexahydropyridazin-1-yl; 2-oxohexahydropyrimidin-1-yl, 4-oxohexahydropyrimidin-1-yl, 3-methylhexahydropyrimidin-1-yl, 6-hydroxymethylhexahydropyrimidin-1-yl; 2-oxo-4-methylhomopiperazino, 3-oxo-4-methylhomopiperazino, 5-oxo-4-methylhomopiperazino, 7-oxo-4-methylhomopiperazino, 2,3-dioxo-4-methylhomopiperazino, 2,7-dioxo-4-methylhomopiperazino, 3,5-dioxo-4-methylhomopiperazino, 3,7-dioxo-4-methylhomopiperazino, 4-methylhomopiperazino, 4-ethylhomopiperazino, 4-cyclopropylhomopiperazino, 2-cyclopropanespiro-4-methylhomopiperazino, 3-cyclopropanespiro-4-methylhomopiperazino, 5-cyclopropanespiro-4-methylhomopiperazino, 7-cyclopropanespiro-4-methylhomopiperazino;and 1,4-oxazepan-4-yl; and 3-methyl-4-oxoimidazolidin-1-yl.

Of the above compounds, the below-listed ones are even more preferred.
3-Dimethylaminoazetidin-1-yl, 2,2-dimethyl-3-dimethylaminoazetidin-1-yl, 2-hydroxymethylazetidin-1-yl, 2-carbamoylazetidin-1-yl, 3-methoxyazetidin-1-yl; 2-oxopyrrolidino, 2-hydroxymethylpyrrolidino, 2-carbamoylpyrrolidino, 3-fluoropyrrolidino, 2-fluoromethylpyrrolidino, 2-fluoromethylpyrrolidino; pyrazolidino, 2-formylpyrazolidin-1-yl, 2-carbamoylpyrazolidin-1-yl; piperidino, 2-hydroxymethylpiperidino, 2-(1-aminocyclopropyl)piperidino, 2-carbamoylpiperidino, 2-methylcarbamoylpiperidino, 2-dimethylcarbamoylpiperidino, 3-methoxypiperidino, 3-fluoropiperidino, 4-fluoropiperidino, 4-methylpiperidino, 4-methoxypiperidino, 3,3-difluoropiperidino, 4,4-difluoropiperidino, 4-fluoromethylpiperidino, 4-methyl-4-methoxypiperidino, 4,4-difluoro-2-carbamoylpiperidino, 4-methylenepiperidino, 4-(difluoromethylene)piperidino; 3-oxo-4-methylpiperazino, 3-oxo-4-ethylpiperazino, 4-methylpiperazino, 4-ethylpiperazino, 4-isopropylpiperazino, 4-cyclopropylpiperazino, 2,4-dimethylpiperazino, 3,4-dimethylpiperazino, 3-cyclopropyl-4-methylpiperazino, 3,4,5-trimethylpiperazino, 2,2,4-trimethylpiperazino, 3,3,4-trimethylpiperazino, 4,6-dimethyl-3-oxopiperazino, 3,5-dioxo-4-methylpiperazino, 4-methoxypiperazino, 2-cyclopropanespiro-4-methylpiperazino; morpholino, 3-carbamoylmorpholino, 3-methylmorpholino; 1,1-dioxothiomorpholino, thiomorpholino; 2-methylhexahydropyridazin-1-yl, 3-methylhexahydropyridazin-1-yl; 3-oxo-4-methylhomopiperazino, 5-oxo-4-methylhomopiperazino, 4-methylhomopiperazino, 4-ethylhomopiperazino, 4-cyclopropylhomopiperazino; 1,4-oxazepan-4-yl; piperidino, 4-methoxypiperidino, 4,4-difluoropiperidino, 3,3-difluoropiperidino, 3-fluoropiperidino, 4-fluoropiperidino, 4-fluoromethylpiperidino; 2-dimethylaminomethylpyrrolidino, 3-dimethylaminopyrrolidino, 3-methoxypyrrolidino; 3-methyl-4-oxoimidazolidin-1-yl; hexahydropyridazin-1-yl, 2-acetylhexahydropyridazin-1-yl, and 2-carbamoylhexahydropyridazin-1-yl.

Of the above compounds, the most preferred ones are the following:
3-Dimethylaminoazetidin-1-yl, 3-methoxyazetidin-1-yl; 2-fluoromethylpyrrolidino, 3-fluoropyrrolidino; pyrazolidino, 2-formyl-pyrazolidino; piperidino, 2-(1-aminocyclopropyl)piperidino, 2-carbamoylpiperidino, 2-methylcarbamoylpiperidino, 2-dimethylcarbamoylpiperidino, 3-methoxypiperidino, 3-fluoropiperidino, 4-fluoromethylpiperidino, 4-methoxypiperidino, 4-fluoropiperidino, 4,4-difluoropiperidino, 4-methyl-4-methoxypiperidino, 4-methylenepiperidino, 4-(difluoromethylene)piperidino; 4-cyclopropylpiperazino, 3-oxo-4-methylpiperazino, 3-oxo-4-ethylpiperazino, 4-methylpiperazino, 4,6-dimethyl-3-oxopiperazino, 3,5-dioxo-4-methylpiperazino; 4-methyl-3-oxohomopiperazino; hexahydropyridazin-1-yl, 2-carbamoylhexahydropyridazin-1-yl; and morpholino.

All the compounds (I) of the present invention do not necessarily form salts. However, when a compound (I) has a carboxyl group, an amino group, or a like group, or when Ar₁ or Ar₂ is a pyridine ring or an analogous ring, the compound can form a salt, and in some cases, the salt may form a solvate. Examples of the salt include salts of inorganic acids such as hydrochloric acid, hydrobromic acid, sulfuric acid, and nitric acid; salts of organic acids such as methanesulfonic acid, p-toluenesulfonic acid, fumaric acid, and trifluoroacetic acid; and salts of alkali metal ions or alkaline earth metal ions, such as sodium ion, potassium ion, or calcium ion.

The solvate of a present compound (I) and the solvate of a salt of the present compound (I) encompass not only those formed through addition of a solvent employed in a crystalization step but also those formed through absorption of moisture in air. Examples of the solvent include water; lower alcohols such as methanol and ethanol; and other organic solvents such as acetone and acetonitrile.

The compounds (I) of the present invention may be prepared according to the process described below. A typical production method of the compounds (I) of the present invention is as follows:

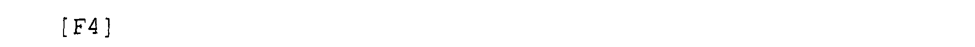

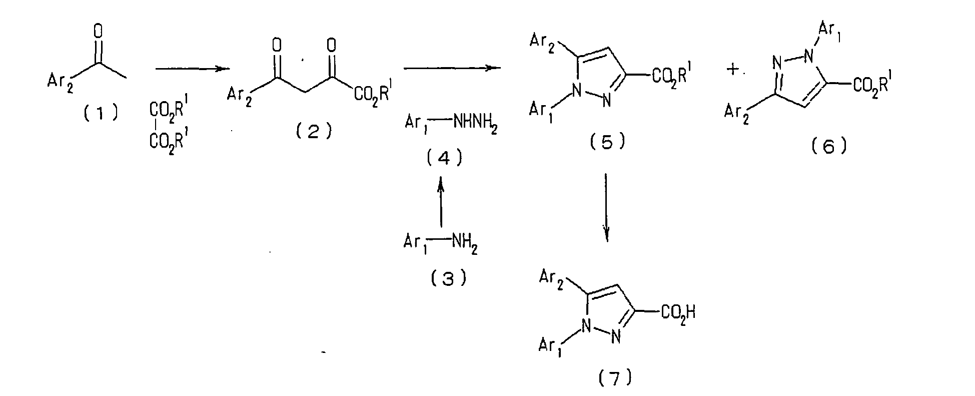

(wherein Ar₁ and Ar₂ have the same meanings as described above, and R¹ represents a methyl group or an ethyl group).
Briefly, an aromatic ketone (1) and oxalic acid dialkyl ester are treated in an alcoholic solvent (e.g., methanol or ethanol) in the presence of a sodium alkoxide (e.g., sodium methoxide or sodium ethoxide), to thereby yield a compound (2). The reaction temperature is preferably -10 to 100°C.
The compound (2) may alternatively be prepared by dissolving or suspending a compound (1) and oxalic acid dialkyl ester in a suitable solvent (such as N,N-dimethylformamide), and then causing reaction with sodium hydride in an argon stream at -20 to 20°C.
Alternatively, the compound (2) may be prepared by dissolving the compound (1) in an inert solvent such as tetrahydrofuran; and under cooling, treated with a base such as lithium bis(trimethylsilyl)amide, and reacted with diethyl oxalate ester. The reaction temperature is preferably -78 to 20°C, more preferably -78°C.
The compound (1) may be a commercial product. Alternatively, the compound (1) may be produced through a method described in the Referential Examples below or similar methods.

When the compound (1) has functional groups such as hydroxyl and amino, preferably, they are protected by suitable protective groups in advance. Exemplary protective groups for hydroxyl include tert-butyl and benzyl; and exemplary protective groups for amino group include trifluoroacetyl, tert-butoxycarbonyl, and benzyloxycarbonyl. These protective groups may be removed under conditions suitable for the respective protective groups.

Subsequently, the compound (2) is dissolved in alcohol (methanol or ethanol). To the resultant solution, a hydrazine derivative (4) or a salt thereof is added at room temperature. After addition of a suitable volume of acetic acid, the mixture is refluxed with heat, to thereby yield a compound (5). At the time of production, a regioisomer (6) is by-produced, but through silica gel column chromatography or a similar step, the target compound (5) can be easily isolated and purified.
The hydrazine derivative (4) or a salt thereof may be produced as follows. An aromatic amine (3) is dissolved in concentrated hydrochloric acid. To the resultant solution, sodium nitrite is added while cooled on ice, to thereby yield a diazo derivative, which is treated with tin(II) chloride. the reaction temperature is preferably -10 to 20°C.
The hydrazine derivative (4) may be a commercial product or may be prepared by a method in which halogenated Ar₁ is reacted with hydrazine (see the Referential Examples) or a similar method. The aromatic amine (3) may be a commercial compound or may be prepared by a method described in the Referential Examples section or a similar method.

In the above-described pyrazole ring formation reaction, acetic acid may be replaced by a suitable amount of triethylamine or concentrated hydrochloric acid, followed by reflux under heat. Alternatively and in some cases, a compound (5) may be obtained without addition of acetic acid, triethylamine, or concentrated hydrochloric acid.

Also, in the pyrazole ring formation reaction, together with a compound (5), a compound of 4,5-dihydro-5-hydroxy-1H-pyrazole form may be produced as an intermediate. In such a case, by dissolving the resultant mixture in a solvent such as methylene chloride, and then adding while stirring methanesulfonyl chloride, triethylamine and 4-dimethylaminopyridine, the intermediate may be transformed into a compound (5).

The above compound (5) may be transformed into an intermediate (7) based on common knowledge in organic chemistry by way of ester hydrolysis using hydrochloric acid, Lewis acid, or a similar acid. Examples of the base include a hydroxide of an alkali metal (such as lithium, sodium, or potassium). Examples of the Lewis acid include boron tribromide. The reaction temperature is preferably -20 to 100°C, more preferably -5 to 50°C
The compound (5) may be transformed into a variety of derivatives through chemical modification based on common knowledge in organic chemistry. For example, derivatives such as alcohols, triflates, and nitriles (5b to d) can be produced from compound (5a):

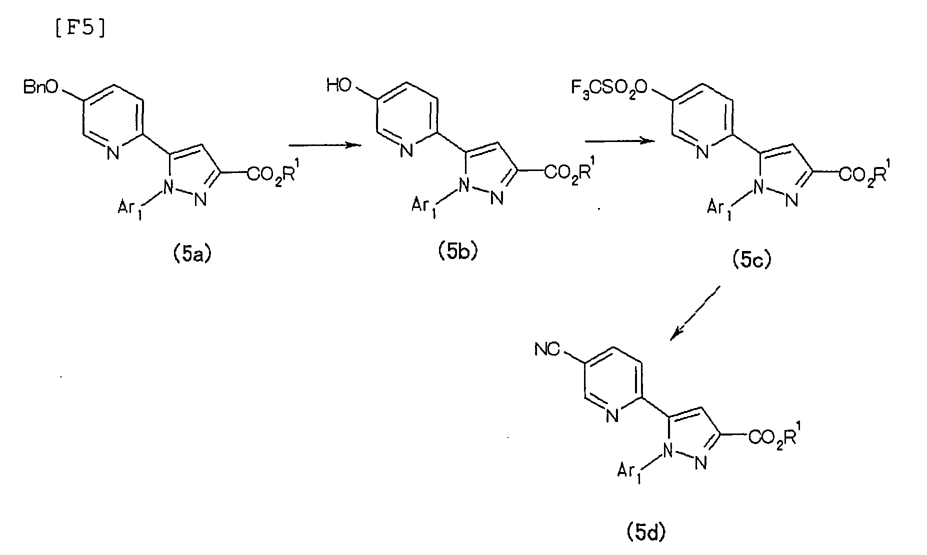

(wherein Ar₁ has the same meaning as defined above, Bn denotes a benzyl group, R¹ denotes a methyl group or an ethyl group).
Specifically, a benzyloxy compound (5a) is dissolved in ethanol or a similar solvent, followed by catalytic hydrogenation using 10% palladium-carbon as a catalyst, to thereby yield a hydroxy compound (5b). The hydroxy compound (5b) is then dissolved in methylene chloride or a similar solvent, and in the presence of a base such as pyridine, the solution is reacted with trifluoromethanesulfonic acid anhydride at -50 to 50°C, to thereby yield a compound (5c). Next, the compound (5c) is dissolved in dichloroethane or a similar solvent, and the resultant solution is reacted with cyanotri(n-butyl)tin and tetrakis(triphenylphosphine)palladium (0), whereby a cyano compound (5d) is produced. The reaction temperature is preferably from 10 to 100°C. The reaction conditions, reagents, etc. are appropriately selected based on common knowledge in organic chemistry.

Moreover, as described below, a compound (5e) may be transformed into different derivatives of carboxylic acid, amide, and amine (5f, 5g, and 5h):

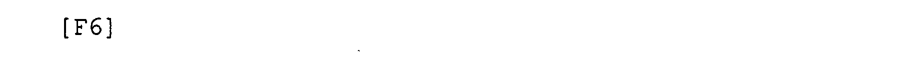

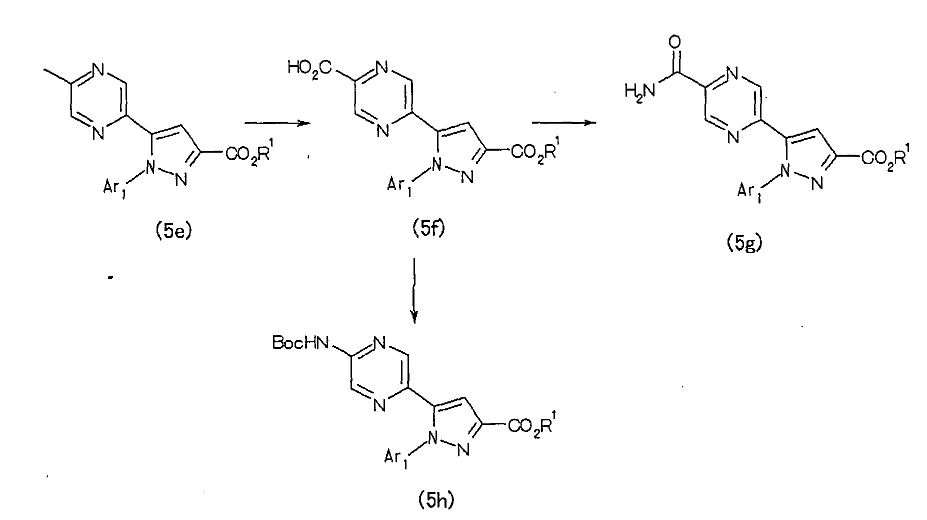

(wherein Ar₁ and R¹ have the same meanings as defined hereinabove and Boc denotes a tert-butoxycarbonyl group).
Specifically, methylpyrazine (5e) is dissolved into pyridine or a similar solvent. Selenium dioxide is added to the resultant solution at room temperature, and the mixture is refluxed under heat, to thereby yield carboxylic acid (5f). The carboxylic acid (5f) is subjected to fusion reaction with, for example, aqueous ammonia, ammonium chloride, and an approapriate fusion agent, whereby an amide (5 g) can be prepared. An amine (5h) is prepared through the following process. That is, carboxylic acid (5f) is dissolved in, for example, dioxane. At room temperature, tert-butanol, triethylamine, and diphenylphosphorylazide are added, and the resultant mixture is refluxed under heat. The reaction conditions, reagents, etc. are appropriately selected based on common knowledge in organic chemistry.
The compound (5) can be transformed into an intermediate (7) through ester hydrolysis as described above.

Fusion reaction between the intermediate (7) and amine (8) provides compound (I) of the present invention:

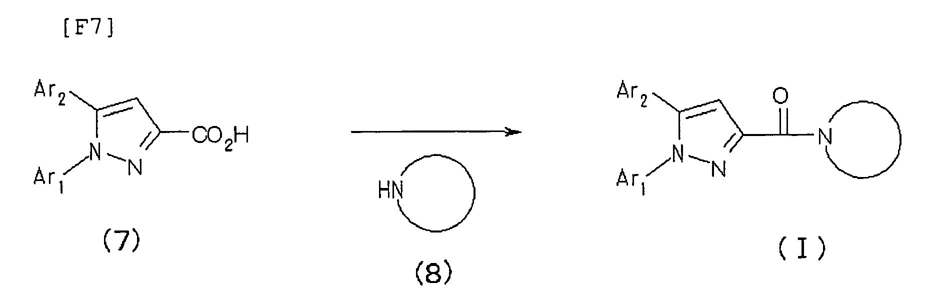

(wherein Ar₁ and Ar₂ have the same meanings as described above).
The fusion process described above may be performed through a method generally used for peptide synthesis. Examples of peptide synthesis methods include the azide method, the acid chloride method, the acid anhydride method, the DCC (dicyclohexylcarbodiimide) method, the active ester method, the carbonyldiimidazole method, the DCC/HOBT(1-hydroxybenzotriazole) method, a method using water-soluble carbodiimide, and a method using diethyl cyanophosphate. These methods are described in, for example, M. Bodanszky, Y. S. Klausner, and M.A. Ondetti, "Peptide Synthesis," A Wiley-interscience publication, New York, 1976; G. R. Pettit, "Synthetic Peptides," Elsevier Scientific Publication Company, New York, 1976; and "Lectures on Experimental Chemistry 4th ed., vol. 22, Organic Synthesis IV," edited by the Japanese Society of Chemistry, Maruzen Publishing, 1992. Examples of a solvent used in the fusion reaction include N,N-dimethylformamide, pyridine, chloroform, methylene chloride, tetrahydrofuran, dioxane, acetonitrile, and a solvent mixture thereof. The reaction temperature is preferably -20 to 50°C, more preferably -10 to 30°C. The amine compound (8) may be a commercial product or may be produced through a method described in literature or in the Referential Examples section herein or a similar method.

In the above fusion reaction, when the amine compound (8) has a functional group such as a hydroxyl group, an amino group, or a carboxyl group, the functional group is preferably protected with a suitable protective group in advance. When the functional group is a hydroxyl group, the amine compound (8) may be transformed to a tert-butyl compound or a benzyl compound prior to the fusion reaction; when the functional group is an amino group, before fusion reaction, a trifluoroacetyl compound, a tert-butoxycarbonyl compound, or a benzyloxycarbonyl group is formed; and when the functional group is a carboxyl group, before fusion reaction, a methyl ester or a tert-butyl ester is formed.

The thus-produced compound (I) of the present invention may be subjected to a further chemical modification reaction based on common knowledge of organic chemistry, to thereby transform derivatives of compound (I). For example, starting from compound (Ia), derivatives such as an alcohol, triflate, nitrile, and amide (Ib to Id) can be produced, and a methoxy derivative (If) can be produced from alcohol (Ib):

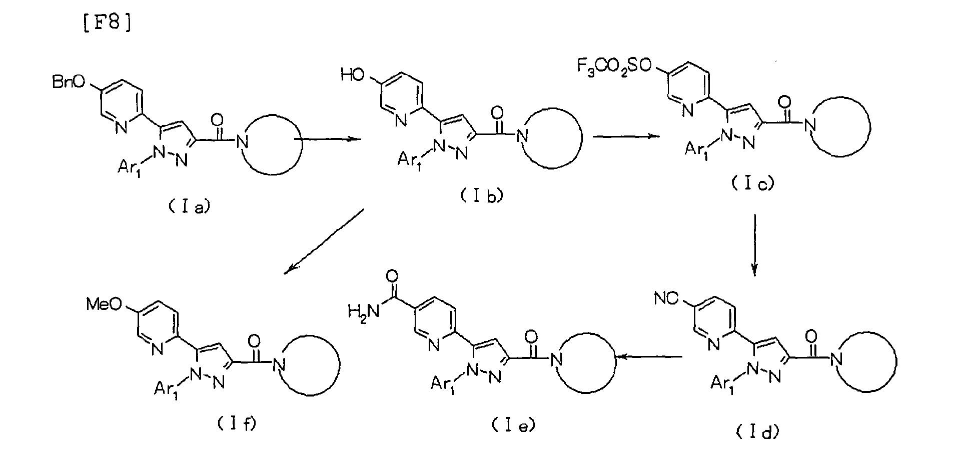

(wherein Ar₁ 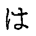 has the same meanings as defined hereinabove, and Bn denotes a benzyl group).
Specifically, a compound (Ia) is dissolved in ethanol or a similar solvent, followed by catalytic hydrogenation using 10% palladium-carbon as a catalyst, to thereby yield an alcohol (Ib). The alcohol (Ib) is then dissolved in methylene chloride or a similar solvent, and in the presence of a base such as pyridine, the solution is reacted with trifluoromethanesulfonic acid anhydride at -50 to 50°C, to thereby yield a compound (Ic). Next, the compound (Ic) is dissolved in dichloroethane or a similar solvent. To the resultant solution is added, under stirring, cyanotri(n-butyl)tin and tetrakis(triphenylphosphine)palladium (0), whereby a nitrile compound (Id) is produced. The reaction temperature is preferably from 10 to 100°C. When the nitrile (Id) is dissolved in a solvent such as methanol or tetrahydrofuran, and then hydrolyzed with sodium hydroxide, an amide (Ie) can be produced. The reaction temperature is preferably from 0 to 100°C. Alternatively, the nitrile (Id) may be transformed into a carboxylic acid, followed by a fusion reaction using, for example, aqueous ammonia, ammonium chloride, and a suitable fusion agent, whereby an amide (Ie) can be produced.

When alcohol (Ib) is dissolved in a solvent mixture of methanol, tetrahydrofuran, methylene chloride, etc., and to the resultant solution is added at room temperature and under stirring a hexane solution of (trimethylsilyl)diazomethane, a methoxy compound (If) can be produced. The reaction temeperature is preferably 0 to 50°C. When protection of the functional groups is needed, conditions under which protection or deprotection is performed appropriately determined based on common knowledge in organic chemistry.

Each of the above-described compounds (Ia to If) of the present invention may be transformed to another derivative falling within the scope of the present invention through chemical modification based on common knowledge in organic chemistry.

The compounds (I) of the present invention, salts thereof, and solvates of the compounds or salts are endowed with potent anti-platelet aggregation activity, and they strongly inhibited thrombus formation in a high shear stress-induced thrombosis model. Therefore, the compounds (I) of the present invention, salts thereof, and solvates of the compounds or salts are useful in humans and other mammals as preventive and/or therapeutic agents for ischemic diseases caused by thrombus or embolus such as myocardial infarction, angina pectoris (chronic stable angina, unstable angina, etc.), ischemic cerebrovascular disorder (transient ischemic attack (TIA), cerebral infarction, etc.), peripheral vascular disease, embolism after replacement with an artificial vessel, thrombotic embolism after coronary artery intervention (coronary artery bypass grafting (CABG), percutaneous transluminal coronary angioplasty (PTCA), stent placement, etc.), diabetic retinopathy and nephropathy, and embolism after replacement with an artificial heart valve, and also as a preventive and/or therapeutic agent against formation of thrombi and emboli associated with vascular operation, blood extracorporeal circulation, and the like. Moreover, the compounds (I) of the present invention, salts thereof, and solvates of the compounds or the salts are useful for ameliorating ischemic conditions such as ulcer, pain, and chill, which accompany chronic arteriosclerosis obliterans.

When any of the compounds (I) of the present invention, salts thereof, and solvates of the compounds or the salts is used as a drug, the daily dose for an adult, which varies depending on the age, sex, symptoms of the patient, etc., is preferably 0.1 mg to 1 g, more preferably 0.5 mg to 500 mg. The daily dose may be divided and administered several times a day. If necessary, the compound/salt/solvate may be administered at a dose exceeding the above daily dose.

No particular limitation is imposed on the administration route and the dosage form of a drug containing a compound (I) of the present invention, a salt of the compound, or a solvate of the compound or the salt, and the drug may be administered via any route and in any dosage form as desired. The dosage form may be determined appropriately depending on the administration route. The drug may be produced through a conventional drug preparation method by incorporating a pharmacologically acceptable carrier as desired.
Examples of oral preparations include solid preparations such as tablets, powders, granules, pills, and capsules, as well as liquid preparations such as solution, syrup, elixir, suspension, and emulsion.
An injection may be prepared by filling a container with a solution of a compound (I), a salt of the compound, or a solvate of the compound or the salt. A solid prepared by, for example, freeze-drying such a solution may also be used as an injection which is restituted before use.
In manufacture of any of the above drug preparations, one or more pharmaceutically acceptable additives selected, in accordance with needs, from among a binder, a disintegrant, a dissolution promoter, a lubricant, a filler, an excipient, and similar additives may be incorporated therein.

### [Examples]

The present invention will next be described in more detail by way of examples, which should not be construed as limiting the invention thereto.

[Referential Example 1] 3-Hydrazinopyridine

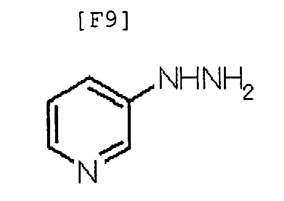

At 5°C or lower, sodium nitrite (10.5 g) in water (39 mL) was added dropwise to 3-aminopyridine (13.0 g) in concentrated hydrochloric acid (104 mL) over 30 minutes, followed by stirring for 15 minutes. The reaction mixture was added dropwise to tin(II) chloride dihydrate (109 g) in concentrated hydrochloric acid (59 mL) at 5°C or lower over 30 minutes, followed by stirring for 1 hour. Under the same temperature conditions, 6N aqueous sodium hydroxide (796 mL) was added dropwise to the reaction mixture, and then a solvent mixture of methanol-chloroform (1:10) was added for partitioning the reaction mixture. The solvent of the organic layer was evaporated under reduced pressure, to thereby give the title compound as a solid product (12.5 g, 83%).

¹H-NMR(400MHz,DMSO-d₆)δ:4.02(2H,br s), 6.89(1H,br s), 7.04-7.12(2H,m), 7.76-7.78(1H,m), 8.08 (1H,m).
EI-MS m/z:109(M⁺).

[Referential Example 2] 2-Hydrazinopyrazine

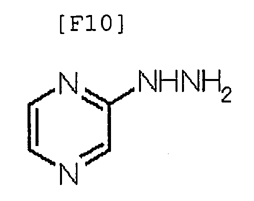

Hydrazine monohydrate (21.80 g) was added to 2-chloropyrazine (10.44 g) in ethanol (65 mL) at room temperature, and the resultant mixture was refluxed for 17 hours, followed by cooling in air. The reaction solvent was evaporated under reduced pressure, and then benzene was added to the residue. The resultant mixture was subjected to decantation, to thereby remove an insoluble matter. The benzene was evaporated under reduced pressure. Hexane was added to the resultant solid, and the mixture was subjected to filtration, to thereby give the title compound (4.67 g, 47%).

¹H-NMR(400MHz,CDCl₃)δ:7.89(1H,d,J=2.7Hz), 7.99-8.05(1H,m), 8.20(1H,d,J=1.5Hz).
ESI-MS m/z:111(M+H)⁺.

[Referential Example 3] 2-Hydrazinopyrimidine

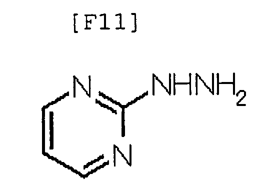

Hydrazine monohydrate (20 mL) was added to a suspension of 2-chloropyrimidine (6.00 g) in ethanol (60 mL) at room temperature, followed by stirring for 80 minutes. The solvent of reaction mixture was evaporated under reduced pressure, and then water (34 mL) was added to the residue. The solid that precipitated was collected through filtration, to thereby give the title compound (2.30 g, 40%).

¹H-NMR(400MHz,DMSO-d₆)δ:4.12(2H,s), 6.57-6.60 (1H,m), 8.12(1H,s), 8.30(2H,d,J=4.9Hz). EI-MS m/z:110(M⁺).

[Referential Example 4] 5-(5-Benzyloxy-2-pyridyl)-1-(3-pyridyl)-1H-pyrazole-3-carboxylic acid

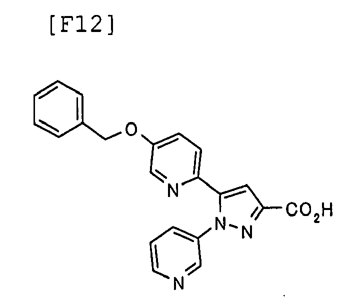

### 1) 5-(5-Benzyloxy-2-pyridyl)-1-(3-pyridyl)-1H-pyrazole-3-carboxylic acid ethyl ester

Acetic acid (6.65 mL) was added at room temperature to a suspension of 4-(5-benzyloxy-2-pyridyl)-2,4-dioxobutanoic acid ethyl ester (7.61 g) and 3-hydrazinopyridine (3.04 g) obtained in Referential Example 1 in ethanol (152 mL). The resultant mixture was refluxed for 64 hours, followed by cooling in air. The reaction mixture was partitioned between saturated aqueous sodium hydrogencarbonate and chloroform. The organic layer was dried over sodium sulfate anhydrate. After a filtration step, the solvent was evaporated under reduced pressure, and the residue was purified by silica gel column chromatography (ethyl acetate - chloroform), to thereby give 5-(5-benzyloxy-2-pyridyl)-1-(3-pyridyl)-1H-pyrazole-3-carboxylic acid ethyl ester as a solid product (7.38 g, 79%).

¹H-NMR(400MHz,CDCl₃)δ:2.48(3H,s), 5.08(2H,s), 7.05(1H,d,J=8.5Hz), 7.16(1H,dd,J=8.5,2.9Hz), 7.31-7.43(5H,m),
8.26(1H,d,J=2.9Hz).
EI-MS m/z:199(M⁺).

### 2) 1-(5-Benzyloxy-2-pyridyl)ethanone

Selenium dioxide (9.20 g) was added at room temperature to 5-benzyloxy-2-methylpyridine (4.13 g) in pyridine (83 mL). The resultant mixture was refluxed for 61 hours, followed by cooling in air. The reaction mixture was partitioned between water and chloroform. The organic layer was dried over magnesium sulfate anhydrate. After a filtration step, the solvent was evaporated under reduced pressure. Triethylamine (6.35 mL) was added at room temperature to a mixture of the resultant residue, N,O-dimethylhydroxylamine hydrochloride (2.22 g), 1-(3-dimethylaminopropyl)-3-ethylcarbodiimide hydrochloride (4.37 g), and 1-hydroxybenzotriazole (3.08 g) in N,N-dimethylformamide (95 mL), followed by stirring for 61 hours. The reaction mixture was partitioned between water and ethyl acetate. The organic layer was dried over sodium sulfate anhydrate. After a filtration step, the solvent was evaporated under reduced pressure, and the residue was purified by silica gel column chromatography (ethyl acetate - hexane), to thereby give 5-benzyloxypyridine-2-carboxylic acid N-methoxy-N-methylamide as an oily product (3.75 g, 66%).
(FAB-MS m/z:273(M+H)⁺.)
In an argon atmosphere at 0°C, methyllithium (1.10M diethyl ether solution, 13.7 mL) was added dropwise to 5-benzyloxypyridine-2-carboxylic acid N-methoxy-N-methylamide (3.74 g) in tetrahydrofuran (75 mL), followed by stirring for 40 minutes. The reaction mixture was partitioned between water and ethyl acetate. The organic layer was dried over sodium sulfate anhydrate. After a filtration step, the solvent was evaporated under reduced pressure, and the residue was purified by silica gel column chromatography (ethyl acetate - hexane), to thereby give 1-(5-benzyloxy-2-pyridyl)ethanone as an oily product (1.47 g, 47%).

¹H-NMR(400MHz,CDCl₃)δ:2.67(3H,s), 5.18(2H,s), 7.30-7.45(6H,m),
8.03(1H,d,J=8.8Hz), 8.39(1H,d,J=2.7Hz).
EI-MS m/z:227(M⁺).

### 3) 4-(5-Benzyloxy-2-pyridyl)-2,4-dioxobutanoic acid ethyl ester

In an argon atmosphere, diethyl oxalate (1.75 mL) and the above-obtained 1-(5-benzyloxy-2-pyridyl)ethanone (1.46 g) in ethanol (15 mL) was added to sodium ethoxide (0.874 g) in ethanol (15 mL), followed by stirring for 7 hours at room temperature. Subsequently, the resultant mixture was stirred for 1 hour at 60°C, followed by cooling in air. Sodium ethoxide (0.874 g) and diethyl oxalate (1.75 mL) were added to the reaction mixture. The mixture was stirred at 60°C for 1 hour, and then was cooled in air. Subsequently, water was added to the reaction mixture, and then was washed with diethyl ether. The aqueous layer was partitioned between a saturated aqueous solution of ammonium chloride and chloroform. The organic layer was dried over sodium sulfate anhydrate. After a filtration step, the solvent was evaporated under reduced pressure, to thereby give 4-(5-benzyloxy-2-pyridyl)-2,4-dioxobutanoic acid ethyl ester as a solid product (1.38 g, 66%).

¹H-NMR(400MHz,CDCl₃)δ:1.38-1.42(3H,m), 4.35-4.42(2H,m), 5.20(2H,s), 7.35-7.44(6H,m), 7.59(1H,s), 8.14(1H,d,J=8.8Hz),
8.44(1H,d,J=2.7Hz).
EI-MS m/z:327(M⁺).

### 4) 5-(5-Benzyloxy-2-pyridyl)-1-(3-pyridyl)-1H-pyrazole-3-carboxylic acid ethyl ester

Acetic acid (6.65 mL) was added at room temperature to a suspension of the above-obtained 4-(5-benzyloxy-2-pyridyl)-2,4-dioxobutanoic acid ethyl ester (7.61 g) and 3-hydrazinopyridine (3.04 g) obtained in Referential Example 1 in ethanol (152 mL). The resultant mixture was refluxed for 64 hours, followed by cooling in air. The reaction mixture was partitioned between a saturated aqueous solution of sodium hydrogencarbonate and chloroform. The organic layer was dried over sodium sulfate anhydrate. After a filtration step, the solvent was evaporated under reduced pressure, and the residue was purified by silica gel column chromatography (ethyl acetate - chloroform), to thereby give 5-(5-benzyloxy-2-pyridyl)-1-(3-pyridyl)-1H-pyrazole-3-carboxylic acid ethyl ester as a solid product (7.38 g, 79%).

¹H-NMR(400MHz,CDCl₃)δ:1.41-1.44(3H,m), 4.93-4.49(2H,m), 5.10(2H,s), 7.20(1H,s), 7.25(1H,dd,J=8.8,2.9Hz), 7.35-7.41(7H,m), 7.82-7.85(1H,m), 8.23(1H,d,J=2.9Hz), 8.52(1H,m),
8.59(1H,dd,J=4.9,1.5Hz).
FAB-MS m/z:401(M+H)⁺.

### 5) Title Compound

1N Aqueous sodium hydroxide (32.2 mL) was added at room temperature to the above-obtained 5-(5-benzyloxy-2-pyridyl)-1-(3-pyridyl)-1H-pyrazole-3-carboxylic acid ethyl ester (5.16 g) in a mixture of methanol (50 mL) and tetrahydrofuran (50 mL), followed by stirring for 160 minutes. The reaction solvent was evaporated under reduced pressure. The residue was dissolved in chloroform and water. The resultant mixture was neutralized with 1N aqueous hydrochloric acid (33 mL). The reaction mixture was partitioned between water and a solvent mixture of methanol-chloroform (1:5). The organic layer was evaporated under reduced pressure, to thereby give the title compound as a solid product (4.61 g, 96%).

¹H-NMR(400MHz,DMSO-d₆)δ:5.19(2H,s), 7.27-7.28(1H,m), 7.35-7.54(7H,m), 7.70(1H,d,J=8.8Hz), 7.78-7.81(1H,m), 8.19(1H,d,J=2.9Hz), 8.51(1H,d,J=2.4Hz), 8.60-8.61(1H,m), 13.10(1H,s).
FAB-MS m/z:373(M+H)⁺.

[Referential Example 5] 5-(5-Chloro-2-pyridyl)-1-(3-pyridyl)-1H-pyrazole-3-carboxylic acid

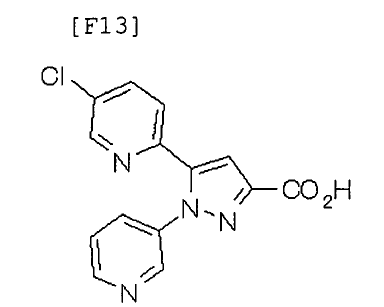

### 1) 2-Bromo-5-chloropyridine

At 0°C, bromine (12 mL) was added to 2-amino-5-chloropyridine (5 g) in 47% hydrobromic acid (50 mL), and then sodium nitrite (15 g) in water (20 mL) was added dropwise to the reaction mixture, followed by stirring for 1 hour. The reaction mixture was partitioned between sodium hydroxide (32 g) in water (80 mL) and ethyl acetate. The organic layer was dried over sodium sulfate anhydrate. After a filtration step, the solvent was evaporated under reduced pressure, to thereby give 2-bromo-5-chloropyridine as a solid product (6.8 g, 91%).

¹H-NMR(400MHz,CDCl₃)δ:7.44(1H,d,J=8.42Hz), 7.54(1H,m), 8.36(1H,s).

### 2) 1-(5-Chloro-2-pyridyl)ethanone

At -78°C, n-butyllithium (1.56M hexane solution, 27 mL) was added dropwise to 2-bromo-5-chloropyridine (6.8 g) in diethyl ether (45 mL), and then N,N-dimethylacetamide (5 mL) was added dropwise to the resultant mixture, followed by stirring for 30 minutes. Subsequently, a saturated aqueous solution of ammonium chloride was added to the reaction mixture. Ethyl acetate was added for partitioning the resultant mixture. The organic layer was dried over sodium sulfate anhydrate. After a filtration step, the solvent was evaporated under reduced pressure and the residue was purified by silica gel column chromatography (hexane - ethyl acetate), to thereby give 1-(5-chloro-2-pyridyl)ethanone as a solid product (3.26 g, 59%).

¹H-NMR(400MHz,CDCl₃)δ:2.70(3H,s), 7.80(1H,dd,J=8.42,2.32Hz), 8.00(1H,d,J=8.42Hz), 8.62(1H,d,J=2.32Hz).

### 3) 4-(5-Chloro-2-pyridyl)-2,4-dioxobutanoic acid ethyl ester

Diethyl oxalate (1.75 mL) was added to sodium ethoxide (0.88 g) in ethanol (30 mL), followed by stirring for 5 minutes. Subsequently, 1-(5-chloro-2-pyridyl)ethanone (1.00 g) was added to the resultant mixture, followed by stirring at room temperature for 1 hour. Water was added to the reaction mixture, and then the resultant mixture was washed with diethyl ether. The aqueous layer was acidified with 1N aqueous solution of hydrochloric acid. Subsequently, chloroform was added for partitioning the resultant mixture. The organic layer was dried over magnesium sulfate anhydrate. After a filtration step, the solvent was evaporated under reduced pressure, to thereby give 4-(5-chloro-2-pyridyl)-2,4-dioxobutanoic acid ethyl ester as a solid product (2.59 g, quantitative amount).

¹H-NMR(400MHz,CDCl₃)δ:1.42(3H,t,J=7.08Hz), 4.41(2H,q,J=7.08Hz), 7.27(1H,s), 7.86(1H,dd,J=8.42,2.44Hz), 8.11(1H,d,J=8.42Hz), 8.67(1H,d,J=2.44Hz).
ESI-MS m/z:256(M+H)⁺.

### 4) 5-(5-Chloro-2-pyridyl)-1-(3-pyridyl)-1H-pyrazole-3-carboxylic acid ethyl ester

Acetic acid (5 mL) was added to the above-obtained 4-(5-chloro-2-pyridyl)-2,4-dioxobutanoic acid ethyl ester (2.59 g) and 3-hydrazinopyridine (1.2 g) obtained in Referential Example 1 in ethanol (100 mL). The resultant mixture was refluxed for 16.5 hours, followed by cooling in air. The reaction mixture was partitioned between water and ethyl acetate. The organic layer was washed with saturated brine, and then was dried sodium sulfate anhydrate. After a filtration step, the solvent was evaporated under reduced pressure, and the residue was purified by silica gel column chromatography (methanol - chloroform), to thereby give 5-(5-chloro-2-pyridyl)-1-(3-pyridyl)-1H-pyrazole-3-carboxylic acid ethyl ester (1.5 g).

¹H-NMR(400MHz,CDCl₃)δ:1.43(3H,t,J=7.08Hz), 4.46(2H,q,J=7.08Hz), 7.29(1H,s), 7.39(1H,dd,J=8.30,4.88Hz), 7.42(1H,d,J=8.30Hz), 7.71(1H,dd,J=8.42,2.44Hz), 7.83(1H, ddd,J=8.42,2.44,1.59Hz), 8.91(1H,d,J=1.59Hz), 8.54(1H,d,J=2.44Hz), 8.62(1H,dd,J=4.88,1.59Hz).
ESI-MS m/z:329(M+H)⁺.

### 5) Title Compound

Sodium methoxide (573 mg) was added to the above-obtained 5-(5-chloro-2-pyridyl)-1-(3-pyridyl)-1H-pyrazole-3-carboxylic acid ethyl ester (500 mg) in methanol (10 mL), followed by stirring at room temperature for 17.5 hours. The reaction mixture was partitioned between 1N aqueous hydrochloric acid and chloroform. The organic layer was dried over sodium sulfate anhydrate. After a filtration step, the solvent was evaporated under reduced pressure, to thereby give the title compound as a solid product (348 mg, 76%).

¹H-NMR(400MHz,CD₃OD/CDCl₃)δ: 7.30(1H,s), 7.33(1H,s), 7.56(1H,m), 7.84(2H,m), 8.38(1H,m), 8.57(2H,m).
ESI-MS m/z:301(M+H)⁺.

[Referential Example 6] 5-(5-Methyl-2-pyrazinyl)-1-(3-pyridyl)-1H-pyrazole-3-carboxylic acid

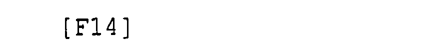

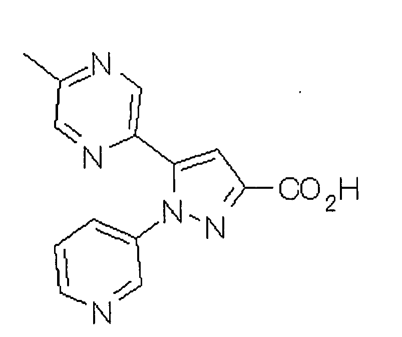

### 1) 5-Methylpyrazine-2-carboxylic acid N-methoxy-N-methylamide

Triethylamine (28.9 mL) was added at room temperature to 5-methylpyrazine-2-carboxylic acid (13.0 g), N,O-dimethylhydroxylamine hydrochloride (10.1 g), 1-(3-dimethylaminopropyl)-3-ethylcarbodiimide hydrochloride (19.8 g), and 1-hydroxybenzotriazole (14.0 g) in N,N-dimethylformamide (130 mL), followed by stirring for 63 hours. The reaction mixture was partitioned between water and ethyl acetate. The organic layer was dried over sodium sulfate anhydrate. After a filtration step, the solvent was evaporated under reduced pressure, and the residue was purified by silica gel column chromatography (hexane - ethyl acetate), to thereby give 5-methylpyrazine-2-carboxylic acid N-methoxy-N-methylamide as a solid product (12.3 g, 72%).

¹H-NMR(400MHz,CDCl₃)δ:2.63(3H,s), 3.41(3H,s), 3.74(3H,s), 8.46(1H,s), 8.82(1H,s).
FAB-MS m/z:182(M+H)⁺.

### 2) 1-(5-Methyl-2-pyrazinyl)ethanone

In an argon atmosphere, methyllithium (1.02M diethyl ether solution, 72.6 mL) was added dropwise to the above-obtained 5-methylpyrazine-2-carboxylic acid N-methoxy-N-methylamide (12.2 g) in tetrahydrofuran (183 mL) at -78°C over 20 minutes, followed by stirring for 130 minutes. At 0°C, the reaction mixture was partitioned between water and ethyl acetate, the organic layer was dried over sodium sulfate anhydrate. After a filtration step, the solvent was evaporated under reduced pressure, and the residue was purified by silica gel column chromatography (ethyl acetate - hexane), to thereby give 1-(5-methyl-2-pyrazinyl)ethanone as an oily product (7.9 g, 86%).

¹H-NMR(400MHz,CDCl₃)δ:2.66(3H,s), 2.70(3H,s), 8.50(1H,m), 9.11(1H,d,J=1.5Hz).
ESI-MS m/z:137(M+H)⁺.

### 3) 4-(5-Methyl-2-pyrazinyl)-2,4-dioxobutanoic acid ethyl ester

In an argon atmosphere, lithium bis(trimethylsilyl)amide (1.0M tetrahydrofuran solution, 63.7 mL) was added dropwise to 1-(5-methyl-2-pyrazinyl)ethanone (7.89 g) in tetrahydrofuran (118 mL) at -78°C over 20 minutes, followed by stirring for 30 minutes. Diethyl oxalate (11.8 mL) was added dropwise to the reaction mixture, followed by stirring for 10 minutes. Subsequently, the resultant mixture was stirred at 0°C for 30 minutes, and then at room temperature for 1.5 hours. The reaction mixture was partitioned between water and diethyl ether. The aqueous layer was partitioned between a saturated aqueous solution of ammonium chloride and chloroform. The organic layer was dried over sodium sulfate anhydrate. After a filtration step, the solvent was evaporated under reduced pressure, to thereby give 4-(5-methyl-2-pyrazinyl)-2,4-dioxobutanoic acid ethyl ester as a solid product (4.92 g, 36%).

¹H-NMR(400MHz,CDCl₃)δ:1.39-1.43(3H,m), 2.69(3H,s), 4.38-4.43(2H,m), 7.60(1H,s), 8.55(1H,m), 9.21(1H,d,J=1.2Hz).
FAB-MS m/z:237(M+H)⁺.

### 4) 5-(5-Methyl-2-pyrazinyl)-1-(3-pyridyl)-1H-pyrazole-3-carboxylic acid ethyl ester

The above-obtained 4-(5-methyl-2-pyrazinyl)-2,4-dioxobutanoic acid ethyl ester (4.91 g) and 3-hydrazinopyridine (2.27 g) obtained in Referential Example 1 in ethanol (98 mL) was refluxed for 40 minutes. Acetic acid (5.95 mL) was added to the reaction mixture. The resultant mixture was refluxed for 14 hours, followed by cooling in air. The reaction mixture was partitioned between a saturated aqueous solution of sodium hydrogencarbonate and chloroform. The organic layer was dried over sodium sulfate anhydrate. After a filtration step, the solvent was evaporated under reduced pressure, and the residue was dissolved in ethanol (99 mL). Concentrated hydrochloric acid (3.3 mL) was added to the reaction mixture. The resultant mixture was refluxed for 1 hour, followed by cooling in air. The reaction mixture was partitioned between a saturated aqueous solution of sodium hydrogencarbonate and chloroform. The organic layer was dried over sodium sulfate anhydrate. After a filtration step, the solvent was evaporated under reduced pressure, and the residue was purified by silica gel column chromatography (acetone - toluene), to thereby give 5-(5-methyl-2-pyrazinyl)-1-(3-pyridyl)-1H-pyrazole-3-carboxylic acid ethyl ester as a solid product (3.16 g, 49%).

¹H-NMR(400MHz,CDCl₃)δ:1.42-1.46(3H,m), 2.58(3H,s), 4.45-4.51(2H,m), 7.34(1H,s), 7.38-7.41(1H,m), 7.83-7.86(1H,m), 8.30-8.31(1H,m), 8.54(1H,d,J=2.4Hz), 8.63(1H,d,J=1.5Hz), 8.64(1H,d,J=1.5Hz).
FAB-MS m/z:310(M+H)⁺.

### 5) Title Compound

In a manner similar to that employed in step 5) of Referential Example 4, the title compound in solid form (0.525 g, 96%) was prepared from 5-(5-methyl-2-pyrazinyl)-1-(3-pyridyl)-1H-pyrazole-3-carboxylic acid ethyl ester (0.60 g) and 1N sodium hydroxide (4.85 mL).

1H-NMR(400MHz,DMSO-d₆)δ:2.50(3H,s), 7.50-7.53(2H,m), 7.84-7.87 (1H; m), 8.36(1H,s 8.58 (1H, d, J=2.4Hz), 8.62-8.64(1H,m), 8.92 (1H, d, J=1.2Hz), 13.17(1H,br s).
FAB-MS m/z:282(M+H)⁺.

[Referential Example 7] 5-(5-Carbamoyl-2-pyrazinyl)-1-(3-pyridyl)-1H-pyrazole-3-carboxylic acid

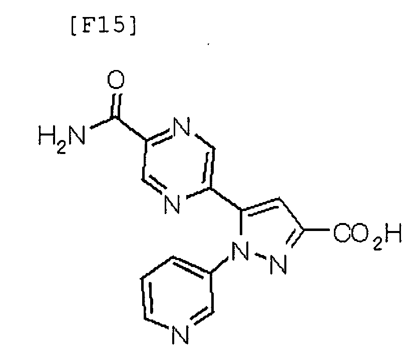

### 1) 5-(5-Carboxy-2-pyrazinyl)-1-(3-pyridyl)-1H-pyrazole-3-carboxylic acid ethyl ester

Selenium dioxide (3.66 g) was added at room temperature to 5-(5-methyl-2-pyrazinyl)-1-(3-pyridyl)-1H-pyrazole-3-carboxylic acid ethyl ester (2.55 g) obtained in step 4) of Referential Example 6 in pyridine (51 mL). The resultant mixture was refluxed for 67 hours, followed by cooling in air. The reaction mixture was partitioned between water and chloroform. The organic layer was dried over sodium sulfate anhydrate. After a filtration step, the solvent was evaporated under reduced pressure, and the resultant solid was washed with diethyl ether, to thereby give 5-(5-carboxy-2-pyrazinyl)-1-(3-pyridyl)-1H-pyrazole-3-carboxylic acid ethyl ester (2.51 g, 90%).

¹H-NMR(400MHz,DMSO-d₆)δ:1.34-1.37(3H,m), 4.36-4.41(2H,m), 7.54(1H,dd,J=8.2,4.8Hz), 7.84(1H,s), 7.92-7.94(1H,m), 8.65-8.68(2H,m), 8.90-8.91(1H,m), 9.24-9.25(1H,m).
FAB-MS m/z:390(M+H)⁺.

### 2) 5-(5-Carbamoyl-2-pyrazinyl)-1-(3-pyridyl)-1H-pyrazole-3-carboxylic acid ethyl ester

In a manner similar to that employed in step 1) of Referential Example 6 in an argon atmosphere, 5-(5-carbamoyl-2-pyrazinyl)-1-(3-pyridyl)-1H-pyrazole-3-carboxylic acid ethyl ester in solid form (0.540 g, 32%) was prepared from the above-obtained 5-(5-carboxy-2-pyrazinyl)-1-(3-pyridyl)-1H-pyrazole-3-carboxylic acid ethyl ester (1.70 g) and ammonium chloride (2.68 g).

¹H-NMR(400MHz,DMSO-d₆)δ:1.35(3H,t,J=7.1Hz), 4.38(2H,q,J=7.1Hz), 7.53(1H,dd,J=8.3,4.9Hz), 7.82(1H,s), 7.88(1H,br s), 7.90-7.93(1H,m), 8.28(1H,br s), 8.64(1H,d,J=2.4Hz), 8.67(1H,dd,J=4.9,1.5Hz), 8.90-8.91(1H,m), 9.13(1H,d,J=1.5Hz).
FAB-MS m/z:339(M+H)⁺.

### 3) Title Compound

Lithium hydroxide monohydrate (72.3 mg) in water (5.5 mL) was added dropwise at room temperature to a suspension of 5-(5-carbamoyl-2-pyrazinyl)-1-(3-pyridyl)-1H-pyrazole-3-carboxylic acid ethyl ester (0.530 g) in tetrahydrofuran (11 mL), followed by stirring for 105 minutes. The reaction mixture was neutralized with 1N aqueous hydrochloric acid (1.72 mL). The solid that precipitated was collected by filtration, to thereby give the title compound (0.445 g, 92%).

¹H-NMR(400MHz,DMSO-d₆)δ:7.53(1H,dd,J=8.3,4.9Hz), 7.76(1H,s), 7.87-7.91(2H,m), 8.28(1H,br s), 8.63-8.66(2H,m), 8.91(1H,s), 9.12(1H,s), 13.28(1H,br s).
FAB-MS m/z:311(M+H)⁺.

[Referential Example 8] 5-(5-Cyano-2-pyridyl)-1-(3-pyridyl)-1H-pyrazole-3-carboxylic acid

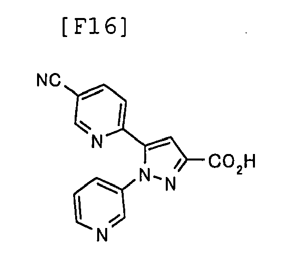

### Method A)

### 1) 5-(5-Hydroxy-2-pyridine)-1-(3-pyridyl)-1H-pyrazole-3-carboxylic acid ethyl ester

10% Palladium-carbon (4.63 g) was added to 5-(5-benzyloxy-2-pyridyl)-1-(3-pyridyl)-1H-pyrazole-3-carboxylic acid ethyl ester (4.63 g) obtained in step 4) of Referential Example 4 in a mixture of ethanol (46 mL) and ethyl acetate (46 mL). The resultant mixture was stirred in the presence of hydrogen at room temperature for 6 hours. The catalyst was removed from the reaction mixture by filtration, and the solvent of the filtrate was evaporated under reduced pressure, to thereby give 5-(5-hydroxy-2-pyridyl)-1-(3-pyridyl)-1H-pyrazole-3-carboxylic acid ethyl ester as a solid product (3.48 g, 97%).

¹H-NMR(400MHz,DMSO-d₆)δ:1.32(3H,t,J=7.1Hz), 4.33(2H,q,J=7.1Hz), 7.20-7.25(2H,m), 7.49(1H,dd,J=8.2,4.8Hz), 7.55(1H,d,J=8.5Hz), 7.76-7.79(1H,m), 7.93(1H,d,J=2.9Hz), 8.49(1H,d,J=2.7Hz), 8.58-8.60(1H,m), 10.31(1H,br s).
FAB-MS m/z:311(M+H)⁺.

### 2) 1-(3-Pyridyl)-5-(5-trifluoromethanesulfonyloxy-2-pyridyl)-1H-pyrazole-3-carboxylic acid ethyl ester

In an argon atmosphere, trifluoromethanesulfonic acid anhydrate (2.26 mL) was added dropwise at room temperature to 5-(5-hydroxy-2-pyridyl)-1-(3-pyridyl)-1H-pyrazole-3-carboxylic acid ethyl ester (3.47 g) in a mixture of dichloromethane (69 mL) and pyridine (23 mL), followed by stirring for 85 minutes. The reaction mixture was partitioned between water and chloroform. The organic layer was dried over sodium sulfate anhydrate. After a filtration step, the solvent was evaporated under reduced pressure, and the residue was purified by silica gel column chromatography (ethyl acetate - chloroform), to thereby give 1-(3-pyridyl)-5-(5-trifluoromethanesulfonyloxy-2-pyridyl)-1H-pyrazole-3-carboxylic acid ethyl ester as a solid product (4.95 g, quantitative amount).

¹H-NMR(400MHz,CDCl₃)δ:1.42-1.45(3H,m), 4.45-4.50(2H,m), 7.35(1H,s), 7.38-7.42(1H,m), 7.59-7.61(1H,m), 7.67-7.70(1H,m), 7.79-7.82(1H,m), 8.41(1H,d,J=2.7Hz), 8.56(1H,d,J=2.4Hz), 8.65(1H,dd,J=4.6,1.5Hz).
FAB-MS m/z:443(M+H)⁺.

### 3) Title Compound

In an argon atmosphere, a suspension of tri-n-butyltin cyanide (14.1 g) and tetrakis(triphenylphosphine)palladium(0) (19.4 g) in dichloroethane (133 mL) was refluxed for 2 hours. 1-(3-Pyridyl)-5-(5-trifluoromethanesulfonyloxy-2-pyridyl)-1H-pyrazole-3-carboxylic acid ethyl ester (4.94 g) in dichloroethane (109 mL) was added dropwise to the reaction mixture. The resultant mixture was refluxed for 13 hours, followed by cooling in air. A saturated aqueous solution of sodium hydrogencarbonate was added to the reaction mixture, and the resultant mixture was filtered through Celite. The filtrate was partitioned between water and chloroform. The organic layer was dried over sodium sulfate anhydrate. After a filtration step, the solvent was evaporated under reduced pressure, and the residue was purified by silica gel column chromatography (ethyl acetate - chloroform), to thereby give 5-(5-cyano-2-pyridyl)-1-(3-pyridyl)-1H-pyrazole-3-carboxylic acid ethyl ester. Lithium hydroxide monohydrate (0.470 g) in water (43 mL) was added dropwise at room temperature to a suspension of the obtained ethyl ester compound in tetrahydrofuran (87 mL), followed by stirring for 40 minutes. The reaction mixture was partitioned between water and chloroform. The aqueous layer was neutralized with 1N aqueous hydrochloric acid (11.2 mL), and then a solvent mixture of methanol-chloroform (1:10) was added for partitioning the resultant mixture. The solvent of the organic layer was evaporated under reduced pressure, to thereby give the title compound as a solid product (2.52 g, 77%).

¹H-NMR(400MHz,DMSO-d₆)δ:7.50-7.54(1H,m), 7.62(1H,s), 7.85-7.87(1H,m), 8.04(1H,d,J=8.3Hz), 8.41-8.44(1H,m), 8.58(1H,d,J=2.4Hz), 8.65(1H,d,J=4.6Hz), 8.80-8.81(1H,m), 13.23(1H,s).
FAB-MS m/z:292(M+H)⁺.

### Method B)

### 1) 2-Acetyl-5-cyanopyridine

At room temperature, ammonium peroxodisulfate (10.3 g) was gradually added to 3-cyanopyridine (3.12 g), pyruvic acid (6.23 mL), and silver nitrate (1.27 g) in a mixture of dichloromethane (150 mL) and water (150 mL). Sulfuric acid (3.2 mL) was gradually added to the reaction mixture under cooling on ice, followed by stirring at 40°C for 1.5 hours. Subsequently, the reaction mixture was alkalinized with 1M aqueous solution of sodium hydroxide under cooling on ice, and then the resultant mixture was extracted with dichloromethane. The organic layer was dried over sodium sulfate anhydrate. After a filtration step, the solvent was evaporated under reduced pressure, and the residue was purified by silica gel column chromatography (ethyl acetate - hexane), to thereby give 2-acetyl-5-cyanopyridine as a solid product (903 mg, 21%).

¹H-NMR(400MHz,CDCl₃)δ:2.75(3H,s), 8.13-8.16(2H,m), 8.95(1H,d,J=1.2Hz).

### 2) 4-(5-Cyano-2-pyridyl)-2,4-dioxobutanoic acid ethyl ester

In a manner similar to that employed in step 3) of Referential Example 6, 4-(5-cyano-2-pyridyl)-2,4-dioxobutanoic acid ethyl ester in solid form (1.188 g, 78%) was prepared from 2-acetyl-5-cyanopyridine (900 mg) and diethyl oxalate (1.67 mL).
¹H-NMR(400MHz,CDCl₃)δ:1.43(3H,t,J=7.1Hz), 4.42(2H,q,J=7.1Hz), 7.69(1H,s), 8.18(1H,dd, J=8.2,2.1Hz), 8.26(1H,d, J=8.1Hz), 8.98 (1H, s).

### 3) 5-(5-Cyano-2-pyridyl)-1-(3-pyridyl)-1H-pyrazole-3-carboxylic acid ethyl ester

In a manner similar to that employed in Method A)-step 3) of Referential Example 9, 5-(5-cyano-2-pyridyl)-1-(3-pyridyl)-1H-pyrazole-3-carboxylic acid ethyl ester in solid form (1.01 g, 45%) was prepared from 4-(5-cyano-2-pyridyl)-2,4-dioxobutanoic acid ethyl ester (1.72 g) and 3-hydrazinopyridine (0.92 g) obtained in Referential Example 1.

¹H-NMR(400MHz,CDCl₃)δ:1.44(3H,t,J=7.1Hz), 4.48(2H,q,J=7.2Hz), 7.41-7.93(1H,m), 7.42(1H,s), 7.61(1H,d,J=8.1Hz), 7.82(1H,d,J=8.1Hz), 8.00(1H,dd,J=8.2,2.1Hz 8.54 (1H, d, J=2. 7Hz) , 8.67-8.68(2H,m).

### 4) Title Compound

In a manner similar to that employed in step 3) of Referential Example 7, the title compound in solid form (5.19 g, quantitative amount) was prepared from 5-(5-cyano-2-pyridyl)-1-(3-pyridyl)-1H-pyrazole-3-carboxylic acid ethyl ester (5.69 g) and lithium hydroxide monohydrate (823 mg).

¹H-NMR(400MHz,DMSO-d₆)δ:7.53(1H,dd,J=8.1,4.9Hz), 7.63(1H,s), 7.87(1H,d,J=8.3Hz), 8.05(1H,d,J=8.3Hz), 8.44(1H,dd,J=8.3,2.2Hz), 8.59(1H,d,J=2.4Hz), 8.65 (1H,d,J=4.6Hz), 8.82(1H,d,J=2.2Hz).

[Referential Example 9] 5-(5-Methyl-2-pyridyl)-1-(3-pyridyl)-1H-pyrazole-3-carboxylic acid

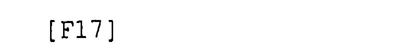

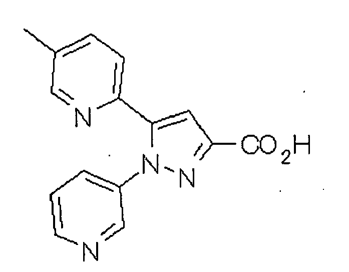

### [Method A]

### 1) 1-(5-Methyl-2-pyridyl)ethanone

At -78°C, n-butyllithium (1.58M hexane solution, 24 mL) was added dropwise to 2-bromo-5-picoline (5.0 g) in diethyl ether (100 mL) over 5 minutes, followed by stirring for 5 minutes. Subsequently, N,N-dimethylacetamide (3.5 mL) was added dropwise to the reaction mixture, followed by stirring for 2 hours. The reaction mixture was partitioned between water and ethyl acetate. The organic layer was dried over magnesium sulfate anhydrate. After a filtration step, the solvent was evaporated under reduced pressure, and the residue was purified by silica gel column chromatography (hexane - ethyl acetate), to thereby give 1-(5-methyl-2-pyridyl)ethanone as an oily product (3.43 g, 87%).

¹H-NMR(400MHz,CDCl₃)δ:2.42(3H,s), 2.71(3H,s), 7.62(1H,dd,J=1.59,7.93Hz), 7.94(1H,d,J=7.93Hz), 8.54(1H,s).

### 2) 4-(5-Methyl-2-pyridyl)-2,4-dioxobutanoic acid ethyl ester

Diethyl oxalate (7 mL) was added dropwise at room temperature to sodium ethoxide (3.5 g) in ethanol (60 mL). 1-(5-Methyl-2-pyridyl)ethanone (3.43 g) in ethanol (40 mL) was added to the reaction mixture, followed by stirring for 2 hours. The reaction mixture was partitioned between water and diethyl ether. The aqueous layer was acidified with 1N aqueous solution of hydrochloric acid, and then chloroform was added for partitioning the reaction mixture. The organic layer was dried over magnesium sulfate anhydrate. After a filtration step, the solvent was evaporated under reduced pressure, to thereby give 4-(5-methyl-2-pyridyl)-2,4-dioxobutanoic acid ethyl ester as a solid product (6.8 g).

¹H-NMR(400MHz,CDCl₃)δ:1.40(3H,t,J=7.08Hz), 2.47(3H,s), 4.39(2H,q,J=7.08Hz), 7.49(1H,br), 7.74(1H,dd,J=1.47,8.06Hz), 8.08(1H,d,J=8.06Hz), 8.58(1H,d,J=0.73Hz).
ESI-MS m/z:236(M+H)⁺.

### 3) 5-(5-Methyl-2-pyridyl)-1-(3-pyridyl)-1H-pyrazole-3-carboxylic acid ethyl ester

3-Hydrazinopyridine (2.0 g) obtained in Referential Example 1 and concentrated hydrochloric acid (2 mL) were added to the above-obtained 4-(5-methyl-2-pyridyl)-2,4-dioxobutanoic acid ethyl ester (3.50 g) in ethanol (30 mL). The resultant mixture was refluxed for 17.5 hours, followed by cooling in air. The reaction mixture was partitioned between saturated aqueous solution of sodium hydrogencarbonate and chloroform. The organic layer was dried over magnesium sulfate anhydrate. After a filtration step, the solvent was evaporated under reduced pressure, and the residue was purified by silica gel column chromatography (chloroform - methanol), to thereby give 5-(5-methyl-2-pyridyl)-1-(3-pyridyl)-1H-pyrazole-3-carboxylic acid ethyl ester (2.51 g, 55%).

¹H-NMR(400MHz,CDCl₃)δ:1.43(3H,t,J=7.14Hz), 2.34(3H,s), 4.46(2H,q,J=7.14Hz), 7.24(1H,s), 7.32(1H,d,J=8.12Hz), 7.37(1H,dd,J=4.82,8.12Hz), 7.51(1H,dd,J=2.08,8.12Hz), 7.84(1H,dd,J=1.44,8.12Hz), 8.30(1H,s), 8.51(1H,d,J=2.56Hz), 8.59(1H,dd,J=1.34,4.82Hz).
ESI-MS m/z:309(M+H)⁺.

### 4) Title Compound

Sodium ethoxide (1.11 g) was added at room temperature to the above-obtained 5-(5-methyl-2-pyridyl)-1-(3-pyridyl)-1H-pyrazole-3-carboxylic acid ethyl ester (2.51 g) in ethanol (80 mL), followed by stirring for 19.5 hours. The reaction mixture was partitioned between water and diethyl ether. The aqueous layer was acidified with 1N aqueous solution of hydrochloric acid, and then chloroform was added for partitioning the resultant mixture. The organic layer was dried over magnesium sulfate anhydrate. After a filtration step, the solvent was evaporated under reduced pressure, to thereby give the title compound (1.33 g, 58%).

¹H-NMR(400MHz,CDCl₃/CD₃OD)δ:2.38(3H,s), 7.26(1H,s), 7.45(2H,m), 7.68(1H,br), 7.85(1H,ddd,J=1.47,2.44,8.30Hz), 8.30(1H,s), 8.52(1H,d,J=2.56Hz), 8.56(1H,dd,J=1.47,4.76Hz).
ESI-MS m/z:281(M+H)⁺.

### [Method B]

### 1) 4-(5-Methyl-2-pyridyl)-2,4-dioxobutanoic acid methyl ester

Dimethyl oxalate (10.4 g) was added at room temperature to sodium methoxide (4.74 g) in methanol (200 mL), followed by stirring for 5 minutes. Subsequently, 1-(5-methyl-2-pyridyl)ethanone (5.93 g) was added at room temperature to the reaction mixture, followed by stirring for 5 hours. The reaction mixture was partitioned between water and diethyl ether. The aqueous layer was acidified with 1N aqueous solution of hydrochloric acid, and then the resultant mixture was extracted with chloroform. The organic layer was dried over magnesium sulfate anhydrate. After a filtration step, the solvent was evaporated under reduced pressure, to thereby give 4-(5-methyl-2-pyridyl)-2,4-dioxobutanoic acid methyl ester as a solid product (7.31 g, 75%).

¹H-NMR(400MHz,CDCl₃)δ:2.46(3H,s), 3.92(3H,s), 7.58(1H,br), 7.70 (1H, dd, J=8.06,1.83Hz), 8.08(1H,d,J=8.06Hz), 8.54(1H,d,J=1.22Hz).

### 2) 5-(5-Methyl-2-pyridyl)-1-(3-pyridyl)-1H-pyrazole-3-carboxylic acid methyl ester

3-Hydrazinopyridine (3.0 g) obtained in Referential Example 1 and concentrated hydrochloric acid (4 mL) were added to the above-obtained 4-(5-methyl-2-pyridyl)-2,4-dioxobutanoic acid methyl ester (4.0 g) in methanol (250 mL). The resultant mixture was refluxed for 2.5 hours, followed by cooling in air. The reaction mixture was partitioned between a saturated aqueous solution of sodium hydrogencarbonate and chloroform. The organic layer was dried over magnesium sulfate anhydrate. After a filtration step, the solvent was evaporated under reduced pressure, and the residue was purified by silica gel column chromatography (methanol - chloroform), to thereby give 5-(5-methyl-2-pyridyl)-1-(3-pyridyl)-1H-pyrazole-3-carboxylic acid methyl ester as a solid product (3.24 g, 61%).

¹H-NMR(400MHz,CDCl₃)δ:2.34(3H,s), 3.99(3H,s), 7.27(1H,s), 7.33(1H,d,J=8.06Hz), 7.37(1H,ddd, J=8.18,4.76,0.73Hz), 7.53(1H,ddd,J=8.06,2.20,0.73Hz), 7.84(1H,ddd,J=8.18,2.56,1.47Hz), 8.30(1H,d,J=1.47Hz), 8.50(1H,d,J=2.32Hz), 8.59(1H,dd,J=4.76,1.47Hz).
ESI-MS m/z:295(M+H)⁺.

### 3) Title Compound

1N Aqueous solution of sodium hydroxide (16 mL) was added at room temperature to 5-(5-methyl-2-pyridyl)-1-(3-pyridyl)-1H-pyrazole-3-carboxylic acid methyl ester (3.24 g) in tetrahydrofuran (100 mL), followed by stirring for 2 hours. The reaction mixture was partitioned between 1N aqueous solution of hydrochloric acid and chloroform. The organic layer was dried over magnesium sulfate anhydrate. After a filtration step, the solvent was evaporated under reduced pressure, to thereby give the title compound as a solid product (2.0 g, 65%).

¹H-NMR(400MHz,DMSO-d₆)δ:2.28(3H,s), 7.30(1H,s), 7.49(1H,dd,J=8.18,4.76Hz), 7.63(1H,d,J=8.18Hz), 7.70(1H,dd,J=8.18,1.58Hz), 7.79(1H,d,J=8.18Hz), 8.24(1H,s), 8.49(1H,d,J=1.56Hz), 8.59(1H,dd,J=4.76,1.59Hz).
ESI-MS m/z:281(M+H)⁺.

[Referential Example 10] 5-(5-Methyl-2-pyridyl)-1-(3-pyridazinyl)-1H-pyrazole-3-carboxylic acid

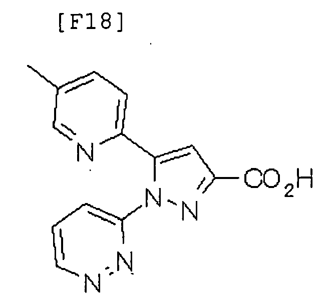

### 1) 1-(6-Chloro-3-pyridazinyl)-5-(5-methyl-2-pyridyl)-1H-pyrazole-3-carboxylic acid ethyl ester

Concentrated hydrochloric acid (1.2 mL) was added at room temperature to 6-chloro-3-hydrazinopyridazine (3.44 g) and 4-(5-methyl-2-pyridyl)-2,4-dioxobutanoic acid ethyl ester (5.56 g) obtained in Method A-step 2) of Referential Example 9 in ethanol (60 mL). The resultant mixture was refluxed for 16 hours, followed by cooling in air. The reaction mixture was neutralized with 1N aqueous solution of sodium hydroxide. The solid that precipitated was collected by filtration, to thereby give 1-(6-chloro-3-pyridazinyl)-5-(5-methyl-2-pyridyl)-1H-pyrazole-3-carboxylic acid ethyl ester (4.48 g, 55%).

¹H-NMR(400MHz,CDCl₃)δ:1.43(3H,t,J=7.0Hz), 2.34(3H,s), 4.46(2H,q,J=7.0Hz), 7.19(1H,s), 7.51(1H,d,J=7.8Hz), 7.57(1H,dd,J=1.0,7.8Hz), 7.68 (1H, d, J=9.0Hz), 8.08(1H,d,J=9.0Hz), 8.21(1H, d, J=1.0Hz).
FAB-MS m/z:344 (M+H)⁺.

### 2) 5-(5-Methyl-2-pyridyl)-1-(3-pyridazinyl)-1H-pyrazole-3-carboxylic acid ethyl ester

10% Palladium-carbon (1.27 g) and ammonium formate (2.615 g) were added at room temperature to 1-(6-chloro-3-pyridazinyl)-5-(5-methyl-2-pyridyl)-1H-pyrazole-3-carboxylic acid ethyl ester (2.58 g) in ethanol(70 mL). The resultant mixture was stirred at 75°C for 1 hour, followed by cooling in air. The insoluble solid was removed by filtration, and then the solvent of the filtrate was evaporated under reduced pressure. The residue was partitioned between water and chloroform. The aqueous layer was extracted with chloroform. The organic layers were combined, followed by washing with saturated brine. The resultant mixture was dried over sodium sulfate anhydrate. After a filtration step, the solvent was evaporated under reduced pressure, and the residue was purified by silica gel column chromatography (methanol - chloroform), to thereby give 5-(5-methyl-2-pyridyl)-1-(3-pyridazinyl)-1H-pyrazole-3-carboxylic acid ethyl ester as a solid product (1.126 g, 49%).

¹H-NMR(400MHz,CDCl₃)δ:1.43(3H,t,J=7.1Hz), 2.32(3H,s), 4.47(2H,q,J=7.1Hz), 7.21(1H,s), 7.51(1H,d like,J=8.0Hz), 7.55-7.61(1H,m), 7.66(1H,dd,J=4.9,8.5Hz), 8.07-8.13(1H,m), 8.19(1H,br), 9.12(1H,dd,J=2.5,4.9Hz).
ESI-MS m/z:310(M+H)⁺.

### 3) Title Compound

Lithium hydroxide monohydrate (0.173 g) was added at room temperature to 5-(5-methyl-2-pyridyl)-1-(3-pyridazinyl)-1H-pyrazole-3-carboxylic acid ethyl ester (1.126 g) in a mixture of tetrahydrofuran (20 mL), ethanol (10 mL), and water (20 mL), followed by stirring for 2.5 hours. The reaction mixture was acidified to pH 5 with 1N aqueous solution of hydrochloric acid. Subsequently, chloroform-methanol (15:1) was added for partitioning the resultant mixture. The aqueous layer was extracted with chloroform-methanol (15:1). The organic layers were combined, followed by drying over sodium sulfate anhydrate. After a filtration step, the solvent was evaporated under reduced pressure, to thereby give the title compound as a solid product (0.688 g, 69%).

¹H-NMR(400MHz,DMSO-d₆)δ:2.27(3H,s), 7.37(1H,s like), 7.67-7.79(2H,m), 7.97(1H,dd,J=4.6,8.3Hz), 8.08(1H,d,J=8.3Hz), 8.14(1H,br s), 9.29(1H,d,J=4.6Hz).
ESI-MS m/z:282(M+H)⁺.

[Referential Example 11] 4-Methoxypiperidine trifluoroacetic acid salt

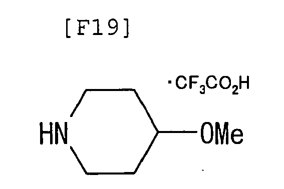

### 1) 4-Methoxypiperidine-1-carboxylic acid tert-butyl ester

In an argon atmosphere, 4-hydroxypiperidine-1-carboxylic acid tert-butyl ester (2.00 g) in N,N-dimethylformamide (20 mL) was added dropwise at room temperature to a suspension of sodium hydride (60%, 0.477 g) in N,N-dimethylformamide (20 mL), followed by stirring for 15 minutes. Subsequently, methyl iodide (0.742 mL) was added dropwise to the reaction mixture, followed by stirring for 2 hours. The reaction mixture was partitioned between water and ethyl acetate. The organic layer was dried over sodium sulfate anhydrate. After a filtration step, the solvent was evaporated under reduced pressure, and the residue was purified by silica gel column chromatography (ethyl acetate - hexane), to thereby give 4-methoxypiperidine-1-carboxylic acid tert-butyl ester as an oily product (1.43 g, 67%).

¹H-NMR(400MHz,CDCl₃)δ:1.39-1.54(2H,m), 1.46(9H,s), 1.81-1.84(2H,m), 3.05-3.12(2H,m), 3.31-3.39(1H,m), 3.35(3H,s), 3.74-3.77(2H,m).

### 2) Title Compound

Trifluoroacetic acid (14 mL) was added at room temperature to the above-obtained 4-methoxypiperidine-1-carboxylic acid tert-butyl ester (1.42 g) in dichloromethane (28 mL), followed by stirring for 2.5 hours. The solvent of the reaction mixture was evaporated under reduced pressure, to thereby give the title compound as an oily product (2.65 g, quantitative amount).

¹H-NMR(400MHz,CDCl₃)δ:1.98-2.02(4H,m), 3.19-3.23(2H,m), 3.30-3.42(2H,m), 3.37(3H,s), 3.54-3.60(1H,m)

[Referential Example 12] 4,4-Difluoropiperidine hydrochloride

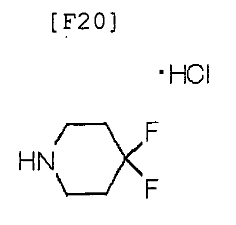

### 1) 1-Benzyl-4,4-difluoropiperidine

In an argon atmosphere, diethylaminosulfur trifluoride (8.38 mL) was added dropwise to 1-benzyl-4-piperidone (5.00 g) in benzene (200 mL) at 0°C, and then the resultant mixture was stirred for 30 minutes, followed by refluxing for 18 hours. At 0°C, the resultant mixture was partitioned between a saturated aqueous solution of sodium hydrogencarbonate and ethyl acetate, and then the organic layer was dried over sodium sulfate anhydrate. After a filtration step, the solvent was evaporated under reduced pressure, and the residue was purified by silica gel column chromatography (hexane - ethyl acetate), to thereby give 1-benzyl-4,4-difluoropiperidine as an oily product (4.67 g, 84%).

¹H-NMR(400MHz,CDCl₃)δ:1.93-2.04(4H,m), 2.53-2.55(4H,m), 3.54(2H,s), 7.24-7.34(5H,m).
EI-MS m/z:211(M⁺).

### 2) Title Compound

In an argon atmosphere, 1-chloroethyl chloroformate (2.62 mL) was added dropwise to the above-obtained 1-benzyl-4,4-difluoropiperidine (4.66 g) in dichloromethane (93 mL) at 0°C. The resultant mixture was refluxed for 2 hours, followed by cooling in air. The reaction solvent was evaporated under reduced pressure, and then the residue was dissolved in methanol (93 mL). The resultant mixture was refluxed for 4 hours, followed by cooling in air. The reaction solvent was evaporated under reduced pressure, to thereby give the title compound as a solid product (3.03 g, 87%).

¹H-NMR(400MHz,D₂O)δ:2.31-2.41(4H,m), 3.43-3.46(4H,m).
FAB-MS m/z:122(M+H)⁺.

[Referential Example 13] 4-Fluoropiperidine hydrochloride

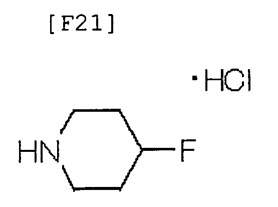

### 1) 4-Fluoropiperidine-1-carboxylic acid tert-butyl ester

In an argon atmosphere, [bis(2-methoxyethyl)amino]sulfur trifluoride (7.33 mL) was added dropwise at -78°C to 4-hydroxy-1-piperidinecarboxylic acid tert-butyl ester (4.00 g) in dichloromethane (80 mL), and the resultant mixture was stirred for 30 minutes, at 0°C for 30 minutes, and then at room temperature for 2 hours. The reaction mixture was partitioned between a saturated aqueous solution of sodium hydrogencarbonate and chloroform. The organic layer was dried over sodium sulfate anhydrate. After a filtration step, the solvent was evaporated under reduced pressure, and the residue was purified by silica gel column chromatography (chloroform - ethyl acetate), to thereby give 4-fluoropiperidine-1-carboxylic acid tert-butyl ester as an oily product (1.77 g, 44%).

¹H-NMR(400MHz,CDCl₃)δ:1.45(9H,s), 1.76-1.86(4H,m), 3.41-3.54(4H,m), 4.70-4.87(1H,m).
EI-MS m/z:203(M⁺).

### 2) Title Compound

4N HCl-dioxane (12 mL) was added at room temperature to the above-obtained 4-fluoropiperidine-1-carboxylic acid tert-butyl ester (1.74 g) in dichloromethane (35 mL), followed by stirring for 40 minutes. The solvent of the reaction mixture was evaporated under reduced pressure. Subsequently, diethyl ether was added to the residue, and then the solid that precipitated was collected through filtration, to thereby give the title compound as a solid product (0.870 g, 73%).

¹H-NMR(400MHz,DMSO-d₆)δ:1.92-2.13(4H,m), 3.01-3.12(4H,m), 4.83-4.97(1H,m).
FAB-MS m/z:104(M+H)⁺.

[Referential Example 14] Hexahydropyridazine hydrochloride

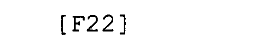

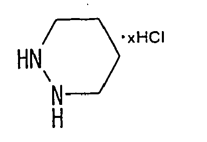

### 1) 3,6-Dihydropyridazine-1,2-dicarboxylic acid dibenzyl ester

1,3-Butadiene (14.2 g) was bubbled into 1,2-azodicarboxylic acid dibenzyl ester (10.28 g) in benzene (50 mL) at -10°C, followed by stirring at room temperature for 16 hours. The reaction solvent was evaporated under reduced pressure, and the residue was purified by silica gel column chromatography (ethyl acetate - hexane), to thereby give 3,6-dihydropyridazine-1,2-dicarboxylic acid dibenzyl ester as an oily product (2.57 g, 21%).

¹H-NMR(400MHz,CDCl₃)δ:3.70-3.85(2H,br), 4.35-4.52(2H,br), 5.05-5.25(4H,br), 5.78(2H,br), 7.03-7.40(10H,m).
FAB-MS m/z:353(M+H)⁺.

### 2) Hexahydropyridazine

10% Palladium-carbon (0.754 g) was added to the above-obtained 3,6-dihydropyridazine-1,2-dicarboxylic acid dibenzyl ester (2.57 g) in methanol (25 mL), followed by stirring in the presence of hydrogen for 19 hours. The catalyst was removed from the reaction mixture by filtration. The solvent of the filtrate was evaporated under reduced pressure, to thereby give hexahydropyridazine as an oily product (0.629 g, quantitative amount).
¹H-NMR(400MHz,DMSO-d₆)δ:1.67-1.75(2H,m),
1.96-2.05(2H,m), 2.60-3.10(4H,m).
ESI-MS m/z:87(M+H)⁺.

### 3) Hexahydropyridazine-1-carboxylic acid tert-butyl ester Method A)

Di-tert-butoxydicarbonate (2.10 g) was added to hexahydropyridazine (0.72 g) in methanol (20 mL) at room temperature, followed by stirring for 15 hours. The reaction solvent was evaporated under reduced pressure, and the residue was purified by silica gel column chromatography (acetone - chloroform), to thereby give hexahydropyridazine-1-carboxylic acid tert-butyl ester as an oily product (0.671 g, 43%).
¹H-NMR(400MHz,CDCl₃)δ:1.48(9H,s), 1.50-1.73(4H,m), 2.87(2H,t like,J=4.5Hz), 3.51(2H,t like,J=4.5Hz), 4.65(1H,br).

### Method B)

10% Palladium-carbon (50% wet, 5.21 g) was added to 2-(tert-butoxycarbonyl)hexahydropyridazine-1-carboxylic acid benzyl ester (Bioorg. Med. Chem., 2002, 10, 953, 26.94 g) in methanol (250 mL), followed by stirring in a hydrogen atmosphere for 4 hours. After a filtration step, the solvent of the filtrate was evaporated under reduced pressure, and the residue was purified by silica gel column chromatography (chloroform - methanol), to thereby give hexahydropyridazine-1-carboxylic acid tert-butyl ester as an oily product (18.24 g, 85%).
¹H-NMR(400MHz,CDCl₃)δ:1.98(9H,s), 1.50-1.73(4H,m), 2.87(2H,t like,J=4.5Hz), 3.51(2H,t like,J=4.5Hz).

### 4) Title Compound

4N HCl-dioxane (4 mL) was added at room temperature to the above-obtained hexahydropyridazine-1-carboxylic acid tert-butyl ester (0.671 g) in dichloromethane (8 mL), followed by stirring for 1 hour. Diethyl ether and pentane ware added to the reaction mixture. The supernatant was removed through decantation. The residue was dried under reduced pressure, to thereby give the title compound as a solid product (0.292 g, 66%).
¹H-NMR(400MHz,DMSO-d₆)δ:1.66(2H,br), 2.50(2H,br), 2.98(2H,br), 4.35(4H,br).
ESI-MS m/z:87(M+H)⁺.

[Referential Example 15] 1-Methylpiperazin-2-one hydrochloride

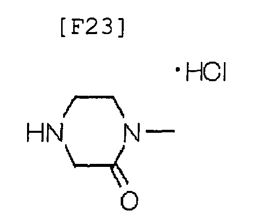

### 1) 3-Oxopiperazine-1-carboxylic acid tert-butyl ester

Triethylamine (3.83 mL) and di-tert-butoxydicarbonate (6.32 mL) were added at room temperature to piperazin-2-one (2.5 g) in a mixture of tetrahydrofuran (50 mL) and methanol (50 mL), followed by stirring for 4 hours. The reaction solvent was evaporated under reduced pressure. The residue was partitioned between water and ethyl acetate. The organic layer was sequentially washed with water and saturated brine. The aqueous layers obtained through washing were combined, and the combined layer was extracted with ethyl acetate. The organic layers were combined, followed by drying over magnesium sulfate anhydrate. After a filtration step, the solvent was evaporated under reduced pressure. Subsequently, ethyl acetate-hexane was added to the residue, followed by drying to solid, to thereby give 3-oxopiperazine-1-carboxylic acid tert-butyl ester (3.6 g, 72%).

¹H-NMR(400MHz,CDCl₃)δ:1.48(9H,s), 3.37-3.40(2H,m), 3.62-3.65(2H,m), 4.01(2H,s), 6.32(1H,br s).

### 2) 4-Methyl-3-oxopiperazine-1-carboxylic acid tert-butyl ester

Sodium hydride (60%, 960 mg) was added at 0°C to the above-obtained 3-oxopiperazine-1-carboxylic acid tert-butyl ester (3.0 g) in N,N-dimethylformamide (50 mL). Methyl iodide (2.33 mL) was added to the reaction mixture, followed by stirring at room temperature for 15 hours. The reaction mixture was partitioned between water and ethyl acetate. The organic layer was sequentially washed with water and saturated brine. The aqueous layers obtained through washing were combined, and the combined layer was extracted with ethyl acetate. The organic layers were combined, followed by drying over magnesium sulfate anhydrate. After a filtration step, the solvent was evaporated under reduced pressure, to thereby give 4-methyl-3-oxopiperazine-1-carboxylic acid tert-butyl ester as an oily product (2.32 g, 72%).
¹H-NMR(400MHz,CDCl₃)δ:1.47(9H,s), 3.01(3H,s), 3.34(2H,t,J=5.6Hz), 3.65(2H,t,J=5.6Hz), 4.07(2H,s).

### 3) Title Compound

4N HCl-dioxane (20 mL) was added to the above-obtained 4-methyl-3-oxopiperazine-1-carboxylic acid tert-butyl ester (2.06 g), followed by stirring at room temperature for 1 hour. The reaction solvent was evaporated under reduced pressure. Toluene was added to the residue, and the solvent was evaporated through azeotropy under reduced pressure. The residue was dried, to thereby give the title compound as an oily product (1.44 g, 99%).

¹H-NMR(400MHz,DMSO-d₆)δ:2.86(3H,s), 3.34(2H,br m), 3.50(2H, m), 3.64(2H,s).
MS(ESI) m/z:115(M+H)⁺.

[Referential Example 16] 1-Methylpiperazin-2-one trifluoroacetic acid salt
4-Methyl-3-oxopiperazine-1-carboxylic acid tert-butyl ester (0.308 g) obtained in step 2) of Referential Example 15 was dissolved in dichloromethane (6 mL). Trifluoroacetic acid (3 mL) was added to the resultant mixture at room temperature, followed by stirring for 1.5 hours. The reaction solvent was evaporated under reduced pressure, and the residue was dried, to thereby give the title compound (0.485 g, quantitative amount).

¹H-NMR(400MHz,CDCl₃-CD₃OD(15:1))δ:2.98(3H,s), 3.39(2H,t-like,J=6.1Hz), 3.54(2H,t-like,J=6.1Hz), 3.72(2H,s).
MS(EI) m/z:114(M⁺).

[Referential Example 17] 1-Cyclopropylpiperazine hydrochloride

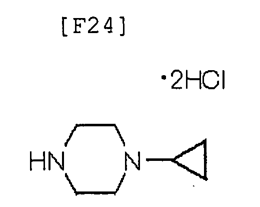

### 1) 4-Cyclopropylpiperazine-1-carboxylic acid tert-butyl ester

Sodium cyanoborohydride (1.89 g) was added at room temperature to piperazine-1-carboxylic acid tert-butyl ester (1.87 g), [(1-ethoxycyclopropyl)oxy]trimethylsilane (8.05 mL), and acetic acid (5.72 mL) in methanol (60 mL), followed by stirring for 5 days. The reaction solvent was evaporated under reduced pressure. Diethyl ether was added to the residue, and then the insoluble matter was removed by filtration. 1N Aqueous solution of sodium hydroxide was added for partitioning the solvent of the filtrate. The organic layer was washed with saturated brine, followed by drying over magnesium sulfate anhydrate. After a filtration step, the solvent was evaporated under reduced pressure, and the residue was purified by silica gel column chromatography (hexane - ethyl acetate), to thereby give 4-cyclopropylpiperazine-1-carboxylic acid tert-butyl ester as a solid product (1.62 g, 71%).

¹H-NMR(400MHz,CDCl₃)δ:0.41-0.48(4H,m), 1.46(9H,s), 2.54-2.56(4H,m), 3.37-3.44 (4H,m).
MS(ESI) m/z:268(M+MeCN)⁺.

### 2) Title Compound

In a manner similar to that employed in step 3) of Referential Example 15, the title compound in solid form (1.30 g, 93%) was prepared from the above-obtained 4-cyclopropylpiperazine-1-carboxylic acid tert-butyl ester (1.61 g).

¹H-NMR(400MHz,DMSO-d₆)δ:0.79-0.81(2H,m), 1.14(2H,br s), 3.52(8H,br s), 9.94 (2H,br).LC-MS m/z:127(M+H)⁺.

[Referential Example 18] Azetidin-3-yldimethylamine hydrochloride

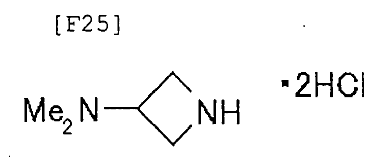

### 1) 1-Benzhydrylazetidin-3-one

Pyridinesulfonic acid (19.7 g) in dimethyl sulfoxide (84 mL) was added dropwise to 1-benzhydrylazetidin-3-ol (4.79 g) in triethylamine (27.9 mL) under cooling on ice, followed by stirring at 50°C for 40 minutes. The reaction mixture was partitioned between ice-water and ethyl acetate. The organic layer was washed with saturated brine, followed by drying over magnesium sulfate anhydrate. After a filtration step, the solvent was evaporated under reduced pressure, and the residue was purified by silica gel column chromatography (hexane - ethyl acetate), to thereby give 1-benzhydrylazetidin-3-one as a solid product (2.85 g, 60%).

¹H-NMR(400MHz,CDCl₃)δ:4.00(4H,s), 4.59(1H,s), 7.19-7.49(10H,m).

### 2) (1-Benzhydrylazetidin-3-yl)dimethylamine

5% Palladium-carbon (1.5 g) was added to the above-obtained 1-benzhydrylazetidin-3-one (1.50 g) and 40% aqueous solution of dimethylamine (4 mL) in methanol (30 mL). The resultant mixture was subjected to catalytic reduction overnight in a hydrogen atmosphere. The catalyst was removed from the reaction mixture by filtration. The solvent of the filtrate was evaporated under reduced pressure, and the residue was purified by silica gel column chromatography (chloroform - methanol), to thereby give (1-benzhydrylazetidin-3-yl)dimethylamine as a solid product (1.55 g, 92%).

¹H-NMR(400MHz,CDCl₃)δ:2.08(6H,s), 2.80-2.87(3H,m), 3.36-3.42(2H,m), 4.37(1H,s), 7.15-7.41(10H,m).
MS(ESI) m/z:267(M+H)⁺.

### 3) Title Compound

20% Palladium hydroxide-carbon (533 mg) was added to the above-obtained (1-benzhydrylazetidin-3-yl)dimethylamine (533 mg) in ethanol (15 mL), followed by stirring in a hydrogen atmosphere for 18 hours. The catalyst was removed from the reaction mixture by filtration. 1N HCl-ethanol (4 mL) was added to the solvent of the filtrate. The solvent of the reaction mixture was evaporated, and then diethyl ether was added to the residue. The solid that precipitated was collected by filtration, to thereby give the title compound (300 mg, 87%).

¹H-NMR(400MHz,DMSO-d₆)δ:2.70(6H,m), 4.05-4.10(2H,m), 4.25-4.31(1H,m), 4.38-4.43(2H,m).
LC-MS m/z:101(M+H)⁺.

[Referential Example 19] Piperidine-2-carboxamide

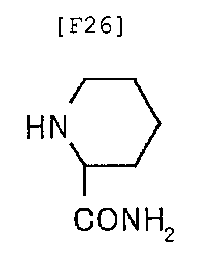

Concentrated aqueous ammonia (3 mL) and triethylamine (2 mL) were added at room temperature to N-benzyloxycarbonylpiperidine-2-carboxylic acid (2.0 g), 1-hydroxybenzotriazole (1.6 g), and 1-(3-dimethylaminopropyl)-3-ethylcarbodiimide hydrochloride (2.3 g) in dichloromethane (20 mL), followed by stirring for 3 days. The reaction mixture was partitioned between water and dichloromethane. The organic layer was dried over magnesium sulfate anhydrate. After a filtration step, the solvent was evaporated under reduced pressure. The residue was dissolved in methanol (30 mL). 10% Palladium-carbon (1 g, 50% wet) was added to the resultant mixture, followed by stirring in the presence of hydrogen for 20 hours. The catalyst was removed from the reaction mixture by filtration. The solvent of the filtrate was evaporated under reduced pressure, to thereby give the title compound as a solid product (970 mg, quantitative amount).

MS (ESI) m/z:128(M⁺).

[Referential Example 20] 5-[5-(tert-Butoxycarbonylamino)-2-pyridyl]-1-(3-pyridyl)-1H-pyrazole-3-carboxylic acid

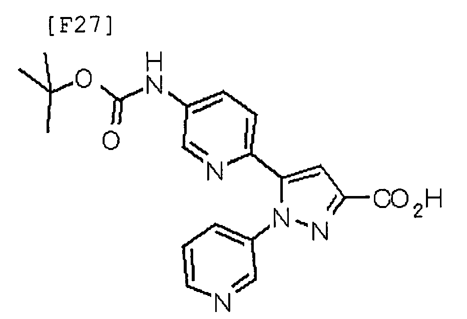

### 1) 2-Acetyl-5-aminopyridine

Methylmagnesium bromide (0.93M tetrahydrofuran solution, 200 mL) was added dropwise to 5-amino-2-cyanopyridine (10.13 g) in tetrahydrofuran (200 mL) in a nitrogen atmosphere under cooling on ice over 25 minutes, followed by stirring at room temperature for 5 hours. Under cooling on ice, a saturated aqueous solution of ammonium chloride was added to the reaction mixture, and then sulfuric acid (20 mL) was added dropwise to the resultant mixture, followed by stirring at room temperature for 80 minutes. Sodium hydroxide (20 g) in water (100 mL) was added dropwise to the reaction mixture under cooling on ice. Subsequently, ethyl acetate was added for partitioning the resultant mixture. The organic layer was washed with saturated brine, followed by drying over sodium sulfate anhydrate. After a filtration step, the solvent was evaporated under reduced pressure, and the residue was purified by silica gel column chromatography (methanol - chloroform), to thereby give 2-acetyl-5-aminopyridine as a solid product (7.68 g, 66%).

¹H-NMR(400MHz,CDCl₃)δ:2.64(3H,s), 4.00-4.30(2H,br), 6.98(1H,dd,J=2.7,8.5Hz), 7.91(1H,dd,J=0.5, 8.5Hz), 8.06(1H,dd,J=0.5,2.7Hz).
ESI-MS m/z:137(M+H)⁺.

### 2) 2-Acetyl-5-(tert-butoxycarbonylamino)pyridine

Di-tert-butoxydicarbonate (11.10 g) in dichloromethane (30 mL) was added to 2-acetyl-5-aminopyridine (6.30 g) and 4-(dimethylamino)pyridine (5.65 g) in dichloromethane (150 mL) under cooling on ice, followed by stirring at room temperature for 1 hour. The solid that precipitated was removed from the resultant mixture by filtration. The solvent of the filtrate was evaporated under reduced pressure, and the residue was purified by silica gel column chromatography (methanol - chloroform), to thereby give 2-acetyl-5-(tert-butoxycarbonylamino)pyridine as a solid product (8.04 g, 73%).

¹H-NMR(400MHz,CDCl₃)δ:1.54(9H,s), 2.68(3H,s), 6.74 (1H,br s), 8.03 (1H, d, J=8.5Hz), 8.11(1H,dd,J=8.5,2.4Hz), 8.46(1H,dd,J=2.4,0.5Hz).
ESI-MS m/z:237(M+H)⁺.

### 3) 4-[5-(tert-Butoxycarbonylamino)-2-pyridyl]-2,4-dioxobutanoic acid ethyl ester

Diethyl oxalate (9.2 mL) was added to sodium ethoxide (4.63 g) in ethanol (340 mL). At room temperature, 2-acetyl-5-(tert-butoxycarbonylamino)pyridine (8.04 g) in ethanol (60 mL) was added to the reaction mixture, followed by stirring for 45 minutes. The resultant mixture was refluxed for 30 minutes, followed by cooling in air. The reaction solvent was evaporated under reduced pressure. The residue was partitioned between 5% aqueous citric acid and ethyl acetate. The organic layer was sequentially washed water and saturated brine, followed by drying over sodium sulfate anhydrate. After a filtration step, the solvent was evaporated under reduced pressure, and the residue was purified by silica gel column chromatography (methanol - chloroform), to thereby give 4-[5-(tert-butoxycarbonylamino)-2-pyridyl]-2,4-dioxobutanoic acid ethyl ester as a solid product (1.70 g, 14.8%).

¹H-NMR(400MHz,CDCl₃)δ:1.41(3H,t,J=7.1Hz), 1.55(9H,s), 4.40(2H,q,J=7.1Hz), 6.78(1H,br s), 7.59(1H,s), 8.14(1H,d,J=8.8Hz), 8.19(1H,dd,J=8.8,2.4Hz), 8.51(1H,d,J=2.4Hz).
ESI-MS m/z:337(M+H)⁺.

### 4) 5-[5-(tert-Butoxycarbonylamino)-2-pyridyl]-4,5-dihydro-5-hydroxy-1-(3-pyridyl)-1H-pyrazole-3-carboxylic acid ethyl ester

4-[5-(tert-Butoxycarbonylamino)-2-pyridyl]-2,4-dioxobutanoic acid ethyl ester (1.59 g) and 3-hydrazinopyridine (0.52 g) obtained in Referential Example 1 in ethanol (100 mL) was refluxed for 17 hours, followed by cooling in air. The reaction solvent was evaporated under reduced pressure, and the residue was purified by silica gel column chromatography (methanol - chloroform), to thereby give 5-[5-(tert-butoxycarbonylamino)-2-pyridyl]-4,5-dihydro-5-hydroxy-1-(3-pyridyl)-1H-pyrazole-3-carboxylic acid ethyl ester as an amorphous product (1.382 g, 68%).

¹H-NMR(400MHz,CDCl₃)δ:1.40(3H,t,J=7.1Hz), 1.53(9H,s), 3.47(1H,d,J=19.0Hz), 3.63(1H,d,J=19.0Hz), 4.39(2H,q,J=7.1Hz), 6.71(1H,br s), 6.78(1H,s), 7.06(1H,ddd,J=8.3,4.6,0.7Hz), 7.35(1H,d,J=8.8Hz), 7.46(1H,ddd,J=8.3,2.7,1.5Hz), 7.98-8.03(1H,m), 8.14(1H,dd,J=4.6,1.5Hz), 8.19(1H,dd,J=2.7,0.7Hz), 8.49(1H,dd,J=2.7,0.5Hz).
ESI-MS m/z:428(M+H)⁺.

### 5) 5-[5-(tert-Butoxycarbonylamino)-2-pyridyl]-1-(3-pyridyl)-1H-pyrazole-3-carboxylic acid ethyl ester

Triethylamine (1.96 mL) and methanesulfonyl chloride (327 µL) were added at room temperature to 5-[5-(tert-butoxycarbonylamino)-2-pyridyl]-4,5-dihydro-5-hydroxy-1-(3-pyridyl)-1H-pyrazole-3-carboxylic acid ethyl ester (1.205 g) and 4-(dimethylamino)pyridine (344 mg) in N,N-dimethylformamide (30 mL), followed by stirring for 4 hours. The reaction solvent was evaporated under reduced pressure. The residue was partitioned between water and ethyl acetate. The organic layer was sequentially washed water and saturated brine, followed by drying over sodium sulfate anhydrate. After a filtration step, the solvent was evaporated under reduced pressure, and the residue was purified by silica gel column chromatography (hexane - ethyl acetate). The product was further purified by silica gel thin-layer chromatography (hexane - ethyl acetate), to thereby give 5-[5-(tert-butoxycarbonylamino)-2-pyridyl]-1-(3-pyridyl)-1H-pyrazole-3-carboxylic acid ethyl ester as a solid product (506 mg, 43%).

¹H-NMR(400MHz,CDCl₃) δ:1.43(3H,t,J=7.1Hz), 1.52(9H,s), 4.46(2H,q,J=7.1Hz), 6.59(1H,br s), 7.23(1H,s), 7.36(2H,ddd,J=8.1,4.6,0.7Hz), 7.38(2H,d,J=8.1Hz), 7.81(1H,ddd,J=8.1,2.4,1.5Hz), 8.02-8.08(1H,br m), 8.26(1H,d,J=2.7Hz), 8.53(1H,d,J=2.4Hz), 8.59(1H,dd,J=4.9,1.5Hz).
ESI-MS m/z:410(M+H)⁺.

### 6) Title Compound

1N Aqueous sodium hydroxide (3.7 mL) was added at room temperature to a suspension of 5-[5-(tert-butoxycarbonylamino)-2-pyridyl]-1-(3-pyridyl)-1H-pyrazole-3-carboxylic acid ethyl ester (505 mg) in ethanol (20 mL). The resultant mixture was refluxed for 10 minutes, followed by cooling in air. The solvent was evaporated under reduced pressure, and the residue was partitioned between water and ethyl acetate. The aqueous layer was acidified with 5% aqueous citric acid. The solid that precipitated was collected by filtration, to thereby give the title compound as a solid product (357 mg, 75.8%).

¹H-NMR(400MHz,DMSO-d₆)δ:1.48(9H,s), 7.27(1H,s), 7.50(1H,dd,J=8.1,4.9Hz), 7.65(1H,d,J=8.5Hz), 7.79(1H,ddd,J=8.1,2.4,1.5Hz), 7.96(1H,dd,J=8.8,2.4Hz), 8.41(1H,d,J=2.4Hz),8.50(1H,d,J=2.4Hz), 8.60(1H,dd,J=4.9,1.2Hz), 9.71(1H,s).
ESI-MS m/z:382(M+H)⁺.

[Referential Example 21] 5-[5-(tert-Butoxycarbonylamino)-2-pyridyl]-1-(2-pyrazinyl)-1H-pyrazole-3-carboxylic acid

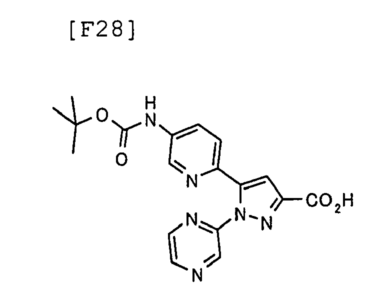

### 1) 5-[5-(tert-Butoxycarbonylamino)-2-pyridyl]-1-(2-pyrazinyl)-1H-pyrazole-3-carboxylic acid ethyl ester

4-[5-(tert-Butoxycarbonylamino)-2-pyridyl]-2,4-dioxobutanoic acid ethyl ester (1.009 g) obtained in step 3) of Referential Example 20 and 2-hydrazinopyrazine (330 mg) obtained in Referential Example 2 were dissolved in ethanol (30 mL). The solution was refluxed for 88 hours, followed by cooling in air. The reaction solvent was evaporated under reduced pressure, and the residue was purified by silica gel column chromatography (methanol - chloroform). The product was further purified by silica gel thin-layer chromatography (methanol - chloroform), to thereby give 5-[5-(tert-butoxycarbonylamino)-2-pyridyl]-1-(2-pyrazinyl)-1H-pyrazole-3-carboxylic acid ethyl ester as an amorphous product (590 mg, 47%).

¹H-NMR(400MHz,CDCl₃)δ:1.43(3H,t,J=7.1Hz), 1.52(9H,s), 4.47(2H,q,J=7.1Hz), 6.55(1H,br s), 7.21(1H,s), 7.51(1H,d,J=8.5Hz), 8.01-8.08(1H,br m), 8.20(1H,dd,J=2.7,0.5Hz), 8.29(1H,dd,J=2.7,1.5Hz), 8.56(1H,d,J=2.7Hz), 9.02(1H,dd,J=1.5,0.5Hz).
ESI-MS m/z:411(M+H)⁺.

### 2) Title Compound

1N Aqueous sodium hydroxide (4.30 mL) was added at room temperature to the above-obtained 5-[5-(tert-butoxycarbonylamino)-2-pyridyl]-1-(2-pyrazinyl)-1H-pyrazole-3-carboxylic acid ethyl ester (589 mg) in ethanol (10 mL), followed by stirring for 2 hours. The reaction solvent was evaporated under reduced pressure, and then water was added to the residue. The resultant mixture was acidified with 5% aqueous citric acid. The solid that precipitated was collected by filtration, to thereby give the title compound (441 mg, 80%).

¹H-NMR(400MHz,DMSO-d₆)δ:1.48(9H,s), 7.31(1H,s), 7.72(1H,d,J=8.8Hz), 7.95(1H,dd,J=8.8,2.4Hz), 8.34(1H,d,J=2.4Hz), 8.49(1H,dd,J=2.4,1.5Hz), 8.74(1H,d,J=2.4Hz), 8.95(1H,d,J=1.5Hz), 9.68(1H,br s).
ESI-MS m/z:383(M+H)⁺.

[Referential Example 22] 5-(6-Methoxy-3-pyridyl)-1-(3-pyridyl)-1H-pyrazole-3-carboxylic acid

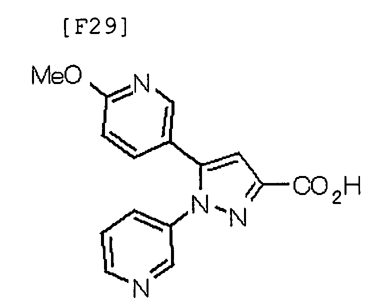

### 1) 3-Acetyl-6-methoxypyridine

1N Aqueous sodium hydroxide (140 mL) was added at room temperature to 6-methoxynicotinic acid methyl ester (20.07 g) in methanol (200 mL), followed by stirring for 16 hours. The solvent of the reaction mixture was evaporated under reduced pressure. 1N Aqueous hydrochloric acid was added to the residue making the pH condition to 4. The solid that precipitated was collected by filtration, to thereby give 6-methoxynicotinic acid (15.12 g, 82%). (ESI-MS m/z:154(M+H)⁺).
N,O-Dimethylhydroxylamine hydrochloride (11.5 g), 1-(3-dimethylaminopropyl)-3-ethylcarbodiimide hydrochloride (41.0 g), 1-hydroxybenzotriazole (14.5 g), and triethylamine (54 mL) were added at room temperature to the obtained 6-methoxynicotinic acid (15.0 g) in dichloromethane (600 mL), followed by stirring for 16 hours. The reaction mixture was partitioned between water and dichloromethane. The aqueous layer was extracted with dichloromethane, and then the organic layers were combined. The combined organic layer was washed with saturated brine, followed by drying over sodium sulfate anhydrate. After a filtration step, the solvent was evaporated under reduced pressure, and the residue was purified by silica gel column chromatography (acetone - chloroform), to thereby give 6-methoxynicotinic acid N-methoxy-N-methylamide as an oily product (19.2 g, quantitative amount). (ESI-MS m/z:197(M+H)⁺).
Methyllithium (0.98M diethyl ether solution, 135 mL) was added dropwise at -78°C to the obtained 6-methoxynicotinic acid N-methoxy-N-methylamide (19.21 g) in tetrahydrofuran (400 mL) over 30 minutes, followed by stirring for 30 minutes. A saturated aqueous solution of ammonium chloride, water, and ethyl acetate were added for partitioning the reaction mixture. The aqueous layer was extracted with ethyl acetate, and then the organic layers were combined. The combined organic layer was washed with saturated brine, followed by drying over sodium sulfate anhydrate. After a filtration step, the solvent was evaporated under reduced pressure. Diethyl ether was added to the residue, and the solid that precipitated was collected by filtration, to thereby give the title compound (10.86 g, 73%).

¹H-NMR(400MHz,CDCl₃)δ:2.57(3H,s), 4.01(3H,s), 6.79(1H,d,J=8.8Hz), 8.14(1H,dd,J=2.5,8.8Hz), 8.78(1H,d,J=2.5Hz).
ESI-MS m/z:152(M+H)⁺.

### 2) 4-(6-Methoxy-3-pyridyl)-2,4-dioxobutanoic acid methyl ester

### [Method A]

Sodium methoxide (0.229 g) was added at room temperature to 3-acetyl-6-methoxypyridine (0.309 g) and dimethyl oxalate (0.484 g) in methanol (15 mL). The resultant mixture was stirred for 1.5 hours, and then at 45°C for 20 hours, followed by cooling in air. The solid that precipitated was collected by filtration, followed by washing with diethyl ether. The solid was dissolved in chloroform and water. Subsequently, 1N aqueous hydrochloric acid was added to the resultant mixture for acidification. The aqueous layer was extracted with chloroform, and then the organic layers were combined. The combined organic layer was washed with saturated brine, followed by drying over sodium sulfate anhydrate. After a filtration step, the solvent was evaporated under reduced pressure, to thereby give 4-(6-methoxy-3-pyridyl)-2,4-dioxobutanoic acid methyl ester as a solid product (0.294 g, 61%).

¹H-NMR(400MHz,CDCl₃)δ:3.94(3H,s), 4.03(3H,s), 6.83(1H,d like, J=8.8Hz), 7.00 (1H, s), 8.15 (1H, dd, J=8.8,2.5Hz), 8.84(1H,d,J=2.5Hz).
ESI-MS m/z:238(M+H)⁺.

### [Method B]

Sodium hydride (55%, 0.185 g) was added at 0°C to 3-acetyl-6-methoxypyridine (0.321 g) in N,N-dimethylformamide (6.0 mL), followed by stirring for 25 minutes. Dimethyl oxalate (0.498 g) was added at 0°C to the reaction mixture, followed by stirring at room temperature for 1 hour. The reaction mixture was partitioned between water and diethyl ether. The aqueous layer was acidified with 1N aqueous hydrochloric acid to pH 4, followed by extraction with ethyl acetate. The aqueous layer was further extracted with ethyl acetate, and then the organic layers were combined. The combined organic layer was washed with saturated brine, followed by drying over sodium sulfate anhydrate. After a filtration step, the solvent was evaporated under reduced pressure, to thereby give 4-(6-methoxy-3-pyridyl)-2,4-dioxobutanoic acid methyl ester as a solid product (0.504 g, quantitative amount).

### 3) 5-(6-Methoxy-3-pyridyl)-1-(3-pyridyl)-1H-pyrazole-3-carboxylic acid methyl ester

3-Hydrazinopyridine (3.45 g) obtained in Referential Example 1 was added at room temperature to 4-(6-methoxy-3-pyridyl)-2,4-dioxobutanoic acid methyl ester (6.80 g) in methanol (120 mL). The resultant mixture was refluxed for 30 minutes, followed by cooling in air. Acetic acid (6.5 mL) was added to the resultant mixture. The resultant mixture was refluxed for 14 hours, followed by cooling in air. The solvent of the reaction mixture was evaporated under reduced pressure. Water and chloroform were added to the residue. 1N Aqueous sodium hydroxide was added to the resultant mixture for neutralization. The aqueous layer was further extracted with chloroform, and the organic layers were combined. The combined organic layer was washed with saturated brine, followed by drying over sodium sulfate anhydrate. After a filtration step, the solvent was evaporated under reduced pressure, and the residue was purified by silica gel column chromatography (acetone - chloroform), to thereby give 5-(6-methoxy-3-pyridyl)-1-(3-pyridyl)-1H-pyrazole-3-carboxylic acid methyl ester as an oily product (4.53 g, 51%).

¹H-NMR(400MHz,CDCl₃)δ:3.99(3H,s), 4.03(3H,s), 6.71(1H,d,J=8.5Hz), 7.06(1H,s), 7.32-7.35(2H,m), 7.72-7.80(1H,m), 8.09(1H,d,J=8.5Hz), 8.58(1H,d,J=2.4Hz), 8.62(1H,dd,J=2.4,4.8Hz).
FAB-MS m/z:311(M+H)⁺.

### 4) Title Compound

Lithium hydroxide monohydrate (0.730 g) was added at room temperature to 5-(6-methoxy-3-pyridyl)-1-(3-pyridyl)-1H-pyrazole-3-carboxylic acid methyl ester (4.89 g) in a mixture of tetrahydrofuran (30 mL), methanol (15 mL), and water (30 mL), followed by stirring for 1.5 hours. The reaction solvent was evaporated under reduced pressure. 1N Aqueous hydrochloric acid was added to the residue making the pH condition to 5 to 6. The solid that precipitated was collected by filtration, to thereby give the title compound (3.278 g, 70%).

¹H-NMR(400MHz,DMSO-d₆)δ:3.86(3H,s), 6.83(1H,d,J=8.8Hz), 7.16(1H,s), 7.52-7.60(2H,m), 7.83-7.91(1H,m), 8.17(1H,d,J=2.4Hz), 8.58(1H,d,J=2.4Hz), 8.64(1H,dd,J=4.9,1.5Hz).
ESI-MS m/z:297(M+H)⁺.

[Referential Example 23] 5-(6-Methyl-3-pyridazinyl)-1-(3-pyridyl)-1H-pyrazole-3-carboxylic acid

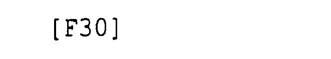

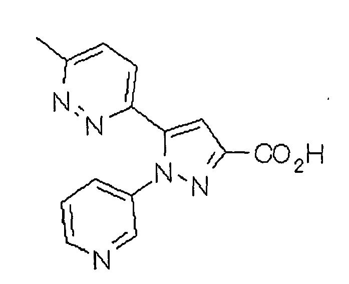

### 1) 3-Acetyl-6-methylpyridazine

Methylmagnesium iodide (2.0M diethyl ether solution, 30 mL) was added dropwise at -15°C to 6-methyl-3-pyridazinecarbonitrile (6.00 g) in a mixture of diethyl ether (100 mL) benzene (20 mL), followed by stirring for 1.5 hours. 1N Aqueous hydrochloric acid (60 mL) was added to the reaction mixture, followed by stirring for 15 minutes. Subsequently, the resultant mixture was partitioned. The aqueous layer was alkalinized with a saturated aqueous solution of sodium hydrogencarbonate, followed by extraction with dichloromethane. The organic layer was dried with magnesium sulfate anhydrate. After a filtration step, the solvent was evaporated under reduced pressure, and the residue was purified by silica gel column chromatography (ethyl acetate - hexane), to thereby give 3-acetyl-6-methylpyridazine as a solid product (4.84 g, 71%).

¹H-NMR(400MHz,CDCl₃)δ:2.82(3H,s), 2.88(3H,s), 7.47(1H,d,J=8.5Hz), 8.03(1H,d,J=8.5Hz).
LC-MS m/z:137(M+H)⁺.

### 2) 4-(6-Methyl-3-pyridazinyl)-2,9-dioxobutanoic acid methyl ester

Lithium bis(trimethylsilyl)amide (1.0M tetrahydrofuran solution, 33 mL) was added at -78°C to 3-acetyl-6-methylpyridazine (4.03 g) in tetrahydrofuran (100 mL), followed by stirring for 1 hour. Dimethyl oxalate (7.0 g) in tetrahydrofuran (30 mL) was added to the reaction mixture at -78°C, followed by stirring at 0°C for 2 hours. The reaction mixture was partitioned between water and diethyl ether. The aqueous layer was acidified with 1N aqueous hydrochloric acid to pH 4, followed by extraction with chloroform. The aqueous layer was further extracted with chloroform, the organic layers were combined. The combined organic layer was washed with saturated brine, followed by drying over sodium sulfate anhydrate. After a filtration step, the solvent was evaporated under reduced pressure, to thereby give 4-(6-methyl-3-pyridazinyl)-2,4-dioxobutanoic acid methyl ester as a solid product (5.42 g, 82%).

¹H-NMR(400MHz,CDCl₃)δ:2.78(3H,s), 3.89(3H,s), 7.47(1H,d,J=8.5Hz), 7.84(1H,s), 8.07(1H,d,J=8.5Hz).
ESI-MS m/z:223(M+H)⁺.

### 3) 5-(6-Methyl-3-pyridazinyl)-1-(3-pyridyl)-1H-pyrazole-3-carboxylic acid methyl ester

3-Hydrazinopyridine (3.00 g) obtained in Referential Example 1 was added at room temperature to 14-(6-methyl-3-pyridazinyl)-2,4-dioxobutanoic acid methyl ester (5.42 g) in methanol (140 mL). The resultant mixture was refluxed for 30 minutes, followed by cooling in air. Subsequently, acetic acid (5.6 mL) was added to the resultant mixture, followed by refluxing for 14 hours. Concentrated hydrochloric acid (1.2 mL) was further added to the resultant mixture. The resultant mixture was refluxed for 24 hours, followed by cooling in air. 1N Aqueous sodium hydroxide was added to the reaction mixture making the pH condition to 4. The solvent of the reaction mixture was evaporated under reduced pressure. The residue was partitioned between chloroform and water. The aqueous layer was extracted with chloroform, the organic layers were combined. The combined organic layer was washed with saturated brine, followed by drying over sodium sulfate anhydrate. After a filtration step, the solvent was evaporated under reduced pressure, and the residue was purified by silica gel column chromatography (methanol - chloroform), to thereby give 5-(6-methyl-3-pyridazinyl)-1-(3-pyridyl)-1H-pyrazole-3-carboxylic acid methyl ester as a solid product (2.98 g, 41%).

¹H-NMR(400MHz,CDCl₃)δ:2.73(3H,s), 4.00(3H,s), 7.32-7.43(3H,m), 7.46(1H,d,J=8.7Hz), 7.86-7.92(1H,m), 8.53(1H,d like, J=2.5Hz), 8.62(1H,dd,J=4.9,1.5Hz)
FAB-MS m/z:296(M+H)⁺.

### 4) Title Compound

1N Aqueous sodium hydroxide (25 mL) was added to a suspension of 5-(6-methyl-3-pyridazinyl)-1-(3-pyridyl)-1H-pyrazole-3-carboxylic acid methyl ester (2.98 g) in a mixture of tetrahydrofuran (20 mL) and methanol (100 mL). The resultant mixture was stirred at 40°C for 6 hours, followed by cooling in air. The resultant mixture was acidified with 1N aqueous hydrochloric acid to pH 4. The reaction solvent was evaporated under reduced pressure. Diethyl ether was added to the residue, and then the solid that precipitated was collected by filtration, to thereby give the title compound as a solid product (2.84 g, quantitative amount).

¹H-NMR(400MHz,DMSO-d₆)δ:2.61(3H,s), 7.50-7.57(2H,m), 7.68(1H,d,J=8.6Hz), 7.85-7.92(1H,m), 7.96(1H,d,J=8.6Hz), 8.56(1H,d like,J=2.4Hz), 8.62(1H,dd,J=3.4,1.5Hz).
FAB-MS m/z:282(M+H)⁺.

[Referential Example 24] 5-(5-Methyl-2-pyridyl)-1-(2-pyridyl)-1H-pyrazole-3-carboxylic acid

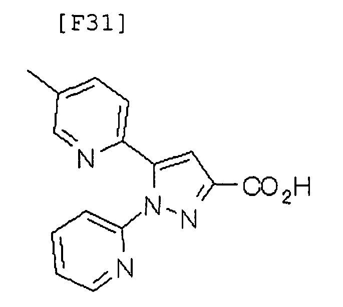

### 1) 5-(5-Methyl-2-pyridyl)-1-(2-pyridyl)-1H-pyrazole-3-carboxylic acid ethyl ester

In a manner similar to that employed in Method A-step 3) of Referential Example 9, 5-(5-methyl-2-pyridyl)-1-(2-pyridyl)-1H-pyrazole-3-carboxylic acid ethyl ester (1.87 g) was prepared from 4-(5-methyl-2-pyridyl)-2,4-dioxobutanoic acid ethyl ester (3.30 g) obtained in Method A-step 2) of Referential Example 9 and 2-hydrazinopyridine (2.0 g).

¹H-NMR(400MHz,CDCl₃)δ:1.42(3H,t,J=7.08Hz), 2.33(3H,s), 4.45(2H,q,J=7.08Hz), 7.20(1H,s), 7.25(1H,m), 7.33(1H,d,J=7.93Hz), 7.52(1H,d,J=7.93Hz), 7.76(1H,d,J=8.05Hz), 7.85(1H,dd,J=8.05,1.84Hz), 8.28(2H,s).
ESI-MS m/z:309(M+H)⁺.

### 2) Title Compound

5-(5-Methyl-2-pyridyl)-1-(2-pyridyl)-1H-pyrazole-3-carboxylic acid ethyl ester (1.86 g) was treated in a manner similar to that employed in Method A-step 4) of Referential Example 9, to thereby give the title compound (1.17 g, 30% (2 steps)).

¹H-NMR(400MHz,CDCl₃)δ:2.36(3H,s), 7.23(1H,s), 7.29(1H,m), 7.37(1H,d,J=7.93Hz), 7.55(1H,d,J=7.93Hz), 7.79(1H,d,J=8.06Hz), 7.86(1H,dt,J=8.06,1.83Hz), 8.27(1H,ddd,J=4.88,1.83,0.86Hz), 8.35(1H,s).
ESI-MS m/z:281(M+H)⁺.

[Referential Example 25] 5-[5-(tert-Butoxycarbonylamino)-2-pyrazinyl]-1-(3-pyridyl)-1H-pyrazole-3-carboxylic acid

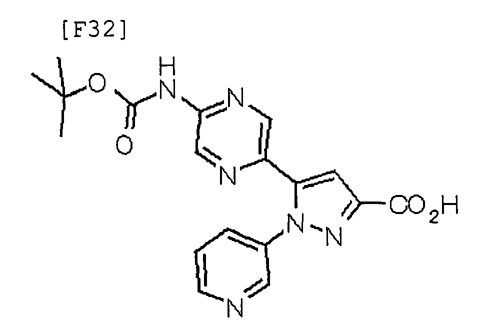

### 1) 5-[5-(tert-Butoxycarbonylamino)-2-pyrazinyl]-1-(3-pyridyl)-1H-pyrazole-3-carboxylic acid ethyl ester

5-(5-Carboxy-2-pyrazinyl)-1-(3-pyridyl)-1H-pyrazole-3-carboxylic acid ethyl ester (5.34 g) obtained in step 1) of Referential Example 7 was suspended in 1,4-dioxane (107 mL), and triethylamine (2.41 mL), diphenylphosphorylazide (3.73 mL), and tert-butanol (3.31 mL) were added to the suspension at room temperature. The mixture was stirred for 20 minutes at 100°C, followed by cooling in air. The reaction solvent was evaporated under reduced pressure, and the residue was purified through silica gel column chromatography (ethyl acetate - chloroform), to thereby give 5-[5-(tert-butoxycarbonylamino)-2-pyrazinyl]-1-(3-pyridyl)-1H-pyrazole-3-carboxylic acid ethyl ester as a solid product (4.63 g, 72%).

¹H-NMR(400MHz,CDCl₃)δ:1.42-1.46(3H,m), 1.53(9H,s), 4.45-4.50(2H,m), 7.29 (1H, d, J=0.5Hz), 7.37-7.44 (2H,m), 7.82-7.85(1H,m), 8.35-8.36(1H,m), 8.56(1H,d,J=2.4Hz), 8.63(1H,dd,J=4.9,1.2Hz), 9.13(1H,d,J=1.2Hz).
ESI-MS m/z:411(M+H)⁺.

### 2) Title Compound

5-[5-(tert-Butoxycarbonylamino)-2-pyrazinyl]-1-(3-pyridyl)-1H-pyrazole-3-carboxylic acid ethyl ester (0.336 g) was suspended in a mixture of methanol (6.7 mL) and tetrahydrofuran (6.7 mL). 1N Sodium hydroxide (2.05 mL) was added to the suspension at room temperature, and the mixture was stirred for 3 hours. The reaction mixture was neutralized with 1N aqueous hydrochloric acid (2.05 mL), and the mixture was partitioned between water and methanol-chloroform (1:10). The solvent of the organic layer was evaporated under reduced pressure, to thereby give the title compound as a solid product (0.286 g, 91%).

¹H-NMR(400MHz,DMSO-d₆) δ:1.47(9H,s), 7.45(1H,s), 7.51(1H,dd,J=8.2,4.8Hz), 7.83-7.86(1H,m), 8.57(1H,d,J=2.4Hz), 8.61-8.62(1H,m), 8.68(1H,m), 8.81(1H,m), 10.38(1H,s), 13.13(1H,br s).
EI-MSm/z:382 (M⁺).

[Referential Example 26] 5-(5-Methyl-2-pyridyl)-1-(2-pyrimidinyl)-1H-pyrazole-3-carboxylic acid

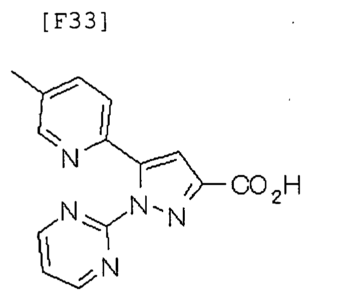

### 1) 5-(5-Methyl-2-pyridyl)-1-(2-pyrimidinyl)-1H-pyrazole-3-carboxylic acid ethyl ester

4-(5-Methyl-2-pyridyl)-2,4-dioxobutanoic acid ethyl ester (2.14 g) obtained in Method A-step 2) of Referential Example 9 and 2-hydrazinopyrimidine (1.00 g) obtained in Referential Example 3 were suspended in ethanol (43 mL), and acetic acid (2.60 mL) was added to the suspension at room temperature. The mixture was refluxed for 16 hours, followed by cooling in air. Concentrated hydrochloric acid (2.9 mL) was added to the reaction mixture, and the resultant mixture was refluxed for 110 minutes, followed by cooling in air. The reaction mixture was partitioned between saturated aqueous sodium hydrogencarbonate and chloroform, and the organic layer was dried over sodium sulfate anhydrate. After a filtration step, the solvent was evaporated under reduced pressure, and the residue was purified through silica gel column chromatography (acetone - toluene), to thereby give 5-(5-methyl-2-pyridyl)-1-(2-pyrimidinyl)-1H-pyrazole-3-carboxylic acid ethyl ester as a solid product (1.22 g, 43%).

¹H-NMR(400MHz,CDCl₃)δ:1.42(3H,t,J=7.2Hz), 2.34(3H,s), 4.45(2H,q,J=7.2Hz), 7.22(1H,s), 7.29-7.31(1H,m), 7.44(1H,d,J=8.1Hz), 7.54(1H,dd,J=8.1,2.2Hz) 8.21-8.22(1H,m), 8.72(2H,d,J=4.9Hz).
EI-MSm/z:309(M⁺).

### 2) Title Compound

5-(5-Methyl-2-pyridyl)-1-(2-pyrimidinyl)-1H-pyrazole-3-carboxylic acid ethyl ester (1.21 g) was dissolved in tetrahydrofuran (24 mL), and a solution of lithium hydroxide monohydrate (0.181 g) in water (12 mL) was added dropwise to the solution at room temperature, followed by stirring for 3 hours. The reaction mixture was neutralized with 1N aqueous hydrochloric acid (4.30 mL), and the mixture was partitioned between water and a methanol-chloroform (1:10) solvent mixture. The solvent of the organic layer was evaporated under reduced pressure, and the formed solid was collected through filtration by use of diethyl ether, to thereby give the title compound (1.01 g, 92%).

¹H-NMR(400MHz,DMSO-d₆)δ:2.27(3H,s), 7.31(1H,s), 7.61-7.72(3H,m), 8.11(1H,s), 8.86(2H,d,J=4.9Hz), 13.11(1H,br s).
EI-MSm/z:281 (M⁺).

[Referential Example 27] 4-Methoxypiperidine hydrochloride
4-Methoxypiperidine-1-carboxylic acid tert-butyl ester (5.34 g) obtained in step 1) of Referential Example 11 was dissolved in 1,4-dioxane (10 mL), and 4N HCl-dioxane (10 mL) was added to the solution at room temperature, followed by stirring for 30 minutes. 4N HC1-dioxane (20 mL) was added to the reaction mixture, and the resultant mixture was stirred for 30 minutes. The reaction solvent was evaporated under reduced pressure, and the resultant solid was collected through filtration by use of ethyl acetate, to thereby give the title compound (3.55 g).

¹H-NMR(400MHz,DMSO-d₆)δ:1.68(2H,m), 1.93(2H,m), 2.91(2H,m), 3.08(2H,m), 3.23(3H,s), 3.42(1H,q,J=3.90Hz).

[Referential Example 28] (3S)-3-Fluoropyrrolidine hydrochloride

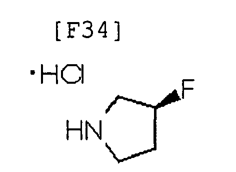

### 1) (3S)-Fluoropyrrolidine-1-carboxylic acid tert-butyl ester

(3R)-Hydroxypyrrolidine-1-carboxylic acid tert-butyl ester (2.62 g) was dissolved in dichloromethane (50 mL), and diethylaminosulfur trifluoride (2.22 mL) was added to the solution at -78°C, followed by stirring for 70 minutes at room temperature. The reaction mixture was poured to ice-water for partitioning the mixture, and the organic layer was dried over magnesium sulfate anhydrate. After a filtration step, the solvent was removed under reduced pressure, and the residue was purified through silica gel column chromatography (hexane - ethyl acetate), to thereby give (3S)-fluoropyrrolidine-1-carboxylic acid tert-butyl ester as an oily product (676 mg, 26%).

¹H-NMR(400MHz,CDCl₃)δ:1.45(9H,s), 2.17-2.26(1H,m), 3.52-3.68(5H,m), 5.20(1H,dt,J=52.7,3.4Hz).

### 2) Title Compound

The above (3S)-3-fluoropyrrolidine-1-carboxylic acid tert-butyl ester (600 mg) was dissolved in dichloromethane (10 mL), and 4N HCl in dioxane (5 mL) was added to the solution at room temperature, followed by stirring for 1 hour. Diethyl ether was added to the reaction mixture, and the precipitated solid was collected through filtration, to thereby give the title compound (341 mg, 86%).

¹H-NMR(400MHz,DMSO-d₆)δ:2.00-2.26(2H,m), 3.15-3.56(4H,m), 5.43(1H,dt,J=52.9,3.8Hz), 9.83(2H,brs).

[Referential Example 29] 4-Fluoromethylpiperidine hydrochloride

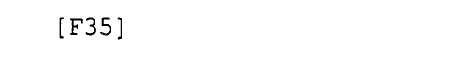

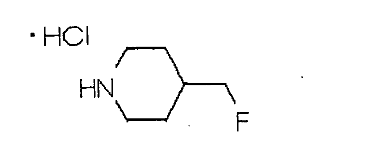

### 1) 4-Fluoromethylpiperidine-1-carboxylic acid tert-butyl ester

[bis(2-Methoxyethyl)amino]sulfur trifluoride (1.2 mL) and [bis(2-methoxyethyl)amino]sulfur trifluoride (50% tetrahydrofuran solution, 3 mL) were added dropwise, under cooling with ice, to a solution of 4-hydroxymethylpiperidine-1-carboxylic acid tert-butyl ester (1.17 g) in dichloromethane (8 mL), and the mixture was stirred for 17 hours at room temperature. Water, saturated aqueous sodium hydrogencarbonate, and ethyl acetate were added to the reaction mixture for partitioning the mixture, and the organic layer was dried over magnesium sulfate anhydrate. After a filtration step, the solvent was evaporated under reduced pressure, and the residue was purified through silica gel column chromatography (hexane - ethyl acetate), to thereby give 4-fluoromethylpiperidine-1-carboxylic acid tert-butyl ester as a solid product (597 mg, 51%).

¹H-NMR(400MHz,CDCl₃)δ:1.22(2H,m), 1.46(9H,s), 1.70(2H,d,J=12.94Hz), 1.83(1H,m), 2.71(2H,br), 4.13(2H,br), 4.21(1H,d,J=6.10Hz), 4.32(1H,d,J=6.10Hz).

### 2) Title Compound

4-Fluoromethylpiperidine-1-carboxylic acid tert-butyl ester (635 mg) was treated in a manner similar to that employed in Referential Example 28, to thereby give the title compound as a solid product (526 mg, quantitative amount).

¹H-NMR(400MHz,CDCl₃)δ:1.76(3H,m), 1.96(2H,d,J=13.4Hz), 2.90(2H,br), 3.54(2H,d,J=12.1Hz), 4.26(1H,d,J=6.10Hz), 4.37(1H,d,J=6.10Hz).

[Referential Example 30] (3R)-3-Fluoropiperidine hydrochloride

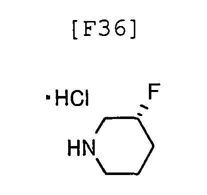

### [Method A]

(2S)-2-Hydroxymethylpyrrolidine-1-carboxylic acid tert-butyl ester (3.00 g) and diethylaminosulfur trifluoride (2.95 mL) were treated in a manner similar to that employed in step 1) of Referential Example 28, to thereby give (3R)-3-fluoropiperidine-1-carboxylic acid tert-butyl ester (346 mg) as an oily product. The ester compound was dissolved in dichloromethane (20 mL), and 4N HCl in dioxane (7 mL) was added to the solution at room temperature, followed by stirring for 30 minutes. Diethyl ether was added to the reaction mixture, and the precipitated product was collected through filtration; to thereby give the title compound as a solid product (162 mg, 8% (two steps)).

¹H-NMR(400MHz,DMSO-d₆)δ:1.65-1.93(4H,m), 3.03-3.20(4H,m), 4.97(1H,d,J=45.9Hz), 9.34(2H,brs).

### [Method B]

Diethylaminosulfur trifluoride (20.6 mL) was added at 0°C to a solution of (2S)-1-benzyl-2-hydroxymethylpyrrolidine (19.87 g) in dichloromethane (300 mL), and the mixture was stirred for 90 minutes at room temperature. The reaction mixture was added to saturated aqueous sodium hydrogencarbonate, and the mixture was extracted with dichloromethane. The organic layer was dried over magnesium sulfate anhydrate. After a filtration step, the solvent was evaporated under reduced pressure, and the residue was purified through silica gel column chromatography (ethyl acetate - hexane), to thereby give a mixture of (2S)-1-benzyl-2-fluoromethylpyrrolidine and (3R)-1-benzyl-3-fluoropiperidine as an oily product (14.56 g, 73%). The mixture (7.25 g) and 1-chloroethyl chloroformate (4.50 mL) were dissolved in dichloromethane (100 mL), and the solution was refluxed for 1.5 hours, followed by cooling in air. The reaction solvent was evaporated under reduced pressure, and the residue was dissolved in methanol (50 mL). The solution was refluxed for 45 minutes, followed by cooling in air. The reaction solvent was evaporated under reduced pressure, and the residue was crystallized from methanol-diethyl ether, to thereby give the title compound (1.582 g, 30%).

¹H-NMR(400MHz,DMSO-d₆)δ:1.72-1.81(4H,m), 2.91-3.33(4H,m), 4.98(1H,d,J=46.1Hz), 9.33(2H,br s).

[Referential Example 31] (2S)-2-Fluoromethylpyrrolidine hydrochloride

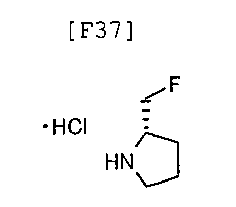

### 1) (2S)-1-Benzoyl-2-hydroxymethylpyrrolidine

(2S)-Hydroxymethylpyrrolidine (3.00 mL) and benzoyl chloride (6.92 mL) were dissolved in a mixture of dichloromethane (100 mL) and water (100 mL), and sodium hydrogencarbonate (7.51 g) was added to the solution at room temperature, followed by stirring for 1.5 hours. Dichloromethane was added to the reaction mixture for partitioning the mixture, and the organic layer was dried over sodium sulfate anhydrate. After a filtration step, the solvent was evaporated under reduced pressure, and the residue was dissolved in tetrahydrofuran (120 mL) and water (60 mL). Lithium hydroxide monohydrate (6.25 g) was added to the reaction mixture at room temperature, and the resultant mixture was stirred overnight. The reaction mixture was partitioned, and the organic layer was dried over sodium sulfate anhydrate. After a filtration step, the solvent was evaporated under reduced pressure, to thereby give (2S)-1-benzoyl-2-hydroxymethylpyrrolidine as an oily product (5.79 g, 95%).

¹H-NMR(400MHz,CDCl₃)δ:1.60-2.27(4H,m), 3.43-3.52(2H,m), 3.71-3.82(2H,m), 4.40(1H,d,J=7.3Hz), 4.92(1H,s), 7.40-7.52(5H,m).

### 2) (2S)-1-Benzoyl-2-fluoromethylpyrrolidine

(2S)-1-Benzoyl-2-hydroxymethylpyrrolidine (1.50 g) was dissolved in dichloromethane (50 mL), and diethylaminosulfur trifluoride (1.93 mL) was added to the solution under cooling with ice, followed by stirring overnight and at room temperature for 6.5 hours. The reaction mixture was partitioned between saturated aqueous sodium hydrogencarbonate and chloroform, and the organic layer was dried over sodium sulfate anhydrate. After a filtration step, the solvent was evaporated under reduced pressure, and the residue was purified through silica gel column chromatography (hexane - ethyl acetate), to thereby give (2S)-1-benzoyl-2-fluoromethylpyrrolidine as an oily product (772 mg, 51%).

¹H-NMR(400MHz,CDCl₃)δ:1.79-2.14(4H,m), 3.49(2H,br s), 4.44-4.90(3H,m), 7.38-7.54(5H,m).

[Referential Example 32] 2-[5-(5-Methyl-2-pyridyl)-1-(3-pyridyl)-1H-pyrazole-3-carbonyl]pyrazolidine-1-carboxylic acid tert-butyl ester

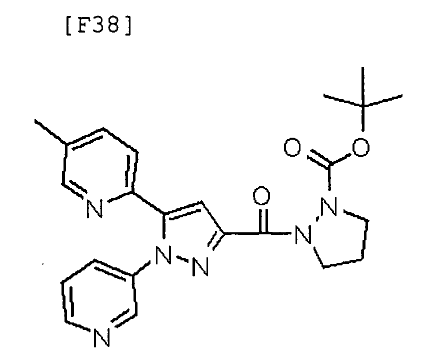

To a solution of 5-(5-methyl-2-pyridyl)-1-(3-pyridyl)-1H-pyrazole-3-carboxylic acid (0.372 g) obtained in Referential Example 9 in dichloromethane (10 mL), at room temperature, 1-hydroxybenzotriazole (0.123 g), 1-(3-dimethylaminopropyl)-3-ethylcarbodiimide hydrochloride (0.580 g), triethylamine (0.739 mL), and pyrazolidine-1-carboxylic acid tert-butyl ester (0.349 g, Y. Endo et al., Bioorg. Med. Chem., 2002, 10, 953) were added, and the mixture was stirred for 4 days. The reaction mixture was partitioned between water and chloroform, and the aqueous layer was extracted with chloroform. The organic layers were combined, washed with saturated brine, and dried over sodium sulfate anhydrate. After a filtration step, the solvent was evaporated under reduced pressure, and the residue was purified through silica gel column chromatography (methanol - chloroform), to thereby give the title compound as an oily product (0.459 g, 80%).

¹H-NMR(400MHz,CDCl₃)δ:1.35(9H,br), 2.05-2.20(2H,br), 2.33(3H,s), 3.15-3.60(2H,br), 4.00-4.40(2H,br), 7.18(1H,br), 7.29-7.40(2H,m), 7.49-7.56(1H,m), 7.76-7.80(1H,m), 8.30(1H,d like,J=1.5Hz), 8.49(1H,br), 8.55(1H,dd like,J=4.0,1.5Hz).
FAB-MS m/z:435(M+H)⁺.

[Referential Example 33] 4-Methoxy-4-methylpiperidine hydrochloride

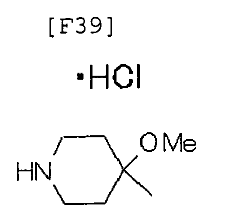

### 1) 4-Hydroxy-4-methylpiperidine-1-carboxylic acid tert-butyl ester

In an argon atmosphere, methyllithium (0.98M diethyl ether solution, 64.5 mL) was added dropwise to a suspension of 4-oxopiperidine-1-carboxylic acid tert-butyl ester (12.0 g) in diethyl ether (120 mL) at -78°C over 15 minutes, and the mixture was stirred for 30 minutes, at 0°C for 15 minutes, and at room temperature for 30 minutes. Water, saturated brine, and chloroform were added for partitioning the reaction mixture, and the organic layer was dried over sodium sulfate anhydrate. After a filtration step, the solvent was evaporated under reduced pressure, and the residue was purified through silica gel column chromatography (ethyl acetate - hexane), to thereby give 4-hydroxy-4-methylpiperidine-1-carboxylic acid tert-butyl ester as an oily product (12.2 g, 94%).

¹H-NMR(400MHz,CDCl₃)δ:1.26(3H,s), 1.46(9H,s), 1.49-1.55(4H,m), 3.20-3.27(2H,m), 3.69-3.72(2H,m).
EI-MSm/z:215 (M⁺).

### 2) 4-Methoxy-4-methylpiperidine-1-carboxylic acid tert-butyl ester

In an argon atmosphere, a solution of the above 4-hydroxy-4-methylpiperidine-1-carboxylic acid tert-butyl ester (8.61 g) in N,N-dimethylformamide (86 mL) was added dropwise to a suspension of sodium hydride (55%, 2.09 g) in N,N-dimethylformamide (86 mL) at 0°C over 15 minutes, and the mixture was stirred for 1 hour. A solution of methyl iodide (2.99 mL) in N,N-dimethylformamide (10 mL) was added dropwise to the reaction mixture, and the mixture was stirred for 15 minutes and at room temperature for 6 hours. The reaction mixture was partitioned between water and ethyl acetate, and the organic layer was dried over sodium sulfate anhydrate. After a filtration step, the solvent was evaporated under reduced pressure, and the residue was purified through silica gel column chromatography (ethyl acetate - hexane), to thereby give 4-methoxy-4-methylpiperidine-1-carboxylic acid tert-butyl ester as an oily product (6.79 g, 74%).

¹H-NMR(400MHz,CDCl₃)δ:1.15(3H,s), 1.37-1.49(2H,m), 1.45(9H,s), 1.69-1.73(2H,m), 3.01-3.22(2H,m), 3.18(3H,s), 3.67-3.70(2H,m).
FAB-MS m/z:230(M+H)⁺.

### 3) Title Compound

In an argon atmosphere, 4N HCl-dioxane (45 mL) was added at 0°C to a solution of 4-methoxy-4-methylpiperidine-1-carboxylic acid tert-butyl ester (6.78 g) in dichloromethane (136 mL), and the mixture was stirred for 5 hours at room temperature. The solvent of the reaction mixture was evaporated under reduced pressure, and the resultant solid was washed with diethyl ether and then collected through filtration, to thereby give the title compound (4.04 g, 82%).

¹H-NMR(400MHz,DMSO-d₆)δ:1.13(3H,s), 1.63-1.71(2H,m), 1.81-1.86(2H,m), 2.86-2.93(2H,m), 3.01-3.06(2H,m), 3.11(3H,s).
EI-MSm/z:129(M⁺).

[Referential Example 34] 3-Methoxypiperidine hydrochloride

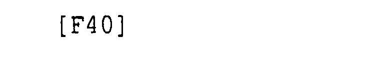

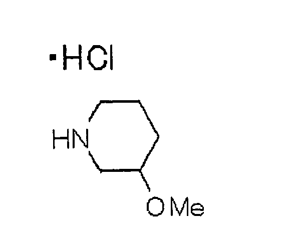

### 1) 3-Hydroxypiperidine-1-carboxylic acid tert-butyl ester

Triethylamine (15.2 mL) and a solution of di-tert-butoxydicarbonate (11.9 g) in methanol (50 mL) were added to a solution of 3-hydroxypiperidine (5.00 g) in methanol (50 mL) at room temperature, and the mixture was stirred for 15 hours. The solvent of the reaction mixture was evaporated under reduced pressure, and the residue was purified through silica gel column chromatography (ethyl acetate - chloroform), to thereby give 3-hydroxypiperidine-1-carboxylic acid tert-butyl ester as a solid product (9.86 g, 99%).

¹H-NMR(400MHz,CDCl₃)δ:1.36-1.55(2H,m), 1.45(9H,s), 1.71-1.78(1H,m), 1.88(1H,m), 3.02-3.13(2H,m), 3.52(1H,m), 3.72-3.76(2H,m).
EI-MS m/z:201 (M⁺).

### 2) 3-Methoxypiperidine-1-carboxylic acid tert-butyl ester

3-Hydroxypiperidine-1-carboxylic acid tert-butyl ester (9.86 g) and methyl iodide (3.66 mL) were treated in a manner similar to that employed in step 2) of Referential Example 33, to thereby give 3-methoxypiperidine-1-carboxylic acid tert-butyl ester as an oily product (9.24 g, 88%).

¹H-NMR(400MHz,CDCl₃)δ:1.30-1.54(2H,m), 1.46(9H,s), 1.72-1.73(1H,m), 1.92(1H,m), 3.04-3.21(3H,m), 3.37(3H,s), 3.55-3.74 (2H,m).
EI-MS m/z:215(M⁺).

### 3) Title Compound

3-Methoxypiperidine-1-carboxylic acid tert-butyl ester (9.24 g) was treated in a manner similar to that employed in step 3) of Referential Example 33, to thereby give the title compound as a solid product (6.36 g, 98%).

¹H-NMR(400MHz,DMSO-d₆)δ:1.58-1.61(2H,m), 1.76-1.81(2H,m), 2.88-2.94(3H,m), 3.09-3.13(1H,m), 3.28(3H,s), 3.51-3.55(1H,m).
EI-MS m/z:115(M⁺).

[Referential Example 35] 5-(5-Methyl-2-pyridyl)-1-(2-pyrazinyl)-1H-pyrazole-3-carboxylic acid

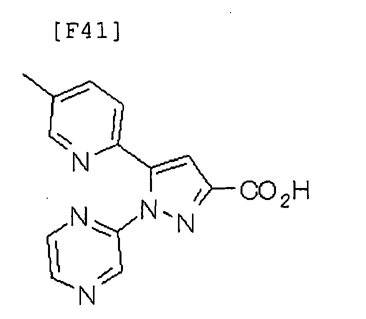

### 1) 5-(5-Methyl-2-pyridyl)-1-(2-pyrazinyl)-1H-pyrazole-3-carboxylic acid ethyl ester

2-Hydrazinopyrazine (2.363 g) obtained in Referential Example 2 and a solution of 4-(5-methyl-2-pyridyl)-2,4-dioxobutanoic acid ethyl ester (5.04 g) obtained in Method A-step 2) of Referential Example 9 in ethanol (100 mL) were refluxed for 2 hours, followed by cooling in air. To the reaction mixture, concentrated hydrochloric acid (2.65 mL) was added, and the mixture was refluxed for 1 hour, followed by cooling in air. The reaction mixture was neutralized with 1N aqueous sodium hydroxide and then extracted with chloroform. Further, the aqueous layer was extracted with chloroform, and the organic layers were combined, followed by washing with saturated brine and drying over sodium sulfate anhydrate. After a filtration step, the solvent was evaporated under reduced pressure, and the residue was purified through silica gel column chromatography (acetone - chloroform), to thereby give 5-(5-methyl-2-pyridyl)-1-(2-pyrazinyl)-1H-pyrazole-3-carboxylic acid ethyl ester as an amorphous product (1.336 g, 22%).

¹H-NMR(400MHz,CDCl₃)δ:1.43(3H,t,J=6.9Hz), 2.34(3H,s), 4.47(2H,q,J=6.9Hz), 7.23(1H,s), 7.24-7. 30 (1H,m), 7.46(1H,d,J=7.9Hz), 7.56(1H,dd,J=7.9,1.5Hz), 8.21(1H,br s), 8.28-8.32(1H,m), 8.56(1H,d,J=2.4Hz), 9.02(1H,d like, J=1.5Hz).
FAB-MS m/z:310(M+H)⁺.

### 2) Title Compound

5-(5-Methyl-2-pyridyl)-1-(2-pyrazinyl)-1H-pyrazole-3-carboxylic acid ethyl ester (1.470 g) was dissolved in a mixture of tetrahydrofuran (20 mL), ethanol (10 mL), and water (5 mL), and, at room temperature, lithium hydroxide monohydrate (0.222 g) was added to the solution, followed by stirring for 2.5 hours. The reaction mixture was acidified to pH 5 to 6 with 1N aqueous hydrochloric acid, and a chloroform-methanol (15:1) solvent mixture was added for partitioning the resultant mixture. The aqueous layer was extracted with a chloroform-methanol (15:1) solvent mixture, and the organic layers were combined and then dried over sodium sulfate anhydrate. After a filtration step, the solvent was evaporated under reduced pressure, to thereby give the title compound as a solid product (1.315 g, 98%).

¹H-NMR(400MHz,CDCl₃)δ:2.35(3H,s), 7.26(1H,s), 7.48(1H,d,J=8.1Hz), 7.58(1H,d,J=8.1,1.0Hz), 8.24(1H,br s), 8.29(1H,t like,J=2.4Hz), 8.58(1H,d,J=2.4Hz), 9.04(1H,s like).
FAB-MS m/z:282(M+H)⁺.

[Referential Example 36] 5-(6-Methoxy-3-pyridazinyl)-1-(3-pyridyl)-1H-pyrazole-3-carboxylic acid

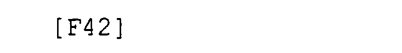

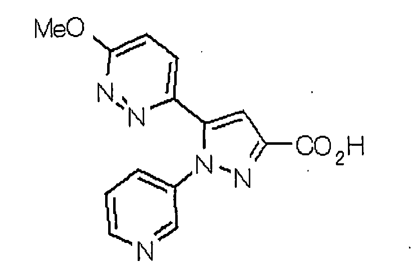

### 1) 3-Methoxypyridazine

In a hydrogen atmosphere, at room temperature, 10% palladium-carbon (wet. 3.12 g) was added to a solution of 3-chloro-6-methoxypyridazine (30.0 g) in methanol (200 mL), and the mixture was stirred for 17 hours. The reaction mixture was subjected to filtration, and the solvent of the filtrate was evaporated under reduced pressure. The residue was purified through silica gel column chromatography (ethyl acetate), to thereby give 3-methoxypyridazine as an oily product (16.3 g, 71%).

¹H-NMR(400MHz,CDCl₃)δ:4.14(3H,s), 6.98(1H,d,J=9.0Hz), 7.37 (1H, dd, J=9.0,4.4Hz), 8.84 (1H, d, J=9.4Hz).

### 2) 3-Methoxypyridazine-1-oxide

To a solution of 3-methoxypyridazine (16.2 g) in dichloromethane (300 mL), at room temperature, m-chlorobenzoyl peroxide (44.0 g) was added, and the mixture was stirred for 16 hours. A solution of sodium hydrogencarbonate (9.27 g) in water (100 mL) was added to the reaction mixture, followed by stirring for 15 minutes. The resultant mixture was partitioned between saturated aqueous sodium hydrogencarbonate and chloroform, and the organic layer was dried over sodium sulfate anhydrate. After a filtration step, the solvent was evaporated under reduced pressure, to thereby give 3-methoxypyridazine-1-oxide as a solid product (15.8 g, 85%).

¹H-NMR(400MHz,CDCl₃)δ:4.02(3H,s), 6.66(1H,d,J=8.5Hz), 7.48(1H,dd,J=8.5,5.9Hz), 7.92(1H,d,J=5.9Hz).

### 3) 6-Cyano-3-methoxypyridazine

3-Methoxypyridazine-1-oxide (9.89 g) and dimethylsulfuric acid (9.45 mL) were mixed together and then stirred for 1 hour at 80°C, followed by cooling in air. Dioxane (100 mL) was added to the reaction mixture, and at 0°C, a solution of potassium cyanide (8.77 g) in water (30 mL) was added thereto, followed by stirring for 4.5 hours at room temperature. The reaction mixture was added to saturated aqueous sodium hydrogencarbonate, and the mixture was extracted with chloroform. The organic layer was washed with water and then dried over sodium sulfate anhydrate. After a filtration step, the solvent was evaporated under reduced pressure, and the residue was purified through silica gel column chromatography (hexane - ethyl acetate), to thereby give 6-cyano-3-methoxypyridazine as a solid product (8.74 g, 72%).

¹H-NMR(400MHz,CDCl₃)δ:4.24(3H,s), 7.09(1H,d,J=9.0Hz), 7.68(1H,d,J=9.0Hz).

### 4) 3-Acetyl-6-methoxypyridazine

6-Cyano-3-methoxypyridazine (5.61 g) was dissolved in a mixture of diethyl ether (100 mL) and benzene (20 mL). Under cooling at -10°C, methylmagnesium iodide (0.84M diethyl ether solution, 60 mL) was added dropwise thereto, and the resultant mixture was stirred for 1 hour at the same temperature. At 0°C, the reaction mixture was acidified with 1N aqueous hydrochloric acid to pH 4 for partitioning the mixture. The aqueous layer was made weak alkali (pH 9) with saturated aqueous sodium hydrogencarbonate, and the mixture was extracted with dichloromethane. Subsequently, the aqueous layer was extracted with dichloromethane, and the organic layers were combined, followed by washing with saturated brine and drying over sodium sulfate anhydrate. After a filtration step, the solvent was evaporated under reduced pressure, and the residue was purified through silica gel column chromatography (ethyl acetate - hexane), to thereby give 3-acetyl-6-methoxypyridazine as a solid product (3.82 g, 61%).

¹H-NMR(400MHz,CDCl₃)δ:2.82(3H,s), 4.23(3H,s), 7.06(1H,d,J=9.3Hz), 8.04(1H,d,J=9.3Hz).
FAB-MS m/z:153(M+H)⁺.

### 5) 4-(6-Methoxy-3-pyridazinyl)-2,4-dioxobutanoic acid methyl ester

3-Acetyl-6-methoxypyridazine (3.82 g) was dissolved in tetrahydrofuran (100 mL), and, at -78°C, lithium bis(trimethylsilyl)amide (1.0M tetrahydrofuran solution, 27 mL) was added to the solution, followed by stirring for 1 hour. At -78°C, dimethyl oxalate (5.9 g) in tetrahydrofuran (20 mL) was added to the reaction mixture, followed by stirring for 2 hours at 0°C. The reaction mixture was partitioned between water and diethyl ether, and the aqueous layer was acidified with 1N aqueous hydrochloric acid to pH 4 and then extracted with chloroform. The aqueous layer was extracted with chloroform, and the organic layers were combined, followed by washing with saturated brine and drying over sodium sulfate anhydrate. After a filtration step, the solvent was evaporated under reduced pressure, to thereby give 4-(6-methoxy-3-pyridazinyl)-2,4-dioxobutanoic acid methyl ester as a solid product (5.38 g, 90%).

¹H-NMR(400MHz,CDCl₃)δ:3.93(3H,s), 4.26(3H,s), 7.12(1H,d,J=9.3Hz), 7.86(1H,s), 8.15(1H,d,J=9.3Hz).
ESI-MS m/z:239(M+H)⁺.

### 6) 5-(6-Methoxy-3-pyridazinyl)-1-(3-pyridyl)-1H-pyrazole-3-carboxylic acid methyl ester

To a solution of 4-(6-methoxypyridazin-3-yl)-2,4-dioxobutanoic acid methyl ester (5.38 g) in methanol (150 mL), at room temperature, 3-hydrazinopyridine (2.82 g) obtained in Referential Example 1 was added, and the mixture was refluxed for 45 minutes. Acetic acid (5.2 mL) was added to the reaction mixture, and the resultant mixture was refluxed for 14 hours, followed by cooling in air. The reaction mixture was neutralized with 1N aqueous sodium hydroxide, and the solvent of the reaction mixture was evaporated under reduced pressure. The residue was partitioned between chloroform and water, and the aqueous layer was extracted with chloroform. The organic layers were combined, followed by washing with saturated brine and drying over sodium sulfate anhydrate. After a filtration step, the solvent was evaporated under reduced pressure, and the residue was purified through silica gel column chromatography (methanol - chloroform), to thereby give 5-(6-methoxy-3-pyridazinyl)-1-(3-pyridyl)-1H-pyrazole-3-carboxylic acid methyl ester as a solid product (0.348 g, 5.0%). Further, the solvent of the second fraction obtained in the above elution process was evaporated under reduced pressure, to thereby give 5-hydroxy-5-(6-methoxy-3-pyridazinyl)-1-(3-pyridyl)-4,5-dihydro-1H-pyrazole-3-carboxylic acid methyl ester as an amorphous product (3.11 g, 44%).

5-(6-Methoxy-3-pyridazinyl)-1-(3-pyridyl)-1H-pyrazole-3-carboxylic acid methyl ester:
¹H-NMR(400MHz,CDCl₃)δ:3.99(3H,s), 4.13(3H,s), 7.00(1H,d,J=9.1Hz), 7.31(1H,s), 7.37-7.46(2H,m), 7.86-7.92(1H,m), 8.54(1H,d like,J=2.0Hz), 8.63(1H,dd,J=4.9,1.5Hz).
FAB-MS m/z:312(M+H.)⁺.
5-Hydroxy-5-(6-methoxy-3-pyridazinyl)-1-(3-pyridyl)-4,5-dihydro-1H-pyrazole-3-carboxylic acid methyl ester:
ESI-MS m/z:330(M+H)⁺.

The thus-obtained 5-hydroxy-5-(6-methoxy-3-pyridazinyl)-1-(3-pyridyl)-4,5-dihydro-1H-pyrazole-3-carboxylic acid methyl ester (2.88 g) was dissolved in dichloromethane (80 mL). To the solution, at room temperature, triethylamine (3.04 mL), methanesulfonyl chloride (1.35 mL), and 4-(dimethylamino)pyridine (0.111 g) were added, and the mixture was stirred for 2.5 hours. Methanol was added to the reaction mixture, and the resultant mixture was partitioned between chloroform and water. The aqueous layer was extracted with chloroform, and the organic layers were combined, followed by washing with saturated brine and drying over sodium sulfate anhydrate. After a filtration step, the solvent was evaporated under reduced pressure, and the residue was purified through silica gel column chromatography (acetone - chloroform), to thereby give 5-(6-methoxy-3-pyridazinyl)-1-(3-pyridyl)-1H-pyrazole-3-carboxylic acid methyl ester as a solid product (1.558 g, 57%).

### 7) Title Compound

5-(6-Methoxy-3-pyridazinyl)-1-(3-pyridyl)-1H-pyrazole-3-carboxylic acid methyl ester (0.399 g) was dissolved in a mixture of tetrahydrofuran (5 mL), methanol (10 mL), and water (5 mL). To the solution, lithium hydroxide monohydrate (62 mg) was added, and the mixture was stirred for 1 hour at 40°C, followed by cooling in air. The reaction mixture was neutralized with 1N aqueous hydrochloric acid, and the solvent of the reaction mixture was evaporated under reduced pressure. 1N Aqueous hydrochloric acid was added to the residue making the pH condition to 4, and the precipitated solid was collected through filtration, to thereby give the title compound as a solid product (0.303 g, 80%).

¹H-NMR(400MHz,DMSO-d₆)δ:4.00(3H,s), 7.34(1H,d,J=7.8Hz), 7.49(1H,s), 7.53(1Hdd,J=4.8,7.8Hz), 7.86-7.91(1H,m), 7.99(1H,d like,J=9.3Hz), 8.60(1H,d,J=2.0Hz), 8.64(1H,d like,J=4.8Hz).
FAB-MS m/z:298(M+H)⁺.

[Referential Example 37] 5-(1-Methyl-1H-pyrrol-2-yl)-1-(3-pyridyl)-1H-pyrazole-3-carboxylic acid

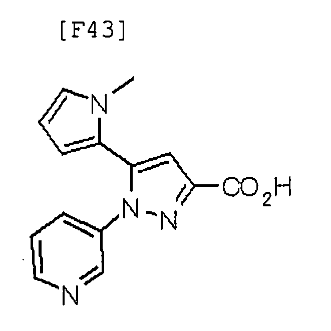

### 1) 5-(1-Methyl-1H-pyrrol-2-yl)-1-(3-pyridyl)-1H-pyrazole-3-carboxylic acid ethyl ester

A solution of 1-(1-methyl-1H-pyrrol-2-yl)-1-ethanone (1.19 mL) in tetrahydrofuran (10 mL) was cooled to -78°C, and lithium bis(trimethylsilyl)amide (1.OM tetrahydrofuran solution, 11.0 mL) was added thereto, followed by stirring for 30 minutes. At the same temperature, diethyl oxalate (2.05 mL) was added to the reaction mixture, and the mixture was stirred for 2 hours, while the temperature was gradually returned to room temperature. Ethanol (50 mL), 3-hydrazinopyridine (1.09 g) obtained in Referential Example 1, and 1M HCl in ethanol (11.0 mL) were added to the reaction mixture, followed by reflux for 16 hours 30 minutes. 1M HCl in ethanol (11.0 mL) was added to the reaction mixture, and the resultant mixture was refluxed for 3 hours, followed by cooling in air. The reaction solvent was evaporated under reduced pressure, and the residue was partitioned between ethyl acetate and saturated aqueous sodium hydrogencarbonate. The aqueous layer was extracted with ethyl acetate. The organic layers were combined and then dried over sodium sulfate anhydrate. After a filtration step, the solvent was evaporated under reduced pressure, and the residue was purified through silica gel column chromatography (ethyl acetate - hexane), to thereby give 5-(1-methyl-1H-pyrrol-2-yl)-1-(3-pyridyl)-1H-pyrazole-3-carboxylic acid ethyl ester as an oily product (2.08 g, 70%).

¹H-NMR(400MHz,CDCl₃)δ:1.44(3H,t,J=7.1Hz), 3.36(3H,s), 4.47(2H,q,J=7.2Hz), 6.08(1H,dd,J=3.7,1.7Hz), 6.15(1H,dd,J=3.7,2.7Hz), 6.71-6.72(1H,m), 7.02(1H,s), 7.30(1H,dd,J=8.3,4.6Hz), 7.64-7.67(1H,m), 8.56(1H,dd,J=4.9,1.5Hz), 8.58(1H,d,J=2.7Hz).
ESI-MS m/z:297(M+H)⁺.

### 2) Title Compound

5-(1-Methyl-1H-pyrrol-2-yl)-1-(3-pyridyl)-1H-pyrazole-3-carboxylic acid ethyl ester (2.08 g) was dissolved in ethanol (21 mL), and, at room temperature, 1N aqueous sodium hydroxide (21.0 mL) was added to the solution, followed by stirring for 4 hours. The reaction mixture was partitioned between 1N aqueous hydrochloric acid (21.0 mL) and ethyl acetate. The aqueous layer was extracted with ethyl acetate. The organic layers were combined and then dried over magnesium sulfate anhydrate. After a filtration step, the solvent was evaporated under reduced pressure, and diethyl ether and hexane were added to the residue. The formed solid was collected through filtration, to thereby give the title compound as a solid product (1.63 g, 86%).

¹H-NMR(400MHz,DMSO-d₆)δ:3.45(3H,s), 5.90(1H,dd,J=3.7,1.7Hz), 6.02(1H,dd,J=3.7,2.7Hz), 6.91-6.92(1H,m), 7.09(1H,s), 7.50(1H,dd,J=8.3,4.9Hz), 7.75-7.78(1H,m), 8.47(1H,d,J=2.9Hz),
8.59(1H,dd,J=4.6,1.5Hz).
ESI-MS m/z:269(M+H)⁺.

[Referential Example 38] 5-(1-Methyl-1H-pyrrol-3-yl)-1-(pyridin-3-yl)-1H-pyrazole-3-carboxylic acid

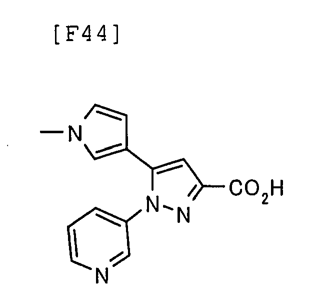

### 1) 5-(1-Methyl-1H-pyrrol-3-yl)-1-(pyridin-3-yl)-1H-pyrazole-3-carboxylic acid ethyl ester

Under cooling at -78°C, lithium bis(trimethylsilyl)amide (1.0M tetrahydrofuran solution, 11.1 mL) was added dropwise to a solution of 3-acetyl-1-methylpyrrole (1.20 mL) in tetrahydrofuran (10 mL), followed by stirring for 30 minutes. Diethyl oxalate (2.06 mL) was added dropwise to the reaction mixture, and the temperature was elevated to room temperature, followed by stirring for 1 hour at room temperature. 3-Hydrazinopyridine (1.30 g) obtained in Referential Example 1, acetic acid (635 µL), and ethanol (50 mL) were added to the reaction mixture, and the resultant mixture was refluxed for 16 hours. Subsequently, 3-hydrazinopyridine (650 mg) was added to the reaction mixture, followed by reflux for 3 hours. Concentrated hydrochloric acid (0.60 mL) was added to the reaction mixture, and the resultant mixture was refluxed for 24 hours, followed by cooling in air. The reaction solvent was evaporated under reduced pressure, and saturated aqueous sodium hydrogencarbonate (100 mL), water (50 mL), and ethyl acetate (100 mL) were added to the residue for partitioning the mixture. Subsequently, the aqueous layer was extracted twice with ethyl acetate, and the organic layers were combined, followed by drying over sodium sulfate anhydrate. After a filtration step, the solvent was evaporated under reduced pressure, and the residue was purified through silica gel column chromatography (dichloromethane-ethyl acetate), to thereby give 5-(1-methyl-1H-pyrrol-3-yl)-1-(pyridin-3-yl)-1H-pyrazole-3-carboxylic acid ethyl ester as a solid product (747 mg, 25%).

¹H-NMR(400MHz,CDCl₃)δ:1.42(3H,t,J=7.2Hz), 3.59(3H,s), 4.44(2H,q,J=7.2Hz), 5.88(1H,dd,J=2.7,2.0Hz), 6.43(1H,t,J=2.0Hz), 6.52 (1H, t, J=2.7Hz), 6.92(1H,s), 7.39(1H,dd,J=8.2,4.8Hz), 7.85(1H,ddd,J=8.2,2.9,1.7Hz), 8.65(1H, dd, J=4.8,1.7Hz), 8.72 (1H, d, J=2.4Hz).
EI-MS m/z:296 (M⁺).

### 2) Title Compound

The above 5-(1-methyl-1H-pyrrol-3-yl)-1-(pyridin-3-yl)-1H-pyrazole-3-carboxylic acid ethyl ester (630 mg) was suspended in tetrahydrofuran (10 mL), and, at room temperature, 1M aqueous sodium hydroxide (2.8 mL) was added to the suspension, followed by stirring for 2 hours. Subsequently, 1M aqueous sodium hydroxide (1 mL) was added thereto, and the mixture was stirred for 19 hours. The reaction mixture was partitioned between water and diethyl ether, and hydrochloric acid was added to the aqueous layer, followed by extraction with chloroform. The organic layer was dried over magnesium sulfate anhydrate. After a filtration step, the solvent was evaporated under reduced pressure; to thereby give the title compound (332 mg, 58%).

¹H-NMR(400MHz,DMSO-d₆)δ:3.54(3H,s), 5.74(1H,s), 6.69(2H,m), 6.86(1H,s), 7.57(1H,m), 7.90(1H,m), 8.62(1H,s), 8.68(1H,s), 12.87(1H,br).
EI-MS m/z:269(M⁺).
[Referential Example 39] 1-Methyl-2,6-dioxopiperazine hydrochloride

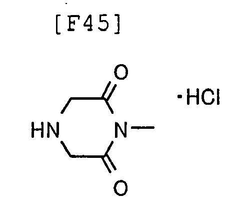

### 1) 4-Benzylpiperazine-2,6-dione

At room temperature, ammonium formate (4.5 g) was added to a solution of N-benzyliminodiacetic acid (5.25 g) in N,N-dimethylformamide (75 mL), and the mixture was refluxed for 22 hours, followed by cooling in air. Water, saturated aqueous sodium hydrogencarbonate, and ethyl acetate were added to the reaction mixture for partitioning the mixture, and the aqueous layer was extracted with ethyl acetate. The organic layers were combined, washed with saturated brine, and dried over sodium sulfate anhydrate. After a filtration step, the solvent was evaporated under reduced pressure, to thereby give 4-benzylpiperazine-2,6-dione as an oily product (4.53 g, 94%).

¹H-NMR(400MHz,CDCl₃)δ:3.38(4H,s), 3.68(2H,s),7.25-7.39(5H,m), 8.26(1H,br).
ESI-MS m/z:205(M+H)⁺.

### 2) Piperazine-2,6-dione hydrochloride

The above 4-benzylpiperazine-2,6-dione (4.53 g) was dissolved in methanol (120 mL) and water (50 mL), and, at room temperature, 1.0M HCl-ethanol (22 mL) and 10% palladium-carbon (1.45 g) were added to the solution, followed by stirring for 20 hours in a hydrogen atmosphere. After a filtration step, the solvent was evaporated under reduced pressure, and toluene was added to the residue. The solvent was evaporated under reduced pressure, to thereby give piperazine-2,6-dione hydrochloride as a solid product (1.85 g, 51%).

¹H-NMR(400MHz,DMSO-d₆)δ:3.93(9H,s), 10.46(2H,br), 11.77(1H,s).

### 3) 4-tert-Butoxycarbonylpiperazine-2,6-dione

The above piperazine-2,6-dione hydrochloride (1.58 g) was suspended in tetrahydrofuran (15 mL), methanol (15 mL), and N,N-dimethylformamide (30 mL), and, at room temperature, triethylamine (3.21 mL) and di-tert-butyl dicarbonate (2.51 g) were added thereto, followed by stirring for 17 hours. The reaction solvent was evaporated under reduced pressure, and water, 1M aqueous hydrochloric acid, and ethyl acetate were added to the residue for partitioning the residue. The aqueous layer was extracted with ethyl acetate, and the organic layers were combined, followed by washing with saturated brine and drying over sodium sulfate anhydrate. After a filtration step, the solvent was evaporated under reduced pressure, to thereby give 4-tert-butoxycarbonylpiperazine-2,6-dione as a solid product (1.75 g, 78%).

¹H-NMR(400MHz,CDCl₃)δ:1.48(9H,s), 4.30(2H,s), 8.14(1H,br).

### 4) 4-tert-Butoxycarbonyl-1-methylpiperazine-2,6-dione

The above 4-tert-butoxycarbonylpiperazine-2,6-dione (0.438 g) is dissolved in N,N-dimethylformamide (10 mL), and, at 0°C, 60% sodium hydride (0.100 g) and methyl iodide (0.190 mL) were added to the solution, followed by stirring for 1 hour at room temperature. The reaction mixture was partitioned between water and ethyl acetate, and the aqueous layer was extracted with ethyl acetate. The organic layers were combined, washed with saturated brine, and dried over sodium sulfate anhydrate. After a filtration step, the solvent was evaporated under reduced pressure, and the residue was purified through silica gel column chromatography (chloroform - acetone), to thereby give 4-tert-butoxycarbonyl-1-methylpiperazine-2,6-dione as an oily product (0.144 g, 31%).
¹H-NMR(400MHz,CDCl₃)δ:1.47(9H,s), 3.18(3H,s), 4.34(4H,s).

### 5) Title Compound

The above 4-tert-butoxycarbonyl-1-methylpiperazine-2,6-dione (0.144 g) was dissolved in dichloromethane (5.0 mL), and, at 0°C, 4M HCl-dioxane (2.5 mL) was added to the solution, followed by stirring for 2 hours at room temperature. Diethyl ether was added to the reaction mixture, and the precipitated solid was collected through filtration, to thereby give the title compound as a solid product (53.4 mg, 51%).
¹H-NMR(400MHz;DMSO-d₆)δ:3.02(3H,s), 4.05(4H,s), 10.00(2H,br).

[Referential Example 40] 1-Ethyl-2-oxopiperazine hydrochloride

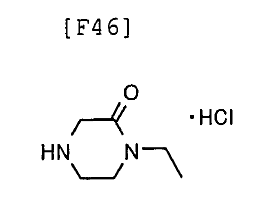

### 1) 3-Oxopiperazine-1-carboxylic acid tert-butyl ester

Piperazin-2-one (5.07 g) was dissolved in tetrahydrofuran (50 mL) and methanol (50 mL), and, at room temperature, triethylamine (7.76 mL) and di-tert-butyl dicarbonate (12.17 g) were added to the solution, followed by stirring for 4 hours. The reaction solvent was evaporated under reduced pressure, and diethyl ether was added to the residue. The precipitated solid was collected through filtration, to thereby give 3-oxopiperazine-1-carboxylic acid tert-butyl ester as a solid product (9.36 g, 92%).

¹H-NMR(400MHz,DMSO-d₆)δ:1.40(9H,s), 3.15(2H,br), 3.45(2H,br), 3.81(2H,br), 8.03(1H,br).
ESI-MS m/z:201(M+H)⁺.

### 2) 4-Ethyl-3-oxopiperazine-1-carboxylic acid tert-butyl ester

The above 3-oxopiperazine-1-carboxylic acid tert-butyl ester (9.36 g) was dissolved in N,N-dimethylformamide (105 mL), and, at 0°C, sodium hydride (1.361 g) was added to the solution, followed by stirring for 25 minutes. Ethyl iodide (4.48 mL) was added to the reaction mixture, and the resultant mixture was stirred for 15 hours at 40°C, followed by cooling in air. The reaction mixture was partitioned between water and ethyl acetate, and the aqueous layer was extracted with ethyl acetate. The organic layers were combined, washed with saturated brine, and dried over sodium sulfate anhydrate. After a filtration step, the solvent was evaporated under reduced pressure, and the residue was purified through silica gel column chromatography (chloroform - acetone), to thereby give 4-ethyl-3-oxopiperazine-1-carboxylic acid tert-butyl ester as an oily product (8.69 g, 81%).

¹H-NMR(400MHz,CDCl₃)δ:1.15(3H,t,J=7.1Hz), 1.46(9H,s), 3.33(2H,t,J=5.4Hz), 3.46(2H,q,J=7.1Hz), 3.64(2H,t,J=5.4Hz), 4.06(2H,s).
EI-MS m/z:228(M⁺).

### 3) Title Compound

The above tert-butyl 4-ethyl-3-oxopiperazine-1-carboxylic acid ester (8.69 g) and 4M HCl-dioxane (60 mL) were treated in a manner similar to that employed in step 5) of Referential Example 39, to thereby give the title compound as an oily product (5.88 g, 94%).

¹H-NMR(400MHz,DMSO-d₆)δ:1.04(3H,t,J=7.1Hz), 3.29-3.39(4H,m), 3.53(2H,t like,J=5.6Hz), 3.63(2H,br), 10.00(2H,br).
FAB-MS m/z:129(M+H)⁺.

[Referential Example 41] 3-Methoxyazetidine hydrochloride

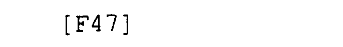

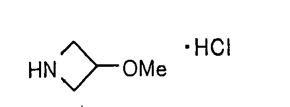

### 1) 1-Benzhydryl-3-methoxyazetidine

To a solution of 1-benzhydrylazetidin-3-ol (4.60 g) in N,N-dimethylformamide (80 mL), at 0°C, 60% sodium hydride (0.852 g) and methyl iodide (1.43 mL) were added, and the mixture was stirred for 3 hours. The reaction mixture was partitioned between water and ethyl acetate, and the aqueous layer was extracted with ethyl acetate. The organic layers were combined, washed with saturated brine, and dried over sodium sulfate anhydrate. After a filtration step, the solvent was evaporated under reduced pressure, and the residue was purified through silica gel column chromatography (hexane - ethyl acetate), to thereby give 1-benzhydryl-3-methoxyazetidine as an oily product (4.27 g, 88%). ¹H-NMR(400MHz,CDCl₃)δ:2.87-2.95(2H,m), 3.22(3H,s), 3.46-3.53(2H,m), 4.01-4.08(1H,m), 4.35(1H,s), 7.14-7.31(6H,m), 7.36-7.43(4H,m).
ESI-MS m/z:259 (M+H)⁺.

### 2) Title Compound

The above 1-benzhydryl-3-methoxyazetidine (4.27 g) was dissolved in methanol (90 mL), and, at room temperature, 1M HCl in ethanol (22 mL) and palladium hydroxide-carbon (20% wet, 2.11 g) were added to the solution, followed by stirring for 18 hours in a hydrogen atmosphere. The catalyst was removed through filtration, and the solvent was evaporated under reduced pressure. The residue was washed with diethyl ether and hexane, and the organic layer was removed through decantation. The residue was dried under reduced pressure, to thereby give the title compound as an amorphous product (1.82 g, 88%).

¹H-NMR(400MHz,DMSO-d₆)δ:3.19(3H,s), 3.69-3.80(2H,m), 4.01-4.12(2H,m), 4.17-4.27(1H,m), 9.37(2H,br).

[Referential Example 42] 5-(1-Methyl-1H-imidazol-4-yl)-1-(pyridin-3-yl)-1H-pyrazole-3-carboxylic acid ethyl ester

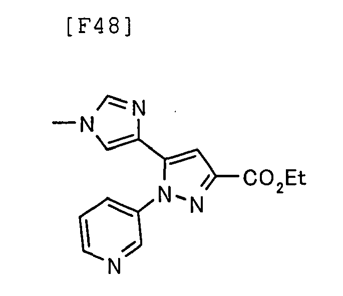

### 1) N-Methoxy-N-methyl-1-methyl-1H-imidazole-4-carboxamide

1-Methyl-1H-imidazole-4-carboxylic acid (1.26 g), N,O-dimethylhydroxylamine hydrochloride (1.17 g), 1-hydroxybenzotriazole (1.84 g), triethylamine (2.09 mL), and 1-ethyl-3-(3-dimethylaminopropyl)carbodiimide hydrochloride (2.30 g) were dissolved in N,N-dimethylformamide (50 mL), and the solution was stirred overnight at room temperature. The reaction solvent was evaporated under reduced pressure, and ethanol was added to the residue. Insoluble matter was removed through filtration, and the solvent was evaporated under reduced pressure. The residue was purified through silica gel column chromatography (chloroform - methanol - water, lower phase), to thereby give N-methoxy-N-methyl-1-methyl-1H-imidazole-4-carboxamide as a solid product (1.08 g, 64%).

¹H-NMR(400MHz,CDCl₃)δ:3.45(3H,s), 3.73(3H,s), 3.78(3H,s), 7.45(1H,s),7.54(1H,s).

### 2) 4-Acetyl-1-methyl-1H-imidazole

In an argon atmosphere and under cooling at -78°C, methyllithium (0.98M diethyl ether solution, 6.84 mL) was added dropwise to a solution of the above N-methoxy-N-methyl-1-methyl-1H-imidazole-4-carboxamide (1.08 g) in tetrahydrofuran (30 mL), and the mixture was stirred for 15 minutes and at 0°C for 75 minutes. The reaction mixture was partitioned between water and chloroform-methanol (10:1), and the organic layer was dried over sodium sulfate anhydrate. After a filtration step, the solvent was evaporated under reduced pressure, and the residue was purified through silica gel thin-layer chromatography (chloroform - methanol - water, lower phase), to thereby give 4-acetyl-1-methyl-1H-imidazole as a solid product (309 mg, 39%).

¹H-NMR(400MHz,CDCl₃)δ:2.56(3H,s), 3.75(3H,s), 7.44(1H,s), 7.56(1H,s)

### 3) Title Compound

Under cooling at -78°C, lithium bis(trimethylsilyl)amide (1.0M tetrahydrofuran solution, 12.5 mL) was added to a solution of the above 4-acetyl-1-methyl-1H-imidazole (1.41 g) in tetrahydrofuran (100 mL), and the mixture was stirred for 35 minutes. Diethyl oxalate (2.31 mL) was added to the reaction mixture, and the mixture was stirred for 15 minutes and at room temperature for 3 hours. The reaction mixture was partitioned between diethyl ether and water. 1M Aqueous hydrochloric acid (13 mL) was added to the aqueous layer, and the mixture was extracted 5 times with 10% methanol-dichloromethane solvent mixture. The organic layers were combined and then dried over sodium sulfate anhydrate. After a filtration step, the solvent was evaporated under reduced pressure, to thereby give a crude product of 4-(1-methyl-1H-imidazol-4-yl)-2,4-dioxobutanoic acid ethyl ester (2.16 g, 85%). The ethyl ester compound (2.16 g) was suspended in ethanol (50 mL), and a solution of 3-hydrazinopyridine (1.05 g) obtained in Referential Example 1 in ethanol (50 mL) was added to the suspension, followed by reflux for 15 hours and then cooling in air. The reaction mixture was partitioned between dichloromethane and saturated aqueous sodium hydrogencarbonate, and the aqueous layer was extracted 3 times with dichloromethane and then once with 10% methanol-dichloromethane. The organic layers were combined and then dried over sodium sulfate anhydrate. After a filtration step, the solvent was evaporated under reduced pressure, and the residue was purified through silica gel flash column chromatography (methanol - dichloromethane), to thereby give the title compound as a solid product (0.946 g, 33%).

¹H-NMR(400MHz,CDCl₃)δ:1.42(3H,t,J=7.1Hz), 3.65(3H,s), 4.45(2H,q,J=7.1Hz), 6.68(1H,d,J=1.2Hz), 7.15(1H,s) 7.40(1H,s), 7.42(1H,dd,J=8.1,4.9Hz) 7.93(1H,ddd,J=8.1,2.4,1.5Hz), 8.65(1H,dd,J=4.9,1.5Hz), 8.68(1H,d,J=2.4Hz).
ESI-MS m/z:298(M+H)⁺.

[Referential Example 43] 4-Methylenepiperidine hydrochloride

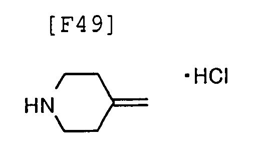

4M HCl-Ethyl acetate (5 mL) was added to 4-methylene-1-piperidinecarboxylic acid tert-butyl ester (0.230 g) at room temperature, and the mixture was stirred for 4.5 hours. The reaction solvent was evaporated under reduced pressure, to thereby give a crude product of the title compound.

[Referential Example 44] (2S)-2,4-Dimethyl-5-oxo-l-piperazinecarboxylic acid tert-butyl ester

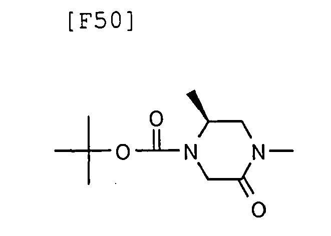

### 1) (1S)-1-Methyl-2-(methylamino)-2-oxoethylcarbamic acid tert-butyl ester

At room temperature, triethylamine (11.1 mL) and 1-(3-dimethylaminopropyl)-3-ethylcarbodiimide hydrochloride (7.66 g) were added to a suspension of (2S)-2-[(tert-butoxycarbonyl)amino]propionic acid (3.78 g), methylamine hydrochloride (2.70 g), and 1-hydroxybenzotriazole (6.12 g) in N,N-dimethylformamide (100 mL), followed by stirring for 17.5 hours. The reaction solvent was evaporated under reduced pressure, and the residue was partitioned between ethyl acetate and saturated aqueous sodium hydrogencarbonate. The aqueous layer was extracted twice with ethyl acetate. The organic layers were combined and then dried over sodium sulfate anhydrate. After a filtration step, the solvent was evaporated under reduced pressure, to thereby give (1S)-1-methyl-2-(methylamino)-2-oxoethylcarbamic acid tert-butyl ester as a solid product (4.33 g, quantitative amount).

¹H-NMR(400MHz,CDCl₃)δ:1.36(3H,d,J=7.1Hz), 1.45(9H,s), 2.82(3H,d,J=4.9Hz), 4.09-4.18(1H,m), 4.99(1H,brs), 6.18(1H,brs).
ESI-MSm/z:103(M-Boc+H)⁺.

### 2) Title Compound

The above (1S)-1-methyl-2-(methylamino)-2-oxoethylcarbamic acid tert-butyl ester (4.33 g) was dissolved in tetrahydrofuran (140 mL), and, at room temperature, boran-dimethylsulfide complex (5.69 mL) was added to the solution, followed by stirring for 21.5 hours. 3M Aqueous hydrochloric acid (20 mL) was added to the reaction mixture, and tetrahydrofuran (200 mL), water (50 mL), and potassium hydroxide (8.00 g) were added to the mixture. The resultant mixture was refluxed for 4.5 days, followed by cooling in air. The organic solvent of the reaction mixture was evaporated under reduced pressure, and the resultant aqueous solution was extracted 3 times with dichloromethane. The organic layers were combined and then dried over sodium sulfate anhydrate. After a filtration step, the solvent was evaporated under reduced pressure, to thereby give a crude product of (1S)-1-methyl-2-(methylamino)ethylcarbamic acid tert-butyl ester (3.00 g, 79%) as an oily product. Under cooling at 0°C, 1M aqueous sodium hydrogencarbonate (47.8 mL) and chloroacetyl chloride (1.90 mL) were added to a solution of the carbamic acid tert-butyl ester compound (3.00 g) in ethyl acetate (50 mL), and the mixture was stirred for 1.5 days at room temperature. The reaction mixture was partitioned between water and ethyl acetate, and the aqueous layer was extracted with ethyl acetate. The organic layers were combined and then dried over sodium sulfate anhydrate. After a filtration step, the solvent was evaporated under reduced pressure, and the residue was purified through silica gel flash column chromatography (ethyl acetate - n-hexane), to thereby give a crude product of (1S)-2-[(2-chloroacetyl)(methyl)amino]-1-methylethylcarbamic acid tert-butyl ester (2.29 g, 54%) as a solid product. The methylethylcarbamic acid tert-butyl ester compound (2.29 g) was dissolved in N,N-dimethylformamide (50 mL), and, under cooling at 0°C, cesium carbonate (4.23 g) was added to the solution, followed by stirring for 3.5 days at room temperature. The reaction solvent was evaporated under reduced pressure, and the residue was partitioned between ethyl acetate and saturated aqueous sodium hydrogencarbonate. The aqueous layer was extracted with ethyl acetate. The organic layers were combined and then dried over sodium sulfate anhydrate. After a filtration step, the solvent was evaporated under reduced pressure, and the residue was purified through silica gel flash column chromatography (ethyl acetate - n-hexane), to thereby give the title compound as an oily product (1.66 g, 84%).

¹H-NMR(400MHz,CDCl₃)δ:1.24(3H,d,J=6.8Hz), 1.47(9H,s), 2.98(1H,dd,J=12.5,1.7Hz), 3.01(3H,s), 3.68(1H,dd,J=12.5,4.5Hz), 3.78(1H,d,J=18.3Hz),
4.28(1H,d,J=18.3Hz), 4.43-4.49(1H,m).
ESI-MS m/z:173(M-tBu+H)⁺.

[Referential Example 45] 1-(3-Pyridyl)-5-{5-[2-(trimethylsilyl)ethynyl]-2-pyridyl}-1H-pyrazole-3-carboxylic acid ethyl ester

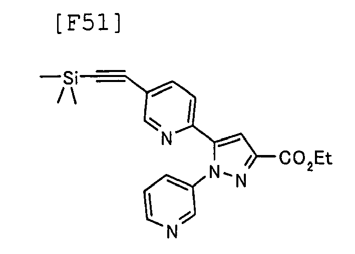

1-(3-Pyridyl)-5-(5-trifluoromethanesulfonyloxy-2-pyridyl)-1H-pyrazole-3-carboxylic acid ethyl ester (1.85 g) obtained in Method A)-step 2) of Referential Example 8 was dissolved in N,N-dimethylformamide (10 mL) and triethylamine (3.6 mL), and, at room temperature, trimethylsilyl acetylene (0.630 mL) was added to the solution, followed by stirring for 15 minutes. Bis(triphenylphosphine)palladium(II) dichloride (58.7 mg) was added to the reaction mixture, and the resultant mixture was stirred for 1.5 days at 60°C, followed by cooling in air. Water was added to the reaction mixture, and the resultant mixture was extracted 5 times with ethyl acetate. The organic layers were combined and then dried over sodium sulfate anhydrate. After a filtration step, the solvent was evaporated under reduced pressure, and the residue was purified through silica gel flash column chromatography (ethyl acetate - n-hexane), to thereby give the title compound as a solid product (0.470 g, 28%).

¹H-NMR(400MHz, CDCl₃)δ:0.25(9H,s), 1.43(3H,t,J=7.2Hz), 4.47(2H,q,J=7.1Hz), 7.31(1H,s), 7.35-7.41(2H,m), 7.75(1H,dd,J=8.1,2.0Hz), 7.78-7.81(1H,m), 8.50(1H,d,J=2.0Hz), 8.54(1H,d,J=2.7Hz), 8.61(1H,dd,J=4.9,1.5Hz).
ESI-MS m/z:391(M+H)⁺.

[Referential Example 46] 5-(5-Cyanopyridin-2-yl)-1-phenyl-1H-pyrazole-3-carboxylic acid

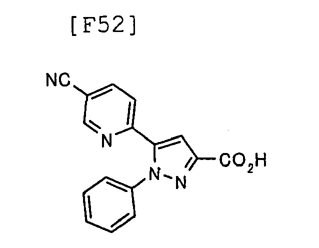

### 1) 5-(5-Benzyloxy-2-pyridyl)-1-phenyl-1H-pyrazole-3-carboxylic acid ethyl ester

4-(5-Benzyloxy-2-pyridyl)-2,4-dioxobutanoic acid ethyl ester (3.14 g) obtained in step 3) of Referential Example 4 and phenylhydrazine (1.16 mL) were treated in a manner similar to that employed in Step 4) of Referential Example 4, to thereby give 5-(5-benzyloxy-2-pyridyl)-1-phenyl-1H-pyrazole-3-carboxylic acid ethyl ester as a solid product (2.95 g, 77%).

¹H-NMR(400MHz,CDCl₃)δ:1.41(3H,t,J=7.1Hz), 4.44(2H,q,J=7.2Hz), 5.10(2H,s), 7.09-7.41(13H,m), 8.32(1H,d,J=2.7Hz).

### 2) 5-(5-Hydroxy-2-pyridyl)-1-phenyl-1H-pyrazole-3-carboxylic acid ethyl ester

5-(5-Benzyloxy-2-pyridyl)-1-phenyl-1H-pyrazole-3-carboxylic acid ethyl ester (2.83 g) was dissolved in ethanol (30 mL) and ethyl acetate (30 mL), and 10% palladium-carbon (1.50 g) was added to the solution, followed by stirring overnight in a hydrogen atmosphere. The reaction mixture was filtrated, and the solvent of the filtrate was evaporated under reduced pressure, to thereby give 5-(5-hydroxy-2-pyridyl)-1-phenyl-1H-pyrazole-3-carboxylic acid ethyl ester as a solid product (1.98 g, 90%).

¹H-NMR(400MHz,CDCl₃)δ:1.39(3H,t,J=7.2Hz), 4.43(2H,q,J=7.1Hz), 7.05(2H,d,J=3.7Hz), 7.17(1H,s), 7.27-7.30(6H,m), 8.05(1H,s).
ESI-MSm/z:310(M+H)⁺.

### 3) 1-Phenyl-5-(5-trifluoromethanesulfonyloxy-2-pyridyl)-1H-pyrazole-3-carboxylic acid ethyl ester

5-(5-Hydroxy-2-pyridyl)-1-phenyl-1H-pyrazole-3-carboxylic acid ethyl ester (1.98 g) and trifluoromethanesulfonic acid anhydrate (1.29 mL) were treated in a manner similar to that employed in Method A)-step 2) of Referential Example 8, to thereby give 1-phenyl-5-(5-trifluoromethanesulfonyloxy-2-pyridyl)-1H-pyrazole-3-carboxylic acid ethyl ester as an oily product (2.60 g, 92%).

¹H-NMR(400MHz,CDCl₃)δ:1.43(3H,t,J=7.1Hz), 4.46(2H,q,J=7.2Hz), 7.29-7.93(7H,m), 7.57(1H,dd,J=8.8,2.7Hz), 8.51(1H,d,J=2.7Hz).

### 4) Title Compound

Tri-n-butyltin cyanide (7.42 g), tetrakis (triphenylphosphine) palladium(0) (10.17 g), and 1-phenyl-5-(5-trifluoromethanesulfonyloxy-2-pyridyl)-1H-pyrazole-3-carboxylic acid ethyl ester (2.59 g) were treated in a manner similar to that employed in Method A-step 3) of Referential Example 8, to thereby give 5-(5-cyano-2-pyridyl)-1-phenyl-1H-pyrazole-3-carboxylic acid ethyl ester (2.708 g) as a solid product. The cyano compound (2.68 g) and lithium hydroxide monohydrate (369 mg) were treated in a manner similar to that employed in Method A-step 3) of Referential Example 8, to thereby give the title compound as a solid product (951 mg, 56%).
ESI-MSm/z:291(M+H)⁺.

[Referential Example 47] 5-(1-Methyl-1H-pyrazol-4-yl)-1-(3-pyridyl)-1H-pyrazole-3-carboxylic acid

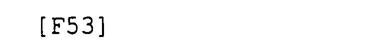

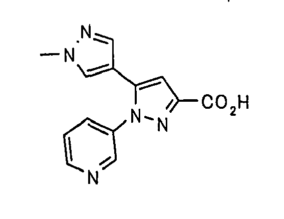

### 1) 1-Methyl-1H-pyrazole-4-carboaldehyde

In an argon atmosphere, phosphorus oxychloride (65.3 mL) was added dropwise to N,N-dimethylformamide (54.2 mL) at 0°C over 30 minutes, and the mixture was stirred at room temperature for 1 hour and 80°C for 10 minutes. Subsequently, 1-methylpyrazole (25.0 g) was added dropwise to the reaction mixture over 30 minutes. The reaction mixture was stirred at 85°C for 1 hour, at 100°C for 3 hours, and at 115°C for 1 hour, and then allowed to cool in air. The reaction mixture was added ice-water (1 L), and the mixture was stirred for 20 hours. The reaction mixture was partitioned between 1M aqueous sodium hydroxide (2 L) and chloroform, and the organic layer was dried over sodium sulfate anhydrate. After a filtration step, the solvent was evaporated under reduced pressure, and the residue was purified through silica gel column chromatography (chloroform - methanol), to thereby give 1-methyl-1H-pyrazole-4-carboaldehyde as an oily product (22.1 g, 66%).

¹H-NMR(400MHz,CDCl₃)δ:3.97(3H,s), 7.91(1H,s), 7.96(1H,s), 9.85(1H,s).
ESI-MSm/z:111(M+H)⁺.

### 2) 1-(1-Methyl-1H-pyrazol-4-yl) ethanol

In an argon atmosphere and under cooling at -78°C, methylmagnesium bromide (0.84M tetrahydrofuran solution, 250 mL) was added dropwise to a solution of the above 1-methyl-1H-pyrazole-4-carboaldehyde (22.0 g) in tetrahydrofuran (220 mL) over 50 minutes, and the mixture was stirred for 20 minutes and at 0°C for 50 minutes. Water and chloroform were added to the reaction mixture, and the resultant mixture was stirred. Insoluble matter in the reaction mixture was removed through filtration by celite. The filtrate was partitioned between water and chloroform, and the organic layer was dried over sodium sulfate anhydrate. After a filtration step, the solvent was removed under reduced pressure, and the residue was purified through silica gel column chromatography (methanol - chloroform), to thereby give 1-(1-methyl-1H-pyrazol-4-yl)ethanol as an oily product (20.2 g, 80%).

¹H-NMR(400MHz,CDCl₃)δ:1.49-1.51(3H,m), 3.87(3H,s), 4.87-4.92(1H,m), 7.33(1H,s), 7.44(1H,s).
ESI-MSm/z:127(M+H)⁺.

### 3) 1-(1-Methyl-1H-pyrazol-4-yl) ethanone

In an argon atmosphere and at 0°C, manganese(IV) oxide (activated, <5 micron, 209 g) was added to a solution of the above 1-(1-methyl-1H-pyrazol-4-yl)ethanol (20.2 g) in dichloromethane (202 mL), and the mixture was stirred for 14 hours at room temperature. Solid matter in the reaction mixture was removed through filtration, and the solvent of the filtrate was evaporated under reduced pressure, to thereby give 1-(1-methyl-1H-pyrazol-4-yl)ethanone as a solid product (18.1 g, 91%).

¹H-NMR(400MHz,CDCl₃)δ:2.42(3H,s), 3.94(3H,s), 7.86(1H,s), 7.89(1H,s).
ESI-MSm/z:125(M+H)⁺.

### 4) 4-(1-Methyl-1H-pyrazol-4-yl)-2,4-dioxobutanoic acid ethyl ester

In an argon atmosphere and at room temperature, diethyl oxalate (17.5 mL) was added to a solution of sodium ethoxide (8.77 g) in ethanol (80 mL), and the mixture was stirred for 10 minutes at room temperature. A solution of the above 1-(1-methyl-1H-pyrazol-4-yl)ethanone (8.00 g) in ethanol (80 mL) was added to the reaction mixture, and the resultant mixture was stirred for 90 minutes at room temperature. The reaction mixture was partitioned between water and diethyl ether. Saturated aqueous ammonium chloride was added to the aqueous layer, and the mixture was extracted with chloroform. The organic layer was dried over sodium sulfate anhydrate. After a filtration step, the solvent was removed under reduced pressure, to thereby give 4-(1-methyl-1H-pyrazol-4-yl)-2,4-dioxobutanoic acid ethyl ester as a solid product (11.4 g, 79%).

¹H-NMR(400MHz,CDCl₃)δ:1.39-1.42(3H,m), 3.98(3H,s), 4.36-4.41(2H,m), 6.69(1H,s), 7.98(2H,m).
EI-MSm/z:224 (M⁺).

### 5) 5-(1-Methyl-1H-pyrazol-4-yl)-1-(3-pyridyl)-1H-pyrazole-3-carboxylic acid ethyl ester

The above 4-(1-methyl-1H-pyrazol-4-yl)-2,4-dioxobutanoic acid ethyl ester (2.8 g) and 3-hydrazinopyridine (2.8 g) obtained in Referential Example 1 were dissolved in ethanol (50 mL), and the solution was refluxed for 30 minutes. Acetic acid (3.6 mL) was added to the reaction mixture, and the mixture was refluxed for 13 hours, followed by cooling in air. The reaction mixture was partitioned between saturated aqueous sodium hydrogencarbonate and dichloromethane, and the organic layer was dried over magnesium sulfate anhydrate. After a filtration step, the solvent was removed under reduced pressure, and the residue was purified through silica gel column chromatography (dichloromethane - methanol), to thereby give 5-(1-methyl-1H-pyrazol-4-yl)-1-(3-pyridyl)-1H-pyrazole-3-carboxylic acid ethyl ester as a solid product (3.2 g, 86%).

¹H-NMR(400MHz,CDCl₃)δ:1.42(3H,t,J=7.3Hz), 3.87(3H,s), 4.45(2H,q,J=7.3Hz), 6.98(1H,s), 7.17(1H,s) 7.30(1H,s), 7.41-7.44(1H,m), 7.78-7.83(1H,m), 8.69-8.75(2H,m).
EI-MSm/z:297 (M⁺).

### 6) Title Compound

The above 5-(1-methyl-1H-pyrazol-4-yl)-1-(3-pyridyl)-1H-pyrazole-3-carboxylic acid ethyl ester (3.2 g) was dissolved in tetrahydrofuran (40 mL) and water (20 mL), and lithium hydroxide monohydrate (500 mg) was added to the solution at room temperature, followed by stirring for 17 hours. The reaction mixture was partitioned between water and ethyl acetate. The aqueous layer was acidified with 1M aqueous hydrochloric acid, and the precipitated solid was collected through filtration, to thereby give the title compound as a solid product (2.2 g, 76%).

¹H-NMR(400MHz,DMSO-d₆)δ:3.79(3H,s), 7.02(1H,s), 7.30(1H,s), 7.58-7.61(1H,m), 7.68(1H,s), 7.92-7.95(1H,m), 8.66-8.33(1H,m), 13.00(1H,s).
EI-MSm/z: 269 (M⁺).

[Referential Example 48] 5-(5-Methyl-2-pyridyl)-1-(1,3-thiazol-2-yl)-1H-pyrazole-3-carboxylic acid ethyl ester

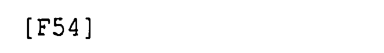

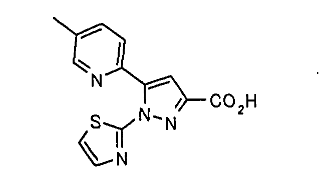

### 1) 5-(5-Methyl-2-pyridyl)-1-(1,3-thiazol-2-yl)-1H-pyrazole-3-carboxylic acid ethyl ester

4-(5-Methyl-2-pyridyl)-2,4-dioxobutanoic acid ethyl ester (1.18 g) obtained in step 2) of Referential Example 9 and 2-hydrazino-1,3-thiazole (Fortuna Haviv et al., J. Med. Chem., 1988, 31, 1719-1728, 0.576 g) were dissolved in ethanol (50 mL), and the solution was refluxed for 26.5 hours. 1M Hydrochloric acid in ethanol (2.50 mL) was added to the reaction mixture, and the resultant mixture was refluxed for 3 hours, followed by cooling in air. The reaction mixture was partitioned between ethyl acetate and saturated aqueous sodium hydrogencarbonate. Subsequently, the aqueous layer was extracted with ethyl acetate, and the organic layers were combined and then dried over sodium sulfate anhydrate. After a filtration step, the solvent was evaporated under reduced pressure, and the residue was purified through silica gel column chromatography (ethyl acetate - n-hexane), to thereby give 5-(5-methyl-2-pyridyl)-1-(1,3-thiazol-2-yl)-1H-pyrazole-3-carboxylic acid ethyl ester as an oily product (0.734 g, 46%).

¹H-NMR(400MHz,CDCl₃)δ:1.43(3H,t,J=7.1Hz), 2.38(3H,s), 4.45(2H,q,J=7.1Hz), 7.18(1H,s), 7.27(1H,d,J=3.4Hz), 7.44(1H,d,J=7.8Hz), 7.48(1H,d,J=3.9Hz), 7.54-7.57(1H,m), 8.40-8.41(1H,m).
ESI-MSm/z:315(M+H)⁺.

### 2) Title Compound

The above 5-(5-methyl-2-pyridyl)-1-(1,3-thiazol-2-yl)-1H-pyrazole-3-carboxylic acid ethyl ester (0.734 g) was dissolved in ethanol (10 mL), and, at room temperature, 1M aqueous sodium hydroxide (3.50 mL) was added to the solution, followed by stirring for 85 minutes. 1M Aqueous hydrochloric acid (3.50 mL) was added to the reaction mixture, and the formed solid was collected through filtration, to thereby give the title compound as a solid product (0.493 g, 73%).

¹H-NMR(400MHz,DMSO-d₆)δ:2.33(3H,s), 7.22(1H,s), 7.58(1H,d,J=3.4Hz), 7.61(1H,d,J=7.8Hz), 7.69-7.72(1H,m), 7.76(1H,d,J=3.4Hz), 8.34-8.36(1H,m).
ESI-MSm/z:287 (M+H)⁺.

[Referential Example 49] 1-Methyl-2-oxo[1.4]diazepane hydrochloride

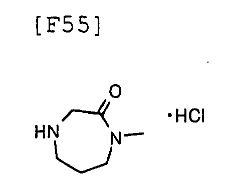

### 1) [N-(3-Benzyloxycarbonylaminopropyl)]aminoacetic acid benzyl ester

N-Benzyloxycarbonyl-1,3-diaminopropane hydrochloride (4.64 g) was suspended in N,N-dimethylformamide (80 mL), and, at room temperature, triethylamine (3.72 mL) and bromoacetic acid benzy ester (4.5 g) were added to the suspension, followed by stirring for 18 hours. The reaction mixture was cooled to 0°C, and water, 1M aqueous hydrochloric acid, and diethyl ether were added for partitioning the mixture. Saturated aqueous sodium hydrogencarbonate was added to the aqueous layer, and the mixture was extracted with ethyl acetate. The resultant aqueous layer was further extracted with ethyl acetate, and the organic layers were combined, followed by washing with saturated brine and drying over sodium sulfate anhydrate. After a filtration step, the solvent was evaporated under reduced pressure, and the residue was purified through silica gel column chromatography (chloroform - methanol), to thereby give [N-(3-benzyloxycarbonylaminopropyl)]aminoacetic acid benzyl ester as an oily product (4.72 g, 67%).

¹H-NMR(400MHz,CDCl₃)δ:1.62-1.74(2H,m), 2.69(2H,t,J=6.6Hz), 3.29(2H,q like,J=6.6Hz), 3.44(2H,s), 5.09(2H,s), 5.16(2H,s), 7.25-7.42(10H,m).
ESI-MSm/z:357(M+H)⁺.

### 2) [N-(3-Benzyloxycarbonylaminopropyl)-N-(tert-butoxycarbonyl)]aminoacetic acid benzyl ester

The above [N-(3-benzyloxycarbonylaminopropyl)]aminoacetic acid benzyl ester (4.72 g) was dissolved in dichloromethane (100 mL), and, at room temperature, triethylamine (2.23 mL) and di-tert-butyl dicarbonate (3.19 g) were added to the solution, followed by stirring for 24 hours. The reaction mixture was partitioned between water and chloroform. Subsequently, the aqueous layer was extracted with chloroform, and the organic layers were combined, followed by washing with saturated brine and drying over sodium sulfate anhydrate. After a filtration step, the solvent was evaporated under reduced pressure, and the residue was purified through silica gel column chromatography (chloroform - acetone), to thereby give [N-(3-benzyloxycarbonylaminopropyl)-N-(tert-butoxycarbonyl)]aminoacetic acid benzyl ester as an oily product (4.77 g, 79%).

¹H-NMR(400MHz,CDCl₃)δ:1.55(9H,s), 1.60-1.75(2H,m), 3.22(2H,q,J=6.1Hz), 3.30-3.38(2H,m), 3.86(2H,br), 5.09(2H,s), 5.16(2H,s), 5.64(1H,br), 7.25-7.40(10H,m).
ESI-MSm/z:357(M-Boc+H)⁺.

### 3) [N-(3-Aminopropyl)-N-(tert-butoxycarbonyl)]aminoacetic acid

The above [N-(3-benzyloxycarbonylaminopropyl)-N-(tert-butoxycarbonyl)]aminoacetic acid benzyl ester (0.534 g) was dissolved in methanol (10 mL), and, at room temperature, 10% palladium-carbon (wet, 85.9 mg) was added to the solution, followed by stirring for 24 hours in a hydrogen atmosphere. Insoluble matter in the reaction mixture was removed through filtration, and the solvent of the filtrate was evaporated under reduced pressure, to thereby give [N-(3-aminopropyl)-N-(tert-butoxycarbonyl)]aminoacetic acid as a solid product (WO2004037169, 0.270 g, 99%).

¹H-NMR(400MHz,DMSO-d₆)δ:1.35(9H×3/5,s), 1.40(9H×2/5,s), 1.69-1.82(2H,m), 2.74-2.83(2H,m), 3.21-3.33(2H,m), 3.83(2H,s).
FAB-MSm/z:233(M+H)⁺.

### 4) Title Compound

The above [N-(3-aminopropyl)-N-(tert-butoxycarbonyl)]aminoacetic acid (0.279 g) was treated through the method described in WO2004037169 (BIFTU, Tesfaye *et al.),* to thereby give the title compound as a solid product.

¹H-NMR(400MHz,DMSO-d₆)δ:1.84-1.93(2H,m), 2.89(3H,s), 3.14-3.22(2H,m), 3.52-3.58(2H,m), 3.81-3.86(2H,m), 9.65(2H,br).
FAB-MSm/z:129(M+H)⁺.

[Referential Example 50] (1-Piperidin-2-ylcyclopropyl)carbamic acid tert-butyl ester

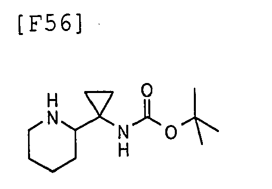

### 1) 2-(1-Aminocyclopropyl)pyridine

In an argon atmosphere, titanium chlorotriisopropoxide (10.3 mL) was added to a solution of 2-cyanopyridine (3.75 g) in tetrahydrofuran (200 mL) at room temperature, and the mixture was stirred for 7 minutes. Ethylmagnesium bromide (0.97M tetrahydrofuran solution, 86 mL) was added dropwise to the reaction mixture at room temperature over 5 minutes, and the resultant mixture was stirred for 55 minutes. Boron trifluoride diethyl ether complex (10.9 mL) was added to the reaction mixture at room temperature, and the resultant mixture was stirred for 55 minutes. The reaction mixture was made basic through use of 1M aqueous sodium hydroxide, and the resultant mixture was extracted with chloroform. The organic layer was dried over sodium sulfate anhydrate. After a filtration step, the solvent was evaporated under reduced pressure, and the residue was purified through silica gel column chromatography (chloroform - methanol), to thereby give 2-(1-aminocyclopropyl)pyridine as an oily product (2.554 g, 53%).
ESI-MSm/z:135(M+H)⁺.

### 2) (1-Pyridin-2-ylcyclopropyl)carbamic acid tert-butyl ester

The above 2-(.1-aminocyclopropyl)pyridine (513 mg) and di-tert-butyl dicarbonate (1.25 g) was dissolved in dichloromethane (50 mL), and, at room temperature, triethylamine (1.06 mL) was added to the solution, followed by stirring for 3 days. The reaction solvent was evaporated under reduced pressure, and the residue was purified through silica gel column chromatography (hexane - ethyl acetate), to thereby give (1-pyridin-2-ylcyclopropyl)carbamic acid tert-butyl ester as a solid product (563 mg, 63%).
ESI-MSm/z:235(M+H)⁺.

### 3) Title Compound

The above (1-pyridin-2-ylcyclopropyl)carbamic acid tert-butyl ester (277 mg) and 5% rhodium-alumina (200 mg) were suspended in acetic acid (1 mL) and ethanol (10 mL), and the suspension was stirred for 2 hours at room temperature in a hydrogen atmosphere (6 atm). The reaction mixture was filtrated through celite, and the solvent was evaporated under reduced pressure. The residue was partitioned between saturated aqueous sodium hydrogencarbonate and a chloroform-methanol (10:1) solvent mixture. The organic layer was dried over sodium sulfate anhydrate. After a filtration step, the solvent was evaporated under reduced pressure, to thereby give the title compound as a solid product (297 mg, quantitative amount).
ESI-MSm/z:241 (M+H)⁺.

[Example 1] 1-[5-(5-Cyano-2-pyridyl)-1-(3-pyridyl)-1H-pyrazole-3-carbonyl]-4-methylpiperazine

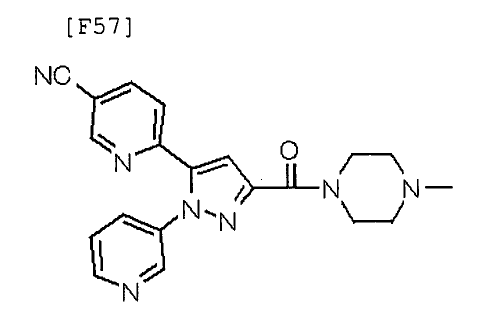

N-Methylpiperazine (0.200 mL) and triethylamine (0.184 mL) were added at room temperature to a solution of 5-(5-cyano-2-pyridyl)-1-(3-pyridyl)-1H-pyrazole-3-carboxylic acid (0.350 g) obtained from Referential Example 8, 1-(3-dimethylaminopropyl)-3-ethylcarbodiimide hydrochloride (0.253 g), and 1-hydroxybenzotriazole (0.179 g) in N,N-dimethylformamide (7 mL), and the mixture was stirred for 20 hours. The reaction mixture was partitioned between water and chloroform, and the solvent of the organic layer was evaporated under reduced pressure. The residue was purified by silica gel column chromatography (methanol - chloroform), to thereby give the title compound (0.372 g, 81%) as a solid product.

¹H-NMR(400MHz,CDCl₃)δ:2.34(3H,s), 2.46-2.53(4H,m), 3.86(2H,m), 4.11(2H,m), 7.30(1H,s), 7.39-7.92(1H,m), 7.65(1H,dd,J=8.3,1.0Hz), 7.71-7.74(1H,m), 8.01(1H,dd,J=8.3,2.2Hz), 8.54-8.55(1H,m), 8.64-8.68(2H,m).
EI-MSm/z:373(M⁺).
Elementary analysis: as C₂₀H₁₉N₇O·0.5H₂O
Calculated: C,62.81;H,5.27;N,25.64.
Found: C,62.68;H,5.00;N,25.54.

[Example 2] 1-[5-(5-Cyano-2-pyridyl)-1-(3-pyridyl)-1H-pyrazole-3-carbonyl]-4-cyclopropylpiperazine

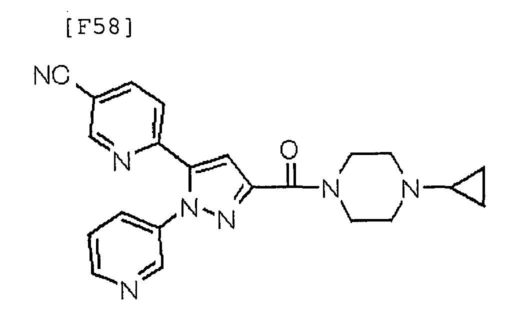

In a manner similar to that employed in Example 1, the title compound (0.316 g, 74%) in solid form was prepared from 5-(5-cyano-2-pyridyl)-1-(3-pyridyl)-1H-pyrazole-3-carboxylic acid (0.30 g) obtained in Referential Example 8 and 1-cyclopropylpiperazine hydrochloride (0.308 g) obtained in Referential Example 17.

¹H-NMR(400MHz,CDCl₃)δ:0.43-0.52(4H,m), 1.64-1.69(1H,m), 2.67-2.74(4H,m), 3.80-3.82(2H,m), 4.04-4.06(2H,m), 7.31(1H,s), 7.40-7.43(1H,m), 7.66(1H,dd,J=8.3,1.0Hz), 7.72-7.75(1H,m), 8.02(1H,dd,J=8.3,2.2Hz), 8.55-8.56(1H,m), 8.66(1H,dd,J=4.6,1.5Hz), 8.68-8.69(1H,m).
EI-MSm/z:399 (M⁺).

[Example 3] 1-[5-(5-Cyano-2-pyridyl)-1-(3-pyridyl)-1H-pyrazole-3-carbonyl]-4-fluoropiperidine

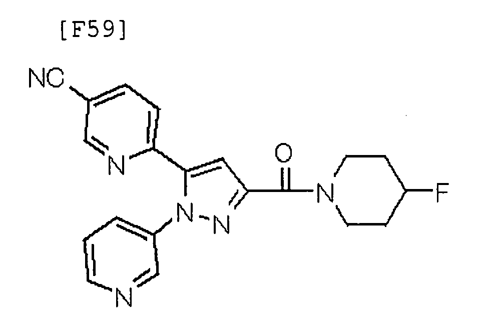

In a manner similar to that employed in Example 1, the title compound (0.277 g, 71%) in solid form was prepared from 5-(5-cyano-2-pyridyl)-1-(3-pyridyl)-1H-pyrazole-3-carboxylic acid (0.30 g) obtained in Referential Example 8 and 4-fluoropiperidine hydrochloride (0.216 g) obtained in Referential Example 13.

¹H-NMR(400MHz,CDCl₃)δ:1.95-2.01(4H,m), 3.69-4.28(4H,m), 4.88-5.02(1H,m), 7.31(1H,s), 7.42(1H,dd,J=8.2,4.8Hz), 7.66(1H,d,J=7.8Hz), 7.72-7.75(1H,m), 8.01-8.03(1H,m), 8.56(1H,d,J=2.4Hz), 8.66-8.67(1H,m), 8.69(1H,d,J=1.7Hz).
EI-MSm/z:376(M⁺).

[Example 4] 1-[5-(5-Cyano-2-pyridyl)-1-(3-pyridyl)-1H-pyrazole-3-carbonyl]-4-methoxypiperidine

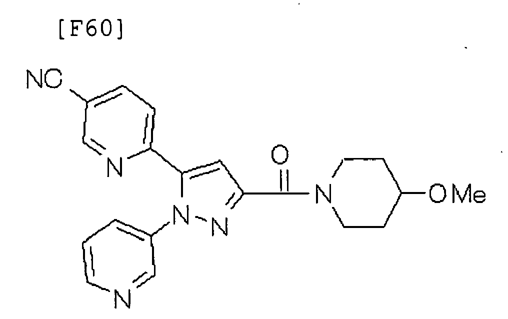

In a manner similar to that employed in Example 1, the title compound (0.300 g, 37%) in solid form was prepared from 5-(5-cyano-2-pyridyl)-1-(3-pyridyl)-1H-pyrazole-3-carboxylic acid (0.60 g) obtained in Referential Example 8 and 4-methoxypiperidine trifluoroacetic acid salt (0.944 g) obtained in Referential Example 11.

¹H-NMR(400MHz,CDCl₃)δ:1.70 (2H,m) , 1.95-1.96(2H,m), 3.39(3H,s), 3.49-3.60(2H,m), 3.76-3.80(1H,m), 4.06-4.08(1H,m), 4.26-4.28(1H,m), 7.28(1H,s), 7.39-7.42(1H,m), 7.65(1H,dd,J=8.2,0.9Hz), 7.72-7.75(1H,m), 8.01(1H,dd,J=8.2,2.1Hz), 8.54-8.55(1H,m), 8.65(1H,dd,J=4.6,1.5Hz), 8.67-8.68(1H,m).
EI-MSm/z:388 (M⁺).

[Example 5] 4-[5-(5-Cyano-2-pyridyl)-1-(3-pyridyl)-1H-pyrazole-3-carbonyl]morpholine

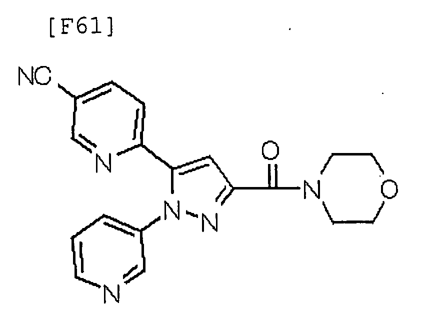

In a manner similar to that employed in Example 1, the title compound (0.355 g, 82%) in solid form was prepared from 5-(5-cyano-2-pyridyl)-1-(3-pyridyl)-1H-pyrazole-3-carboxylic acid (0.350 g) obtained in Referential Example 8 and morpholine (0.157 mL).

¹H-NMR(400MHz,CDCl₃)δ:3.74-3.83(6H,m), 4.16-4.18(2H,m), 7.34(1H,m), 7.41(1H,dd,J=8.2,4.8Hz), 7.65-7.67(1H,m), 7.71-7.74(1H,m), 8.00-8.03(1H,m), 8.54(1H,d,J=2.4Hz), 8.65-8.68(2H,m).
EI-MSm/z:360(M⁺).

[Example 6] 1-[5-(5-Cyano-2-pyridyl)-1-(3-pyridyl)-1H-pyrazole-3-carbonyl]piperidine

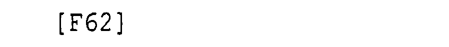

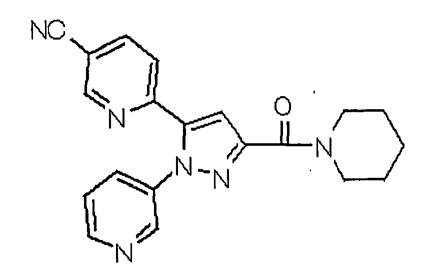

In a manner similar to that employed in Example 1, the title compound (0.353 g, 81%) in solid form was prepared from 5-(5-cyano-2-pyridyl)-1-(3-pyridyl)-1H-pyrazole-3-carboxylic acid (0.350 g) obtained in Referential Example 8 and piperidine (0.178 mL).

¹H-NMR(400MH,CDCl₃)δ:1.61-1.70(6H,m), 3.75-3.77(2H,m), 3.91-3.94(2H,m), 7.25(1H,s), 7.40(1H,dd,J=8.2,4.8Hz), 7.63-7.65(1H,m), 7.72-7.75(1H,m), 7.99-8.02(1H,m), 8.54(1H,d,J=2.4Hz), 8.64(1H,dd,J=4.8,1.6Hz), 8.67-8.68(1H,m).
EI-MSm/z:358(M⁺).
Elementary analysis: as C₂₀H₁₈N₆O·0.25H₂O
Calculated: C,66.19;H,5.14;N,23.16.
Found: C,66.28;H,5.00;N,23.25.

[Example 7] 1-[5-(5-Carbamoyl-2-pyrazinyl)-1-(3-pyridyl)-1H-pyrazole-3-carbonyl]-4,4-difluoropiperidine

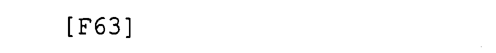

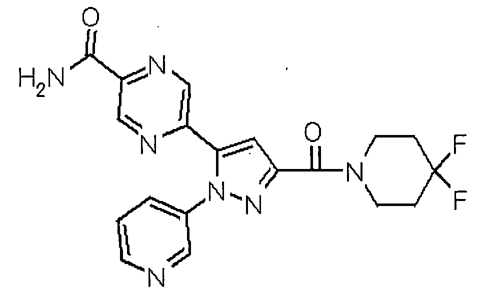

In a manner similar to that employed in Example 1, the title compound (0.238 g, 82%) in solid form was prepared from 5-(5-carbamoyl-2-pyrazinyl)-1-(3-pyridyl)-1H-pyrazole-3-carboxylic acid (0.215 g) obtained in Referential Example 7 and 4,4-difluoropiperidine hydrochloride (0.164 g) obtained in Referential Example 12.

¹H-NMR(400MHz,DMSO-d₆)δ:2.08(4H,m), 3.81(2H,m), 4.07(2H,m), 7.50-7.53(1H,m), 7.64(1H,s), 7.89-7.92(2H,m), 8.28(1H,br s), 8.64(1H,dd,J=4.9,1.2Hz), 8.67(1H,d,J=2.7Hz), 8.93(1H,d,J=1.2Hz), 9.10(1H,d,J=1.2Hz).
EI-MSm/z:413(M⁺).

[Example 8] 1-[5-(5-Carbamoyl-2-pyridyl)-1-(3-pyridyl)-1H-pyrazole-3-carbonyl]-4-fluoropiperidine

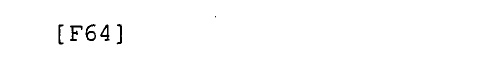

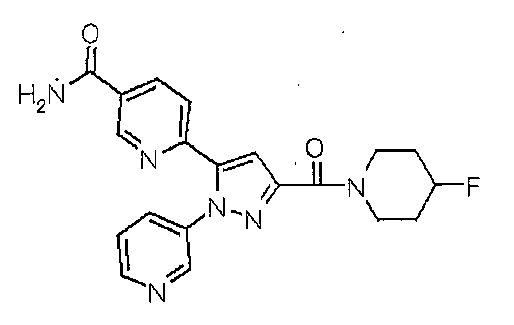

1N Aqueous sodium hydroxide (2.51 mL) was added at room temperature to a solution of 1-[5-(5-cyano-2-pyridyl)-1-(3-pyridyl)-1H-pyrazole-3-carbonyl]-4-fluoropiperidine (0.190 g) obtained from Example 3 in a solvent mixture of methanol (3.8 mL) and tetrahydrofuran (3.8 mL), and the resultant mixture was stirred at 80°C for 15 minutes. The reaction mixture was cooled in air, and was partitioned between water and methanol - chloroform (1:10) solvent mixture. The solvent of the organic layer was evaporated under reduced pressure, and the residue was purified by silica gel column chromatography (methanol - chloroform), to thereby give the title compound (0.129 g, 64%) as a solid product.

¹H-NMR(400MHz,DMSO-d₆)δ:1.77-1.96(4H,m), 3.73-3.94(4H,m), 4.88-5.01(1H,m), 7.35(1H,s), 7.49-7.52(1H,m), 7.63(1H,br s), 7.81-7.84(1H,m), 7.88(1H,d,J=8.3Hz), 8.15(1H,br s), 8.28-8.31(1H,m), 8.57(1H,d,J=2.4Hz), 8.61(1H,dd,J=9.8,1.3Hz), 8.83(1H,d,J=1.7Hz).
EI-MSm/z:394 (M⁺).
Elementary analysis: as C₂₀H₁₉FN₆O₂·0.5H₂O
Calculated: C,59.55;H,5.00;N,20.83;F,4.71.
Found: C,59.62;H,4.87;N,20.77;F,4.68.
[Example 9] 1-[5-(5-Carbamoyl-2-pyridyl)-1-(3-pyridyl)-1H-pyrazole-3-carbonyl]-4-methoxypiperidine

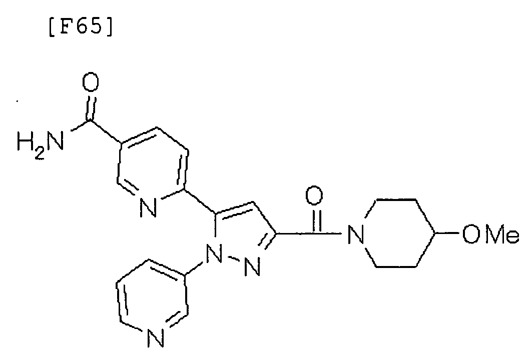

In a manner similar to that employed in Example 8, the title compound (0.125 g, 63%) in solid form was prepared from 1-[5-(5-cyano-2-pyridyl)-1-(3-pyridyl)-1H-pyrazole-3-carbonyl]-4-methoxypiperidine (0.190 g) obtained in Example 4.

¹H-NMR(400MHz,DMSO-d₆)δ:1.47-1.50(2H,m), 1.88(2H,m), 3.28(3H,s), 3.33-3.63(3H,m), 3.97(1H,m), 4.14(1H,m), 7.32(1H,s), 7.49-7.52(1H,m), 7.62(1H,br s), 7.80-7.83(1H,m), 7.87(1H,dd,J=8.3,0.7Hz), 8.14(1H,br s), 8.28-8.30(1H,m), 8.56 (1H, d, J=2.2Hz), 8.61 (1H, dd, J=4.9,1.5Hz), 8.82-8.83(1H,m).
EI-MSm/z: 406 (M⁺).
Elementary analysis: as C₂₁H₂₂N₆O₃·0.25H₂O
Calculated: C,61.38;H,5.52;N,20.45.
Found: C,61.36;H,5.44;N,20.49.

[Example 10] 1-[5-(5-Carbamoyl-2-pyridyl)-1-(3-pyridyl)-1H-pyrazole-3-carbonyl]piperidine

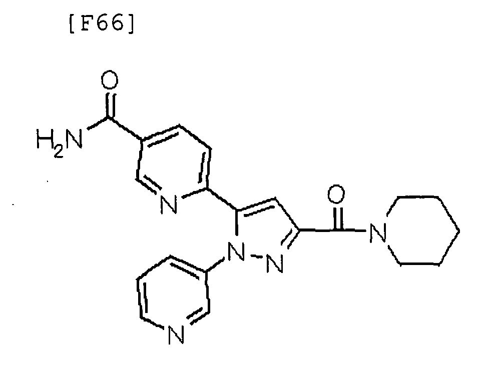

In a manner similar to that employed in Example 8, the title compound (0.197 g, 64%) in solid form was prepared from 1-[5-(5-cyano-2-pyridyl)-1-(3-pyridyl)-1H-pyrazole-3-carbonyl]piperidine (0.294 g) obtained in Example 6.

¹H-NMR(400MHz,DMSO-d₆)δ:1.56-1.65(6H,m), 3.61-3.64(2H,m), 3.80-3.83(2H,m), 7.29(1H,s), 7.48-7.51(1H,m), 7.62(1H,br s), 7.79-7.82(1H,m), 7.86(1H,dd,J=8.2,0.9Hz), 8.19(1H,br s), 8.27-8.29(1H,m), 8.54-8.55(1H,m), 8.60(1H,dd,J=4.6,1.5Hz), 8.81-8.82(1H,m).
EI-MSm/z:376 (M⁺).
Elementary analysis: as C₂₀H₂₀N₆O₂·0.25H₂O
Calculated: C,63.06;H,5.42;N,22.06.
Found: C,62.81;H,5.28;N,21.88.

[Example 11] 1-[5-(5-Hydroxy-2-pyridyl)-1-(3-pyridyl)-1H-pyrazole-3-carbonyl]-4,4-difluoropiperidine

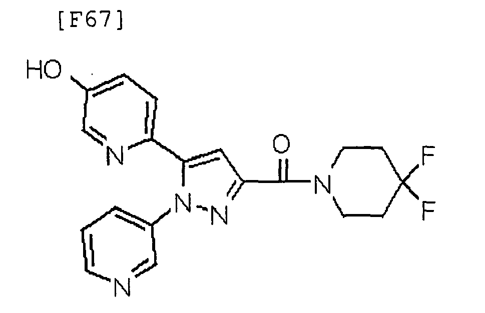

### 1) 1-[5-(5-Benzyloxy-2-pyridyl)-1-(3-pyridyl)-1H-pyrazole-3-carbonyl]-4,4-difluoropiperidine

In a manner similar to that employed in Example 1, 1-[5-(5-benzyloxy-2-pyridyl)-1-(3-pyridyl)-1H-pyrazole-3-carbonyl]-4,4-difluoropiperidine (2.34 g, 95%) in solid form was prepared from 5-(5-benzyloxy-2-pyridyl)-1-(3-pyridyl)-1H-pyrazole-3-carboxylic acid (1.93 g) obtained in Referential Example 4 and 4,4-difluoropiperidine hydrochloride (0.980 g) obtained in Referential Example 12.

¹H-NMR(400MHz,CDCl₃)δ:2.06-2.12 (4H,m), 3.92(2H,m), 4.20(2H,m), 5.11(2H,s), 7.08(1H,s), 7.26-7.30(1H,m), 7.34-7.42(7H,m), 7.71-7.74(1H,m), 8.24(1H,d,J=2.9Hz), 8.53(1H,d,J=2.7Hz), 8.58(1H,dd,J=4.6,1.5Hz).
EI-MSm/z: 475 (M⁺).

### 2) Title Compound

10% Palladium-carbon (2.33 g) was added to a solution of 1-[5-(5-benzyloxy-2-pyridyl)-1-(3-pyridyl)-1H-pyrazole-3-carbonyl]-4,4-difluoropiperidine (2.33 g) in a solvent mixture of methanol (47 mL) and ethyl acetate (47 mL), and the resultant mixture was stirred in a hydrogen flow at room temperature for 1.5 hours. The catalyst was removed from the reaction mixture by filtration, and the solvent of the filtrate was evaporated under reduced pressure. The residue was purified by silica gel column chromatography (methanol - chloroform), to thereby give the title compound (1.68 g, 89%) as a solid product.

¹H-NMR(400MHz,DMSO-d₆)δ:2.06(4H,m), 3.77(2H,m), 4.05(2H,m), 7.08(1H,s), 7.22(1H,dd,J=8.5,2.9Hz), 7.45-7.48(1H,m), 7.52-7.54(1H,m), 7.73-7.76(1H,m), 7.95(1H,d,J=2.4Hz), 8.51(1H,d,J=2.4Hz), 8.56(1H,dd,J=4.9,1.5Hz), 10.33(1H,br s).
EI-MSm/z: 385 (M⁺).

[Example 12] 1-[5-(5-Cyano-2-pyridyl)-1-(3-pyridyl)-1H-pyrazole-3-carbonyl]-4,4-difluoropiperidine

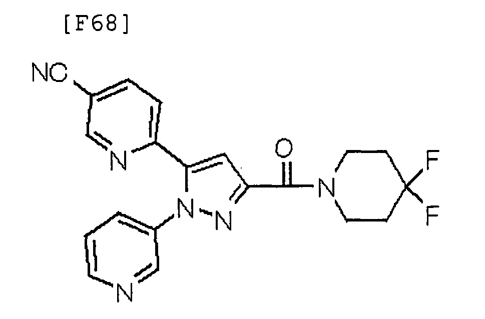

### 1) 1-[5-(5-Trifluoromethanesulfonyloxy-2-pyridyl)-1-(3-pyridyl)-1H-pyrazole-3-carbonyl]-4,4-difluoropiperidine

In a manner similar to that employed in step 2) of Referential Example 8, 1-[5-(5-trifluoromethanesulfonyloxy-2-pyridyl)-1-(3-pyridyl)-1H-pyrazole-3-carbonyl]-4,4-difluoropiperidine (1.67 g, 98%) in solid form was prepared from 1-[5-(5-hydroxy-2-pyridyl)-1-(3-pyridyl)-1H-pyrazole-3-carbonyl]-4,4-difluoropiperidine (1.27 g) obtained in Example 11 and trifluoromethanesulfonic acid anhydrate (0.665 mL).

¹H-NMR(400MHz,CDCl₃)δ:2.09(4H,m), 3.93(2H,m), 4.21(2H,m), 7.26(1H,m), 7.38-7.41(1H,m), 7.63-7.71(3H,m), 8.41(1H,m), 8.56-8.57(1H,m), 8.64-8.65(1H,m).
EI-MSm/z:517(M⁺).

### 2) Title Compound

In an argon atmosphere, a suspension of cyanotri(n-butyl)tin (4.06 g) and tetrakis(triphenylphosphine)palladium(0) (5.56 g) in dichloroethane (45 mL) was refluxed for 2 hours. To the reaction mixture was added dropwise a solution of 1-[5-(5-trifluoromethanesulfonyloxy-2-pyridyl)-1-(3-pyridyl)-1H-pyrazole-3-carbonyl]-4,4-difluoropiperidine (1.66 g) in dichloroethane (37 mL), and the resultant mixture was refluxed for 22 hours, followed by cooling in air. Saturated aqueous sodium hydrogencarbonate was added to the reaction mixture, and the resultant mixture was filtered through Celite. The filtrate was partitioned between water and chloroform, and the organic layer was dried over sodium sulfate anhydrate. After a filtration step, the solvent was evaporated under reduced pressure, and the residue was purified by silica gel column chromatography (hexane - ethyl acetate), to thereby give the title compound (1.03 g, 80%) as a solid product.

¹H-NMR(400MHz,CDCl₃)δ:2.10(4H,m), 3.93(2H,m), 4.21(2H,m), 7.33-7.34(1H,m), 7.40-7.43(1H,m), 7.64-7.66(1H,m), 7.71-7.73(1H,m), 8.00-8.03(1H,m), 8.54(1H,d,J=2.4Hz), 8.66-8.68(2H,m).
EI-MSm/z:394(M⁺).
Elementary analysis: as C₂₀H₁₆F₂N₆O·0.5H₂O
Calculated: C,59.55;H,4.25;N,20.83;F,9.42.
Found: C,59.42;H,4.12;N,20.67;F,9.57.

[Example 13] 1-[5-(5-Carbamoyl-2-pyridyl)-1-(3-pyridyl)-1H-pyrazole-3-carbonyl]-4,4-difluoropiperidine

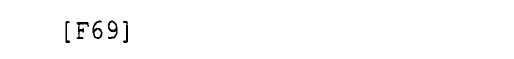

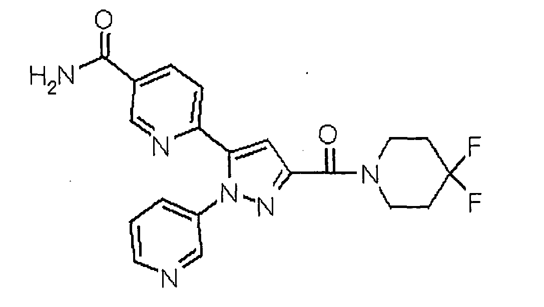

In a manner similar to that employed in Example 8, the title compound (0.109 g, 27%) in solid form was prepared from 1-[5-(5-cyano-2-pyridyl)-1-(3-pyridyl)-1H-pyrazole-3-carbonyl]-4,4-difluoropiperidine (0.40 g) obtained in Example 12.

¹H-NMR(400MHz,DMSO-d₆)δ:2.07(4H,m), 3.79(2H,m), 4.06(2H,m), 7.39(1H,s), 7.49-7.53(1H,m), 7.64(1H,br s), 7.82-7.85(1H,m), 7.89(1H,dd,J=8.2,0.9Hz), 8.16(1H,br s), 8.29-8.31(1H,m), 8.58-8.59(1H,m), 8.62(1H,dd,J=4.6,1.5Hz), 8.82-8.83(1H,m).
EI-MSm/z : 412 (M⁺).
Elementary analysis: as C₂₀H₁₈F₂Nr₆O₂
Calculated: C,58.25;H,4.40;N,20.38;F,9.21.
Found: C,58.03;H,4.36;N,20.26;F,9.31.

[Example 14] 1-[5-(5-Methoxy-2-pyridyl)-1-(3-pyridyl)-1H-pyrazole-3-carbonyl]-4,4-difluoropiperidine

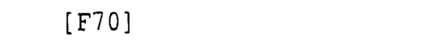

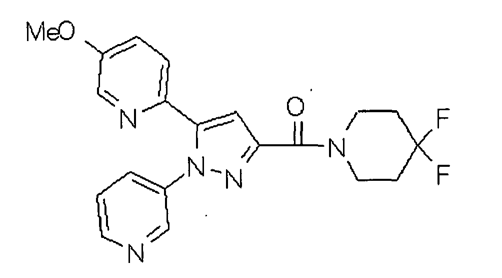

In an argon atmosphere, (trimethylsilyl)diazomethane (2.0M hexane solution, 0.623 mL) was added dropwise at room temperature to a solution of 1-[5-(5-hydroxy-2-pyridyl)-1-(3-pyridyl)-1H-pyrazole-3-carbonyl]-4,4-difluoropiperidine (0.40 g) obtained in Example 11 in a solvent mixture of methanol (8m), tetrahydrofuran (8 mL), and dichloromethane (8 mL), and the resultant mixture was stirred for 3 hours. (Trimethylsilyl)diazomethane (2.0M hexane solution, 1.246 m) was further added to the reaction mixture, and the resultant mixture was stirred for 2.5 hours. The reaction solvent was evaporated under reduced pressure, and the residue was partitioned between saturated aqueous sodium hydrogencarbonate and chloroform. The organic layer was dried over sodium sulfate anhydrate. After a filtration step, the solvent was evaporated under reduced pressure, and the residue was purified by silica gel column chromatography (chloroform - ethyl acetate), to thereby give the title compound (0.194 g, 46%) as a solid product.

¹H-NMR(400MHz,CDCl₃)δ:2.06-2.12(4H,m),3.87(3H,s), 3.93(2H,m), 4.20(2H,m), 7.08(1H,s), 7.21-7.24(1H,m), 7.34-7.37(1H,m), 7.42(1H,d,J=8.8Hz), 7.71-7.74(1H,m), 8.17(1H,d,J=2.9Hz), 8.53(1H,d,J=2.4Hz), 8.58(1H,dd,J=4.6,1.5Hz).
EI-MSm/z:399(M⁺).

[Example 15] 1-[5-(5-Methyl-2-pyridyl)-1-(3-pyridyl)-1H-pyrazole-3-carbonyl]-4,4-difluoropiperidine

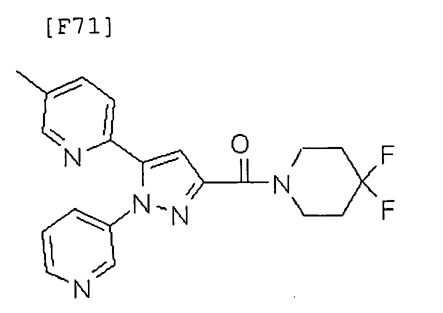

In a manner similar to that employed in Example 1, the title compound (360 mg, 40%) in solid form was prepared from 5-(5-methyl-2-pyridyl)-1-(3-pyridyl)-1H-pyrazole-3-carboxylic acid (665 mg) obtained in Referential Example 9 and 4,4-difluoropiperidine hydrochloride (681 mg) obtained in Referential Example 12.

¹H-NMR(400MHz,CDCl₃)δ:2.08 (4H,m), 2.35(3H,s), 3.93(2H,br), 4.20(2H,br), 7.13(1H,s), 7.35(2H,dd,J=8.18,4.70Hz), 7.53(1H,d,J=8.06Hz), 7.73(1H,ddd,J=8.18,2.44,1.53Hz) 8.31(1H,s), 8.52(1H,d,J=2.44Hz), 8.58(1H,dd,J=4.70,1.53Hz).
FAB-MS m/z:384(M+H)⁺.
Elementary analysis: as C₂₀H₁₉F₂N₅O
Calculated: C,62.65;H,5.00;N,18.27;F,9.91.
Found: C,62.52;H,4.85;N,18.21;F,9.71.

[Example 16] 1-[5-(5-Chloro-2-pyridyl)-1-(3-pyridyl)-1H-pyrazole-3-carbonyl]-4,4-difluoropiperidine

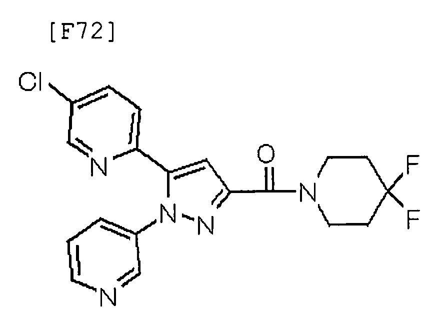

In a manner similar to that employed in Example 1, the title compound (115 mg, 26%) in solid form was prepared from 5-(5-chloro-2-pyridyl)-1-(3-pyridyl)-1H-pyrazole-3-carboxylic acid (325 mg) obtained in Referential Example 5 and 4,4-difluoropiperidine hydrochloride (196 mg) obtained in Referential Example 12.

¹H-NMR(400MHz,CDCl₃)δ:2.10(4H,m), 3.93(2H,br), 4.21(2H,br), 7.19(1H,s), 7.38(1H,dd,J=8.18,4.76Hz), 7.46(1H,d,J=8.42Hz), 7.71(1H,dd,J=8.18,1.47Hz), 7.72(1H,dd,J=8.42,2.44Hz), 8.41(1H,d,J=2.44Hz), 8.54(1H,d,J=2.44Hz), 8.62(1H,dd,J=4.76,1.47Hz).
FAB-MS m/z:404(M+H)⁺.

[Example 17] 1-[5-(5-Methyl-2-pyrazinyl)-1-(3-pyridyl)-1H-pyrazole-3-carbonyl]-4-methoxypiperidine

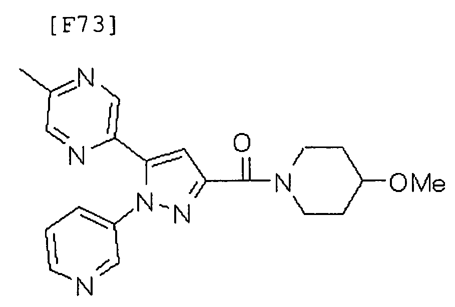

In a manner similar to that employed in Example 1, the title compound (0.304 g, 88%) in solid form was prepared from 5-(5-methyl-2-pyrazinyl)-1-(3-pyridyl)-1H-pyrazole-3-carboxylic acid (0.250 g) obtained in Referential Example 6 and 4-methoxypiperidine hydrochloride (0.150 g) obtained in Referential Example 27.

¹H-NMR(400MHz,CDCl₃)δ:1.70(2H,m), 1.95(2H,m), 2.58(3H,s), 3.39(3H,s), 3.49-3.59(2H,m), 3.75-3.79(1H,m), 4.09(1H,m), 4.28(1H,m), 7.19(1H,s), 7.37-7.40(1H,m), 7.74-7.77(1H,m), 8.31(1H,d,J=1.5Hz), 8.55(1H,d,J=2.0Hz), 8.61(1H,dd,J=4.9,1.5Hz), 8.66(1H,d,J=1.5Hz).
EI-MSm/z:378 (M⁺).
Elementary analysis: as C₂₀H₂₂N₆O₂·0.5H₂O
Calculated: C,62.00;H,5.98;N,21.69.
Found: C,61.89;H,5.70;N,21.50.

[Example 18] 1-[5-(5-Methyl-2-pyridyl)-1-(2-pyrazinyl)-1H-pyrazole-3-carbonyl]-4-methoxypiperidine

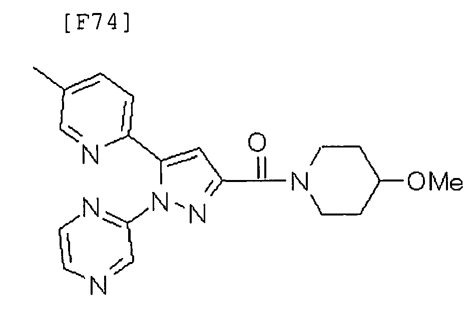

1-(3-Dimethylaminopropyl)-3-ethylcarbodiimide hydrochloride (0.374 g), 1-hydroxybenzotriazole (0.139 g), 4-methoxypiperidine hydrochloride (0.216 g) obtained from Referential Example 27, and triethylamine (0.565 mL) were added at room temperature to a solution of 5-(5-methyl-2-pyridyl)-1-(2-pyrazinyl)-1H-pyrazole-3-carboxylic acid (0.253 g) obtained from Referential Example 35 in dichloromethane (8 mL), and the mixture was stirred for 22 hours. The reaction mixture was partitioned between water and chloroform, and the aqueous layer was further extracted with chloroform. The organic layers were combined, and the combined organic layer was washed with saturated brine, followed by drying over sodium sulfate anhydrate. After a filtration step, the solvent was evaporated under reduced pressure, and the residue was purified by silica gel column chromatography (methanol - chloroform), to thereby give the title compound (0.149 g, 44%) as a solid product.

¹H-NMR(400MHz,CDCl₃)δ:1.55-1.78(2H,m), 1.87-2.04(2H,m), 2.35(3H,s), 3.38(3H,s), 3.47-3.63(2H,m), 3.72-3.84(1H,m), 4.02-4.13(1H,m), 4.20-4.32(1H,m), 7.05(1H,s), 7.45(1H,d,J=7.8Hz), 7.56(1H,dd,J=7.8,2.0Hz), 8.20-8.30(2H,m), 8.51(1H,d,J=2.5Hz), 8.97(1H,d,J=1.4Hz).
ESI-MS m/z:379(M+H)⁺.

[Example 19] 1-[5-(5-Methyl-2-pyridyl)-1-(2-pyrazinyl)-1H-pyrazole-3-carbonyl]-4,4-difluoropiperidine

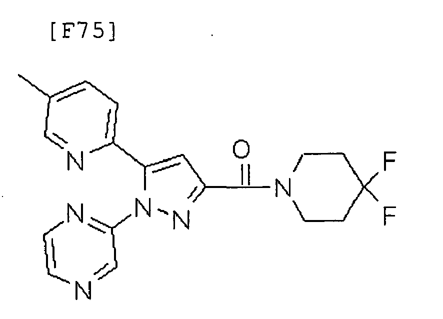

In a manner similar to that employed in Example 18, the title compound (0.204 g, 63%) in solid form was prepared from 5-(5-methyl-2-pyridyl)-1-(2-pyrazinyl)-1H-pyrazole-3-carboxylic acid (0.238 g) obtained in Referential Example 35 and 4,4-difluoropiperidine hydrochloride (0.200 g) obtained from Referential Example 12.

¹H-NMR(400MHz,CDCl₃)δ:2.03-2.17(4H,m), 2.35(3H,s), 3.90-3.97(2H,br), 4.16-4.25(2H,br), 7.11(1H,s), 7.46(1H,d like,J=7.8Hz), 7.55-7.61(1H,m), 8.21-8.25(1H,m), 8.25-8.29(1H,m), 8.54(1H,d,J=2.7Hz), 8.94(1H,d,J=1.5Hz).
ESI-MS m/z:385(M+H)⁺.

[Example 20] 1-[5-(5-Chloro-2-pyridyl)-1-(3-pyridyl)-1H-pyrazole-3-carbonyl]hexahydropyridazine

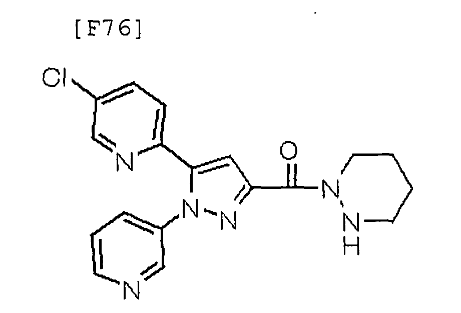

In a manner similar to that employed in Example 18, the title compound (0.204 g, 58%) in solid form was prepared from 5-(5-chloro-2-pyridyl)-1-(3-pyridyl)-1H-pyrazole-3-carboxylic acid (0.288 g) obtained in Referential Example 5 and hexahydropyridazine hydrochloride (0.14 g) obtained in Referential Example 14.

¹H-NMR(400MHz,CDCl₃)δ:1.60-1.80(4H,m), 3.00(2H,br), 3.84(2H×1/3,br), 4.23(2H×2/3,br), 6.64(1H×2/3,s), 7.10-7.45 (2H+1H×1/3,m), 7.62-7.80(2H,m), 8.35-8.61(3H,m).
ESI-MS m/z:369(M+H)⁺.
Elementary analysis: as C₁₈H₁₇ClN₆O
Calculated: C,58.62;H,4.65;N,22.79;Cl,9.61.
Found: C,58.48;H,4.69;N,22.71;Cl,9.70.

[Example 21] 1-[5-(5-Chloro-2-pyridyl)-1-(3-pyridyl)-1H-pyrazole-3-carbonyl]-2-carbamoylhexahydropyridazine

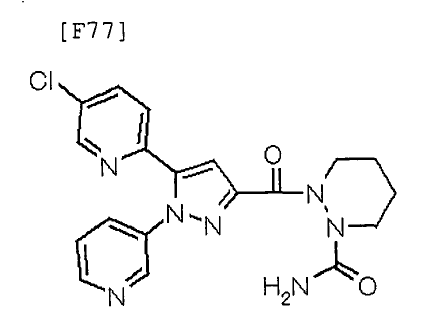

Trimethylsilyl isocyanate (1.20 mL) was added at room temperature to a solution of 1-[5-(5-chloro-2-pyridyl)-1-(3-pyridyl)-1H-pyrazole-3-carbonyl]hexahydropyridazine (0.153 g) obtained from Example 20 in 1,4-dioxane (2.0 mL), and the mixture was stirred in a sealed tube at an external temperature of 110°C for 24 hours. The reaction mixture was cooled in air, and methanol was added thereto. The reaction solvent was evaporated under reduced pressure, and the residue was partitioned between saturated aqueous sodium hydrogencarbonate and methanol - chloroform (1:20) solvent mixture. The aqueous layer was further extracted with methanol - chloroform (1:20) solvent mixture, and the organic layers were combined. The combined organic layer was washed with saturated brine, and was dried over sodium sulfate anhydrate. After a filtration step, the solvent was evaporated under reduced pressure, and the residue was purified by silica gel column chromatography (methanol - chloroform), to thereby give the title compound (0.103 g, 61%) as a solid product.

¹H-NMR(400MHz,CDCl₃)δ:1.63-2.00(4H,m), 2.89(1H,br t,J=11.5Hz), 3.06(1H,br), 4.42(1H,br d,J=13.0Hz), 4.67(1H,br), 5.46(2H,br), 7.10(1H,s), 7.35(1H,dd,J=4.9,8.1Hz), 7.39(1H,d,J=8.3Hz), 7.69(1H,dd,J=2.5,8.3Hz), 7.74-7.81(1H,m), 8.40(1H,d like,J=2.5Hz), 8.46(1H,d like,J=2.2Hz), 8.57(1H,d like,J=3.7Hz).
ESI-MS m/z:412 (M+H)⁺.

[Example 22] 1-[5-(5-Amino-2-pyridyl)-1-(2-pyrazinyl)-1H-pyrazole-3-carbonyl]-4,4-difluoropiperidine

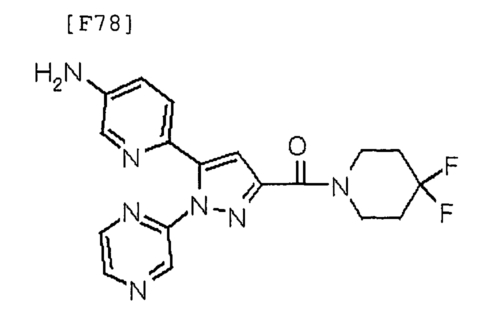

### 1) 1-[5-(5-tert-Butoxycarbonylamino-2-pyridyl)-1-(2-pyrazinyl)-1H-pyrazole-3-carbonyl]-4,4-difluoropiperidine

In a manner similar to that employed in Example 1, 1-[5-(5-tert-butoxycarbonylamino-2-pyridyl)-1-(2-pyrazinyl)-1H-pyrazole-3-carbonyl]-4,4-difluoropiperidine (87 mg, 94%) in solid form was prepared from 5-(5-tert-butoxycarbonylamino-2-pyridyl)-1-(2-pyrazinyl)-1H-pyrazole-3-carboxylic acid (73 mg) obtained in Referential Example 20 and 4,4-difluoropiperidine hydrochloride (90 mg) obtained in Referential Example 12.

¹H-NMR(400MHz,CDCl₃)δ:1.53(9H,s), 2.04-2.16(4H,m), 3.90-3.96(2H,m), 4.16-4.24(2H,m), 6.56(1H,br s), 7.10(1H,s), 7.51(1H,d,J=8.5Hz), 8.00-8.06(1H,m), 8.24(1H,d,J=2.4Hz), 8.28(1H,dd,J=2.4,1.5Hz), 8.55(1H,d,J=2.4Hz), 8.94(1H,d,J=1.5Hz).
ESI-MS m/z:486(M+H)⁺.

### 2) Title Compound

Trifluoroacetic acid (2 mL) was added at room temperature to a solution of the above-prepared 1-[5-(5-tert-butoxycarbonylamino-2-pyridyl)-1-(2-pyrazinyl)-1H-pyrazole-3-carbonyl]-4,4-difluoropiperidine (85 mg) in dichloromethane (2 mL), and the mixture was stirred for 30 minutes. The reaction solvent was evaporated under reduced pressure, and the residue was partitioned between saturated aqueous sodium hydrogencarbonate and ethyl acetate. The organic layer was washed with saturated brine, and was dried over sodium sulfate anhydrate. After a filtration step, the solvent was evaporated under reduced pressure, to thereby give the title compound (72 mg, 97%) as a solid product.

¹H-NMR(400MHz,CDCl₃)δ:2.03-2.15(4H,m), 3.82-3.97(4H,br m), 4.16-4.23(2H,br m), 7.01(1H,dd,J=8.5,2.7Hz), 7.03(1H,s) 7.35(1H,d,J=8.5Hz), 7.85(1H,d,J=2.7Hz), 8.32(1H,dd,J=2.4,1.2Hz), 8.54(1H,d,J=2.4Hz), 8.88(1H,d,J=1.2Hz).
ESI-MS m/z:386(M+H)⁺.

[Example 23] 1-[5-(5-Chloro-2-pyridyl)-1-(3-pyridyl)-1H-pyrazole-3-carbonyl]-4-methylpiperazine

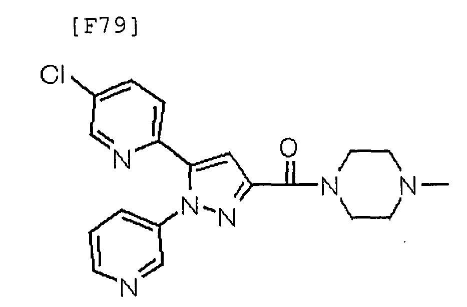

In a manner similar to that employed in Example 1, the title compound (62 mg, 16%) in solid form was prepared from 5-(5-chloro-2-pyridyl)-1-(3-pyridyl)-1H-pyrazole-3-carboxylic acid (300 mg) obtained in Referential Example 5 and N-methylpiperazine (100 mg).

¹H-NMR(400MHz,CDCl₃)δ:2.33(3H,s), 2.46-2.35(4H,m), 3.80-3.90(2H,m), 4.05-4.15(2H,m), 7.16(1H,s), 7.35-7.39(1H,m), 7.42-7.46(1H,m), 7.70-7.74(2H,m), 8.40-8.41(1H,m), 8.54(1H,d,J=2.4Hz), 8.60-8.61(1H,m).
EI-MSm/z:382 (M⁺).

[Example 24] 1-[5-(5-Chloro-2-pyridyl)-1-(3-pyridyl)-1H-pyrazole-3-carbonyl]-4-methyl-3-oxopiperazine

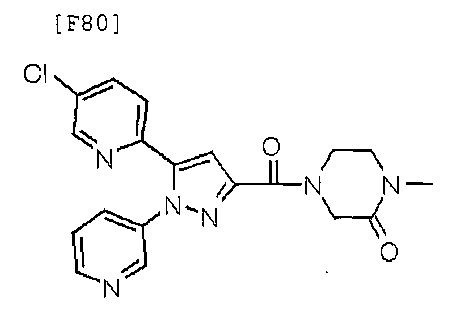

In a manner similar to that employed in Example 1, the title compound (101 mg, 25.5%) in solid form was prepared from 5-(5-chloro-2-pyridyl)-1-(3-pyridyl)-1H-pyrazole-3-carboxylic acid (300 mg) obtained in Referential Example 5 and 1-methylpiperazin-2-one hydrochloride (151 mg) obtained in Referential Example 15.

¹H-NMR(400MHz,CDCl₃)δ:3.07(3H,s), 3.42-3.50(2H,m), 4.00-4.10(1H,m), 4.40-4.50(2H,m), 4.80-4.90 (1H,m), 7.20-7.27(1H,m), 7.35-7.50(2H,m), 8.40(1H,d,J=2.3Hz), 8.49-8.63(2H,m).
EI-MSm/z:396(M⁺).

[Example 25] 1-[5-(5-Chloro-2-pyridyl)-1-(3-pyridyl)-1H-pyrazole-3-carbonyl]-2-carbamoylpiperidine

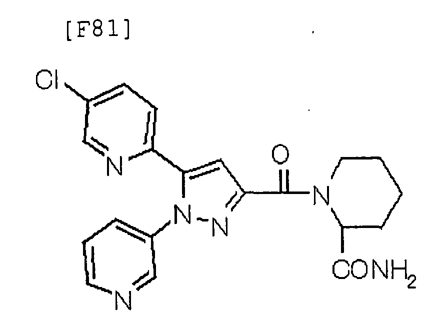

In a manner similar to that employed in Example 1, the title compound (37 mg, 9%) in solid form was prepared from 5-(5-chloro-2-pyridyl)-1-(3-pyridyl)-1H-pyrazole-3-carboxylic acid (300 mg) obtained in Referential Example 5 and piperidine-2-carboxamide (130 mg) obtained in Referential Example 19.

¹H-NMR(400MHz,CDCl₃)δ:1.50-1.90(6H,m), 2.30-2.40(1H,m), 2.80-2.90(1/2×1H,m), 4.70-4.80(1H,m), 7.18(1H,d,J=15.1Hz), 7.36-7.39(1H,m), 7.65-7.80(2H,m), 8.41(1H,s), 8.49(1/2×1H,s), 8.55(1/2×1H,s), 8.61(1H,d,J=4.6Hz).
EI-MSm/z:410(M⁺).

[Example 26] 1-[5-(5-Chloro-2-pyridyl)-1-(3-pyridyl)-1H-pyrazole-3-carbonyl]-3-dimethylaminoazetidine

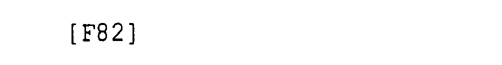

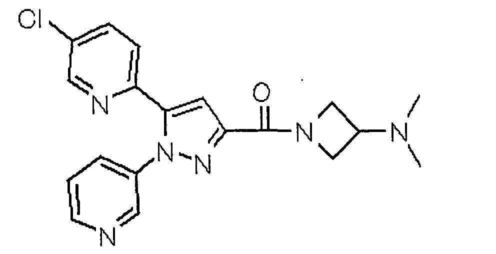

In a manner similar to that employed in Example 1, the title compound (78 mg, 20%) in solid form was prepared from 5-(5-chloro-2-pyridyl)-1-(3-pyridyl)-1H-pyrazole-3-carboxylic acid (300 mg) obtained in Referential Example 5 and azetidine-3-yldimethylamine hydrochloride (174 mg) obtained in Referential Example 18.

¹H-NMR(400MHz,CDCl₃)δ:2.21(6H,s), 3.10-3.20(1H,m), 4.00-4.07(1H,m), 4.20-4.25(1H,m), 4.40-4.45(1H,m), 4.60-4.70(1H,m), 7.32-7.35(1H,m), 7.47(1H,d,J=8.5Hz), 7.68-7.72(2H,m), 8.39(1H,d,J=2.4Hz), 8.57-8.62(2H,m).
FAB-MS m/z:383(M+H)⁺.

[Example 27] 1-[5-(5-Methyl-2-pyridyl)-1-(3-pyridyl)-1H-pyrazole-3-carbonyl]-4-fluoromethylpiperidine

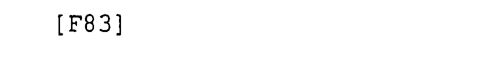

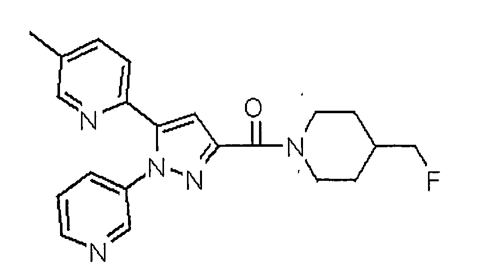

In a manner similar to that employed in Example 18, the title compound (86 mg, 18%) in solid form was prepared from 5-(5-methyl-2-pyridyl)-1-(3-pyridyl)-1H-pyrazole-3-carboxylic acid (350 mg) obtained in Referential Example 9 and 4-fluoromethylpiperidine hydrochloride (384 mg) obtained in Referential Example 29.

¹H-NMR(400MHz,CDCl₃)δ:1.40(2H,m), 1.83(2H,d,J=5.75Hz), 2.04(1H,m), 2.34(3H,s), 2.82(1H,t,J=12.21Hz), 3.18(1H,t,J=12.21Hz), 4.25(1H,q,J=2.81H), 4.37(1H,q,J=2.81Hz), 4.82(2H,d,J=13.06Hz), 7.06(1H,s), 7.34(1H,dd,J=8.18,4.76Hz), 7.36(1H,d,J=7.94Hz), 7.53(1H,d,J=7.94,2.32Hz), 7.75(1H,dt,J=8.18,1.59Hz), 8.32(1H,d,J=1.59Hz), 8.51(1H,d,J=2.32Hz), 8.56(1H,dd,J=4.76,1.59Hz).
FAB-MS m/z:380(M+H)⁺.
Elementary analysis: as C₂₁H₂₂FN₅O
Calculated: C,66.47;H,5.84;N,18.46;F,5.01.
Found: C,66.18;H,5.81;N,18.16;F,4.81.

[Example 28] 1-[5-(5-Chloro-2-pyridyl)-1-(3-pyridyl)-1H-pyrazole-3-carbonyl]-4-methoxypiperidine

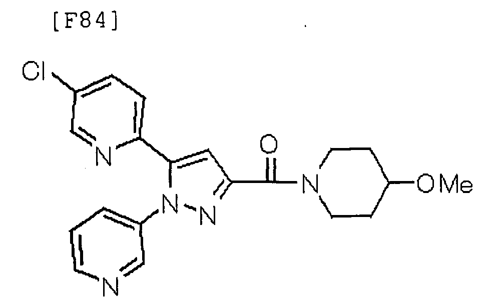

In a manner similar to that employed in Example 18, the title compound (590 mg, 89%) in solid form was prepared from 5-(5-chloro-2-pyridyl)-1-(3-pyridyl)-1H-pyrazole-3-carboxylic acid (500 mg) obtained in Referential Example 5 and 4-methoxypiperidine hydrochloride (380 mg) obtained in Referential Example 27.

¹H-NMR(400Hz,CDCl₃)δ:1.70(2H,m), 1.95(2H,m), 3.38(3H,s), 3.52(2H,m), 3.76(1H,m), 4.08(1H,m), 4.27(1H,m), 7.13(1H,s) 7.37(1H,dd,J=8.18,4.76Hz), 7.44(1H,d,J=8.42Hz), 7.72(1H,dd,J=8.42,2.44Hz), 7.73(1H,dd,J=8.18,2.44Hz), 8.41(1H,d,J=2.44Hz), 8.54(1H,d,J=2.94Hz), 8.60(1H,dd,J=4.76,1.47Hz).
FAB-MS m/z:398(M+H)⁺
Elementary analysis: as C₂₀H₂₀ClN₅O₂
Calculated: C,60.38;H,5.07;N,17.60;Cl,8.91.
Found: C,60.14;H,5.04;N,17.51;Cl,8.91.

[Example 29] 1-[5-(5-Chloro-2-pyridyl)-1-(3-pyridyl)-1H-pyrazole-3-carbonyl]-4-fluoropiperidine

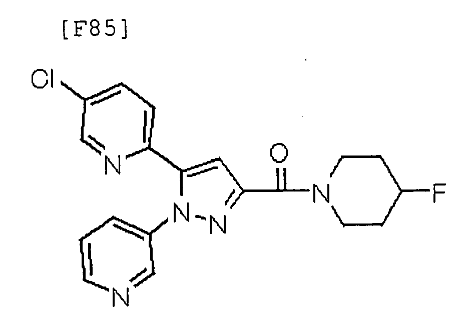

In a manner similar to that employed in Example 18, the title compound (307 mg, 80%) in solid form was prepared from 5-(5-chloro-2-pyridyl)-1-(3-pyridyl)-1H-pyrazole-3-carboxylic acid (300 mg) obtained in Referential Example 5 and 4-fluoropiperidine hydrochloride (170 mg) obtained in Referential Example 13.

¹H-NMR(400MHz,CDCl₃)δ:1.96(4H,m), 3.70(1H,br), 4.00(1H,br), 4.06(1H,br), 4.23(1H,br), 4.88(0.5H,br), 4.99(0.5H,br), 7.14(1H,s), 7.38(1H,m), 7.45(1H,m), 7.72(2H,m), 8.41(1H,br), 8.54(1H,br), 8.61(1H,br).
FAB-MS m/z:386(M+H)⁺.

[Example 30] 1-[5-(5-Methyl-2-pyridyl)-1-(3-pyridyl)-1H-pyrazole-3-carbonyl]-4-methoxypiperidine

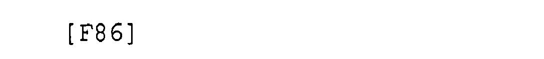

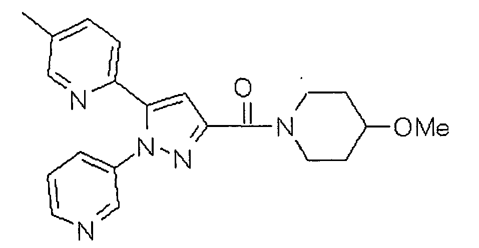

In a manner similar to that employed in Example 18, the title compound (270 mg, 50%) in solid form was prepared from 5-(5-methyl-2-pyridyl)-1-(3-pyridyl)-1H-pyrazole-3-carboxylic acid (400 mg) obtained in Referential Example 9 and 4-methoxypiperidine hydrochloride (328 mg) obtained in Referential Example 27.

¹H-NMR(400MHz,CDCl₃)δ:1.68(2H,br), 1.94(2H,br), 2.34(3H,s), 3.38(3H,s), 3.51(2H,m), 3.74(1H,m), 4.09(1H,br), 4.26(1H,br), 7.06(1H,s), 7.34(1H,dd,J=8.18,4.76Hz), 7.35(1H,d,J=8.06Hz), 7.53(1H,d,J=8.06Hz), 7.74(1H,ddd,J=8.18,2.32,1.47Hz), 8.31(1H,s), 8.51(1H,d,J=2.32Hz), 8.56(1H,dd,J=4.76,1.47Hz).
FAB-MS m/z:378(M+H)⁺.

[Example 31] 4-[5-(5-Methyl-2-pyridyl)-1-(3-pyridyl)-1H-pyrazole-3-carbonyl]morpholine

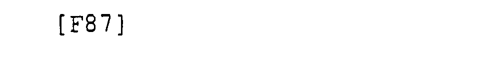

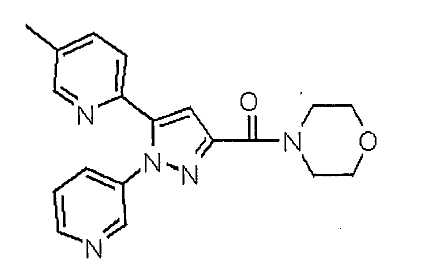

In a manner similar to that employed in Example 18, the title compound (113 mg, 22%) in solid form was prepared from 5-(5-methyl-2-pyridyl)-1-(3-pyridyl)-1H-pyrazole-3-carboxylic acid (400 mg) obtained in Referential Example 9 and morpholine (187 µL).

¹H-NMR(400MHz,CDCl₃)δ:2.34(3H,s), 3.74-3.83(6H,br), 4.15(2H,br), 7.13(1H,s) 7.34(1H,dd,J=4.76,8.18Hz), 7.36(1H,d,J=7.94Hz), 7.53(1H,dd,J=7.94,2.20Hz), 7.74(1H,dd,J=8.18,1.4 8.31(1H,d,J=1.47Hz), 8.52(1H,d,J=2.44Hz),8.57(1H,dd,J=4.76,1.47Hz).
FAB-MS m/z:350(M+H)⁺.

[Example 32] 1-[5-(5-Methyl-2-pyridyl)-1-(2-pyridyl)-1H-pyrazole-3-carbonyl]-4,4-difluoropiperidine

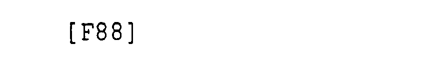

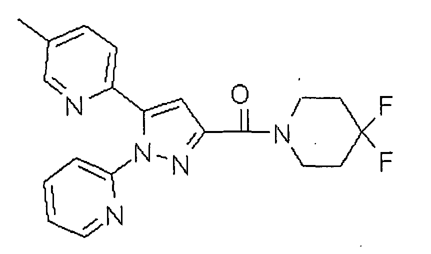

In a manner similar to that employed in Example 18, the title compound (270 mg, 36%) in solid form was prepared from 5-(5-methyl-2-pyridyl)-1-(2-pyridyl)-1H-pyrazole-3-carboxylic acid (556 mg) obtained in Referential Example 24 and 4,4-difluoropiperidine hydrochloride (313 mg) obtained in Referential Example 12.

¹H-NMR(400MHz,CDCl₃)δ:2.09(4H,br), 2.35(3H,s), 3.92(2H,br), 4.19(2H,br), 7.07(1H,s), 7.26(1H,m), 7.35(1H,d,J=7.94Hz), 7.51(1H,ddd,J=7.94,2.20,0.73Hz), 7.65(1H,td,J=8.06,0.98Hz), 7.83(1H,dd,J=8.06,1.96Hz), 8.27(1H,dd,J=1.95,0.86Hz), 8.28(1H,dd,J=2.20,0.86Hz).
FAB-MS m/z:384(M+H)⁺.
Elementary analysis: as C₂₀H₁₉F₂N₅O
Calculated: C,62.65;H,5.00;N,18.27;F,9.91.
Found: C,62.43;H,5.01;N,18.22;F,9.75.

[Example 33] 1-[5-(5-Methyl-2-pyridyl)-1-(2-pyridyl)-1H-pyrazole-3-carbonyl]-4-fluoropiperidine

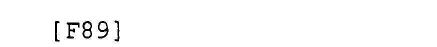

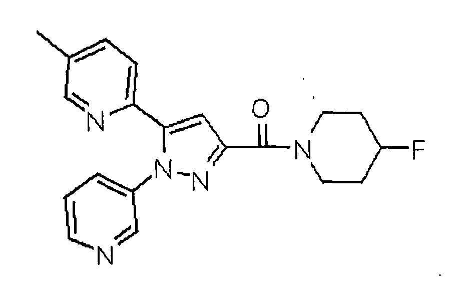

In a manner similar to that employed in Example 18, the title compound (264 mg, 41%) in solid form was prepared from 5-(5-methyl-2-pyridyl)-1-(3-pyridyl)-1H-pyrazole-3-carboxylic acid (500 mg) obtained in Referential Example 9 and 4-fluoropiperidine hydrochloride (324 mg) obtained in Referential Example 13.

¹H-NMR(400MHz,CDCl₃)δ:1.96(4H,m), 2.35(3H,s), 3.71(1H,br), 4.01(2H,br), 4.22(1H,br), 4.87(0.5H,br), 4.99(0.5H,br), 7.09(1H,s), 7.34(1H,dd,J=8.18,4.76Hz), 7.36(1H,d,J=7.93Hz), 7.53(1H,dd,J=7.93,2.20Hz), 7.73(1H,ddd,J=8.18,2.44,1.47Hz), 8.31(1H,d,J=1.47Hz), 8.52(1H,d,J=2.20Hz), 8.57(1H,dd,J=4.76,1.47Hz).
FAB-MS m/z:366(M+H)⁺.
Elementary analysis: as C₂₀H₂₀FN₅O
Calculated: C,65.74;H,5.52;N,19.17;F,5.20.
Found: C,65.79;H,5.44;N,19.15;F,5.13.

[Example 34] 4-[5-(6-Methoxy-3-pyridyl)-1-(3-pyridyl)-1H-pyrazole-3-carbonyl]morpholine

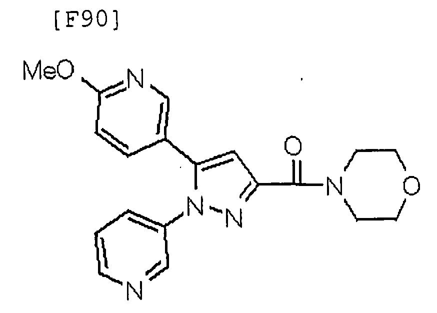

In a manner similar to that employed in Example 18, the title compound (0.189 g, 58%) in solid form was prepared from 5-(6-methoxy-3-pyridyl)-1-(3-pyridyl)-1H-pyrazole-3-carboxylic acid (0.262 g) obtained in Referential Example 22 and morpholine (0.235 mL).

¹H-NMR(400MHz,CDCl₃)δ:3.72-3.92(6H,m), 3.94(3H,s), 4.14-4.25(2H,m), 6.71(1H,d,J=8.6Hz), 6.96(1H,s), 7.32-7.42(2H,m), 7.61-7.67(1H,m), 8.09(1H,d,J=2.5Hz), 8.56-8.65(2H,m).
ESI-MS m/z:366(M+H)⁺.

[Example 35] 1-[5-(5-Methyl-2-pyrazinyl)-1-(3-pyridyl)-1H-pyrazole-3-carbonyl]hexahydropyridazine

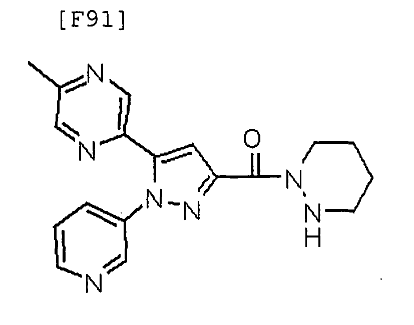

In a manner similar to that employed in Example 18, the title compound (0.132.g, 42%) in solid form was prepared from 5-(5-methyl-2-pyrazinyl)-1-(3-pyridyl)-1H-pyrazole-3-carboxylic acid (0.256 g) obtained in Referential Example 6 and hexahydropyridazine hydrochloride (0.182 g) obtained in Referential Example 14.

¹H-NMR(400MHz,CDCl₃)δ:1.60-1.90(4H,m), 2.57(3H,s), 2.95-3.10(2H,m), 3.82-3.90(1H×1/3,m), 4.19-4.29(2H×2/3,m), 4.66-4.73(1H×1/3,m), 6.67(1H×1/3,s like), 7.15-7.42(2H+1H×2/3,m), 7.70-7.84(1H,m), 8.30(1H×2/3,s), 8.50-8.66(2H+1H×1/3,m).
ESI-MS m/z:350(M+H)⁺.

[Example 36] (2S)-1-[5-(5-Methyl-2-pyridyl)-1-(3-pyridyl)-1H-pyrazole-3-carbonyl]-2-fluoromethylpyrrolidine

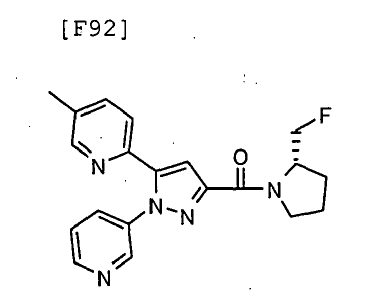

In a manner similar to that employed in Example 18, the title compound (29 mg, 8%) in solid form was prepared from 5-(5-methyl-2-pyridyl)-1-(3-pyridyl)-1H-pyrazole-3-carboxylic acid (280 mg) obtained in Referential Example 9 and (2S)-2-fluoromethylpyrrolidine hydrochloride (154 mg) obtained in Referential Example 31.

¹H-NMR(400MHz,CDCl₃)δ:1.94-2.08(4H,m), 2.34(3H,s), 3.69-4.10(2H,m),4.38-5.20(3H,m), 7.21(1H,s), 7.33-7.37(1H,m), 7.39-7.43(1H,m), 7.53-7.56(1H,m), 7.73-7.77(1H,m), 8.30(1H,d,J=1.5Hz), 8.49-8.58(2H,m).
ESI-MS m/z:366(M+H)⁺.

[Example 37] 1-[5-(5-Methyl-2-pyrazinyl)-1-(3-pyridyl)-1H-pyrazole-3-carbonyl]-4,4-difluoropiperidine

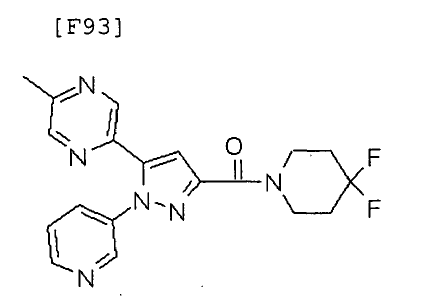

In a manner similar to that employed in Example 1, the title compound (0.304 g, 89%) in solid form was prepared from 5-(5-methyl-2-pyrazinyl)-1-(3-pyridyl)-1H-pyrazole-3-carboxylic acid (0.250 g) obtained in Referential Example 6 and 4,4-difluoropiperidine hydrochloride (0.168 g) obtained in Referential Example 12.

¹H-NMR(400MHz,CDCl₃)δ:2.07-2.13(4H,m), 2.58(3H,s), 3.94(2H,m), 4.21(2H,m), 7.25(1H,s), 7.39(1H,dd,J=8.2,4.8Hz), 7.73-7.75(1H,m), 8.31(1H,m), 8.55(1H,d,J=2.4Hz), 8.62-8.64(1H,m), 8.67(1H,d,J=1.5Hz).
EI-MSm/z:384(M⁺).

[Example 38] 1-[5-(5-Methyl-2-pyridyl)-1-(2-pyrimidinyl)-1H-pyrazole-3-carbonyl]-4,4-difluoropiperidine

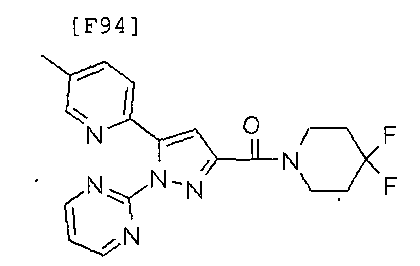

In a manner similar to that employed in Example 1, the title compound (0.270 g, 78%) in solid form was prepared from 5-(5-methyl-2-pyridyl)-1-(2-pyrimidinyl)-1H-pyrazole-3-carboxylic acid (0.250 g) obtained in Referential Example 26 and 4,4-difluoropiperidine hydrochloride (0.168 g) obtained in Referential Example 12.

¹H-NMR(400MHz,CDCl₃)δ:2.03-2.11(4H,m), 2.34(3H,s), 3.91(2H,s), 4.20(2H,s), 7.07(1H,s), 7.28-7.30(1H,m), 7.44(1H,d,J=8.1Hz), 7.53-7.56(1H,m), 8.22(1H,m), 8.70(2H,d,J=4.6Hz).
EI-MSm/z:384 (M⁺).

[Example 39] 1-[5-(5-methyl-2-pyridyl)-1-(2-pyridyl)-1H-pyrazole-3-carbonyl]-4-methoxy-4-methylpiperidine

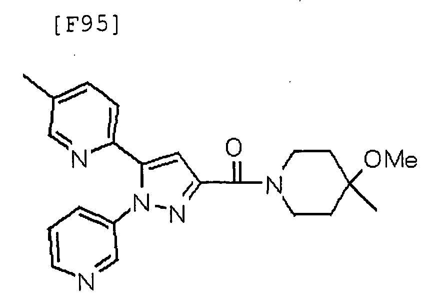

In a manner similar to that employed in Example 1, the title compound (0.211 g, 60%) in solid form was prepared from 5-(5-methyl-2-pyridyl)-1-(3-pyridyl)-1H-pyrazole-3-carboxylic acid (0.250 g) obtained in Referential Example 9 and 4-methoxy-4-methylpiperidine hydrochloride (0.192 g) obtained in Referential Example 33.

¹H-NMR(400MHz,CDC1₃)δ:1.19(3H,s), 1.54-1.61(2H,m), 1.80-1.91(2H,m), 2.34(3H,s), 3.20-3.27(1H,m), 3.23 (3H, s), 3.53-3.60(1H,m), 4.36-4.40(2H,m), 7.05(1H,s), 7.32-7.37(2H,m), 7.52-7.54(1H,m), 7.73-7.76(1H,M), 8.31-8.32(1H,m), 8.52(1H,d,J=2.4Hz), 8.56(1H,dd,J=4.9,1.5Hz).
EI-MSm/z:391(M⁺).

[Example 40] 1-[5-(5-Methyl-2-pyridyl)-1-(3-pyridyl)-1H-pyrazole-3-carbonyl]-3-methoxypiperidine

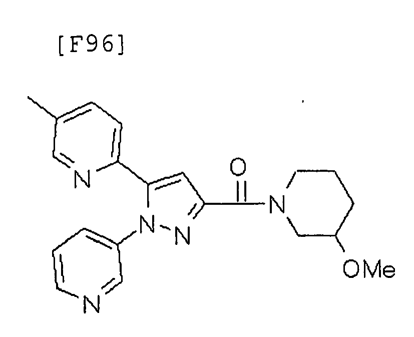

In a manner similar to that employed in Example 1, the title compound (0.133 g, 39%) in solid form was prepared from 5-(5-methyl-2-pyridyl)-1-(3-pyridyl)-1H-pyrazole-3-carboxylic acid (0.250 g) obtained in Referential Example 9 and 3-methoxypiperidine hydrochloride (0.176 g) obtained in Referential Example 34.

¹H-NMR(400MHz,CDCl₃)δ:1.56-1.67(2H,m),1.86(1H,m), 2.02(1H,m), 2.34(3H,s), 3.30(3/2H,s), 3.30-3.62(3H,m), 3.44(3/2H,s), 4.18-4.44(2H,m), 7.06-7.10(1H,m), 7.32-7.39(2H,m), 7.53(1H,d,J=7.8Hz), 7.73(1H,d,J=7.8Hz), 8.30(1H,s),8.50-8.53(1H,m), 8.55(1H,dd,J=4.6,1.5Hz).
EI-MSm/z: 377 (M⁺).
Elementary analysis: as C₂₁H₂₃N₅O₂
Calculated: C,66.83;H,6.14;N,18.55.
Found: C,66.90;H,6.03;N,18.58.

[Example 41] 1-[5-(5-Methyl-2-pyridyl)-1-(3-pyridazinyl)-1H-pyrazole-3-carbonyl]-4,4-difluoropiperidine

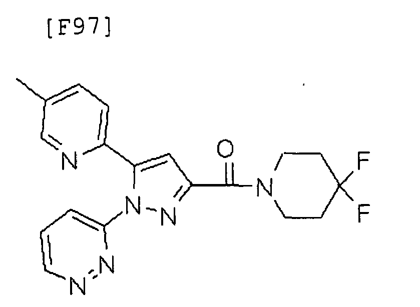

In a manner similar to that employed in Example 18, the title compound (0.280 g, 92%) in solid form was prepared from 5-(5-methyl-2-pyridyl)-1-(3-pyridazinyl)-1H-pyrazole-3-carboxylic acid (0.227 g) obtained in Referential Example 10 and 4,4-difluoropiperidine hydrochloride (0.194 g) obtained in Referential Example 12.

¹H-NMR(400MHz,CDCl₃)δ:2.00-2.20(4H,br), 2.33(3H,s), 3.88-4.00(2H,br), 4.12-4.26(2H,br), 7.10(1H,s), 7.50(1H,d,J=7.8Hz), 7.57(1H,dd,J=7.8,1.5Hz), 7.66(1H,dd,J=8.8,4.9 Hz), 7.93(1H,dd,J=8.0,0.7 Hz), 8.20(1H,br), 9.13(1H,dd,J=4.9,1.5 Hz).
ESI-MS m/z:385(M+H)⁺.

[Example 42] 1-[5-(6-Methyl-3-pyridazinyl)-1-(3-pyridyl)-1H-pyrazole-3-carbonyl]-4,4-difluoropiperidine

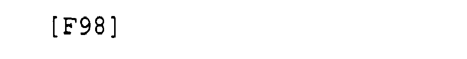

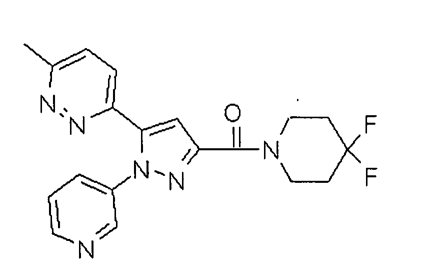

In a manner similar to that employed in Example 1, the title compound (0.212 g, 58%) in solid form was prepared from 5-(6-methyl-3-pyridazinyl)-1-(3-pyridyl)-1H-pyrazole-3-carboxylic acid (0.226 g) obtained in Referential Example 23 and 4,4-difluoropiperidine hydrochloride (0.226 g) obtained in Referential Example 12.

¹H-NMR(400MHz,CDCl₃)δ:2.04-2.18(4H,m), 2.73(3H,s), 3.90-4.00(2H,br), 4.16-4.26(2H,br), 7.27(1H,s), 7.36-7.42(2H,m), 7.50(1H,d,J=8.5Hz), 7.78-7.82(1H,m), 8.54(1H,d,J=2.5Hz), 8.62(1H,dd,J=4.9,1.5Hz).
ESI-MS m/z:385 (M+H)⁺.

[Example 43] (3R)-1-[5-(5-chloro-2-pyridyl)-1-(3-pyridyl)-1H-pyrazole-3-carbonyl]-3-fluoropiperidine

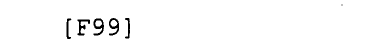

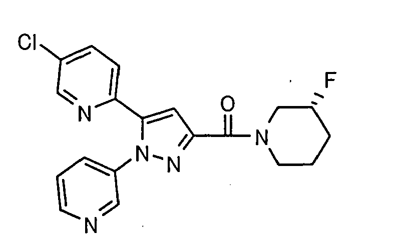

In a manner similar to that employed in Example 18, the title compound (135 mg, 35%) in solid form was prepared from 5-(5-chloro-2-pyridyl)-1-(3-pyridyl)-1H-pyrazole-3-carboxylic acid (301 mg) obtained in Referential Example 5 and (3R)-3-fluoropiperidine hydrochloride (154 mg) obtained in Referential Example 30.

¹H-NMR(400MHz,CDCl₃)δ:1.63(1H,s), 1.91-1.97(3H,m), 3.49(0.5H,br s), 3.90-4.02(3H,m), 4.36-4.43(0.5H,m), 4.67-4.79(1H,m), 7.17(1H,s), 7.37(1H,dd,J=8.2,4.8Hz), 7.49(1H,d,J=8.3Hz), 7.71-7.76(2H,m), 8.41(1H,d,J=2.4Hz), 8.54(1H,s), 8.61(1H,dd,J=4.8,1.6Hz).
ESI-MS m/z:386(M+H)⁺.

[Example 44] (3R)-1-[5-(5-Methyl-2-pyridyl)-1-(3-pyridyl)-1H-pyrazole-3-carbonyl]-3-fluoropiperidine

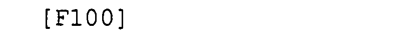

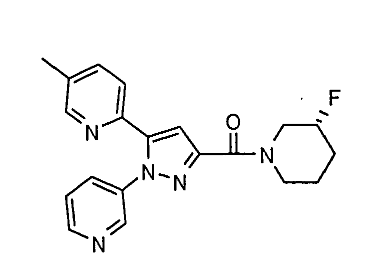

In a manner similar to that employed in Example 18, the title compound (212 mg, 54%) in solid form was prepared from 5-(5-methyl-2-pyridyl)-1-(3-pyridyl)-1H-pyrazole-3-carboxylic acid (302 mg) obtained in Referential Example 9 and (3R)-3-fluoropiperidine hydrochloride (150.4 mg) obtained in Referential Example 30.

¹H-NMR(400MHz,CDCl₃)δ:1.55(1H,br s), 1.98-2.02(3H,m), 2.34(3H,s), 3.50(0.5H,br s), 3.92-4.13(3H,m), 4.35(0.5H,br s), 4.73(1H,dd,J=48.2,20.4Hz), 7.10(1H,s) 7.33-7.39(2H,m), 7.52-7.55(1H,m), 7.76(1H,d,J=7.8Hz), 8.31(1H,s), 8.52(1H,s), 8.57(1H,s).
ESI-MS m/z:366(M+H)⁺.

[Example 45] 1-[5-(5-Amino-2-pyridyl)-1-(3-pyridyl)-1H-pyrazole-3-carbonyl]-4,4-difluoropiperidine

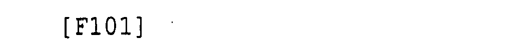

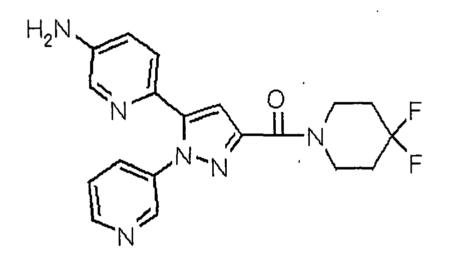

### 1) 1-[5-(5-tert-Butoxycarbonylamino-2-pyridyl)-1-(3-pyridyl)-1H-pyrazole-3-carbonyl]-4,4-difluoropiperidine

In a manner similar to that employed in Example 18, 1-[5-(5-tert-butoxycarbonylamino-2-pyridyl)-1-(3-pyridyl)-1H-pyrazole-3-carbonyl]-4,4-difluoropiperidine (466 mg, 96%) in amorphous form was prepared from 5-(5-tert-butoxycarbonylamino-2-pyridyl)-1-(3-pyridyl)-1H-pyrazole-3-carboxylic acid (381 mg) obtained in Referential Example 20 and 4,4-difluoropiperidine hydrochloride (173 mg) obtained in Referential Example 12.

¹H-NMR(400MHz,CDCl₃)δ:1.53(9H,s), 2.07(4H,br s), 3.92(2H,s), 4.20(2H,s), 6.63(1H,s), 7.11(1H,s), 7.35(1H,dd,J=8.2,4.8Hz), 7.42(1H,d,J=8.5Hz), 7.70(1H,dt,J=8.5,2.0Hz), 8.04(1H,s), 8.29(1H,d,J=2.0Hz), 8.57(2H,dt,J=11.7,4.3Hz).
ESI-MS m/z:485(M+H)⁺.

### 2) Title Compound

4N HCl-dioxane (10 mL) was added at room temperature to a solution of 1-[5-(5-tert-butoxycarbonylamino-2-pyridyl)-1-(3-pyridyl)-1H-pyrazole-3-carbonyl]-4,4-difluoropiperidine (458 mg) in dichloromethane (30 mL), and the mixture was stirred for 30 minutes. The reaction solvent was evaporated under reduced pressure, and saturated aqueous sodium hydrogencarbonate was added to the residue, to thereby neutralize the residue. The resultant mixture was extracted with chloroform-methanol (10:1), and the organic layer was dried over sodium sulfate anhydrate. After a filtration step, the solvent was evaporated under reduced pressure, and the residue was purified by silica gel thin-layer chromatography (methanol - chloroform), to thereby give the title compound (254 mg, 70%) as a solid product.

¹H-NMR(400MHz,CDCl₃)δ:2.07-2.10(4H,m), 3.87-3.92(4H,m), 4.14(2H,brs), 6.97(1H,dt,J=9.4,1.5Hz), 7.02(1H,d,J=0.5Hz), 7.24-7.26(1H,m), 7.34(1H,dd,J=8.1, 4.9Hz), 7.71-7.74 (1H,m), 7.94(1H,d,J=2.9Hz), 8.56(2H,td,J=4.8,1.5Hz).
ESI-MS m/z:385 (M+H)⁺.
Elementary analysis: as C₁₉H₁₈F₂N₆O
Calculated: C,59.37;H,4.72;N,21.86;F,9.89.
Found: C,59.28;H,4.68;N,21.71;F,9.77.

[Example 46] (3S)-1-[5-(5-Methyl-2-pyridyl)-1-(3-pyridyl)-1H-pyrazole-3-carbonyl]-3-fluoropyrrolidine

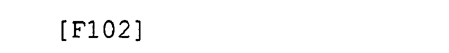

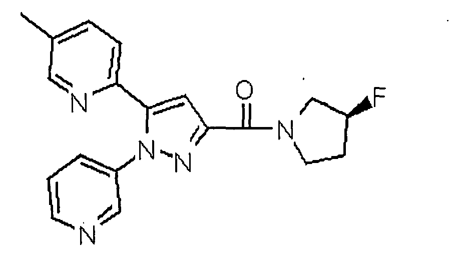

In a manner similar to that employed in Example 18, the title compound (107 mg, 31%) in solid form was prepared from 5-(5-methyl-2-pyridyl)-1-(3-pyridyl)-1H-pyrazole-3-carboxylic acid (280 mg) obtained in Referential Example 9 and (3S)-3-fluoropyrrolidine hydrochloride (153 mg) obtained in Referential Example 28.

¹H-NMR(400MHz,CDCl₃)δ:1.98-2.18 (1H,m), 2.28-2.41 (1H ,m), 2.35(3H,s), 3.77-4.20(3H,m), 4.42-4.52(1H,m), 5.25-5.41(1H,m), 7.25-7.26(1H,m) 7.35(1H,dd,J=8.2,4.8Hz), 7.41(1H,dd,J=8.1,2.2Hz) 7.55(1H,dd,J=7.9,1.6Hz), 7.73-7.76(1H,m), 8.30(1H,s), 8.55-8.57(2H,m).
ESI-MS m/z:352(M+H)⁺.
Elementary analysis: as C₁₉H₁₈FN₅O-0.25H₂O
Calculated: C,64.12;H,5.24;N,19.68.
Found: C,64.19;H,5.24;N,19.66.

[Example 47] (2S)-1-[5-(5-Methyl-2-pyrazinyl)-1-(3-pyridyl)-1H-pyrazole-3-carbonyl]-2-fluoromethylpyrrolidine

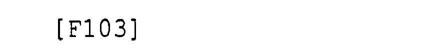

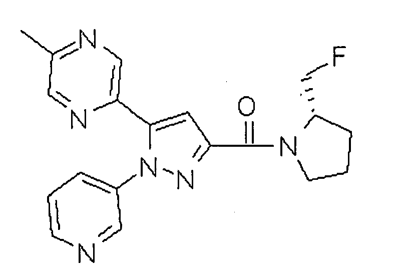

In a manner similar to that employed in Example 18, the title compound (188 mg, 51%) in solid form was prepared from 5-(5-methyl-2-pyrazinyl)-1-(3-pyridyl)-1H-pyrazole-3-carboxylic acid (281 mg) obtained in Referential Example 6 and (2S)-2-fluoromethylpyrrolidine hydrochloride (139 mg) obtained in Referential Example 31.

¹H-NMR(400MHz,CDCl₃)δ:1.94-2.15(4H,m), 2.58(3H,s), 3.72-4.10(2H,m), 4.39-5.20(3H,m), 7.34(1H,s), 7.36-7.41(1H,m), 7.73-7.77(1H,m), 8.30(1H,s), 8.53-8.71(3H,m).
ESI-MS m/z:367(M+H)⁺.
Elementary analysis: as C₁₉H₁₉FN₆O
Calculated: C,62.28;H,5.23;N,22.94.
Found: C,62.00;H,5.15;N,22.76.

[Example 48] 1-[5-(5-Methyl-2-pyridyl)-1-(3-pyridyl)-1H-pyrazole-3-carbonyl]pyrazolidine

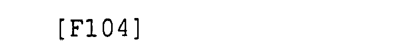

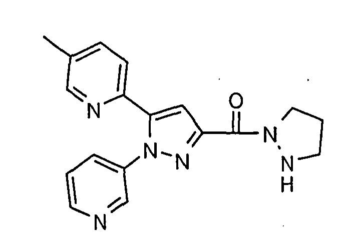

A 4N solution of HCl in dioxane (4.5 mL) was added at 0°C to a solution of 2-[5-(5-methyl-2-pyridyl)-1-(3-pyridyl)-1H-pyrazole-3-carbonyl]pyrazolidine-1-carboxylic acid tert-butyl ester (0.459 g) obtained from Referential Example 32 in dichloromethane (13.5 mL), and the mixture was stirred for 1.5 hours. The reaction mixture was partitioned between 1N aqueous sodium hydroxide and chloroform, and the aqueous layer was further extracted with chloroform. The organic layers were combined, and the combined organic layer was washed with saturated brine, followed by drying over sodium sulfate anhydrate. After a filtration step, the solvent was evaporated under reduced pressure, and the residue was purified by silica gel column chromatography (methanol - chloroform), to thereby give the title compound (0.284 g, 80%) as an amorphous product.

¹H-NMR(400MHz,CDCl₃)δ:2.10-2.35(2H,m), 2.33(3H,s), 3.12-3.27(2H,m), 3.75-3.86(2H×2/3,m), 4.12-4.22(2H×1/3,m), 4.70-4.80(1H×2/3,m), 5.42-5.50(1H×1/3,m), 7.20-7.60(4H,m), 7.68-7.84(1H,m), 8.30(1H, br), 8.51-8.60(2H,m).
FAB-MS m/z:335(M+H)⁺.

[Example 49] 1-[5-(5-Methyl-2-pyridyl)-1-(3-pyridyl)-1H-pyrazole-3-carbonyl]-2-formylpyrazolidine

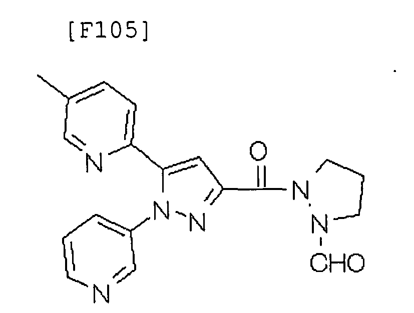

4-(Dimethylamino)pyridine (0.186 g) and trifluoromethanesulfonic acid anhydrate (0.199 mL) were added at 0°C to a solution of 1-[5-(5-methyl-2-pyridyl)-1-(3-pyridyl)-1H-pyrazole-3-carbonyl]pyrazolidine (0.264 g) obtained from Example 48 in N,N-dimethylformamide (5.0 mL), and the mixture was stirred for 1 hour. The reaction mixture was partitioned between saturated aqueous sodium hydrogencarbonate and ethyl acetate, and the aqueous layer was extracted with ethyl acetate. The organic layers were combined, and the combined organic layer was washed with saturated brine, followed by drying over sodium sulfate anhydrate. After a filtration step, the solvent was evaporated under reduced pressure, and the residue was purified by silica gel column chromatography (methanol - chloroform), to thereby give the title compound (0.156 g, 55%) as a solid product.

¹H-NMR(400MHz,CDCl₃)δ:2.10-2.25(2H,m), 2.34(3H,s), 3.65-3.75(2H,m), 4.02-4.18(2H,m), 7.18-7.30(1H,m), 7.32-7.40(2H,m), 7.50-7.56(1H,m), 7.73-7.81(1H,m), 8.31(1H,br), 8.47(1H,br), 8.54-8.62(2H,m).
ESI-MS m/z:363(M+H)⁺.

[Example 50] 4-[5-(1-Methyl-1H-pyrrol-2-yl)-1-(3-pyridyl)-1H-pyrazole-3-carbonyl]morpholine

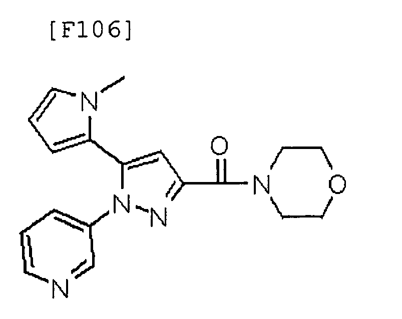

In a manner similar to that employed in Example 1, the title compound (0.211 g, 80%) in solid form was prepared from 5-(1-methyl-1H-pyrrol-2-yl)-1-(3-pyridyl)-1H-pyrazole-3-carboxylic acid (0.209 g) obtained in Referential Example 37 and morpholine (0.123 mL).

¹H-NMR(400MHz,CDCl₃)δ:3.36(3H,s), 3.76-3.86(6H,m), 4.18-4.21(2H,m), 6.10(1H,dd,J=3.7,1.7Hz), 6.15-6.17(1H,m), 6.72-6.73(1H,m), 6.95(1H,s), 7.27-7.30(1H,m), 7.49-7.52(1H,m),
8.55(1H,d,J=4.2Hz), 8.60(1H,d,J=2.4Hz).
ESI-MS m/z:338(M+H)⁺.

[Example 51] 1-[5-(1-Methyl-1H-pyrrol-2-yl)-1-(3-pyridyl)-1H-pyrazole-3-carbonyl]-4-methoxypiperidine

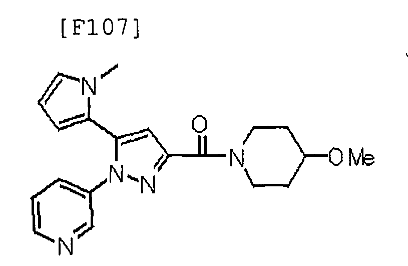

In a manner similar to that employed in Example 1, the title compound (0.247 g, 86%) in amorphous form was prepared from 5-(1-methyl-1H-pyrrol-2-yl)-1-(3-pyridyl)-1H-pyrazole-3-carboxylic acid (0.209 g) obtained in Referential Example 37 and 4-methoxypiperidine hydrochloride (0.218 g) obtained in Referential Example 27.

¹H-NMR(400Hz,CDCl₃)δ:1.66-1.75(2H,m), 1.92-2.01(2H,m), 3.35(3H,s), 3.39(3H,s), 3.50-3.59(2H,m), 3.75-3.81(1H,m), 4.07-4.13(1H,m), 4.29-4.35(1H,m), 6.11(1H,dd,J=3.7,1.7Hz), 6.15-6.17(1H,m), 6.72-6.73(1H,m), 6.89(1H,s), 7.26-7.29(1H,m), 7.50-7.53(1H,m), 8.53(1H,dd,J=4.6,1.2Hz), 8.60(1H,d,J=2.4Hz).
ESI-MS m/z:366(M+H)⁺.

[Example 52] 1-[5-(6-Methoxy-3-pyridazinyl)-1-(3-pyridyl)-1H-pyrazole-3-carbonyl]-4,4-difluoropiperidine

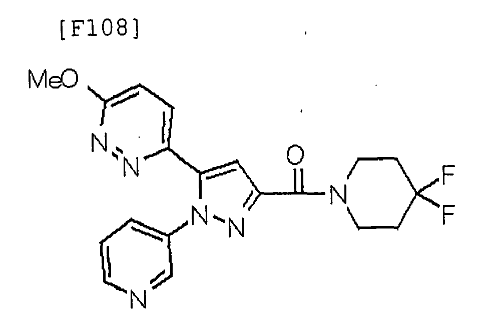

In a manner similar to that employed in Example 1, the title compound (0.254 g, 66%) in solid form was prepared from 5-(6-methoxy-3-pyridazinyl)-1-(3-pyridyl)-1H-pyrazole-3-carboxylic acid (0.300 g) obtained in Referential Example 36 and 4,4-difluoropiperidine hydrochloride (0.235 g) obtained in Referential Example 12.

¹H-NMR(400MHz,DMSO-d₆)δ:2.02-2.18(4H,m), 3.75-3.85(2H,m), 4.00(3H,s), 4.00-4.12(2H,m), 7.35(1H,d like,J=9.3Hz), 7.38 (1H, s like), 7.52(1H,dd,J=8.0,4.9Hz), 7.86-7. 91 (1H,m), 7.98(1H,d like,J=9.3Hz), 8.60-8.70(2H,m).
ESI-MS m/z:401 (M+H)⁺.

[Example 53] 4-[5-(6-Methoxy-3-pyridazinyl)-1-(3-pyridyl)-1H-pyrazole-3-carbonyl]morpholine

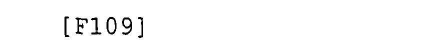

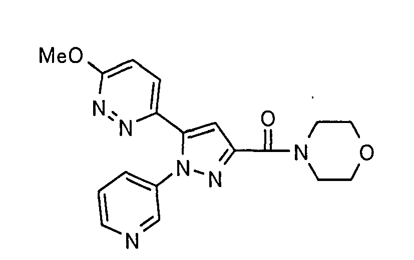

In a manner similar to that employed in Example 1, the title compound (0.118 g, 39%) in solid form was prepared from 5-(6-methoxy-3-pyridazinyl)-1-(3-pyridyl)-1H-pyrazole-3-carboxylic acid (0.259 g) obtained in Referential Example 36 and morpholine (0.145 mL).

¹H-NMR(400MHz,CDCl₃)δ:3.72-3.89(6H,m), 4.13(3H,s), 4.15-4.24(2H,m), 7.01(1H,d,J=9.0Hz), 7.21(1H,s), 7.38(1H,dd,J=8.6,4.9Hz), 7.47(1H,d,J=9.0Hz), 7.76-7.82(1H,m), 8.55(1H,d like,J=2.4Hz), 8.61(1H,dd,J=4.9,1.5Hz).
ESI-MS m/z:367(M+H)⁺.

[Example 54] 1-[5-(5-Amino-2-pyrazinyl)-1-(3-pyridyl)-1H-pyrazole-3-carbonyl]-4,4-difluoropiperidine

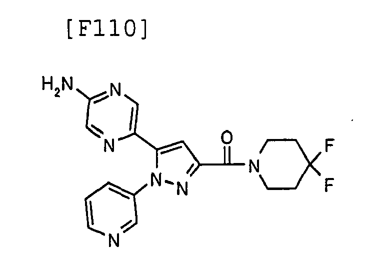

### 1) 1-[5-(5-tert-Butoxycarbonylamino-2-pyrazinyl)-1-(3-pyridyl)-1H-pyrazole-3-carbonyl]-4,4-difluoropiperidine

In a manner similar to that employed in Example 1, 1-[5-(5-tert-butoxycarbonylamino-2-pyrazinyl)-1-(3-pyridyl)-1H-pyrazole-3-carbonyl]-4,4-difluoropiperidine (0.494 g, 87%) in solid form was prepared from 5-[5-(tert-butoxycarbonylamino)-2-pyrazinyl]-1-(3-pyridyl)-1H-pyrazole-3-carboxylic acid (0.450 g) obtained in Referential Example 25 and 4,4-difluoropiperidine hydrochloride (0.223 g) obtained in Referential Example 12.

¹H-NMR(400MHz,CDCl₃)δ:1.54(9H,s), 2.07-2.13(4H,m), 3.94(2H,m), 4.22(2H,m), 7.20(1H,s), 7.37-7.40(1H,m), 7.53(1H,s), 7.73-7.76(1H,m), 8.40(1H,d,J=1.7Hz), 8.57(1H,m), 8.63(1H,dd,J=4.9,1.5Hz), 9.15(1H,d,J=1.5Hz).
EI-MSm/z:485(M⁺).

### 2) Title Compound

Trifluoroacetic acid (4.9 mL) was added at room temperature to a solution of 1-[5-(5-tert-butoxycarbonylamino-2-pyrazinyl)-1-(3-pyridyl)-1H-pyrazole-3-carbonyl]-4,4-difluoropiperidine (0.486 g) in dichloromethane (9.7 mL), and the mixture was stirred for 3 hours. The reaction mixture was partitioned between saturated aqueous sodium hydrogencarbonate and chloroform, and the organic layer was dried over sodium sulfate anhydrate. After a filtration step, the solvent was evaporated under reduced pressure, and the residue was purified by silica gel column chromatography (methanol - chloroform), to thereby give the title compound (0.342 g, 88%) as a solid product.

¹H-NMR(400MHz,CDCl₃)δ:2.06 (9H,m), 3.78(2H,m), 4.06(2H,m), 6.80(2H,s), 7.09(1H,m), 7.48-7.51(1H,m), 7.68(1H,m), 7.79-7.82(1H,m), 8.21(1H,s), 8.58(2H,m).
EI-MSm/z:385 (M⁺).

[Example 55] 4-[5-(1-Methyl-1H-pyrrol-3-yl)-1-(3-pyridyl)-1H-pyrazole-3-carbonyl]morpholine

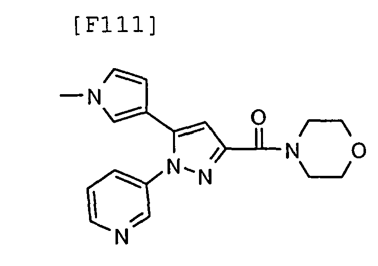

1-Hydroxybenzotriazole (84 mg), morpholine (70 µL), and triethylamine (0.3 mL) were added at room temperature to a solution of (1-methyl-1H-pyrrol-3-yl)-1-(3-pyridyl)-1H-pyrazole-3-carboxylic acid (165 mg) obtained from Referential Example 38 in dichloromethane (10 mL), and 1-(3-dimethylaminopropyl)-3-ethylcarbodiimide hydrochloride (178 mg) was added to the reaction mixture, followed by stirring at room temperature for 23.5 hours. The reaction mixture was partitioned between water and chloroform, and the organic layer was dried over magnesium sulfate anhydrate. After a filtration step, the solvent was evaporated under reduced pressure, and the residue was purified by silica gel thin-layer chromatography (chloroform - methanol), to thereby give the title compound (146 mg, 70%) as a solid product.

¹H-NMR(400MHz,CDCl₃)δ:3.61(3H,s), 3.73-3.82(6H,br), 4.15(2H,br), 5.87(1H,t,J=1.83Hz), 6.49(1H,t,J=1.95Hz), 6.52(1H,t,J=2.56Hz) 6.80(1H,s), 7.37(1H,dd,J=8.18,4.76Hz), 7.78(1H,ddt,J=8.18,2.44,1.59Hz), 8.62(1H,dd,J=4.76,1.59Hz), 8.71(1H,d,J=2.44Hz).
FAB-MS m/z:338(M+H)⁺.

[Example 56] 1-[5-(1-Methyl-1H-pyrrol-3-yl)-1-(3-pyridyl)-1H-pyrazole-3-carbonyl]-4-methoxypiperidine

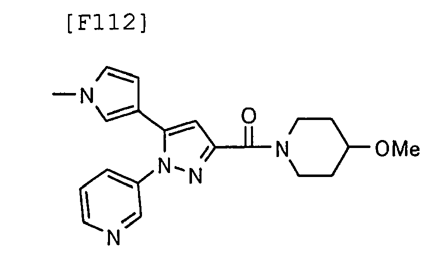

1-Hydroxybenzotriazole (84 mg), 4-methoxypiperidine hydrochloride (123 mg) obtained from Referential Example 27, and triethylamine (0.3 mL) were added at room temperature to a solution of (1-methyl-1H-pyrrol-3-yl)-1-(3-pyridyl)-1H-pyrazole-3-carboxylic acid (165 mg) obtained from Referential Example 38 in dichloromethane (10 mL), and 1-(3-dimethylaminopropyl)-3-ethylcarbodiimide hydrochloride (178 mg) was added to the reaction mixture, followed by stirring at room temperature for 23 hours. The reaction mixture was partitioned between water and chloroform, and the organic layer was dried over magnesium sulfate anhydrate. After a filtration step, the solvent was evaporated under reduced pressure, and the residue was purified by silica gel thin-layer chromatography (chloroform - methanol), to thereby give the title compound as an oily product.

¹H-NMR(400MHz,CDCl₃)δ:1.66(2H,br), 1.94(2H,br), 3.38(3H,s), 3.50(2H,m), 3.61(3H,s), 3.72(1H,br), 4.10(1H,br) 4.29(1H,br), 5.87(1H,t,J=1.95Hz), 6.51(2H,m), 6.74(1H,s), 7.36(1H,dd,J=8.18,4.76Hz), 7.79(1H,dt,J=8.18,1.9Hz), 8.61 (1H, dd, J=4.76,1.09Hz), 8.72(1H, d, J=2.32Hz).
FAB-MS m/z:366(M+H)⁺.

[Example 57] 1-[5-(5-Methylpyridin-2-yl)-1-(pyridin-3-yl)-1H-pyrazole-3-carbonyl]hexahydropyridazine

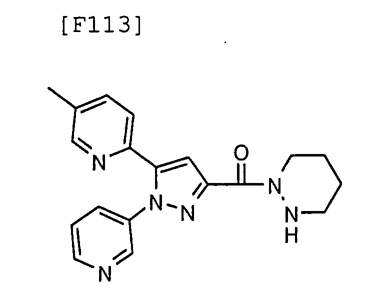

1-Hydroxybenzotriazole (0.167 g), 1-(3-dimethylaminopropyl)-3-ethylcarbodiimide hydrochloride (0.462 g), triethylamine (0.944 mL), and hexahydropyridazine hydrochloride (0.269 g) obtained from Referential Example 14 were added at room temperature to a solution of 5-(5-methylpyridin-2-yl)-1-(pyridin-3-yl)-1H-pyrazole-3-carboxylic acid (0.316 g) obtained from Referential Example 9 in dichloromethane (7.5 mL) and N,N-dimethylformamide (3 mL), and the mixture was stirred for 21 hours. The reaction mixture was partitioned between water and ethyl acetate, and the aqueous layer was extracted with ethyl acetate. The organic layers were combined, and the combined organic layer was washed with saturated brine, followed by drying over sodium sulfate anhydrate. After a filtration step, the solvent was evaporated under reduced pressure, and the residue was purified by silica gel column chromatography (chloroform - methanol), to thereby give the title compound (0.226 g, 58%) as a solid product.

¹H-NMR(400MHz,CDCl₃)δ:1.65-1.90(4H,m), 2.34(3H,s), 2.98-3.09(2H,m), 3.87(2H×1/3,br), 4.25(2H×2/3,br), 7.14-7.42(3H,m), 7.48-7.57(1H,m), 7.73-7.87(1H,m), 8.31(1H,br), 8.50-8.61(1H,m).
ESI-MS m/z:349(M+H)⁺.

[Example 58] 2-[5-(5-Methylpyridin-2-yl)-1-(pyridin-3-yl)-1H-pyrazole-3-carbonyl]hexahydropyridazine-1-carboxamide

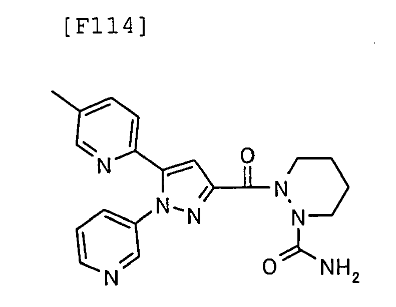

Trimethylsilyl isocyanate (1.35 mL) was added at room temperature to a solution of 1-[5-(5-methylpyridin-2-yl)-1-(pyridin-3-yl)-1H-pyrazole-3-carbonyl]hexahydropyridazine (0.289 g) obtained from Example 57 in 1,4-dioxane (2.5 mL), and the mixture was refluxed for 2 days. After the reaction mixture was cooled in air, methanol was added thereto, and the solvent was evaporated under reduced pressure. The residue was purified by silica gel column chromatography (chloroform - methanol), to thereby give the title compound (0.178 g, 55%) as a solid product.

¹H-NMR(400MHz,CDCl₃)δ:1.65-1.92(4H,m), 2.35(3H,s), 2.82-3.15(2H,m), 4.39-4.47(2H×3/5,m), 4.63-4.75(2H×2/5,m), 5.13(2H,br), 7.11(1H,s), 7.38(1H,d,J=8.1Hz), 7.45-7.59(2H,m), 7.96-8.03(1H,m), 8.48(1H,d,J=2.2Hz), 8.53-8.60(1H,m).
ESI-MS m/z:392(M+H)⁺.

[Example 59] 1-[5-(5-Methylpyridin-2-yl)-1-(pyridin-3-yl)-1H-pyrazole-3-carbonyl]-4-methyl-3,5-dioxopiperazine

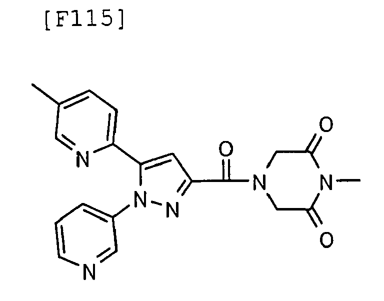

1-Hydroxybenzotriazole (49.5 mg), 1-(3-dimethylaminopropyl)-3-ethylcarbodiimide hydrochloride (0.127 g), triethylamine (0.165 mL), and 1-methyl-2,6-dioxopiperazine hydrochloride (52.5 mg) obtained from Referential Example 39 were added at room temperature to a solution of 5-(5-methylpyridin-2-yl)-1-(pyridin-3-yl)-1H-pyrazole-3-carboxylic acid (83.1 mg) obtained from Referential Example 9 in N,N-dimethylformamide (3.0 mL), and the mixture was stirred for 20 hours. The reaction mixture was partitioned between water and ethyl acetate, and the aqueous layer was further extracted with ethyl acetate. The organic layers were combined, and the combined organic layer was washed with saturated brine, followed by drying over sodium sulfate anhydrate. After a filtration step, the solvent was evaporated under reduced pressure, and the residue was purified by silica gel thin-layer chromatography (chloroform - methanol), to thereby give the title compound (89.4 mg, 78%) as a solid product.

¹H-NMR(400MHz,CDCl₃)δ:2.39(3H,s), 3.20(3H,s), 4.72(2H,br), 5.24(2H,br), 7.22(1H,s), 7.36-7.43(2H,m), 7.54(1H,dd,J=2.2,8.0Hz), 7.77-7.83(1H,m), 8.29-8.33(1H,m), 8.50(1H,d,J=2.5Hz), 8.60(1H,dd,J=1.5,4.9Hz).
ESI-MSm/z:391(M+H)⁺.

[Example 60] 1-[5-(5-Methylpyridin-2-yl)-1-(pyridin-3-yl)-1H-pyrazole-3-carbonyl]-3-oxopiperazine

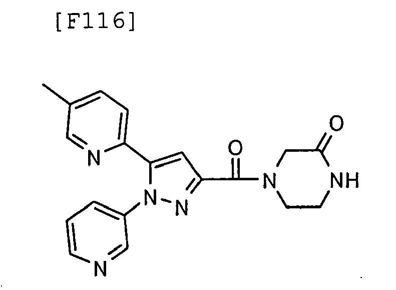

1-Hydroxybenzotriazole (0.125 g), 1-(3-dimethylaminopropyl)-3-ethylcarbodiimide hydrochloride (0.342 g), triethylamine (0.350 mL), and piperazin-2-one (0.126 g) were added at room temperature to a solution of 5-(5-methylpyridin-2-yl)-1-(pyridin-3-yl)-1H-pyrazole-3-carboxylic acid (0.234 g) obtained from Referential Example 9 in N,N-dimethylformamide (6.0 mL), and the mixture was stirred for 3 days. The reaction mixture was partitioned between water and ethyl acetate, and the aqueous layer was further extracted with ethyl acetate-methanol (10:1) solvent mixture. The organic layers were combined, and the combined organic layer was washed with saturated brine, followed by drying over sodium sulfate anhydrate. After a filtration step, the solvent was evaporated under reduced pressure, and the residue was purified by silica gel thin-layer chromatography (chloroform - methanol), to thereby give the title compound (73.1 mg, 24%) as a solid product.

¹H-NMR(400MHz,DMSO-d₆)δ:2.29(3H,s), 3.20-3.45(2H+1H×1/5,m), 3.83(1H×4/5,br), 4.10-4.22(2H+1H×1/5,m), 4.57(1H×4/5,br), 7.23(1H×4/5,s), 7.26(1H×1/5,s), 7.45-7.53(1H,m), 7.64(1H,d,J=8.1Hz), 7.72(1H,br d, J=7.8Hz), 7.80(1H,br d,J=8.3Hz), 8.13(1H,br), 8.26(1H,br), 8.49-8.62(2H,m).
ESI-MS m/z: 363(M+H)⁺.

[Example 61] 1-[5-(5-Methylpyridin-2-yl)-1-(pyridin-3-yl)-1H-pyrazole-3-carbonyl]-3-methoxyazetidine

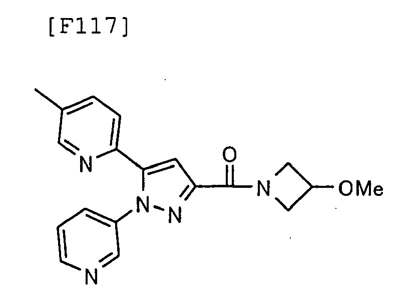

1-Hydroxybenzotriazole (0.0879 g), 1-(3-dimethylaminopropyl)-3-ethylcarbodiimide hydrochloride (0.227 g), triethylamine (0.454 mL), and 3-methoxyazetidine hydrochloride (0.142 g) obtained from Referential Example 41 were added at room temperature to a solution of 5-(5-methylpyridin-2-yl)-1-(pyridin-3-yl)-1H-pyrazole-3-carboxylic acid (0.152 g) obtained from Referential Example 9 in dichloromethane (5.0 mL), and the mixture was stirred for 6 days. The reaction mixture was partitioned between water and chloroform, and the aqueous layer was further extracted with chloroform. The organic layers were combined, and the combined organic layer was washed with saturated brine, followed by drying over sodium sulfate anhydrate. After a filtration step, the solvent was evaporated under reduced pressure, and the residue was purified by silica gel thin-layer chromatography (chloroform - methanol), to thereby give the title compound (74.0 mg, 39%) as a solid product.

¹H-NMR(400MHz,CDCl₃)δ:3.44(3H,s), 4.11(3H,s), 4.06-4.17(1H,m), 4.26-4.67(3H,m), 4.77-4.86(1H,m), 7.21(1H,s), 7.33-7.80(4H,m), 8.29(1H,br), 8.51-8.61(2H,m).
ESI-MSm/z:350(M+H)⁺.

[Example 62] 1- [5- (1-Methyl-1H-pyrrol-3-yl) -1- (pyridin-3-yl) - 1H-pyrazole-3-carbonyl]-4-methyl-3-oxopiperazine

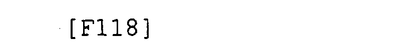

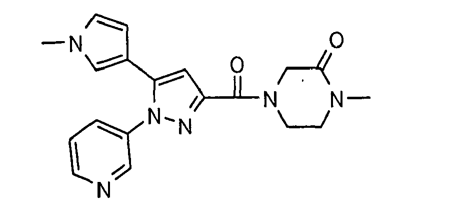

1-Hydroxybenzotriazole (0.132 g), 1-(3-dimethylaminopropyl)-3-ethylcarbodiimide hydrochloride (0.378 g), triethylamine (0.630 mL), and 1-methylpiperazin-2-one hydrochloride (0.200 g) obtained from Referential Example 15 were added at room temperature to a solution of 5-(1-methylpyrrol-3-yl)-1-(pyridin-3-yl)-1H-pyrazole-3-carboxylic acid (0.242 g) obtained from Referential Example 38 in N,N-dimethylformamide (5.0 mL), and the mixture was stirred for 3 days. The reaction mixture was partitioned between water and ethyl acetate, and the aqueous layer was further extracted with ethyl acetate. The organic layers were combined, and the combined organic layer was washed with saturated brine, followed by drying over sodium sulfate anhydrate. After a filtration step, the solvent was evaporated under reduced pressure, and the residue was purified by silica gel column chromatography (chloroform - methanol), to thereby give the title compound (0.243 g, 74%) as an amorphous product.

¹H-NMR(400MHz,CDCl₃)δ:3.01(3H,s), 3.46(2H,br), 3.61(3H,s), 4.03(1H+1H×1/3,m), 4.42(1H+1H×2/3,m), 4.80(1H,br), 5.86(1H,br), 6.46-6.56(2H,m), 6.82(1H×2/3,br), 6.86(1H×1/3,br), 7.37-7.46(1H,m), 7.76-7.95(1H,m), 8.61-8.78(2H,m).
ESI-MS m/z:365(M+H.)⁺.

[Example 63] 1-[5-(5-Methylpyridin-2-yl)-1-(pyridin-3-yl)-1H-pyrazole-3-carbonyl]-4-ethyl-3-oxopiperazine

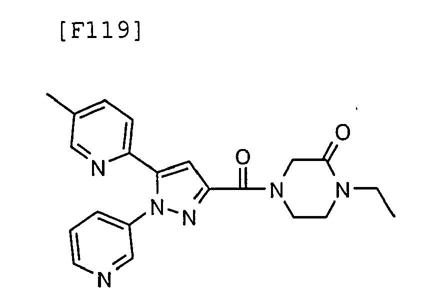

1-Hydroxybenzotriazole (0.127 g), 1-(3-dimethylaminopropyl)-3-ethylcarbodiimide hydrochloride (0.357 g), triethylamine (0.559 mL), and 1-ethylpiperazin-2-one hydrochloride (0.234 g) obtained from Referential Example 40 were added at room temperature to a solution of 5-(5-methylpyridin-2-yl)-5-(pyridin-3-yl)-1H-pyrazole-3-carboxylic acid (0.247 g) obtained from Referential Example 9 in N,N-dimethylformamide (5.0 mL), and the mixture was stirred for 18 hours. The reaction mixture was partitioned between water and ethyl acetate, and the aqueous layer was further extracted with ethyl acetate. The organic layers were combined, and the combined organic layer was washed with saturated brine, followed by drying over sodium sulfate anhydrate. After a filtration step, the solvent was evaporated under reduced pressure, and the residue was purified by silica gel column chromatography (chloroform - methanol), to thereby give the title compound (0.184 g, 53%) as a solid product.

¹H-NMR(400MHz,CDCl₃)δ:1.17(3H,t,J=7.1Hz), 2.34(3H,s), 3.42-3.55(4H,m), 3.99-4.08(1H+1H×1/2,m), 4.36-4.47(1H+1H×1/2,m), 4.82(1H,br), 7.18(1H×1/2,s), 7.20(1H×1/2,s), 7.36-7.59(3H,m), 7.73-7.78(1H×1/2,m), 7.86-7.93(1H×1/2,m), 8.29(1H,br), 8.47-8.62(2H,m).
ESI-MS m/z:391(M+H)⁺.
Elementary analysis: as C₂₁H₂₂N₆O₂
Calculated: C,64.60;H,5.68;N,21.52.
Found: C,64.53;H,5.64;N,21.40.

[Example 64] 1-[5-(5-Aminomethylpyridin-2-yl)-1-(pyridin-3-yl)-1H-pyrazole-3-carbonyl]-4,4-difluoropiperidine

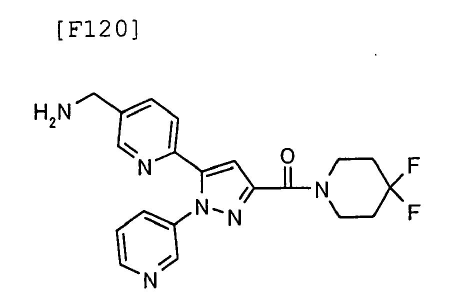

A suspension of 1-[5-(5-cyanopyridin-2-yl)-1-(pyridin-3-yl)-1H-pyrazole-3-carbonyl]-4,4-difluoropiperidine (500 mg) obtained from Example 12 and nickel on silica-alumina (~65%, 280 mg) in 2M ammonia-ethanol (30 mL) was stirred in a hydrogen atmosphere (8 atm) at 120°C for 2 hours. The resultant mixture was cooled in air, and was filtered through Celite. The solvent of the filtrate was evaporated under reduced pressure, and the residue was purified by silica gel thin-layer chromatography (the lower layer of chloroform - methanol - water), to thereby give the title compound (259 mg, 51%) as a white solid product.

¹H-NMR(40OMHz,CDCl₃)δ:2.09(4H,brs), 3.93(4H,s), 4.20(2H,s), 7.16(1H,s), 7.36(1H,dd,J=7.9,4.8Hz), 7.46(1H,d,J=7.8Hz), 7.73-7.77(2H,m), 8.44(1H,s), 8.51(1H,d,J=2.4Hz), 8.58(1H,dd,J=4.6,1.5Hz).
ESI-MS m/z:399(M+H)⁺.

[Example 65] 1-[5-(5-Hydroxymethylpyridin-2-yl)-1-(pyridin-3-yl)-1H-pyrazole-3-carbonyl]-4,4-difluoropiperidine

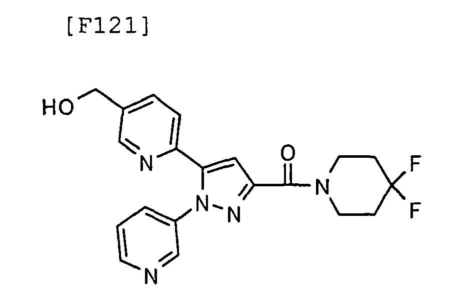

Sodium nitrite (91 mg) was added at room temperature to a solution of 1-[5-(5-aminomethylpyridin-2-yl)-1-(pyridin-3-yl)-1H-pyrazole-3-carbonyl]-4,4-difluoropiperidine (105 mg) obtained from Example 64 in water (3 mL) and acetic acid (1 mL), and the mixture was stirred for 2 hours. Under cooling on ice, saturated aqueous sodium hydrogencarbonate and chloroform-methanol (10:1) solvent mixture was added to the resultant mixture. The organic layer was dried over sodium sulfate anhydrate. After a filtration step, the solvent was evaporated under reduced pressure, and the residue was purified by silica gel thin-layer chromatography (chloroform - methanol), to thereby give the title compound (46 mg, 44%) as a white solid product.

¹H-NMR(400MHz,CDCl₃)δ:2.05-2.13(5H,m), 3.93(2H,brs), 4.16(2H,brs), 4.75(2H,s), 7.17(1H,s), 7.37 (1H,dd,J=8.1,4.9Hz), 7.48(1H,d,J=7.8Hz), 7.73-7.79(2H,m), 8.45(1H,s), 8.52(1H,d,J=2.4Hz), 8.59(1H,d,J=4.6Hz).
ESI-MS m/z:400(M+H)⁺.

[Example 66] 1-[5-(5-Fluoromethylpyridin-2-yl)-1-(pyridin-3-yl)-1H-pyrazole-3-carbonyl]-4,4-difluoropiperidine

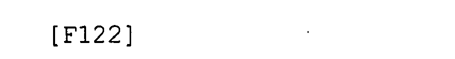

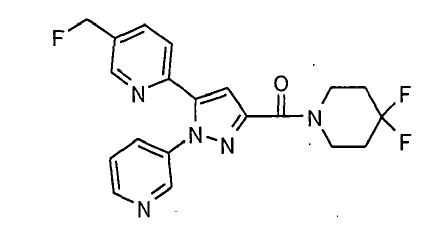

Bis(2-methoxyethyl)aminosulfur trifluoride (223 µL) was added under cooling on ice to a solution of 5-[(5-hydroxymethylpyridin-2-yl)-1-(pyridin-3-yl)-1H-pyrazole-3-carbonyl]-4,4-difluoropiperidine (440 mg) obtained from Example 65 in dichloromethane (30 mL), and the mixture was stirred for 20 minutes. The reaction mixture was partitioned between saturated aqueous sodium hydrogencarbonate and chloroform, and the organic layer was dried over sodium sulfate anhydrate. After a filtration step, the solvent was evaporated under reduced pressure, and the residue was purified by silica gel column chromatography (hexane - ethyl acetate) and NH silica gel thin-layer chromatography (hexane - ethyl acetate), to thereby give the title compound (130 mg, 29%) as a solid product.

¹H-NMR(400MHz,CDCl₃)δ:1.56(4H,brs), 2.04-2.13(4H,m), 3.93(2H,s), 4.21(2H,s), 5.42(2H,d,J=47.4Hz), 7.21(1H,s), 7.37(1H,dd,J=8.2,4.8Hz), 7.54(1H,d,J=8.1Hz), 7.72-7.75(1H,m), 7.77(1H,d,J=8.1Hz), 8.47(1H,s), 8.54 (1H,d,J=2.4Hz), 8.61(1H,dd,J=4.9,1.5Hz).
ESI-MS m/z:402(M+H)⁺.

[Example 67] 1-[5-(5-Methylpyridin-2-yl)-1-(pyridin-3-yl)-1H-pyrazole-3-carbonyl]-4-methylpiperazine

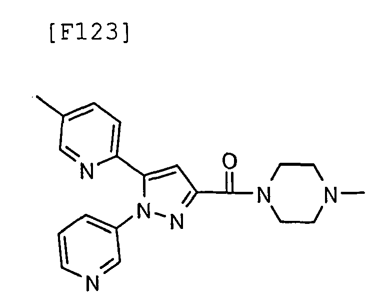

A solution of 5-(5-methylpyridin-2-yl)-1-(pyridin-3-yl)-1H-pyrazole-3-carboxylic acid (280 mg) obtained from Referential Example 9, 1-methylpiperazine (165 µL), 1-hydroxybenzotriazole (54 mg), triethylamine (697 µL), and 1-ethyl-3-(3-dimethylaminopropyl)carbodiimide hydrochloride (288 mg) in dichloromethane (30 mL) was stirred at room temperature overnight. The reaction mixture was partitioned between water and dichloromethane, and the organic layer was dried over sodium sulfate anhydrate. After a filtration step, the solvent was evaporated under reduced pressure, and the residue was purified by silica gel thin-layer chromatography (the lower layer of chloroform - methanol - water), to thereby give the title compound (90 mg, 25%) as a solid product.

¹H-NMR(400MHz,CDCl₃)δ:2.33(3H,s), 2.35(3H,s), 2.95-2.53(4H,m), 3.86(2H,s), 4.09(2H,s), 7.09(1H,s), 7.33-7.37(2H,m), 7.54(1H,d,J=8.1Hz), 7.74(1H,dt,J=8.5,2.1Hz), 8.32 (1H,s), 8.52 (1H,d,J=2.7Hz), 8.57(1H,dd,J=4. 8,1.6Hz).
ESI-MS m/z:363(M+H)⁺.

[Example 68] 1-[5-(5-Aminomethylpyridin-2-yl)-1-phenyl-1H-pyrazole-3-carbonyl]-4,4-difluoropiperidine

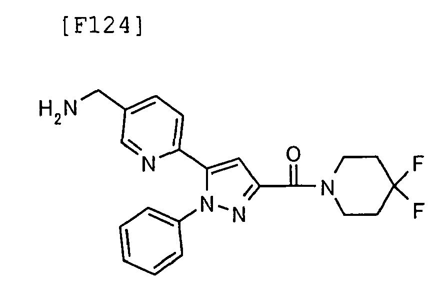

### 1) 1-[5-(5-Cyanopyridin-2-yl)-1-phenyl-1H-pyrazole-3-carbonyl]-4,4-difluoropiperidine

In a manner similar to that employed in Example 1, the title compound (1.1 g, 81%) in solid form was prepared from 5-(5-cyanopyridin-2-yl)-1-phenyl-1H-pyrazole-3-carboxylic acid (1.0 g) obtained in Referential Example 46 and 4,4-difluoropiperidine hydrochloride (0.66 g) obtained in Referential Example 12.

¹H-NMR(400MHz,CDCl₃)δ:2.00-2.15(4H,m), 3.90-3.95(2H,m), 4.18-4.23(2H,m), 7.29-7.46(7H,m), 7.91(1H,dd,J=8.3,2.2Hz), 8.77(1H,dd,J=2.0,0.7Hz).
EI-MSm/z:393(M⁺)

### 2) Title Compound

A suspension of the above-prepared 1-[5-(5-cyanopyridin-2-yl)-1-phenyl-1H-pyrazole-3-carbonyl]-4,4-difluoropiperidine (1.05 g) and nickel on silica-alumina (~65%, 730 mg) in 2M ammonia-ethanol (30 mL) was stirred in a hydrogen atmosphere (8 atm) at 120°C for 40 minutes, and the mixture was stirred at room temperature overnight. The reaction mixture was filtered through Celite, and the solvent of the filtrate was evaporated under reduced pressure. The residue was purified by silica gel thin-layer chromatography (chloroform - methanol), to thereby give the title compound (406 mg, 38%) as a solid product.

¹H-NMR(400MHz,CDCl₃)δ:1.61(2H,br s), 2.05-2.12(4H,m), 3.92(4H,brs), 4.20(2H,s), 7.13(1H,s), 7.23(1H,d,J=8.3Hz), 7.28-7.33(2H,m), 7.34-7.40(3H,m), 7.65(1H,dd,J=8.1,2.2Hz), 8.51(1H,d,J=1.5 Hz).
ESI-MS m/z:398(M+H)⁺.

[Example 69] 1-[5-(5-Methoxymethylpyridin-2-yl)-1-(pyridin-3-yl)-1H-pyrazole-3-carbonyl]-9,4-difluoropiperidine

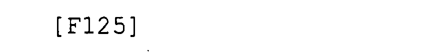

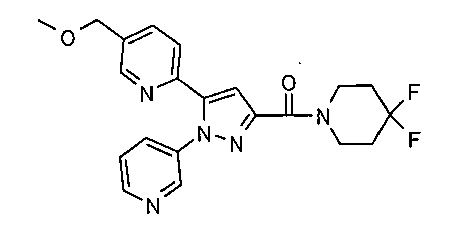

Sodium hydride (as 60% mineral oil suspension, 9.0 mg) was added under cooling on ice to a solution of 1-[5-(5-hydroxymethylpyridin-2-yl)-1-(pyridin-3-yl)-1H-pyrazole-3-carbonyl]-4,4-difluoropiperidine (60 mg) obtained from Example 65 and methyl iodide (0.5 mL) in N,N-dimethylformamide (5 mL), and the mixture was stirred for 1 hour. The reaction mixture was partitioned between water and ethyl acetate. The organic layer was washed with water three times, and subsequently with saturated brine. The organic layer was dried over sodium sulfate anhydrate. After a filtration step, the solvent was evaporated under reduced pressure, and the residue was purified by silica gel thin-layer chromatography (ethyl acetate), to thereby give the title compound (33 mg, 53%) as a solid product.

¹H-NMR(400MHz,CDCl₃)δ:2.04-2.13(4H,m), 3.42(3H,s), 3.93(2H,s), 4.20(2H,s), 4.47(2H,s), 7.17(1H,s), 7.34-7.37(1H,m), 7.48(1H,d,J=8.1Hz), 7.70-7.75(2H,m), 8.43(1H,d,J=1.5Hz), 8.55(1H,d,J=2.0Hz), 8.59(1H,dd,J=4.9,1.5Hz).
ESI-MS m/z:419(M+H)⁺.

[Example 70] 1-(5-(1-Methyl-1H-imidazol-4-yl)-1-(pyridin-3-yl)-1H-pyrazole-3-carbonyl]-4,4-difluoropiperidine

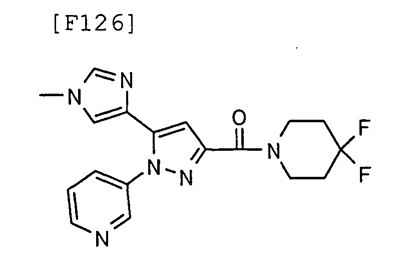

1M Aqueous sodium hydroxide (2.34 mL) was added to a solution of 5-(1-methyl-1H-imidazol-4-yl)-1-(3-pyridyl)-1H-pyrazole-3-carboxylic acid ethyl ester (0.232 g) obtained from Referential Example 42 in ethanol (5 mL) and tetrahydrofuran (5 mL), and the mixture was stirred at room temperature for 2.5 hours. 1M HCl (2.34 mL) was added to the reaction mixture, and the solvent was evaporated under reduced pressure. The residue was suspended in N,N-dimethylformamide (15 mL), and to the suspension were added at room temperature 4,4-difluoropiperidine hydrochloride (0.184 g) obtained from Referential Example 12, 1-hydroxybenzotriazole (0.143 g), triethylamine (0.330 mL), and 1-(3-dimethylaminopropyl)-3-ethylcarbodiimide hydrochloride (0.179 g), followed by stirring at room temperature for 17.5 hours. The reaction solvent was evaporated under reduced pressure, and saturated aqueous sodium hydrogencarbonate was added to the residue, followed by extraction with dichloromethane three times. The organic layers were combined, and the combined organic layer was dried over sodium sulfate anhydrate. After a filtration step, the solvent was evaporated under reduced pressure, and the residue was purified by silica gel flash column chromatography (dichloromethane - methanol), to thereby give the title compound (0.234 g, 80%) as a solid product.

¹H-NMR(400MHz,CDCl₃)δ:2.03-2.13(4H,m), 3.68(3H,s), 3.90-3.94(2H,m), 4.14-4.18(2H,m), 6.81(1H,d,J=1.2Hz), 6.99(1H,s), 7.38-7.41(2H,m), 7.86(1H,ddd,J=8.1,2.4,1.5Hz), 8.62(1H,dd,J=4.8,1.5Hz), 8.68(1H,d,J=2.4Hz).
ESI-MS m/z:373(M+H)⁺.
Elementary analysis: as C₁₈H₁₈N₆OF₂
Calculated: C,58.06;H,4.87;N,22.57;F,10.20.
Found: C,57.89;H,4.82;N,22.38;F.9.95.

[Example 71] 1-[5-(5-Methylpyridin-2-yl)-1-(pyridin-3-yl)-1H-pyrazole-3-carbonyl]-4-methyl-3-oxopiperazine

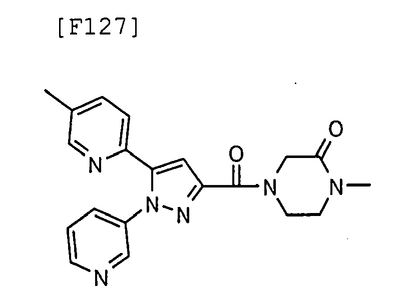

Triethylamine (0.330 mL) and 1-(3-dimethylaminopropyl)-3-ethylcarbodiimide hydrochloride (179 mg) were added at room temperature to a solution of 5-(5-methyl-2-pyridyl)-1-(3-pyridyl)-1H-pyrazole-3-carboxylic acid (0.218 g) obtained from Referential Example 9, 1-methyl-2-piperazinone hydrochloride (176 mg) obtained from Referential Example 15, and 1-hydroxybenzotriazole (143 mg) in N,N-dimethylformamide (15 mL), and the mixture was stirred at room temperature for 15.5 hours. The reaction solvent was evaporated under reduced pressure, and the residue was partitioned between ethyl acetate and saturated aqueous sodium hydrogencarbonate. The aqueous layer was further extracted with ethyl acetate four times. The organic layers were combined, and the combined organic layer was dried over sodium sulfate anhydrate. After a filtration step, the solvent was evaporated under reduced pressure, and the residue was purified by silica gel flash column chromatography (dichloromethane - methanol), to thereby give the title compound (231 mg, 78%) as a solid product.

¹H-NMR(400MHz,CDCl₃)δ:2.34(3H,s), 3.03(3H,s), 3.46-3.50(2H,m), 4.03-4.07(1H,m), 4.41-4.45(2H,m), 4.85(1H,brs), 7.16-7.20(1H,m), 7.34-7.40(2H,m), 7.52-7.56(1H,m), 7.68-7.83(1H,m), 8.30(1H,s), 8.47(1H,brs), 8.59(1H,brs).
ESI-MS m/z:377(M+H)⁺.
Elementary analysis: as C₂₀H₂₀N₆O₂
Calculated: C,63.82;H,5.36;N,22.33.
Found: C,63.65;H,5.28;N,22.24.

[Example 72] 1-[5-(1-Methyl-1H-imidazol-4-yl)-1-(pyridin-3-yl)-1H-pyrazole-3-carbonyl]-4-(difluoromethylene)piperidine

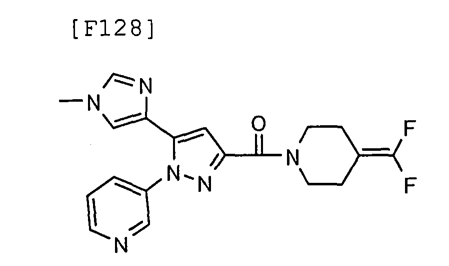

1M Aqueous sodium hydroxide (2.34 mL) was added to a solution of 5-(1-methyl-1H-imidazol-4-yl)-1-(3-pyridyl)-1H-pyrazole-3-carboxylic acid ethyl ester (0.232 g) obtained from Referential Example 42 in ethanol(5 mL) and tetrahydrofuran (5 mL), and the mixture was stirred at room temperature for 4 hours. 1M HCl (2.34 mL) was added to the reaction mixture, and the reaction solvent was evaporated under reduced pressure. The residue, 4-(difluoromethylene)piperidine hydrochloride (Bioorganic & Medicinal Chemistry (2004), 12(7), 1713-1730, 0.130 g), and 1-hydroxybenzotriazole (0.143 g) were suspended in N,N-dimethylformamide (15 mL), and to the suspension were added at room temperature triethylamine (0.330 mL) and 1-(3-dimethylaminopropyl)-3-ethylcarbodiimide hydrochloride (0.179 g), followed by stirring for 2.5 days. The reaction solvent was evaporated under reduced pressure, and the residue was partitioned between ethyl acetate and saturated aqueous sodium hydrogencarbonate. The aqueous layer was further extracted with ethyl acetate, and the organic layers were combined. The combined organic layer was dried over sodium sulfate anhydrate. After a filtration step and an evaporation step under reduced pressure, the residue was purified by silica gel flash column chromatography (dichloromethane - methanol), to thereby give the title compound (0.210 g, 67%) as a solid product.

¹H-NMR(400MHz,CDCl₃)δ:2.27-2.33(4H,m), 3.68(3H,s), 3.77-3.81(2H,m), 3.97-4.01(2H,m), 6.81(1H,s), 6.96(1H,s), 7.37-7.42(2H,m), 7.87 (1H,d, J=8.1Hz), 8.62 (1H,brs), 8.69 (1H,brs).
ESI-MS m/z:385(M+H)⁺.

[Example 73] 1-[5-(1-Methyl-1H-imidazol-4-yl)-1-(pyridin-3-yl)-1H-pyrazole-3-carbonyl]-4-methylenepiperidine

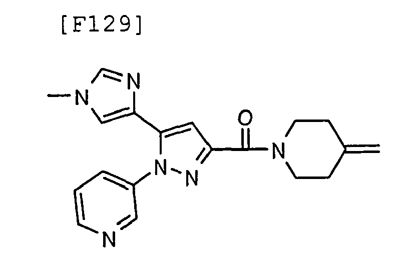

1M Aqueous sodium hydroxide (2.34 mL) was added to a solution of 5-(1-methyl-1H-imidazol-4-yl)-1-(3-pyridyl)-1H-pyrazole-3-carboxylic acid ethyl ester (0.232 g) obtained from Referential Example 42 in methanol (5 mL) and tetrahydrofuran (5 mL), and the mixture was stirred at room temperature for 4.5 hours. 1M HCl (2.34 mL) was added to the reaction mixture, and the reaction solvent was evaporated under reduced pressure. The residue, 4-methylenepiperidine hydrochloride (0.156 g) obtained from Referential Example 43, and 1-hydroxybenzotriazole (0.143 g) was suspended in N,N-dimethylformamide (25 mL), and to the suspension were added at room temperature triethylamine (0.330 mL) and 1-(3-dimethylaminopropyl)-3-ethylcarbodiimide hydrochloride (0.179 g), followed by stirring for 15.5 hours. The reaction solvent was evaporated under reduced pressure. The residue was partitioned between ethyl acetate and saturated aqueous sodium hydrogencarbonate, and the aqueous layer was further extracted with ethyl acetate. The organic layers were combined, and the combined organic layer was dried over sodium sulfate anhydrate. After a filtration step, the solvent was evaporated under reduced pressure, the residue was purified by silica gel flash column chromatography (dichloromethane - methanol), to thereby give the title compound (0.172 g, 62%) as a solid product.

¹H-NMR(400MHz,CDCl₃)δ:2.28-2.37(4H,m), 3.68(3H,s), 3.78-3.82(2H,m), 3.94-3.97(2H,m), 4.79-4.81(2H,m), 6.81(1H,d,J=1.2Hz), 6.94(1H,s), 7.39(1H,dd,J=8.1,4.9Hz), 7.42(1H,s), 7.88-7.91(1H,m), 8.61(1H,dd,J=4.9,1.5Hz), 8.69(1H,d,J=2.4Hz).
ESI-MS m/z:349(M+H)⁺.

[Example 74] 1-[5-(5-Ethynylpyridin-2-yl)-1-(pyridin-3-yl)-1H-pyrazole-3-carbonyl]-4,4-difluoropiperidine

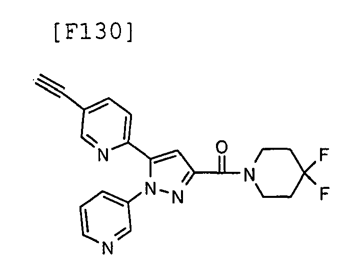

1M Aqueous sodium hydroxide (1.35 mL) was added at room temperature to a solution of 1-(3-pyridyl)-5-{5-[2-(trimethylsilyl)ethynyl]-2-pyridyl}-1H-pyrazole-3-carboxylic acid ethyl ester (0.175 g) obtained from Referential Example 45 in a solvent mixture of ethanol (5 mL) and tetrahydrofuran (5 mL), and the mixture was stirred for 2 hours. 1M HCl (1.35 mL) was added to the reaction mixture, and the reaction solvent was evaporated under reduced pressure. The residue, 4,4-difluoropiperidine hydrochloride (0.127 g) obtained from Referential Example 12, and 1-hydroxybenzotriazole (82.4 mg) was suspended in N,N-dimethylformamide (10 mL), and to the suspension were added triethylamine (0.187 mL) and 1-(3-dimethylaminopropyl)-3-ethylcarbodiimide hydrochloride (0.103 g), followed by stirring at room temperature for 4.5 days. The reaction solvent was evaporated under reduced pressure, and the residue was partitioned between ethyl acetate and saturated aqueous sodium hydrogencarbonate. The aqueous layer was further extracted with ethyl acetate, and the organic layers were combined. The combined organic layer was dried over sodium sulfate anhydrate. After a filtration step, the solvent was evaporated under reduced pressure, and the residue was purified by silica gel flash column chromatography (ethyl acetate - n-hexane), to thereby give the title compound (0.132 g, 74%) as a solid product.

¹H-NMR(400MHz,CDCl₃)δ:2.04-2.15(4H,m), 3.28(1H,s), 3.91-3.95(2H,m), 4.19-4.22(2H,m), 7.22 (1H,s), 7.38(1H,ddd,J=8.3,4.9,0.7Hz), 7.47(1H,dd,J=8.1,0.7Hz), 7.71(1H,ddd,J=8.1,2.4,1.5Hz), 7.82 (1H, dd, J=8.1,2.2Hz), 8.54(1H,dd,J=2.2,0.7Hz), 8.55(1H,d,J=2.4Hz), 8.62(1H,dd,J=4.9,1.5Hz).
ESI-MS m/z:394(M+H)⁺.

[Example 75] 1-[5-(5-Acetylpyridin-2-yl)-1-(pyridin-3-yl)-1H-pyrazole-3-carbonyl]-4,4-difluoropiperidine

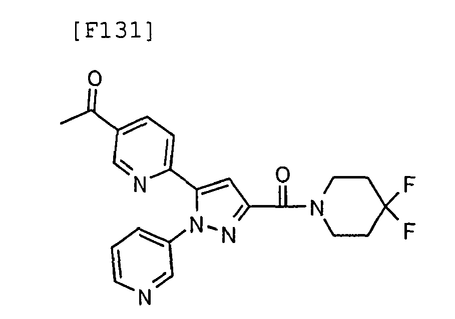

1-[5-(5-Ethynylpyridin-2-yl)-1-(pyridin-3-yl)-1H-pyrazole-3-carbonyl]-4,4-difluoropiperidine (0.160 g) obtained from Example 74 and mercury (II) sulfate (0.120 g) were suspended in 75% aqueous acetone (2 mL), and sulfuric acid (0.0440 mL) was added to the suspension, followed by refluxing for 2 hours. The reaction mixture was cooled in air, and to the mixture were added aqueous potassium carbonate, methanol, and chloroform. The reaction solvent was evaporated under reduced pressure, and the residue was purified by silica gel flash column chromatography (ethyl acetate - n-hexane), to thereby give the title compound (59.2 mg, 34%) as a solid product.

¹H-NMR(400MHz,CDCl₃)δ:2.05-2.15(4H,m), 2.62(3H,s), 3.92-3.96(2H,m), 4.20-4.23(2H,m), 7.30(1H,s), 7.41(1H,ddd,J=8.2,4.9,0.7Hz), 7.63(1H,dd,J=8.3,1.0Hz), 7.75(1H,ddd,J=8.1,2.4,1.5Hz), 8.28(1H,dd,J=8.3,2.2Hz), 8.55(1H,d,J=2.4Hz), 8.64(1H,dd,J=4.9,1.5Hz), 8.97(1H,dd,J=2.2,1.0Hz).
ESI-MS m/z:412(M+H)⁺.

[Example 76] (-)-(6S)-1-[5-(5-Methylpyridin-2-yl)-1-(pyridin-3-yl)-1H-pyrazole-3-carbonyl]-4,6-dimethyl-3-oxopiperazine

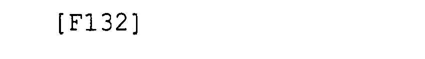

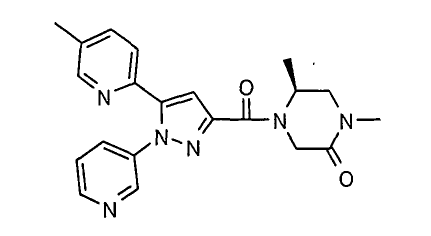

4M HCl-dioxane (5 mL) was added at room temperature to (2S)-2,4-dimethyl-5-oxo-1-piperazinecarboxylic acid tert-butyl ester (0.267 g) obtained from Referential Example 44, and the mixture was stirred for 4 hours. The reaction solvent was evaporated under reduced pressure. The residue, 5-(5-methyl-2-pyridyl)-1-(3-pyridyl)-1H-pyrazole-3-carboxylic acid (0.218 g) obtained from Referential Example 9, and 1-hydroxybenzotriazole (143 mg) were suspended in N,N-dimethylformamide (15 mL), and to the suspension were added at room temperature triethylamine (0.330 mL) and 1-(3-dimethylaminopropyl)-3-ethylcarbodiimide hydrochloride (179 mg), followed by stirring for 1.5 days. The reaction solvent was evaporated under reduced pressure, and the residue was partitioned between dichloromethane and saturated aqueous sodium hydrogencarbonate. The aqueous layer was extracted with 10% methanol-dichloromethane solvent mixture twice, and the organic layers were combined. The combined organic layer was dried over sodium sulfate anhydrate. After a filtration step, the solvent was evaporated under reduced pressure, and the residue was purified by silica gel flash column chromatography (methanol- dichloromethane), to thereby give the title compound (250 mg, 54%) as a solid product.

¹H-NMR(400MHz,CDCl₃)δ:1.41(3H,d,J=6.8Hz), 2.35(3H,s), 3.01-3.12(1H,m), 3.04(3H,s), 3.75-3.99(3/2xH,m), 4.25-4.34(1/2xH,m), 4.82-4.91(1/2xH,m), 5.16-5.33(1H,m 5.53(1/2xH,brs), 7.16(1H,s), 7.35-7.39(2H,m), 7.54(1H,d,J=7.8Hz), 7.68-7.83(1H,m), 8.31(1H,s), 8.44-8.60(1H,m), 8.59(1H,dd,J=4.9,1.5Hz).
ESI-MS m/z:391 (M+H)⁺.
Elementary analysis: as C₂₁H₂₂N₆O₂
Calculated: C,64.60;H,5.68;N,21.52.
Found: C,64.48;H,5.70;N,21.21.

[Example 77] 1-[5-(5-Carbamoylmethoxypyridin-2-yl)-1-(pyridin-3-yl)-1H-pyrazole-3-carbonyl]-4,4-difluoropiperidine

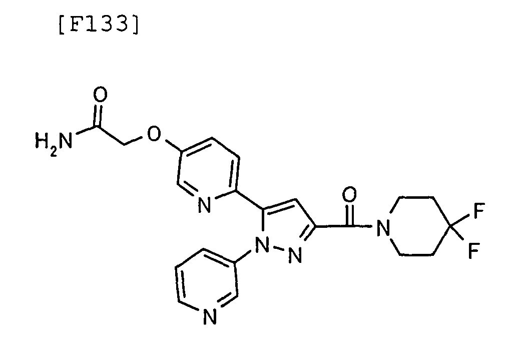

Potassium carbonate (215 mg) and bromoacetamide (129 mg) were added to a solution of 5-(5-hydroxypyridin-2-yl)-1-(pyridin-3-yl)-1H-pyrazole-3-carboxylic acid (300 mg) obtained from Example 11 in N,N-dimethylformamide (5 mL), and the mixture was stirred at 50°C for 16 hours. The reaction mixture was cooled in air, and was partitioned between water and ethyl acetate. The organic layer was washed with water twice, and was dried over magnesium sulfate anhydrate. After a filtration step, the solvent was evaporated under reduced pressure, and the residue was purified by silica gel thin-layer chromatography (dichloromethane - methanol), to thereby give the title compound (240 mg, 70%) as a solid product.

¹H-NMR(400MHz,CDCl₃)δ:2.00-2.15(4H,m), 3.85-3.91(2H,m), 4.15-4.21(2H,m), 4.55(2H,s), 5.78(1H,br s), 6.47(1H,br s), 7.13(1H,s) 7.26-7.29(1H,m), 7.39-7.50(2H,m), 7.77-7.80(1H,m), 8.22(1H,d,J=2.9Hz), 8.51(1H,d,J=2.4Hz), 8.60(1H,dd,J=4.9,1.2Hz).
FAB-MS m/z:443(M+H)⁺.
[Example 78] 1-[5-(5-Methyl-2-pyridyl)-1-(3-pyridyl)-1H-pyrazole-3-carbonyl]-2-carbamoylpiperidine

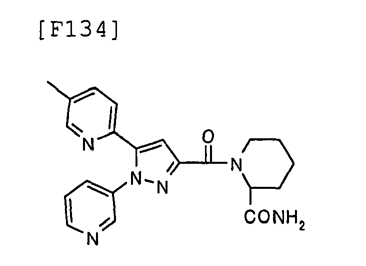

Triethylamine (0.137 mL) was added at room temperature to a suspension of 5-(5-methyl-2-pyridyl)-1-(3-pyridyl)-1H-pyrazole-3-carboxylic acid (0.250 g) obtained from Referential Example 9, piperidine-2-carboxamide(0.126 g) obtained from Referential Example 19, 3-(3-dimethylaminopropyl)-1-ethylcarbodiimide hydrochloride (0.188 g), and 1-hydroxybenzotriazole (0.133 g) in dichloromethane (5 mL), and the mixture was stirred for 15 hours. The reaction mixture was partitioned between water and chloroform, and the solvent of the organic layer was evaporated under reduced pressure. The residue was purified by silica gel column chromatography (methanol - chloroform), to thereby give the title compound (0.229 g, 66%) as a solid product.

¹H-NMR(400MHz,CDCl₃)δ:1.54-1.84(5H,m), 2.32-2.40(1H,m), 2.36(3H,s), 2.82-3.24(1H,m), 4.72-4.75(1H,m), 5.39-5.54(2H,m), 6.36-7.00(1H,m), 7.12-7.15(1H,m 7.37-7.41(2H,m), 7.55-7.57(1H,m), 7.74-7.83(1H,m), 8.32(1H,s), 8.49-8.59(2H,m).
EI-MSm/z: 390 (M⁺).

[Example 79] 4-{[5-(5-Methyl-2-pyridyl)-1-(1,3-thiazol-2-yl)-1H-pyrazol-3-yl]carbonyl}-1-methyl-2-piperazinone

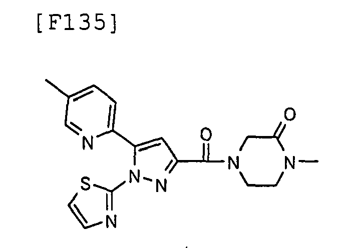

Triethylamine (0.235 mL) and 1-(3-dimethylaminopropyl)-3-ethylcarbodiimide hydrochloride (0.129 g) were added at room temperature to a suspension of 5-(5-methyl-2-pyridyl)-1-(1,3-thiazol-2-yl)-1H-pyrazole-3-carboxylic acid (0.160 g) obtained from Referential Example 48, 1-methyl-2-piperazinone hydrochloride (0.126 g), and 1-hydroxybenzotriazole (0.103 g) in N,N-dimethylformamide (15 mL), and the mixture was stirred for 16.5 hours. The solvent of the reaction mixture was evaporated under reduced pressure, and the residue was partitioned between saturated aqueous sodium hydrogencarbonate and 10% methanol-dichloromethane. The aqueous layer was further extracted with 10% methanol-dichloromethane, and the organic layers were combined. The combined organic layer was dried over sodium sulfate anhydrate. After a filtration step, the solvent was evaporated under reduced pressure, and the residue was purified by silica gel column chromatography (methanol - dichloromethane), to thereby give the title compound (0.185 g, 85%) as a solid product.

¹H-NMR(400MHz,CDCl₃)δ:2.39(3H,s), 3.03(3/2H,s), 3.05(3/2H,s), 3.45-3.53(2H,m), 4.03-4.07(1H,m), 4.36-4.39(1H,m), 4.44(1H,s), 4.84(1H,s), 7.11(1H,s), 7.23(1H,d,J=3.4Hz), 7.45-7.48(2H,m), 7.55-7.60 (1H,m), 8.41-8.42(1H,m).
ESI-MSm/z:383(M+H)⁺.

[Example 80] 4-{[5-(5-Methyl-2-pyridyl)-1-(1,3-thiazol-2-yl)-1H-pyrazol-3-yl]carbonyl}morpholine

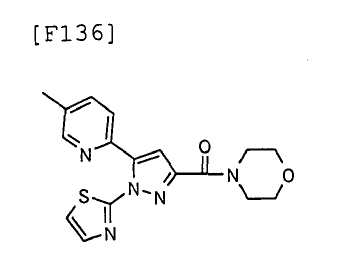

In a manner similar to that employed in Example 79, the title compound (0.146 g, 73%) in solid form was prepared from (5-(5-methyl-2-pyridyl)-1-(1,3-thiazol-2-yl)-1H-pyrazole-3-carboxylic acid (0.160 g) obtained in Referential Example 48 and morpholine (73.0 µL).

¹H-NMR(400MHz,CDCl₃)δ:2.39(3H,s), 3.74-3.85(6H,m), 4.09-4.13(2H,m), 7.05(1H,s), 7.21(1H,d,J=3.4Hz), 7.45-7.47(2H,m), 7.56-7.59(1H,m), 8.42-8.43(1H,m).
ESI-MSm/z:356(M+H)⁺.

[Example 81] 1-{[5-(5-Methyl-2-pyridyl)-1-(1,3-thiazol-2-yl)-1H-pyrazol-3-yl]carbonyl}-4-methoxypiperidine

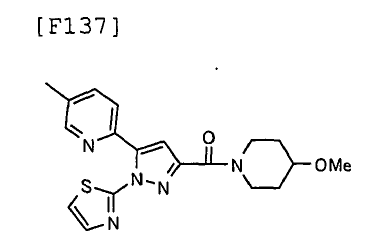

In a manner similar to that employed in Example 79, the title compound (0.167 g, 78%) in solid form was prepared from 5-(5-methyl-2-pyridyl)-1-(1,3-thiazol-2-yl)-1H-pyrazole-3-carboxylic acid (0.160 g) obtained in Referential Example 48 and 4-methoxypiperidine hydrochloride (0.127 g) obtained in Referential Example 11.

¹H-NMR(400MHz,CDCl₃)δ:1.65-1.75(2H,m), 1.90-2.01(2H,m), 2.39(3H,s), 3.39(3H,s), 3.49-3.59(2H,m), 3.68-3.76(1H,m), 4.04-4.11(1H,m), 4.18-4.25(1H,m) 6.98(1H,s), 7.19(1H,d,J=3.4Hz), 7.44(1H,d,J=3.4Hz), 7.46(1H,d,J=8.1Hz), 7.55-7.58(1H,m), 8.43-8.44(1H,m).
ESI-MSm/z:384(M+H)⁺.

[Example 82] 1-[5-(5-Methylpyridin-2-yl)-1-(pyridin-3-yl)-1H-pyrazole-3-carbonyl]-3-oxo-4-methyl[1.4]diazepane

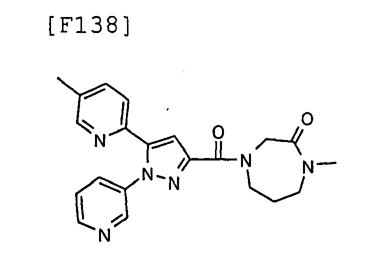

1-Hydroxybenzotriazole (0.135 g), 1-(3-dimethylaminopropyl)-3-ethylcarbodiimide hydrochloride (0.367 g), triethylamine (0.621 mL), and 1-methyl-2-oxo [1.4] diazepane hydrochloride (0.210 g) obtained from Referential Example 49 were added at room temperature to a solution of 5-(5-methyl-2-pyridyl)-1-(3-pyridyl)-1H-pyrazole-3-carboxylic acid (0.258 g) obtained from Referential Example 9 in N,N-dimethylformamide (2 mL) and dichloromethane (7.5 mL), and the mixture was stirred for 15 hours. The reaction mixture was partitioned between water and ethyl acetate. The aqueous layer was further extracted with ethyl acetate, and the organic layers were combined. The combined organic layer was washed with saturated brine, and was dried over sodium sulfate anhydrate. After a filtration step, the solvent was evaporated under reduced pressure, and the residue was purified by silica gel column chromatography (chloroform - methanol), to thereby give the title compound (0.185 g, 52%) as an amorphous product.

¹H-NMR(400MHz,CDCl₃)δ:2.02-2.16(2H,m), 2.32(3H,s), 2.98(3H×1/2,s), 3.01(3H×1/2,s), 3.43-3.50(2H,m), 3.85(1H,t,J=5.8Hz), 4.11(1H,t,J=5.8Hz), 4.50(1H,s), 4.70(1H,s), 7.14(1H×1/2,s), 7.15(1H×1/2,s), 7.37-7.50(2H,m), 7.51-7.56(1H,m), 7.70-7.74(1H×1/2,m), 8.11-8.16(1H×1/2,m), 8.28(1H,br), 8.42-8.58(2H,m).
ESI-MSm/z:391(M+H)⁺.

[Example 83] (2S)-1-[5-(1-Methyl-1H-pyrazole-4-yl)-1-(pyridin-3-yl)-1H-pyrazole-3-carbonyl]-2-fluoromethylpyrrolidine

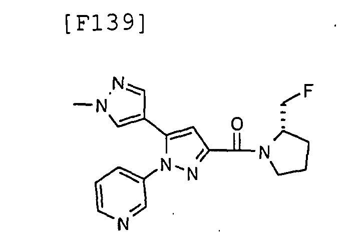

### 1) Title Compound

(2S)-2-Fluoromethylpyrrolidine hydrochloride (160 mg) obtained from Referential Example 31 and triethylamine (0.6 mL) were added at room temperature to a solution of 5-(1-methyl-1H-pyrazole-4-yl)-1-(3-pyridyl)-1H-pyrazole-3-carboxylic acid (250 mg) obtained from Referential Example 47, 3-(3-dimethylaminopropyl)-1-ethylcarbodiimide hydrochloride (270 mg), and 1-hydroxybenzotriazole (190 mg) in dichloromethane (10 mL), and the mixture was.stirred for 63 hours. The reaction mixture was partitioned between water and dichloromethane, and the organic layer was dried over magnesium sulfate anhydrate. After a filtration step, the solvent was evaporated under reduced pressure, and the residue was purified by silica gel thin-layer chromatography (dichloromethane - methanol), to thereby give the title compound (290 mg, 88%) as an amorphous product.

¹H-NMR(400MHz,CDCl₃)δ:1.92-2.26(4H,m), 3.88(3H,s), 3.67-5.20(5H,m), 6.96-7.00(1H,m), 7.24-7.28(2H,m), 7.74-7.77(1H,m),
8.66-8.73(2H,m).
EI-MSm/z:354 (M⁺).

### 2) Hydrochloride salt of the title compound

1M HCl-ethanol (1 mL) was added at room temperature to a solution of the title compound (290 mg) in a solvent mixture of ethanol (5 mL) and methanol (3 mL), and the mixture was stirred for 30 minutes. The reaction solvent was evaporated under reduced pressure, to thereby give a hydrochloride salt of the title compound (260 mg, 65%) as a solid product.
EI-MSm/z:354(M⁺).

[Example 84] 1-[5-(5-Methylpyridin-2-yl)-1-(pyridin-3-yl)-1H-pyrazole-3-carbonyl]-2-(1-aminocyclopropyl)piperidine

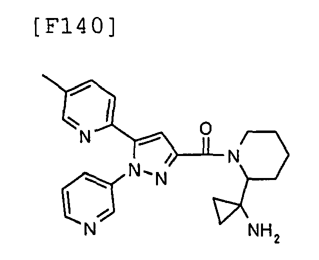

### 1) 1-[5-(5-Methylpyridin-2-yl)-1-(pyridin-3-yl)-1H-pyrazole-3-carbonyl]-2-[1-(N-tert-butoxycarbonylamino)cyclopropyl]piperidine

In a manner similar to that employed in Example 78, 1-[5-(5-methylpyridin-2-yl)-1-(pyridin-3-yl)-1H-pyrazole-3-carbonyl]-2-[1-(N-tert-butoxycarbonylamino)cyclopropyl]piperidine (225 mg, 88%) in amorphous form was prepared from 1-(5-methylpyridin-2-yl)-1-(pyridin-3-yl)-1H-pyrazole-carboxylic acid (143 mg) obtained in Referential Example 9 and (1-piperidin-2-ylcyclopropyl)carbamidic acid tert-butyl ester (123 mg) obtained in Referential Example 50.
ESI-MSm/z:503(M+H)⁺.

### 2) Title Compound

Trifluoroacetic acid (7 mL) was added at room temperature to a solution of the above-prepared 1-[5-(5-methylpyridin-2-yl)-1-(pyridin-3-yl)-1H-pyrazole-3-carbonyl]-2-[1-(N-tert-butoxycarbonylamino)cyclopropyl]piperidine (224 mg) in dichloromethane (20 mL), and the mixture was stirred for 105 minutes. The reaction solvent was evaporated under reduced pressure, and the residue was partitioned between saturated aqueous sodium hydrogencarbonate and chloroform-methanol (10:1) solvent mixture. The organic layer was dried over sodium sulfate anhydrate. After a filtration step, the solvent was evaporated under reduced pressure, to thereby give the title compound (80 mg, 44%) as a solid product.

¹H-NMR(400MHz,CDCl₃)δ:0.83-0.94(3H,m), 1.02-1.05(1H,m), 1.25-1.38(3H,m), 1.53(1H,d,J=10.3Hz), 1.77-1.82(3H,m), 2.31(1H,br s), 2.33(3H,s), 2.58-2.64(1H,m), 3.10(1H,d,J=11.7Hz), 7.19(1H,s), 7.33-7.39(3H,m), 7.52-7.54(1H,m), 7.75-7.78(1H,m), 8.26-8.27(1H,m), 8.51(1H,d,J=2.0Hz), 8.58(1H,dd,J=4.9,1.5Hz).
ESI-MSm/z:403(M+H)⁺.

### [Test Example 1] Platelet aggregation-inhibitory action

Human blood was drawn by use of 3.13% sodium citrate as an anticoagulant in a volume 1/10 the blood volume. The blood was centrifuged at 180xg for 10 minutes to separate platelet rich plasma (PRP) from the blood. Upper layer PRP was removed, and the lower layer was centrifuged at 1600xg for 10 minutes, to thereby recover the upper layer platelet poor plasma (PPP). A solution (1 µL) of compound of the present invention was added to PRP (200 µL), and the mixture was allowed to stand at 37°C for 2 minutes. Subsequently, collagen (2 µL) was added to the resultant mixture so as to induce platelet aggregation. Percent platelet aggregation was measured using PAM-12C (SSR Engineering). Optical transmittance of PPP was regarded as an indicator of 100% aggregation. Percent platelet aggregation values of the respective compounds were determined at different concentrations. IC₅₀ values were calculated. Table 1 shows the results.

### [Test Example 2] Inhibitory effects on cyclooxygenase-1 (COX-1) and cyclooxygenase-2 (COX-2)

COX-1 inhibiting activity and COX-2 inhibiting activity of inventive compounds produced in some of the Examples herein were measured by use of a COX Inhibitor Screening Assay Kit (product of Cayman Chemical Company, Catalog Nos. 560101 and 560121).
Before starting the measurement, a reaction buffer, heme, arachidonic acid, SnCl₂, EIA buffer, washing buffer, prostaglandin (PG) screening EIA standard, PG screening acetylcholine esterase (AchE), tracer (chromogenic enzyme HRP conjugate), and PG screening EIA antiserum were prepared ready for use.
· Production of PGF_{2α} in the presence of COX-1 or COX-2
A reaction mixture containing a compound of the present invention from the Examples (50 µM) and COX-1 or COX-2 was allowed to stand at 37°C for 10 minutes. Arachidonic acid (10 µL) was added thereto, and the resultant mixture was further allowed to stand at 37°C for 2 minutes. 1N-Hydrochloric acid (50 µL) was added to the reaction mixture, to thereby stop the reaction. SnCl₂ solution (100 µL) was added thereto, and the resultant mixture was allowed to stand at room temperature for 5 minutes.

### (2) Quantitative determination of PGF_{2α} using ELISA

Antiserum (rabbit anti-PGF_{2α} antibody, 50 µL) was added to the wells of a 96-well plate that had been coated with mouse anti-rabbit IgG. A solution of PGF_{2α}-containing mixture obtained above (2000-fold dilution, 50 µL) and an AchE tracer (50 µL) were sequentially added to each well, and the mixture was allowed to stand at room temperature for 18 hours. The wells were washed 5 times with the washing buffer to remove an excessive AchE tracer, and an Ellman reagent (200 µL) was added. After keeping the plate in a dark room for 60 minutes, absorbance was measured at 405 nm.

### (3) Calculation of inhibitory activity of the compounds of the present invention

A calibration curve was obtained by use of a PG screening EIA standard, and the production amount of PGF_{2α} was determined from the absorbance. Percent inhibition against COX-1 or COX-2 in the presence of a 50 µM of the compound was calculated. Table 1 shows the results.
Notably, the percentage of the amount of PGF_{2α} produced by use of a reaction mixture containing no compound was regarded as 100% in calculation of percent inhibition.

**[Table 1]**

| Example | Inhibition of collagen-induced platelet aggregation, IC₅₀ (µM) | Inhibitory effect against COX-1 at 50 µM (% inhibition) | Inhibitory effect against COX-2 at 50 µM (% inhibition) |
|---|---|---|---|
| 13 | 0.13 | 10.3 | ND |
| 15 | 0.009 | 8.1 | ND |
| 29 | 0.095 | -1.4 | 5.3 |
| 30 | 0.12 | -21.1 | ND |
| 33 | 0.0313 | -16.7 | ND |
| 57 | 0.14 | -17.4 | ND |
| 65 | 0.13 | 5.9 | ND |
| 67 | 0.19. | -21 | ND |
| 70 | 0.28 | 5.5 | ND |
| 71 | 0.27 | 18.1 | ND |
| 73 | 0.078 | 11.9 | ND |

As is clear from Table 1, the compounds (I) of the present invention, salts thereof, and solvates or the compounds or the salts were confirmed to have strong platelet aggregation inhibitory activity without inhibiting COX-1 or COX-2.

## Claims

1. A compound represented by formula (I): 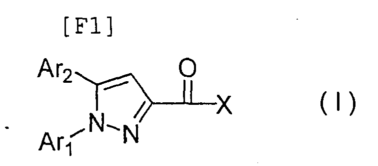 (wherein Ar₁ represents a phenyl group which may have 1 to 3 substituents, or a non-substituted 5- or 6-membered aromatic heterocyclic group; Ar₂ represents (i) a non-substituted phenyl group, (ii) a phenyl group which has been substituted by a lower alkyl group having 1 to 3 groups or atoms selected from among a carbamoyl group which may be substituted, an amino group which may be substituted, a hydroxyl group, a lower alkoxy group, and a halogen atom, or (iii) a 5- or 6-membered aromatic heterocyclic group which has been substituted by 1 to 3 groups or atoms selected from among a lower alkyl group which may be substituted, a lower alkynyl group, a lower alkanoyl group, a carbamoyl group which may be substituted, a cyano group, an amino group which may be substituted, a hydroxyl group, a lower alkoxy group which may be substituted, and a halogen atom; and X represents a group represented by formula (II): 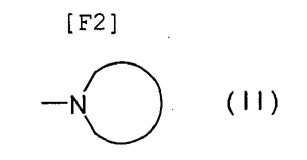 (wherein the ring structure represents a 4- to 7-membered heterocyclic group which may have, in addition to the nitrogen atom shown in formula (II), one heteroatom selected from among nitrogen, oxygen, and sulfur, and which may be substituted by 1 to 4 groups or atoms selected from among a lower alkyl group which may be substituted, an alkylidene group which may be substituted, a carbamoyl group which may be substituted, an amino group which may be substituted, a hydroxyl group, a lower alkoxy group, an oxo group, a lower alkanoyl group, a lower alkylsulfonyl group, and a halogen atom)), a salt thereof, or a solvate of the compound or the salt.

2. The compound according to claim 1, a salt thereof, or a solvate of the compound or the salt, wherein Ar₁ is a non-substituted phenyl group.

3. The compound according to claim 1, a salt thereof, or a solvate of the compound or the salt, wherein Ar₁ is a non-substituted 5- or 6-membered aromatic heterocyclic group.

4. The compound according to claim 3, a salt thereof, or a solvate of the compound or the salt, wherein the 5- or 6-membered aromatic heterocyclic group is a pyrrolyl group, a pyrazolyl group, an imidazolyl group, a thiazolyl group, a pyridyl group, a pyridazinyl group, a pyrimidinyl group, or a pyrazinyl group.

5. The compound according to any one of claims 1 to 4, a salt thereof, or a solvate of the compound or the salt, wherein Ar₂ is a 5- or 6-membered aromatic heterocyclic group which has been substituted by 1 to 3 groups or atoms selected from among a lower alkyl group which may be substituted, a lower alkynyl group, a lower alkanoyl group, a carbamoyl group which may be substituted, a cyano group, an amino group which may be substituted, a hydroxyl group, a lower alkoxy group which may be substituted, and a halogen atom.

6. The compound according to claim 5, a salt thereof, or a solvate of the compound or the salt, wherein the 5- or 6-membered aromatic heterocyclic group is a pyrrolyl group, a pyrazolyl group, an imidazolyl group, a pyridyl group, a pyridazinyl group, a pyrimidinyl group, or a pyrazinyl group.

7. The compound according to any one of claims 1 to 6, a salt thereof, or a solvate of the compound or the salt, wherein a group represented by formula (II) is an azetidino group, a pyrrolidino group, a pyrazolidino group, a piperidino group, a piperazino group, a morpholino group, a thiomorpholino group, a homopiperazino group, or a hexahydropyridazin-1-yl group, which groups may have, in addition to the nitrogen atom shown in formula (II), one heteroatom selected from among nitrogen, oxygen, and sulfur, and which may be substituted by 1 to 4 groups and atoms selected from among an alkylidene group which may be substituted, a carbamoyl group which may be substituted, an amino group which may be substituted, a hydroxyl group, a lower alkoxy group, an oxo group, a lower alkanoyl group, a lower alkylsulfonyl group, and a halogen atom.

8. The compound according to any one of claims 1 to 7, a salt thereof, or a solvate of the compound or the salt, wherein X is a group selected from among 3-dimethylaminoazetidin-1-yl, 3-methoxyazetidin-1-yl, 2-fluoromethylpyrrolidino, 3-fluoropyrrolidino, pyrazolidino, 2-formylpyrazolidino, piperidino, 2-(1-aminocyclopropyl)piperidino, 2-carbamoylpiperidino, 2-methylcarbamoylpiperidino, 2-dimethylcarbamoylpiperidino, 3-methoxypiperidino, 3-fluoropiperidino, 4-fluoromethylpiperidino, 4-methoxypiperidino, 4-fluoropiperidino, 4,4-difluoropiperidino, 4-methyl-4-methoxypiperidino, 4-methylenepiperidino, 4-(difluoromethylene)piperidino, 4-cyclopropylpiperazino, 3-oxo-4-methylpiperazino, 3-oxo-4-ethylpiperazino, 4-methylpiperazino, 4,6-dimethyl-3-oxopiperazino, 3,5-dioxo-4-methylpiperazino, 4-methyl-3-oxohomopiperazinyl, hexahydropyridazin-1-yl, 2-carbamoylhexahydropyridazin-1-yl, and morpholino.

9. A drug containing, as an active ingredient, a compound according to any one of claims 1 to 8, a salt thereof, or a solvate of the compound or the salt.

10. A preventive and/or therapeutic agent for an ischemic disease, containing, as an active ingredient, a compound according to any one of claims 1 to 8, a salt thereof, or a solvate of the compound or the salt.

11. An agent inhibiting platelet aggregation containing, as an active ingredient, a compound according to any one of claims 1 to 8, a salt thereof, or a solvate of the compound or the salt.

12. A pharmaceutical composition containing a compound according to any one of claims 1 to 8, a salt thereof, or a solvate of the compound or the salt.

13. A method for preventing and/or treating an ischemic disease, **characterized by** administering an effective amount of a compound according to any one of claims 1 to 8, a salt thereof, or a solvate of the compound or the salt.

14. A use of a compound according to any one of claims 1 to 8, a salt thereof, or a solvate of the compound or the salt, for production of a preventive and/or therapeutic agent for an ischemic disease.
